# EUROPEAN PATENT SPECIFICATION

(11) **EP 2 242 752 B1**
(45) Date of publication and mention of the grant of the patent: **08.08.2012**
(21) Application number: 08768910.5
(22) Date of filing: 13.02.2008
(51) Int. Cl.: C07D 403/14, C07D 405/14, C07D 417/14, A61P 31/00, A61K 31/41

(54) **IMIDAZOLYL BIPHENYL IMIDAZOLES AS HEPATITIS C VIRUS INHIBITORS**
IMIDAZOLYLBIPHENYLIMIDAZOLE ALS HEPATITIS-C-VIRUS-INHIBITOREN
IMIDAZOLYLE DIPHÉNYLE IMIDAZOLES INHIBITRICES DU VIRUS DE L'HÉPATITE C

(43) Date of publication of application: 27.10.2010
(73) Proprietor: Bristol-Myers Squibb Company, Princeton, NJ 08543-4000 (US)
(72) Inventor: BACHAND, Carol, Candiac, Québec J5R 1B1 (CA); BELEMA, Makonen, Wallingford, CT 06492 (US); DEON, Daniel H., Montreal, Québec H4A 2N4 (CA); GOOD, Andrew C., Wallingford, CT 06492 (US); GOODRICH, Jason, Wallingford, CT 06492 (US); JAMES, Clint A., Longueuil, Québec J4H 2N4 (CA); LAVOIE, Rico, Candiac Quebec J5R 4B7 (CA); LOPEZ, Omar D., Wallingford, CT 06492 (US); MARTEL, Alain, Delson, Québec J5B 1G8 (CA); MEANWELL, Nicholas A., Wallingford, CT 06492 (US); NGUYEN, Van N., Wallingford, CT 06492 (US); ROMINE, Jeffrey Lee, Wallingford, CT 06492 (US); RUEDIGER, Edward H., Greenfield Park, Québec J4V 2K3 (CA); SNYDER, Lawrence B., Wallingford, CT 06492 (US); ST. LAURENT, Denis R., Wallingford, CT 06492 (US); YANG, Fukang, Wallingford, CT 06492 (US); LANGLEY, David R., Wallingford, CT 06492 (US); WANG, Gan, Wallingford, CT 06492 (US); HAMANN, Lawrence G., North Grafton, MA 01536 (US)
(74) Representative: Reitstötter - Kinzebach
(86) International application number: PCT/US2008/053779
(87) International publication number: WO 2009/102325

(56) References cited:
- WO-A-2004/005264
- WO-A-2008/021927
- WO-A-2008/021928
- WO-A-2008/021936
- US-A- 6 080 870
- SCHUBERT HERMANN ET AL: "Diimidazole. III. Synthese von aromatisch überbrückten 4(5),4'(5')-Diimidazolen" JOURNAL FUER PRAKTISCHE CHEMIE, LEIPZIG, DE, vol. 22, no. 3/4, 1 January 1963 (1963-01-01), pages 140-152, XP002473762 ISSN: 0021-8383

## Description

The present disclosure is generally directed to antiviral compounds, and more specifically directed to compounds which can inhibit the function of the NS5A protein encoded by Hepatitis C virus (HCV), compositions comprising such compounds, and methods for inhibiting the function of the NS5A protein.

HCV is a major human pathogen, infecting an estimated 170 million persons worldwide - roughly five times the number infected by human immunodeficiency virus type 1. A substantial fraction of these HCV infected individuals develop serious progressive liver disease, including cirrhosis and hepatocellular carcinoma.

Presently, the most effective HCV therapy employs a combination of alpha-interferon and ribavirin, leading to sustained efficacy in 40% of patients. Recent clinical results demonstrate that pegylated alpha-interferon is superior to unmodified alpha-interferon as monotherapy. However, even with experimental therapeutic regimens involving combinations of pegylated alpha-interferon and ribavirin, a substantial fraction of patients do not have a sustained reduction in viral load. Thus, there is a clear and long-felt need to develop effective therapeutics for treatment of HCV infection.

HCV is a positive-stranded RNA virus. Based on a comparison of the deduced amino acid sequence and the extensive similarity in the 5' untranslated region, HCV has been classified as a separate genus in the Flaviviridae family. All members of the Flaviviridae family have enveloped virions that contain a positive stranded RNA genome encoding all known virus-specific proteins *via* translation of a single, uninterrupted, open reading frame.

Considerable heterogeneity is found within the nucleotide and encoded amino acid sequence throughout the HCV genome. At least six major genotypes have been characterized, and more than 50 subtypes have been described. The major genotypes of HCV differ in their distribution worldwide, and the clinical significance of the genetic heterogeneity of HCV remains elusive despite numerous studies of the possible effect of genotypes on pathogenesis and therapy.

The single strand HCV RNA genome is approximately 9500 nucleotides in length and has a single open reading frame (ORF) encoding a single large polyprotein of about 3000 amino acids. In infected cells, this polyprotein is cleaved at multiple sites by cellular and viral proteases to produce the structural and non-structural (NS) proteins. In the case of HCV, the generation of mature non-structural proteins (NS2, NS3, NS4A, NS4B, NS5A, and NS5B) is effected by two viral proteases. The first one is believed to be a metalloprotease and cleaves at the NS2-NS3 junction; the second one is a serine protease contained within the N-terminal region of NS3 (also referred to herein as NS3 protease) and mediates all the subsequent cleavages downstream of NS3, both in cis, at the NS3-NS4A cleavage site, and in trans, for the remaining NS4A-NS4B, NS4B-NS5A, NS5A-NS5B sites. The NS4A protein appears to serve multiple functions, acting as a cofactor for the NS3 protease and possibly assisting in the membrane localization of NS3 and other viral replicase components. The complex formation of the NS3 protein with NS4A seems necessary to the processing events, enhancing the proteolytic efficiency at all of the sites. The NS3 protein also exhibits nucleoside triphosphatase and RNA helicase activities. NS5B (also referred to herein as HCV polymerase) is a RNA-dependent RNA polymerase that is involved in the replication of HCV.

Compounds useful for treating HCV-infected patients are desired which selectively inhibit HCV viral replication. In particular, compounds which are effective to inhibit the function of the NS5A protein are desired. The HCV NS5A protein is described, for example, in Tan, S.-L., Katzel, M.G. Virology 2001, 284, 1-12; and in Park, K.-J.; Choi, S.-H, J. Biological Chemistry 2003.

WO 2004/005264 discloses imidazole compounds which are useful against hepatitis C virus infections and diseases associated therewith.

Journal für Praktische Chemie, vol. 22, no. 3/4, 140-152 (1963) discloses the synthesis of aromatic bridged 4(5), 4'(5')-diimidazoles.

The invention provides a compound selected from
methyl ((1S)-2-((1R,3S,5R)-3-(4-(4'-(2-((1R,3S,5R)-2-(N-(methoxycarbonyl)-L-alanyl)-2-azabicyclo[3,1,0]hex-3-yl)-1H-imidazol-4-yl)-4-biphenylyl)-1H-imidazol-2-yl)-2-azabicyclo[3,1,0]hex-2-yl)-1-methyl-2-oxoethyl)carbamate;
dimethyl (4,4'-biphenyldiylbis(1H-imidazole-4,2-diyl(1R,3S,5R)-2-azabicyclo[3,1,0]hexane-3,2-diyl((2S)-1-oxo-1,2-butanediyl)))biscarbamate;
methyl (2-((1R,3S,5R)-3-(4-(4'-(2-((1R,3S,5R)-2-(((methoxycarbonyl)amino)acetyl)-2-azabicycto[3.1.0]hex-3-yl)-1H-imidazol-4-yl)-4-biphenylyl)-1H-imidazol-2-yl)-2-azabicyclo[3.1.0]hex-2-yl)-2-oxoethyl) carbamate;
dimethyl (4,4'-biphenyldiylbis(1H-imidazole-4,2-diyl(1R,3S,5R)-2-azabicyclo[3.1.0]hexane-3,2-diyl((1S)-1-cyclopropyl-2-oxo-2,1-ethanediyl)))biscarbamate;
methyl ((1R)-1-(((1R,3S,SR)-3-(4-(4'-(2-((1R,3S,SR)-2-((2R)-2-((methoxycarbonyl)amino)-3-methylbutanoyl)-2-azabicyclo[3.1.0]hex-3-yl)-1H-imidazol-4-yl)-4-biphenylyl)-1H-imidazol-2-yl)-2-azabicyclo[3.1.0]hex-2-yl)carbonyl)-2-methylpropyl)carbamate;
dimethyl (4,4'-biphenyldiylbis(1H-imidazole-4,2-diyl(1R,3S,5R)-2-azabicyclo[3.1.0]hexane-3,2-diyl((1R)-2-oxo-1-phenyl-2,1-ethanediyl)))biscarbamate;
methyl ((1S)-1-(((1R,3S,5R)-3-(4-(4'-(2-((1R,3S,5R)-2-acetyl-2-azabicyclo[3.1.0]hex-3-yl)-1H-imidazol-4-yl)-4-biphenylyl)-1H-imidazol-2-yl)-2-azabicyclo[3.1.0]hex-2-yl)carbonyl)-2-methylpropyl)carbamate;
methyl ((1S)-2-methyl-1-(((1R,3S,5R)-3-(4-(4'-(2-((1R,3S,5R)-2-(5-((3aS,4S,6aR)-2-oxohexahydro-1H-thieno[3,4-d]imidazol-4-yl)pentanoyl)-2-azabicyclo[3.1.0]hex-3-yl)-1H-imidazol-4-yl)-4-biphenylyl)-1H-imidazol-2-yl)-2-azabicyclo[3.1.0]hex-2-yl)carbonyl-2-methylpropyl)carbamate;
N-((1S)-1-(((1R,3S,5R)-3-(4-(4'-(2-((1R,3S,5R)-2-((2S)-2-acetamido-3-methylbutanoyl)-2-azabicyclo[3.1.0]hex-3-yl)-1H-imidazol-4-yl)-4-biphenylyl)-1H-imidazol-2-yl)-2-azabicyclo[3.1.0]hex-2-yl)carbonyl)-2-methylpropyl)acetamide;
tert-butyl ((1S)-1-(((1R,3S,5R)-3-(4-(4'-(2-((1R,3S,5R)-2-((2S)-2-((tert-butoxycarbonyl)amino)-3-methylbutanoyl)-2-azabicyclo[3.1.0]hex-3-yl)-1H-imidazol-4-yl)-4-biphenylyl)-1H-imidazol-2-yl)-2-azabicyclo[3.1.0]hex-2-yl)carbonyl)-2-methylpropyl)carbamate;
(2S)-1-((1R,3S,5R)-3-(4-(4'-(2-((1R,3S,5R)-2-((2S)-2-amino-3-methylbutanoyl)-2-azabicyclo[3.1.0]hex-3-yl)-1H-imidazol-5-yl)-4-biphenylyl)-1H-imidazol-2-yl)-2-azabicyclo[3.1.0]hex-2-yl)-3-methyl-1-oxo-2-butanamine;
N-((1S)-1-(((1R,3S,5R)-3-(4-(4'-(2-((1R,3S,5R)-2-((2S)-2-((cyclopropylcarbonyl)amino)-3-methylbutanoyl)-2-azabicyclo[3.1.0]hex-3-yl)-1H-imidazol-4-yl)-4-biphenylyl)-1H-imidazol-2-yl)-2-azabicyclo[3.1.0]hex-2-yl)carbonyl)-2-methylpropyl)cyclopropanecarbamate;
tert-butyl ((1R)-1-(((1R,3S,5R)-3-(4-(4'-(2-((1R,3S,5R)-2-((2R)-2-((tert-butoxycarbonyl)amino)-3-methylbutanoyl)-2-azabicyclo[3.1.0]hex-3-yl)-1H-imidazol-4-yl)-4-biphenylyl)-1H-imidazol-2-yl)-2-azabicyclo[3.1.0]hex-2-yl)carbonyl)-2-methylpropyl)carbamate;
N-((1R)-1-(((1R,3S,5R)-3-(4-(4'-(2-((1R,3S,5R)-2-((2R)-2-acetarnido-3-methylbutanoyl)-2-azabicyclo[3.1.0]hex-3-yl)-1H-imidazol-4-yl)-4-biphenylyl)-1H-imidazol-2-yl)-2-azabicyclo[3.1.0]hex-2-yl)carbonyl)-2-methylpropyl)acetamide;
N,N'-(4,4'-biphenyldiylbis(1H-imidazole-4,2-diyl(1R,3S,5R)-2-azabicyclo[3.1.0]hexane-3,2-diyl(2S)-3-methyl-1-oxo-1,2-butanediyl))di(2-pyrimidinamine);
methyl ((1S)-1-(((6S)-6-(4-(4'-(2-((6S)-5-((2S)-2-((methoxycarbonyl)amino)-3-methylbutanoyl)-5-azaspiro[2.4]hept-6-yl)-1H-imidazol-4-yl)-4-biphenylyl)-1H-imidazol-2-yl)-5-azaspiro[2.4]hept-5-yl)carbonyl)-2-methylpropyl)carbamate;
methyl ((1S)-1-(((2S,5S)-2-(4-(4'-(2-((2S,5S)-1-((2S)-2-((methoxycarbonyl)amino)-3-methylbutanoyl)-5-methyl-2-pyrrolidinyl)-1H-imidazol-4-yl)-4-biphenylyl)-1H-imidazol-2-yl)-5-methyl-1-pyrrolidinyl)carbonyl)-2-methylpropyl)carbamate;
methyl (2-((6S)-6-(4-(4'-(2-((6S)-5-(((methoxycarbonyl)amino)acetyl)-5-azaspiro[2.4]hept-6-yl)-1H-imidazol-4-yl)-4-biphenylyl)-1H-imidazol-2-yl)-5-azaspiro[2.4]hept-5-yl)-2-oxoethyl)carbamate;
methyl ((1S)-2-((6S)-6-(4-(4'-(2-((6S)-5-(N-(methoxycarbonyl)-L-alanyl)-5-azaspiro[2.4]hept-6-yl)-1H-imidazol-4-yl)-4-biphenylyl)-1H-imidazol-2-yl)-5-azaspiro[2.4]hept-5-yl)-1-methyl-2-oxoethyl)carbamate;
dimethyl (4,4'-biphenyldiylbis(1H-imidazole-4,2-diyl(6S)-5-azaspiro[2.4]heptane-6,5-diyl((2S)-1-oxo-1,2-butanediyl)))biscarbamate;
methyl ((1R)-1-(((6S)-6-(4-(4'-(2-((6S)-5-((2R)-2-((methoxycarbonyl)amino)-3-methylbutanoyl)-5-azaspiro[2.4]hept-6-yl)-1H-imidazol-4-yl)-4-biphenylyl)-1H-imidazol-2-yl)-5-azaspiro[2.4]hept-5-yl)carbonyl)-2-methylpropyl)carbamate;
dimethyl (4,4'-biphenyldiylbis(1H-imidazole-4,2-diyl(6S)-5-azaspiro[2.4]heptane-6,5-diyl((1R)-2-oxo-1-phenyl-2,1-ethanediyl)))biscarbamate;
methyl ((1R)-1-(((2S,SS)-2-(4-(4'-(2-((2S,5S)-1-((2R)-2-((methoxycarbonyl)amino)-3-methylbutanoyl)-5-methyl-2-pyrrolidinyl)-1H-imidazol-4-yl)-4-biphenylyl)-1H-imidazol-2-yl)-5-methyl-1-pyrrolidinyl)carbonyl)-2-methylpropyl)carbamate;
dimethyl (4,4'-biphenyldiylbis(1H-imidazole-4,2-diyl((2S,5S)-5-methyl-2,1-pyrrolidinediyl)((1R)-2-oxo-1-phenyl-2,1-ethanediyl)))biscarbamate;
dimethyl (4,4'-biphenyldiylbis(1H-imidazole-4,2-diyl((2S,5S)-5-methyl-2,1-pyrrolidinediyl)((2S)-1-oxo-1,2-butanediyl)))biscarbamate;
methyl (2-((2S,5S)-2-(4-(4'-(2-((2S,5S)-1-(((methoxycarbonyl)amino)acetyl)-5-methyl-2-pyrrolidinyl)-1H-imidazol-4-yl)-4-biphenylyl)-1H-imidazol-2-yl)-5-methyl-1-pyrrolidinyl)-2-oxoethyl)carbamate;
methyl (2-((2S,5S)-2-(4-(4'-(2-((2S,5S)-1-(2-((methoxycarbonyl)amino)-2-methylpropanoyl)-5-methyl-2-pyrrolidinyl)-1H-imidazol-4-yl)-4-biphenylyl)-1H-imidazol-2-yl)-5-methyl-1-pyrrolidinyl)-1,1-dimethyl-2-oxoethyl)carbamate;
methyl ((1S)-2-((2S,5S)-2-(4-(4'-(2-((2S,5S)-1-(N-(methoxycarbonyl)-L-alanyl)-5-methyl-2-pyrrolidinyl)-1H-imidazol-4-yl)-4-biphenylyl)-1H-imidazol-2-yl)-5-methyl-1-pyrrolidinyl)-1-methyl-2-oxoethyl)carbamate;
4,4'-(4,4'-biphenyldiyl)bis(2-((2S,5S)-5-methyl-1-(3-methylbutanoyl)-2-pyrrolidinyl)-1 H-imidazole);
4,4'-(4,4'-biphenyldiyl)bis(2-((25,5S)-5-methyl-1-(phenylacetyl)-2-pyrrolidinyl)-1H-imidazole);
(2R,2'R)-1,1'-(4,4'-biphenyldiylbis(1H-imidazole-4,2-diyl((2S,5S)-5-methyl-2,1-pyrrolidinediyl)))bis(3-methyl-1-oxo-2-butanol);
(25,2'S)-1,1'-(4,4'-biphenyldiyibis(1H-imidazole-4,2-diyl((2S,5S)-5-methyl-2,1-pyrrolidinediyl)))bis(3-methyl-1-oxo-2-butanol);
2-((2S,5S)-1-acetyl-5-methyl-2-pyrrolidinyl)-4-(4'-(2-((2S,5S)-1-acetyl-5-methyl-2-pyrrolidinyl)-1H-imidazol-5-yl)-4-biphenylyl)-1H-imidazole;
tert-butyl (2S)-2-(4-(4'-(2-((2S)-1-(tert-butoxycarbonyl)-2-pyrrolidinyl)-4-(1,3-dioxan-2-ylmethyl)-1H-imidazol-5-yl)-4-biphenylyl)-1H-imidazol-2-yl)-1-pyrrolidinecarboxylate;
tert-butyl (2S)-2-(4-(4'-(2-((2S)-1-(tert-butoxycarbonyl)-2-pyrrolidinyl)-4-(1,3-dioxolan-2-ylmethyl)-1H-imidazol-5-yl)-4-biphenylyl)-1H-imidazol-2-yl)-1-pyrrolidinecarboxylate;
tert-butyl (2S)-2-(4-(4'-(2-((2S)-1-(tert-butoxycarbonyl)-2-pyrrolidinyl)-1H-imidazol-4-yl)-4-biphenylyl)-5-(2-methoxy-2-oxoethyl)-1H-imidazol-2-yl)-1-pyrrolidinecarboxylate;
tert-butyl (2S)-2-(4-(4'-(2-((2S)-1-(tert-butoxycarbonyl)-2-pyrrolidinyl)-1H-imidazol-4-yl)-4-biphenylyl)-5-propyl-1H-imidazol-2-yl)-1-pyrrolidinecarboxylate;
ethyl 2-((2S)-1-(tert-butoxycarbonyl)-2-pyrrolidinyl)-5-(4'-(2-((2S)-1-(tert-butoxycarbonyl)-2-pyrrolidinyl)-1H-imidazol-5-yl)-4-biphenylyl)-1H-imidazole-4-carboxylate;
tert-butyl (2S)-2-(4-(4'-(2-((2S)-1-(tert-butoxycarbonyl)-2-pyrrolidinyl)-1H-imidazol-4-yl)-3'-fluoro-4-biphenylyl)-1H-imidazol-2-yl)-4,4-difluoro-1-pyrrolidinecarboxylate;
2-((2S)-4,4-difluoro-2-pyrrolidinyl)-4-(3'-fluoro-4'-(2-((2S)-2-pyrrolidinyl)-1H-imidazol-4-yl)-4-biphenylyl)-1H-imidazole;
(1R)-2-((2S)-2-(4-(4'-(2-((2S)-1-((2R)-2-(diethylamino)-2-phenylacetyl)-2-pyrrolidinyl)-1H-imidazol-4-yl)-4-biphenylyl)-5-methyl-1H-imidazol-2-yl)-1-pyrrolidinyl)-N,N-diethyl-2-oxo-1-phenylethanamine;
1-((1R)-2-((2S)-2-(4-(4'-(4-methyl-2-((2S)-1-((2R)-2-phenyl-2-(1-piperidinyl)acetyl)-2-pyrrolidinyl)-1H-imidazol-5-yl)-4-biphenylyl)-1H-imidazol-2-yl)-1-pyrrolidinyl)-2-oxo-1-phenylethyl)piperidine;
methyl ((1R)-2-((2S)-2-(4-(4'-(2-((2S)-1-((2R)-2-((methoxycarbonyl)amino)-2-phenylacetyl)-2-pyrrolidinyl)-1H-imidazol-4-yl)-4-biphenylyl)-5-methyl-1H-imidazol-2-yl)-1-pyrrolidinyl)-2-oxo-1-phenylethyl)carbamate;
methyl ((1S)-2-((25)-2-(4-(4'-(2-((25)-1-N-(methoxycarbonyl)-L-alanyl)-2-pyrrolidinyl)-4-methyl-1H-imidazol-5-yl)-4-biphenylyl)-1H-imidazol-2-yl)-1-pyrrolidinyl)-1-methyl-2-oxoethyl)carbamate;
methyl ((1S)-1-(((2S)-2-(4-(4'-(2-((2S)-1-((2S)-2-((methoxycarbonyl)amino)-3-methylbutanoyl)-2-pyrrolidinyl)-1H-imidazol-4-yl)-4-biphenylyl)-5-methyl-1H-imidazol-2-yl)-1-pyrrolidinyl)carbonyl)-2-methylpropyl)carbamate;
methyl ((1S,2R)-2-methoxy-1-(((2S)-2-(4-(4'-(2-((2S)-1-(N-(methoxycarbonyl)-O-methyl-L-threonyl)-2-pyrrolidinyl)-4-methyl-1H-imidazol-5-yl)-4-biphenylyl)-1H-imidazol-2-yl)-1-pyrrolidinyl)carbonyl)propyl)carbamate;
(1R,1'R)-2,2'-(4,4'-biphenyldiylbis((4-methyl-1H-imidazole-5,2-diyl)(2S)-2,1-pyrrolidinediyl))bis(N,N-dimethyl-2-oxo-1-phenylethanamine);
(1R,1'R)-2,2'-(4,4'-biphenyldiylbis((4-methyl-1H-imidazole-5,2-diyl)(2S)-2,1-pyrrolidinediyl))bis(N,N-diethyl-2-oxo-1-phenylethanamine);
1,1'-(4,4'-biphenyldiylbis((4-methyl-1H-imidazole-5,2-diyl)(2S)-2,1-pyrrolidinediyl((1R)-2-oxo-1-phenyl-2,1-ethanediyl)))dipiperidine;
dimethyl (4,4'-biphenyldiylbis((4-methyl-1H-imidazole-5,2-diyl)(2S)-2,1-pyrrolidinediyl((1R)-2-oxo-1-phenyl-2,1-ethanediyl)))biscarbamate;
methyl ((1S)-2-((2S)-2-(4-(4'-(2-((2S)-1-(N-(methoxycarbonyl)-L-alanyl)-2-pyrrolidinyl)-5-methyl-1H-imidazol-4-yl)-4-biphenylyl)-5-methyl-1H-imidazol-2-yl)-1-pyrrolidinyl)-1-methyl-2-oxoethyl)carbamate;
methyl ((1S)-1-(((2S)-2-(4-(4'-(2-((2S)-1-((2S)-2-((methoxycarbonyl)amino)-3-methylbutanoyl)-2-pyrrolidinyl)-4-methyl-1H-imidazol-5-yl)-4-biphenylyl)-5-methyl-1H-imidazol-2-yl)-l-pyrrolidinyl)carbonyl)-2-methylpropyl)carbamate;
methyl ((1S,2R)-2-methoxy-1-(((2S)-2-(4-(4'-(2-((2S)-1-(N-(methoxycarbonyl)-O-methyl-L-threonyl)-2-pyrrolidinyl)-5-methyl-1H-imidazol-4-yl)-4-biphenylyl)-5-methyl-1H-imidazol-2-yl)-1-pyrrolidinyl)carbonyl)propyl)carbamate;
methyl ((1S)-1-(((2S)-2-(4-(1,3-dioxan-2-ylmethyl)-5-(4'-(2-((2S)-1-((2S)-2-((methoxycarbonyl)amino)-3-methylbutanoyl)-2-pyrrolidinyl)-1H-imidazol-4-yl)-4-biphenylyl)-1H-imidazol-2-yl)-1-pyrrolidinyl)carbonyl)-2-methylpropyl)carbamate;
methyl ((1S,2R)-1-(((2S)-2-(4-(2,2-dimethoxyethyl)-5-(4'-(2-((2S)-1-(N-(methoxycarbonyl)-O-methyl-L-threonyl)-2-pyrrolidinyl)-1 H-imidazol-4-yl)-4-biphenylyl)-1H-imidazol-2-yl)-1-pyrrolidinyl)carbonyl)-2-methoxypropyl)carbamate;
methyl ((1S)-1-(((2S)-2-(4-(2,2-dimethoxyethyl)-5-(4'-(2-((2S)-1-((2S)-2-((methoxycarbonyl)amino)-3-methylbutanoyl)-2-pyrrolidinyl)-1H-imidazol-4-yl)-4-biphenylyl)-1H-imidazol-2-yl)-1-pyrrolidinyl)carbonyl)-2-methylpropyl)carbamate;
methyl (2-((2S)-1-(N-(methoxycarbonyl)-O-methyl-L-threonyl)-2-pyrrolidinyl)-4-(4'-(2-((2S)-1-(N-(methoxycarbonyl)-O-methyl-L-threonyl)-2-pyrrolidinyl)-1H-imidazol-4-yl)-4-biphenylyl)-1H-imidazol-5-yl)acetate;
methyl (2-((2S)-1-(N-(methoxycarbonyl)-L-valyl)-2-pyrrolidinyl)-4-(4'-(2-((2S)-1-(N-(methoxycarbonyl)-L-valyl)-2-pyrrolidinyl)-1H-imidazol-4-yl)-4-biphenylyl)-1H-imidazol-5-yl)acetate;
methyl ((1S)-2-((2S)-2-(4-(4'-(2-((2S)-1-(N-(methoxycarbonyl)-L-alanyl)-2-pyrrolidinyl)-4-propyl-1H-imidazol-5-yl)-4-biphenylyl)-1H-imidazol-2-yl)-1-pyrrolidinyl)-1-methyl-2-oxoethyl)carbamate;
(1R)-2-((2S)-2-(4-(4'-(2-((2S)-1-((2R)-2-(diethylamino)-2-phenylacetyl)-2-pyrrolidinyl)-1H-imidazol-4-yl)-4-biphenylyl)-5-propyl-1H-imidazol-2-yl)-1-pyrrolidinyl)-N,N-diethyl-2-oxo-1-phenylethanamine;
methyl ((1S)-1-(((2S)-2-(4-(4'-(2-((2S)-1-((2S)-2-((methoxycarbonyl)amino)-3-methylbutanoyl)-2-pyrrolidinyl)-1H-imidazol-4-yl)-4-biphenylyl)-5-propyl-1H-imidazol-2-yl)-1-pyrrolidinyl)carbonyl)-2-methylpropyl)carbamate;
methyl ((1S)-1-(((2S)-4,4-difluoro-2-(4-(3'-fluoro-4'-(2-((2S)-1-((2S)-2-((methoxycarbonyl)amino)-3-methylbutanoyl)-2-pyrrolidinyl)-1H-imidazol-4-yl)-4-biphenylyl)-1H-imidazol-2-yl)-1-pyrrolidinyl)carbonyl)-2-methylpropyl)carbamate;
(1R)-2-((2S)-2-(4-(4'-(2-((2S)-1-((2R)-2-(diethylamino)-2-phenylacetyl)-4,4-difluoro-2-pyrrolidinyl)-1H-imidazol-4-yl)-3-fluoro-4-biphenylyl)-1H-imidazol-2-yl)-1-pyrrolidinyl)-N,N-diethyl-2-oxo-1-phenylethanamine;
methyl ((1S)-1-(((2S)-2-(4-(hydroxymethyl)-5-(4'-(2-((2S)-1-((2S)-2-((methoxycarbonyl)amino)-3-methylbutanoyl)-2-pyrrolidinyl)-1H-imidazol-4-yl)-4-biphenylyl)-1H-imidazol-2-yl)-1-pyrrolidinyl)carbonyl)-2-methylpropyl)carbamate;
methyl ((1S)-2-((2S)-2-(4-(4'-(4-(hydroxymethyl)-2-((2S)-1-(N-(methoxycarbonyl)-L-alanyl)-2-pyrrolidinyl)-1H-imidazol-5-yl)-4-biphenylyl)-1H-imidazol-2-yl)-1-pyrrolidinyl)-1-methyl-2-oxoethyl)carbamate;
(N,N'-(4,4'-biphenyldiylbis(1H-imidazole-4,2-diyl(2S)-2,1-pyrrolidinediyl((2S)-3-methyl-1-oxo-1,2-butanediyl)))di(2-pyrimidinamine);
N,N'-(4,4'-biphenyldiylbis(1H-imidazole-4,2-diyl(2S)-2,1-pyrrolidinediyl((1S)-1-cyclopropyl-2-oxo-2,1-ethanediyl)))di(2-pyrimidinamine);
N,N'-(4,4'-biphenyldiylbis(1H-imidazole-4,2-diyl(2S)-2,1-pyrrolidinediyl((2S)-1-oxo-1,2-propanediyl)))di(2-pyrimidinamine);
N,N'-(4,4'-biphenyldiylbis(1H-imidazole-4,2-diyl(2S)-2,1-pyrrolidinediyl((2S)-1-oxo-1,2-butanediyl)))di(2-pyrimidinamine);
N,N'-(4,4'-biphenyldiylbis(1H-imidazole-4,2-diyl(2S)-2,1-pyrrolidinediyl((2S)-3-methoxy-1-oxo-1,2-propanediyl)))di(2-pyrimidinamine); and
N,N'-(4,4'-biphenyldiylbis(1H-imidazole-4,2-diyl(25)-2,1-pyrrolidinediyl((2S,3R)-3-methoxy-1-oxo-1,2-butanediyl)))di(2-pyrimidinamine); 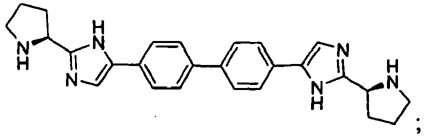 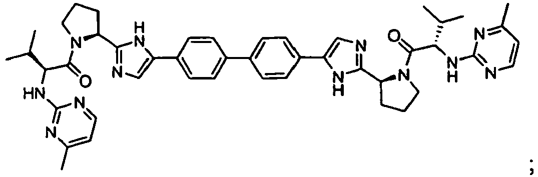 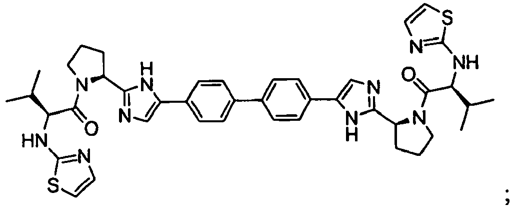 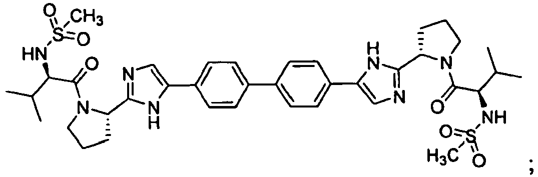 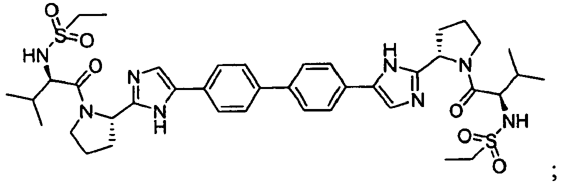 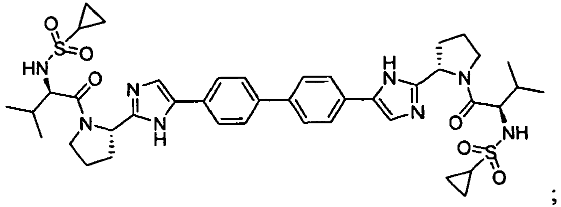 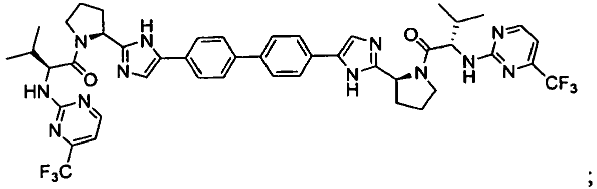 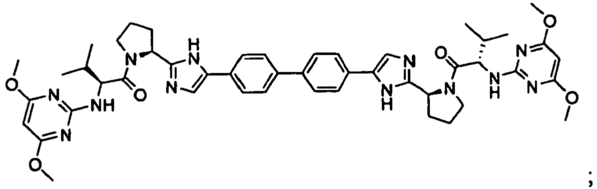 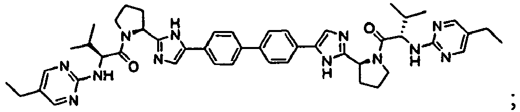 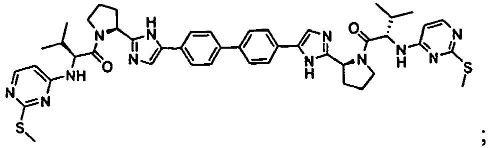 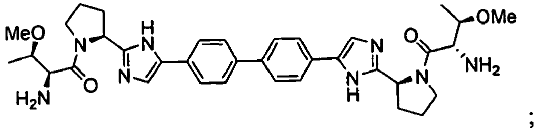 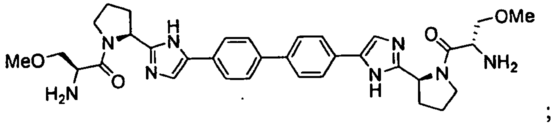 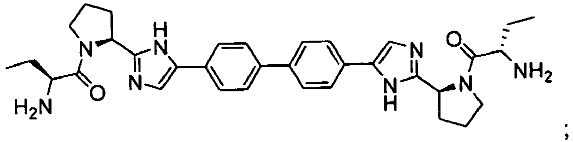 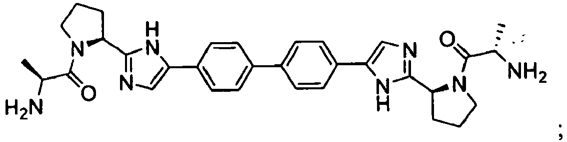 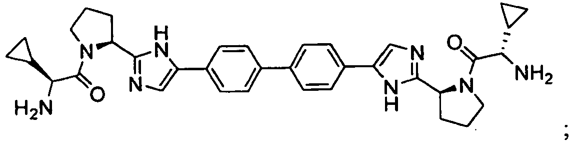 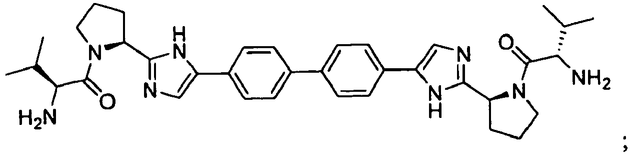 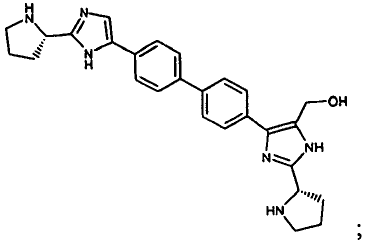 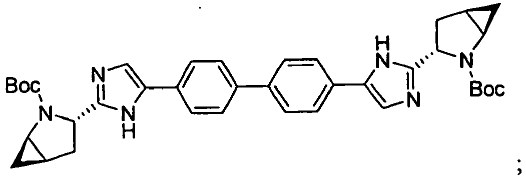 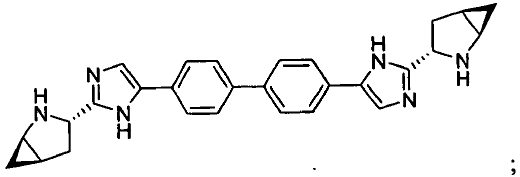 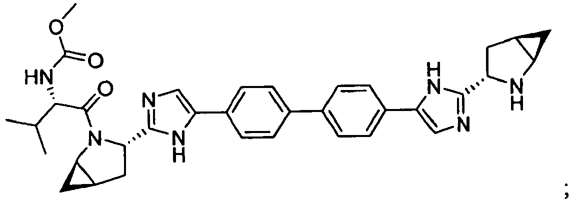 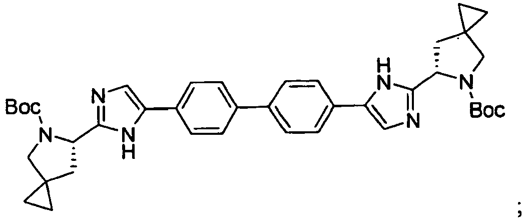 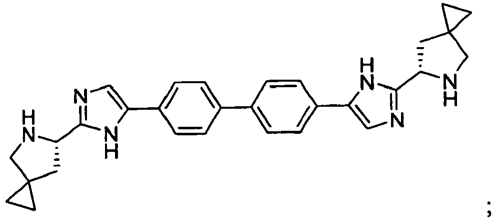 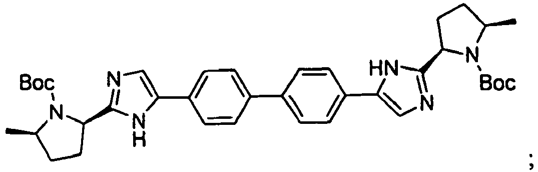 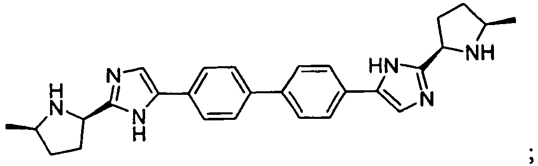 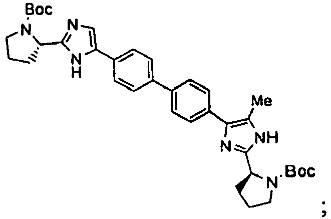 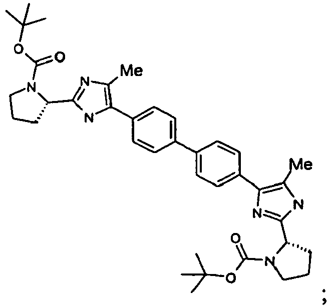 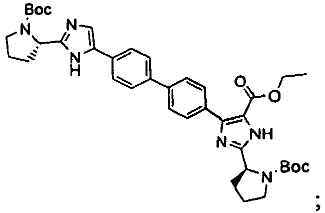 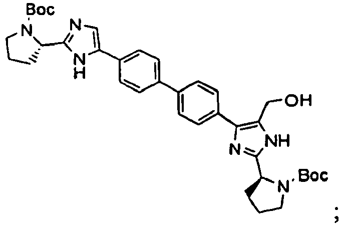 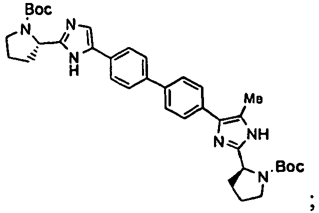 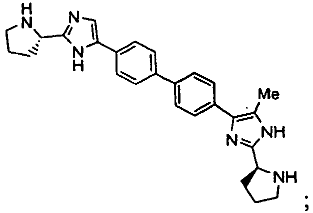 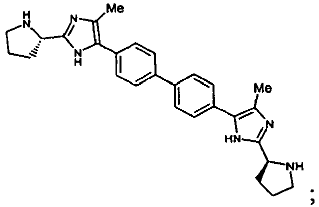 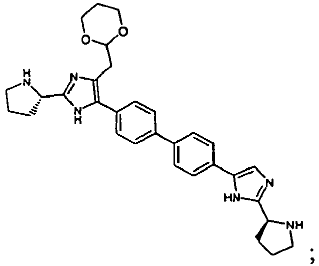 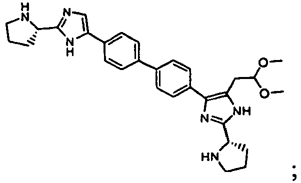 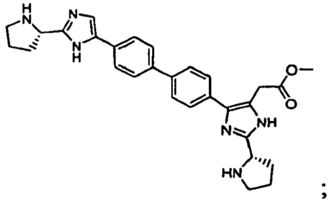 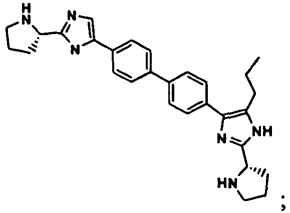 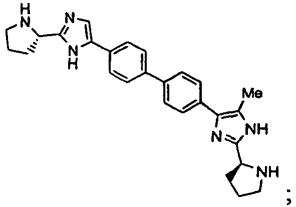 and 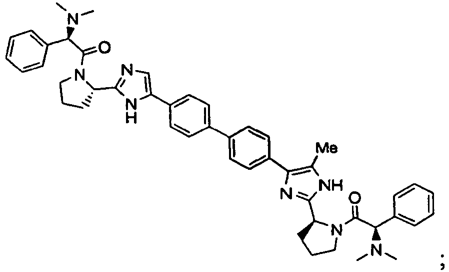 or a pharmaceutically acceptable salt thereof.

In an embodiment the pharmaceutically acceptable salt is a dihydrochloride salt.

The present disclosure further provides a composition comprising a compound of the invention, or a pharmaceutically acceptable salt thereof, and a pharmaceutically acceptable carrier.

In a first embodiment the composition further comprises one or two additional compounds having anti-HCV activity. In a second embodiment at least one of the additional compounds is an interferon or a ribavirin. In a third embodiment the interferon is selected from interferon alpha 2B, pegylated interferon alpha, consensus interferon, interferon alpha 2A, and lymphoblastiod interferon tau.

In a further embodiment the composition further comprises one or two additional compounds having anti-HCV activity wherein at least one of the additional compounds is selected from interleukin 2, interleukin 6, interleukin 12, a compound that enhances the development of a type 1 helper T cell response, interfering RNA, anti-sense RNA, Imiqimod, ribavirin, an inosine 5'-monophospate dehydrogenase inhibitor, amantadine, and rimantadine.

In a further embodiment the composition further comprises one or two additional compounds having anti-HCV activity wherein at least one of the additional compounds is effective to inhibit the function of a target selected from HCV metalloprotease, HCV serine protease, HCV polymerase, HCV helicase, HCV NS4B protein, HCV entry, HCV assembly, HCV egress, HCV NS5A protein, and IMPDH for the treatment of an HCV infection.

The present disclosure further provides a compound for use in a method of treating an HCV infection in a patient, comprising administering to the patient a therapeutically effective amount of a compound of the invention, or a pharmaceutically acceptable salt thereof.

In a first embodiment the method further comprises administering one or two additional compounds having anti-HCV activity prior to, after or simultaneously with the compound of the invention, or a pharmaceutically acceptable salt thereof. In a second embodiment at least one of the additional compounds is an interferon or a ribavirin. In a third embodiment the interferon is selected from interferon alpha 2B, pegylated interferon alpha, consensus interferon, interferon alpha 2A, and lymphoblastiod interferon tau.

In a fourth embodiment the method further comprises administering one or two additional compounds having anti-HCV activity prior to, after or simultaneously with the compound of the invention, or a pharmaceutically acceptable salt thereof, wherein at least one of the additional compounds is selected from interleukin 2, interleukin 6, interleukin 12, a compound that enhances the development of a type I helper T cell response, interfering RNA, anti-sense RNA, Imiqimod, ribavirin, an inosine 5'-monophospate dehydrogenase inhibitor, amantadine, and rimantadine.

In a fifth embodiment the method further comprises administering one or two additional compounds having anti-HCV activity prior to, after or simultaneously with the compound of formula (I), or a pharmaceutically acceptable salt thereof, wherein at least one of the additional compounds is effective to inhibit the function of a target selected from HCV metalloprotease, HCV serine protease, HCV polymerase, HCV helicase, HCV NS4B portein, HCV entry, HCV assembly, HCV egress, HCV NSSA protein, and IMPDH for the treatment of an HCV infection.

Other embodiments of the present disclosure may comprise suitable combinations of two or more of embodiments and/or aspects disclosed herein.

Yet other embodiments and aspects of the disclosure will be apparent according to the description provided below.

The compounds of the present disclosure also exist as tautomers; therefore the present disclosure also encompasses all tautomeric forms.

The description of the present disclosure herein should be construed in congruity with the laws and principals of chemical bonding. In some instances it may be necessary to remove a hydrogen atom in order accommodate a substitutent at any given location. For example, in the structure shown below 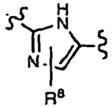 R⁸ may be attached to either the carbon atom in the imidazole ring or, alternatively, R⁸ may take the place of the hydrogen atom on the nitrogen ring to form an N-substituted imidazole.

It should be understood that the compounds encompassed by the present disclosure are those that are suitably stable for use as pharmaceutical agent.

As used in the present specification, the following terms have the meanings indicated:

As used herein, the singular forms "a", "an", and "the" include plural reference unless the context clearly dictates otherwise.

Asymmetric centers exist in the compounds of the present disclosure. These centers are designated by the symbols "R" or "S", depending on the configuration of substituents around the chiral carbon atom. Individual stereoisomers of compounds can be prepared synthetically from commercially available starting materials which contain chiral centers or by preparation of mixtures of enantiomeric products followed by separation such as conversion to a mixture of diastereomers followed by separation or recrystallization, chromatographic techniques, or direct separation of enantiomers on chiral chromatographic columns. Starting compounds of particular stereochemistry are either commercially available or can be made and resolved by techniques known in the art.

Certain compounds of the present disclosure may also exist in different stable conformational forms which may be separable. Torsional asymmetry due to restricted rotation about an asymmetric single bond, for example because of steric hindrance or ring strain, may permit separation of different conformers. The present disclosure includes each conformational isomer of these compounds and mixtures thereof.

The term "compounds of the present disclosure", and equivalent expressions, are meant to embrace said compounds, and pharmaceutically acceptable enantiomers, diastereomers, and salts thereof. Similarly, references to intermediates are meant to embrace their salts where the context so permits.

The compounds of the present disclosure can exist as pharmaceutically acceptable salts. The term "pharmaceutically acceptable salt," as used herein, represents salts or zwitterionic forms of the compounds of the present disclosure which are water or oil-soluble or dispersible, which are, within the scope of sound medical judgment, suitable for use in contact with the tissues of patients without excessive toxicity, irritation, allergic response, or other problem or complication commensurate with a reasonable benefit/risk ratio, and are effective for their intended use The salts can be prepared during the final isolation and purification of the compounds or separately by reacting a suitable nitrogen atom with a suitable acid. Representative acid addition salts include acetate, adipate, alginate, citrate, aspartate, benzoate, benzenesulfonate, bisulfate, butyrate, camphorate, camphorsulfonate; digluconate, dihydrobromide, diydrochloride, dihydroiodide, glycerophosphate, hemisulfate, heptanoate, hexanoate, formate, fumarate, hydrochloride, hydrobromide, hydroiodide, 2-hydroxyethanesulfonate, lactate, maleate, mesitylenesulfonate, methanesulfonate, naphthylenesulfonate, nicotinate, 2-naphthalenesulfonate, oxalate, palmoate, pectinate, persulfate, 3-phenylproprionate, picrate, pivalate, propionate, succinate, tartrate, trichloroacetate, trifluoroacetate, phosphate, glutamate, bicarbonate, para-toluenesulfonate, and undecanoate. Examples of acids which can be employed to form pharmaceutically acceptable addition salts include inorganic acids such as hydrochloric, hydrobromic, sulfuric, and phosphoric, and organic acids such as oxalic, maleic, succinic, and citric.

Basic addition salts can be prepared during the final isolation and purification of the compounds by reacting a carboxy group with a suitable base such as the hydroxide, carbonate, or bicarbonate of a metal cation or with ammonia or an organic primary, secondary, or tertiary amine. The cations of pharmaceutically acceptable salts include lithium, sodium, potassium, calcium, magnesium, and aluminum, as well as nontoxic quaternary amine cations such as ammonium, tetramethylammonium, tetraethylammonium, methylamine, dimethylamine, trimethylamine, triethylamine, diethylamine, ethylamine, tributylamine, pyridine, N,N-dimethylaniline, N-methylpiperidine, N-methylmorpholine, dicyclohexylamine, procaine, dibenzylamine, N,N-dibenzylphenethylamine, and N,N'-dibenzylethylenediamine. Other representative organic amines useful for the formation of base addition salts include ethylenediamine, ethanolamine, diethanolamine, piperidine, and piperazine.

When it is possible that, for use in therapy, therapeutically effective amounts of a compound of the invention as well as pharmaceutically acceptable salts thereof, may be administered as the raw chemical, it is possible to present the active ingredient as a pharmaceutical composition. Accordingly, the disclosure further provides pharmaceutical compositions, which include therapeutically effective amounts of the compounds of the invention or pharmaceutically acceptable salts thereof, and one or more pharmaceutically acceptable carriers, diluents, or excipients. The term "therapeutically effective amount," as used herein, refers to the total amount of each active component that is sufficient to show a meaningful patient benefit, e.g., a reduction in viral load. When applied to an individual active ingredient, administered alone, the term refers to that ingredient alone. When applied to a combination, the term refers to combined amounts of the active ingredients that result in the therapeutic effect, whether administered in combination, serially, or simultaneously. The compounds of the invention and pharmaceutically acceptable salts thereof, are as described above. The carrier(s), diluent(s), or excipient(s) must be acceptable in the sense of being compatible with the other ingredients of the formulation and not deleterious to the recipient thereof. In accordance with another aspect of the present disclosure there is also provided a process for the preparation of a pharmaceutical formulation including admixing a compound of the invention, or a pharmaceutically acceptable salt thereof, with one or more pharmaceutically acceptable carriers, diluents, or excipients. The term "pharmaceutically acceptable," as used herein, refers to those compounds, materials, compositions, and/or dosage forms which are, within the scope of sound medical judgment, suitable for use in contact with the tissues of patients without excessive toxicity, irritation, allergic response, or other problem or complication commensurate with a reasonable benefit/risk ratio, and are effective for their intended use.

Pharmaceutical formulations may be presented in unit dose forms containing a predetermined amount of active ingredient per unit dose. Dosage levels of between about 0.01 and about 250 milligram per kilogram ("mg/kg") body weight per day, preferably between about 0.05 and about 100 mg/kg body weight per day of the compounds of the present disclosure are typical in a monotherapy for the prevention and treatment of HCV mediated disease. Typically, the pharmaceutical compositions of this disclosure will be administered from about 1 to about 5 times per day or alternatively, as a continuous infusion. Such administration can be used as a chronic or acute therapy. The amount of active ingredient that may be combined with the carrier materials to produce a single dosage form will vary depending on the condition being treated, the severity of the condition, the time of administration, the route of administration, the rate of excretion of the compound employed, the duration of treatment, and the age, gender, weight, and condition of the patient. Preferred unit dosage formulations are those containing a daily dose or sub-dose, as herein above recited, or an appropriate fraction thereof, of an active ingredient. Treatment may be initiated with small dosages substantially less than the optimum dose of the compound. Thereafter, the dosage is increased by small increments until the optimum effect under the circumstances is reached. In general, the compound is most desirably administered at a concentration level that will generally afford antivirally effective results without causing any harmful or deleterious side effects.

When the compositions of this disclosure comprise a combination of a compound of the present disclosure and one or more additional therapeutic or prophylactic agent, both the compound and the additional agent are usually present at dosage levels of between about 10 to 150%, and more preferably between about 10 and 80% of the dosage normally administered in a monotherapy regimen.

Pharmaceutical formulations may be adapted for administration by any appropriate route, for example by the oral (including buccal or sublingual), rectal, nasal, topical (including buccal, sublingual, or transdermal), vaginal, or parenteral (including subcutaneous, intracutaneous, intramuscular, intra-articular, intrasynovial, intrasternal, intrathecal, intralesional, intravenous, or intradermal injections or infusions) route. Such formulations may be prepared by any method known in the art of pharmacy, for example by bringing into association the active ingredient with the carrier(s) or excipient(s). Oral administration or administration by injection are preferred.

Pharmaceutical formulations adapted for oral administration may be presented as discrete units such as capsules or tablets; powders or granules; solutions or suspensions in aqueous or non-aqueous liquids; edible foams or whips; or oil-in-water liquid emulsions or water-in-oil emulsions.

For instance, for oral administration in the form of a tablet or capsule, the active drug component can be combined with an oral, non-toxic pharmaceutically acceptable inert carrier such as ethanol, glycerol, water, and the like. Powders are prepared by comminuting the compound to a suitable fine size and mixing with a similarly comminuted pharmaceutical carrier such as an edible carbohydrate, as, for example, starch or mannitol. Flavoring, preservative, dispersing, and coloring agent can also be present.

Capsules are made by preparing a powder mixture, as described above, and filling formed gelatin sheaths. Glidants and lubricants such as colloidal silica, talc, magnesium stearate, calcium stearate, or solid polyethylene glycol can be added to the powder mixture before the filling operation. A disintegrating or solubilizing agent such as agar-agar, calcium carbonate, or sodium carbonate can also be added to improve the availability of the medicament when the capsule is ingested.

Moreover, when desired or necessary, suitable binders, lubricants, disintegrating agents, and coloring agents can also be incorporated into the mixture. Suitable binders include starch, gelatin, natural sugars such as glucose or beta-lactose, corn sweeteners, natural and synthetic gums such as acacia, tragacanth or sodium alginate, carboxymethylcellulose, polyethylene glycol, and the like. Lubricants used in these dosage forms include sodium oleate, sodium chloride, and the like. Disintegrators include, without limitation, starch, methyl cellulose, agar, betonite, xanthan gum, and the like. Tablets are formulated, for example, by preparing a powder mixture, granulating or slugging, adding a lubricant and disintegrant, and pressing into tablets. A powder mixture is prepared by mixing the compound, suitable comminuted, with a diluent or base as described above, and optionally, with a binder such as carboxymethylcellulose, an aliginate, gelating, or polyvinyl pyrrolidone, a solution retardant such as paraffin, a resorption accelerator such as a quaternary salt and/or and absorption agent such as betonite, kaolin, or dicalcium phosphate. The powder mixture can be granulated by wetting with a binder such as syrup, starch paste, acadia mucilage, or solutions of cellulosic or polymeric materials and forcing through a screen. As an alternative to granulating, the powder mixture can be run through the tablet machine and the result is imperfectly formed slugs broken into granules. The granules can be lubricated to prevent sticking to the tablet forming dies by means of the addition of stearic acid, a stearate salt, talc, or mineral oil. The lubricated mixture is then compressed into tablets. The compounds of the present disclosure can also be combined with a free flowing inert carrier and compressed into tablets directly without going through the granulating or slugging steps. A clear or opaque protective coating consisting of a sealing coat of shellac, a coating of sugar or polymeric material, and a polish coating of wax can be provided. Dyestuffs can be added to these coatings to distinguish different unit dosages.

Oral fluids such as solution, syrups, and elixirs can be prepared in dosage unit form so that a given quantity contains a predetermined amount of the compound. Syrups can be prepared by dissolving the compound in a suitably flavored aqueous solution, while elixirs are prepared through the use of a non-toxic vehicle. Solubilizers and emulsifiers such as ethoxylated isostearyl alcohols and polyoxyethylene sorbitol ethers, preservatives, flavor additive such as peppermint oil or natural sweeteners, or saccharin or other artificial sweeteners, and the like can also be added.

Where appropriate, dosage unit formulations for oral administration can be microencapsulated. The formulation can also be prepared to prolong or sustain the release as for example by coating or embedding particulate material in polymers, wax, or the like.

The compounds of the invention and pharmaceutically acceptable salts thereof, can also be administered in the form of liposome delivery systems, such as small unilamellar vesicles, large unilamellar vesicles, and multilamellar vesicles. Liposomes can be formed from a variety of phopholipids, such as cholesterol, stearylamine, or phophatidylcholines.

The compounds of the invention and pharmaceutically acceptable salts thereof may also be delivered by the use of monoclonal antibodies as individual carriers to which the compound molecules are coupled. The compounds may also be coupled with soluble polymers as targetable drug carriers. Such polymers can include polyvinylpyrrolidone, pyran copolymer, polyhydroxypropylmethacrylamidephenol, polyhydroxyethylaspartamidephenol, or polyethyleneoxidepolylysine substituted with palitoyl residues. Furthermore, the compounds may be coupled to a class of biodegradable polymers useful in achieving controlled release of a drug, for example, polylactic acid, polepsilon caprolactone, polyhydroxy butyric acid, polyorthoesters, polyacetals, polydihydropyrans, polycyanoacrylates, and cross-linked or amphipathic block copolymers of hydrogels.

Pharmaceutical formulations adapted for transdermal administration may be presented as discrete patches intended to remain in intimate contact with the epidermis of the recipient for a prolonged period of time. For example, the active ingredient may be delivered from the patch by iontophoresis as generally described in Pharmaceutical Research 1986, 3(6), 318.

Pharmaceutical formulations adapted for topical administration may be formulated as ointments, creams, suspensions, lotions, powders, solutions, pastes, gels, sprays, aerosols, or oils.

Pharmaceutical formulations adapted for rectal administration may be presented as suppositories or as enemas.

Pharmaceutical formulations adapted for nasal administration wherein the carrier is a solid include a course powder having a particle size for example in the range 20 to 500 microns which is administered in the manner in which snuff is taken, *i.e.*, by rapid inhalation through the nasal passage from a container of the powder held close up to the nose. Suitable formulations wherein the carrier is a liquid, for administration as a nasal spray or nasal drops, include aqueous or oil solutions of the active ingredient.

Pharmaceutical formulations adapted for administration by inhalation include fine particle dusts or mists, which may be generated by means of various types of metered, dose pressurized aerosols, nebulizers, or insufflators.

Pharmaceutical formulations adapted for vaginal administration may be presented as pessaries, tampons, creams, gels, pastes, foams, or spray formulations.

Pharmaceutical formulations adapted for parenteral administration include aqueous and non-aqueous sterile injection solutions which may contain anti-oxidants, buffers, bacteriostats, and soutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilized) condition requiring only the addition of the sterile liquid carrier, for example water for injections, immediately prior to use, Extemporaneous injection solutions and suspensions may be prepared from sterile powders, granules, and tablets.

It should be understood that in addition to the ingredients particularly mentioned above, the formulations may include other agents conventional in the art having regard to the type of formulation in question, for example those suitable for oral administration may include flavoring agents.

The term "patient" includes both human and other mammals.

The term "treating" refers to: (i) preventing a disease, disorder or condition from occurring in a patient that may be predisposed to the disease, disorder, and/or condition but has not yet been diagnosed as having it; (ii) inhibiting the disease, disorder, or condition, i.e., arresting its development; and (iii) relieving the disease, disorder, or condition, i.e., causing regression of the disease, disorder, and/or condition.

The compounds of the present disclosure can also be administered with a cyclosporin, for example, cyclosporin A. Cyclosporin A has been shown to be active against HCV in clinical trials (Hepatology 2003, 38, 1282; Biochem. Biophys. Res. Commun. 2004, 313, 42; J. Gastroenterol. 2003, 38, 567).

Table I below lists some illustrative examples of compounds that can be administered with the compounds of this disclosure. The compounds of the disclosure can be administered with other anti-HCV activity compounds in combination therapy, either jointly or separately, or by combining the compounds into a composition.

**Table 1**

| *Brand Name* | *Physiological Class* | *Type of Inhibitor or Target* | *Source Company* |
|---|---|---|---|
| NIM811 | | Cyclophilin Inhibitor | Novartis |
| Zadaxin | | Immunomodulator | Sciclone |
| Suvus | | Methylene blue | Bioenvision |
| Actilon (CPG 10101) | | TLR9 agonist | Coley |
| Batabulin (T67) | Anticancer | β-tubulin inhibitor | Tularik Inc., South San Francisco, CA |
| ISIS 14803 | Antiviral | antisense | ISIS Pharmaceutica ls Inc, Carlsbad, CA/Elan Pharmaceutical s Inc., New York, NY |
| Summetrel | Antiviral | antiviral | Endo Pharmaceutica Is Holdings Inc., Chadds Ford, PA |
| GS-9132 (ACH-806) | Antiviral | HCV Inhibitor | Achillion/Gilead |
| Pyrazolopyrimidine compounds and salts From WO-2005047288 26 May 2005 | Antiviral | HCV Inhibitors | Arrow Therapeutics Ltd. |
| Levovirin | Antiviral | IMPDH inhibitor | Ribapharm Inc., Costa Mesa, CA |
| Merimepodib (VX-497) | Antiviral | IMPDH inhibitor | Vertex Pharmaceutica Is Inc., Cambridge, MA |
| XTL-6865 (XTL-002) | Antiviral | monoclonal antibody | XTL Biopharmaceu ticals Ltd, Rehovot, Isreal |
| Telaprevir (VX-950, LY-570310) | Antiviral | NS3 serine protease inhibitor | Vertex Pharmaceutica Is Inc., Cambridge, MA/ Eli Lilly and Co. Inc., Indianapolis, ION |
| HCV-796 | Antiviral | NS5B Replicase Inhibitor | Wyeth/Viropharma |
| NM-283 | Antiviral | NS5B Replicase Inhibitor | Idenix/Novartis |
| GL-59728 | Antiviral | NS5B Replicase Inhibitor | Gene Labs/Novartis |
| GL-60667 | Antiviral | NS5B Replicase Inhibitor | Gene Labs/Novartis |
| 2'C MeA | Antiviral | NS5B Replicase Inhibitor | Gilead |
| PSI 6130 | Antiviral | NS5B Replicase Inhibitor | Roche |
| R1626 | Antiviral | NS5B Replicase Inhibitor | Roche |
| 2'C Methyl adenosine | Antiviral | NS5B Replicase Inhibitor | Merck |
| JTK-003 | Antiviral | RdRp inhibitor | Japan Tobacco Inc., Tokyo, Japan |
| Levovirin | Antiviral | ribavirin | ICN Pharmaceutica Is, Costa Mesa, CA |
| Ribavirin | Antiviral | ribavirin | Schering-Plough Corporation, Kenilworth, NJ |
| Viramidine | Antiviral | Ribavirin Prodrug | Ribapharm Inc., Costa Mesa, CA |
| Heptazyme | Antiviral | ribozyme | Ribozyme Pharmaceutica Is Inc., Boulder, CO |
| BILN-2061 | Antiviral | serine protease inhibitor | Boehringer Ingelheim Pharma KG, Ingelheim, Germany |
| SCH 503034 | Antiviral | serine protease inhibitor | Schering Plough |
| Zadazim | Immune modulator | Immune modulator | SciClone Pharmaceutica Is Inc., San Mateo, CA |
| Ceplene | Immunomodulator | immune modulator | Maxim Pharmaceutica Is Inc., San Diego, CA |
| CellCept | Immunosuppressant | HCV IgG immunosuppressant | F. Hoffmann-La Roche LTD, Basel, Switzerland |
| Civacir | Immunosuppressant | HCV IgG immunosuppressant | Nabi Biopharmaceu ticals Inc., Boca Raton, FL |
| Albuferon - a | Interferon | albumin IFN-α2b | Human Genome Sciences Inc., Rockville, MD |
| Infergen A | Interferon | IFN alfacon-1 | InterMune Pharmaceutica Is Inc., Brisbane, CA |
| Omega IFN | Interferon | IFN-ω | Intarcia Therapeutics |
| IFN-β and EMZ701 | Interferon | IFN-β and EMZ701 | Transition Therapeutics Inc., Ontario, Canada |
| Rebif | Interferon | IFN-β1a | Serono, Geneva, Switzerland |
| Roferon A | Interferon | IFN-α2a | F. Hoffmann-La Roche LTD, Basel, Switzerland |
| Intron A | Interferon | IFN-α2b | Schering-Plough Corporation, Kenilworth, NJ |
| Intron A and Zadaxin | Interferon | IFN-α2b/α1-thymosin | RegeneRx Biopharmiceu ticals Inc., Bethesda, MD/ SciClone Pharmaceutica Is Inc, San Mateo, CA |
| Rebetron | Interferon | IFN-α2b/ribavirin | Schering-Plough Corporation, Kenilworth, NJ |
| Actimmune | Interferon | INF-γ | InterMune Inc., Brisbane, CA |
| Interferon-β | Interferon | Interferon-β-1a | Serono |
| Multiferon | Interferon | Long lasting IFN | Viragen/Valen tis |
| Wellferon | Interferon | lymphoblastoid IFN-αn1 | GlaxoSmithK1 ine plc, Uxbridge, UK |
| Omniferon | Interferon | natural IFN-α | Viragen Inc., Plantation, FL |
| Pegasys | Interferon | PEGylated IFN-α2a | F. Hoffmann-La Roche LTD, Basel, Switzerland |
| Pegasys and Ceplene | Interferon | PEGylated IFN-α2a/immune modulator | Maxim Pharmaceutica Is Inc., San Diego, CA |
| Pegasys and Ribavirin | Interferon | PEGylated IFN-α2a/ribavirin | F. Hoffinann-La Roche LTD, Basel, Switzerland |

| *Brand Name* | *Physiological Class* | *Type of Inhibitor or Turret* | *Source Company* |
|---|---|---|---|
| PEG-Intron | Interferon | PEGylated IFN-α2b | Schering-Plough Corporation, Kenilworth, NJ |
| PEG-Intron / Ribavirin | Interferon | PEGylated IFN-a2b/ribavirin | Schering-Plough Corporation, Kenilworth, NJ |
| IP-501 | Liver protection | antifibrotic | Indevus Pharmaceutica Is Inc., Lexington, MA |
| IDN-6556 | Liver protection | caspase inhibitor | Idun Pharmaceutica Is Inc., San Diego, CA |
| ITMN-191 (R-7227) | Antiviral | serine protease inhibitor | InterMune Pharmaceutica Is Inc., Brisbane, CA |
| GL-59728 | Antiviral | NS5B Replicase Inhibitor | Genelabs |
| ANA-971 | Antiviral | TLR-7 agonist | Anadys |

The compounds of the present disclosure may also be used as laboratory reagents. Compounds may be instrumental in providing research tools for designing of viral replication assays, validation of animal assay systems and structural biology studies to further enhance knowledge of the HCV disease mechanisms. Further, the compounds of the present disclosure are useful in establishing or determining the binding site of other antiviral compounds, for example, by competitive inhibition.

The compounds of this disclosure may also be used to treat or prevent viral contamination of materials and therefore reduce the risk of viral infection of laboratory or medical personnel or patients who come in contact with such materials, e.g., blood, tissue, surgical instruments and garments, laboratory instruments and garments, and blood collection or transfusion apparatuses and materials.

This disclosure is intended to encompass compounds of the invention when prepared by synthetic processes or by metabolic processes including those occurring in the human or animal body (*in vivo*) or processes occurring *in vitro.*

The abbreviations used in the present application, including particularly in the illustrative schemes and examples which follow, are well-known to those skilled in the art. Some of the abbreviations used are as follows: HATU for O-(7-azabenzotriazol-1-yl)-N,N,N',N'-tetramethyluronium hexafluorophosphate; Boc or BOC for tert-butoxycarbonyl; NBS for N-bromosuccinimide; tBu or t-Bu for tert-butyl; SEM for -(trimethylsilyl)ethoxymethyl; DMSO for dimethylsulfoxide; MeOH for methanol; TFA for trifluoroacetic acid; RT for room temperature or retention time (context will dictate); t_{R} for retention time; EDCI for 1-(3-dimethylaminopropyl)-3-ethylcarbodiimide hydrochloride; DMAP for 4-dimethylaminopyridine; THF for tetrahydrofuran; DBU for 1,8-diazabicyclo[5.4.0]undec-7-ene; *t*-Bu; DEA for diethylamine; HMDS for hexamethyldisilazide; DMF for N,N-dimethylformamide; Bzl for benzyl; EtOH for ethanol; iPrOH or i-PrOH for isopropanol; Me₂S for dimethylsulfide; Et₃N or TEA for triethylamine; Ph for phenyl; OAc for acetate; EtOAc for ethyl acetate; dppf for 1,1'-bis(diphenylphosphino)ferrocene; iPr₂EtN or DIPEA for diisopropylethylamine; Cbz for carbobenzyloxy; n-BuLi for n-butyllithium; ACN for acetonitrile; h or hr for hours; m or min for minutes; s for seconds; LiHMDS for lithium hexamethyldisilazide; DIBAL for diisobutyl aluminum hydride; TBDMSCI for tert-butyldimethylsilyl chloride; Me for methyl; ca. for about; OAc for acetate; iPr for isopropyl; Et for ethyl; Bn for benzyl; and HOAT for 1-hydroxy-7-azabenzotriazole.

The abbreviations used in the present application, including particularly in the illustrative schemes and examples which follow, are well-known to those skilled in the art.

### Compound analysis conditions

Purity assessment and low resolution mass analysis were conducted on a Shimadzu LC system coupled with Waters Micromass ZQ MS system. It should be noted that retention times may vary slightly between machines. The LC conditions employed in determining the retention time (RT) were:

| *Condition 1* | |
|---|---|
| Column | = Phenomenex-Luna 3.0X 50 mm S10 |
| Start %B | = 0 |
| Final %B | = 100 |
| Gradient time | = 2 min |
| Stop time | = 3 min |
| Flow Rate | = 4 mL/min |
| Wavelength | = 220 nm |
| Slovent A | = 0.1% TFA in 10% methanol/90%H₂O |
| Solvent B | = 0.1% TFA in 90% methanol/10% H₂O |
| | |

| *Condition 2* | |
|---|---|
| Column | = Phenomenex-Luna 4.6X50 mm S 10 |
| Start %B | = 0 |
| Final %B | = 100 |
| Gradient time | = 2 min |
| Stop time | = 3 min |
| Flow Rate | = 5 mL/min |
| Wavelength | = 220 nm |
| Slovent A | = 0.1% TFA in 10% methanol/90%H₂O |
| Solvent B | = 0.1% TFA in 90% methanol/10% H₂O |
| | |

| *Condition 3* | |
|---|---|
| Column | = HPLC XTERRA C18 3.0 x 50mm S7 |
| Start %B | = 0 |
| Final %B | = 100 |
| Gradient time | = 3 min |
| Stop time | = 4 min |
| Flow Rate | = 4 mL/min |
| Wavelength | = 220 nm |
| Slovent A | = 0.1% TFA in 10% methanol/90%H₂O |
| Solvent B | = 0.1% TFA in 90% methanol/10% H₂O |
| | |

| *Condition M1* | |
|---|---|
| Column: Luna 4.6X 50 mm S 10 | |
| Start %B | = 0 |
| Final %B | = 100 |
| Gradient time | = 3 min |
| Stop time | = 4 min |
| Flow rate | = 4 mL/min |
| Solvent A: | = 95% H₂O: 5% CH₃CN, 10 mm Ammonium acetate |
| Solvent B: | = 5% H₂O : 95% CH₃CN; 10 mm Ammonium acetate |

### Synthesis of common caps

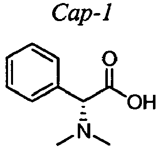

A suspension of 10% Pd/C (2.0g) in methanol (10 mL) was added to a mixture of (R)-2-phenylglycine (10g, 66.2 mmol), formaldehyde (33 mL of 37% wt. in water), 1N HCl (30 mL) and methanol (30 mL), and exposed to H₂ (60 psi) for 3 hours. The reaction mixture was filtered through diatomaceous earth (Celite®), and the filtrate was concentrated *in vacuo.* The resulting crude material was recrystallized from isopropanol to provide the HCl salt of *Cap*-1 as a white needle (4.0 g). Optical rotation: -117.1° [c = 9.95 mg/mL in H₂O; λ = 589 nm]. ¹H NMR (DMSO-d₆, δ = 2.5 ppm, 500 MHz): δ 7.43-7.34 (m, 5H), 4.14 (s, 1H), 2.43 (s, 6H); LC (Cond. 1): RT = 0.25; LC/MS: Anal. Calcd. for [M+H]⁺ C₁₀H₁₄NO₂ 180.10; found 180.17; HRMS: Anal. Calcd. for [M+H]⁺ C₁₀H₁₄NO₂ 180.1025; found 180.1017.

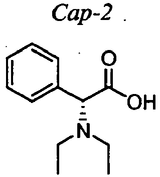

NaBH₃CN (6.22g, 94 mmol) was added in portions over a few minutes to a cooled (ice/water) mixture of (R)-2-Phenylglycine (6.02 g, 39.8 mmol) and MeOH (100 mL), and stirred for 5 min. Acetaldehyde (10 mL) was added drop-wise over 10 min and stirring was continued at the same cooled temperature for 45 min and at ambient temperature for -6.5 hr. The reaction mixture was cooled back with ice-water bath, treated with water (3 mL) and then quenched with a drop-wise addition of concentrated HCl over ∼ 45 min until the pH of the mixture is ∼ 1.5 - 2.0. The cooling bath was removed and the stirring was continued while adding concentrated HCl in order to maintain the pH of the mixture around 1.5-2.0. The reaction mixture was stirred over night, filtered to remove the white suspension, and the filtrate was concentrated *in vacuo.* The crude material was recrystallized from ethanol to afford the HCl salt of *Cap*-2 as a shining white solid in two crops (crop-1: 4.16 g; crop-2: 2.19 g). ¹H NMR (DMSO-d₆, δ = 2.5 ppm, 400 MHz): 10.44 (1.00, br s, 1H), 7.66 (m, 2H), 7.51 (m, 3H), 5.30 (s, 1H), 3.15 (br m, 2H), 2.98 (br m, 2H), 1.20 (app br s, 6H). Crop-1: [α]²⁵ -102.21° (c = 0.357, H₂O); crop-2: [α]²⁵ -99.7° (c = 0.357, H₂O). LC (Cond. 1): RT = 0.43 min; LC/MS: Anal. Calcd. for [M+H]⁺ C₁₂H₁₈NO₂: 208.13; found 208.26

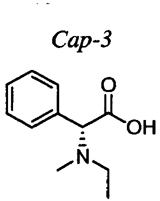

Acetaldehyde (5.0 mL, 89.1 mmol) and a suspension of 10% Pd/C (720 mg) in methanol/H₂O (4mL/1 mL) was sequentially added to a cooled (∼ 15 °C) mixture of (R)-2-phenylglycine (3.096g, 20.48 mmol), 1N HCl (30 mL) and methanol (40 mL). The cooling bath was removed and the reaction mixture was stirred under a balloon of H₂ for 17 hours. An additional acetaldehyde (10 mL, 178.2 mmol) was added and stirring continued under H₂ atmosphere for 24 hours [Note: the supply of H₂ was replenished as needed throughout the reaction]. The reaction mixture was filtered through diatomaceous earth (Celite^{®}), and the filtrate was concentrated *in vacuo.* The resulting crude material was recrystallized from isopropanol to provide the HCl salt of (R)-2-(ethylamino)-2-phenylacetic acid as a shining white solid (2.846g). ¹H NMR (DMSO-d₆, δ = 2.5 ppm, 400 MHz): 8 14.15 (br s, 1H), 9.55 (br s, 2H), 7.55-7.48 (m, 5H), 2.88 (br m, 1H), 2.73 (br m, 1H), 1.20 (app t, J = 7.2, 3H). LC (Cond. 1): RT = 0.39 min; >95 % homogeneity index; LC/MS: Anal. Calcd. for [M+H]⁺ C₁₀H₁₄NO₂: 180.10; found 180.18.

A suspension of 10% Pd/C (536 mg) in methanol/H₂O (3 mL/1 mL) was added to a mixture of (R)-2-(ethylamino)-2-phenylacetic acid/HCl (1.492g, 6.918 mmol), formaldehyde (20 mL of 37% wt. in water), 1N HCl (20 mL) and methanol (23 mL). The reaction mixture was stirred under a balloon of H₂ for ∼72 hours, where the H₂ supply was replenished as needed. The reaction mixture was filtered through diatomaceous earth (Celite^{®}) and the filtrate was concentrated *in vacuo.* The resulting crude material was recrystallized from isopropanol (50 mL) to provide the HCl salt of *Cap*-3 as a white solid (985 mg). ¹H NMR (DMSO-d₆, δ = 2.5 ppm, 400 MHz): δ 10.48 (br s, 1H), 7.59-7.51 (m, 5H), 5.26 (s, 1H), 3.08 (app br s, 2H), 2.65 (br s, 3H), 1.24 (br m, 3H). LC (Cond. 1): RT = 0.39 min; >95 % homogeneity index; LC/MS: Anal. Calcd. for [M+H]⁺ C₁₁H₁₆NO₂: 194.12; found 194.18; HRMS: Anal. Calcd. for [M+H]⁺ C₁₁H₁₆NO₂:194.1180; found 194.1181.

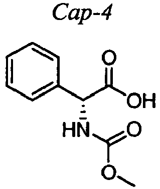

ClCO₂Me (3.2 mL, 41.4 mmol) was added dropwise to a cooled (ice/water) THF (410 mL) semi-solution of (R)-tert-butyl 2-amino-2-phenylacetate/HCl (9.877 g, 40.52 mmol) and diisopropylethylamine (14.2 mL, 81.52 mmol) over 6 min, and stirred at similar temperature for 5.5 hours. The volatile component was removed *in vacuo,* and the residue was partitioned between water (100 mL) and ethyl acetate (200 mL). The organic layer was washed with 1N HCl (25 mL) and saturated NaHCO₃ solution (30 mL), dried (MgSO₄), filtered, and concentrated *in vacuo.* The resultant colorless oil was triturated from hexanes, filtered and washed with hexanes (100 mL) to provide (R)-tert-butyl 2-(methoxycarbonylamino)-2-phenylacetate as a white solid (7.7 g). ¹H NMR (DMSO-d₆, δ = 2.5 ppm, 400 MHz): 7.98 (d, *J* = 8.0, 1H), 7.37-7.29 (m, 5H), 5.09 (d, *J* = 8, 1H), 3.56 (s, 3H), 1.33 (s, 9H). LC (Cond. 1): RT = 1.53 min; ∼90 % homogeneity index; LC/MS: Anal. Calcd. for [M+Na]⁺ C₁₄H₁₉NNaO₄: 288.12; found 288.15.

TFA (16 mL) was added dropwise to a cooled (ice/water) CH₂Cl₂ (160 mL) solution of the above product over 7 minutes, and the cooling bath was removed and the reaction mixture was stirred for 20 hours. Since the deprotection was still not complete, an additional TFA (1.0 mL) was added and stirring continued for an additional 2 hours. The volatile component was removed *in vacuo,* and the resulting oil residue was treated with diethyl ether (15 mL) and hexanes (12 mL) to provide a precipitate. The precipitate was filtered and washed with diethyl ether/hexanes (∼1:3 ratio; 30 mL) and dried *in vacuo* to provide *Cap*-4 as a fluffy white solid (5.57 g). Optical rotation: -176.9° [c = 3.7 mg/mL in H₂O; λ = 589 nm]. ¹H NMR (DMSO-d₆, δ = 2.5 ppm, 400 MHz): δ 12.84 (br s, 1H), 7.96 (d, *J* = 8.3, 1H)_{;} 7.41-7.29 (m, 5H), 5.14 (d, *J* = 8.3, 1H), 3.55 (s, 3H). LC (Cond. 1): RT = 1.01 min; >95 % homogeneity index; LC/MS: Anal. Calcd. for [M+H]+C₁₀.H₁₂NO₄ 210.08; found 210.17; HRMS: Anal. Calcd. for [M+H]⁺C₁₀H₁₂NO₄ 210.0766; found 210.0756.

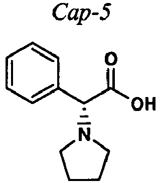

A mixture of (R)- 2-phenylglycine (1.0 g, 6.62 mmol), 1,4-dibromobutane (1.57 g, 7.27 mmol) and Na₂CO₃ (2.10 g, 19.8 mmol) in ethanol (40 mL) was heated at 100 °C for 21 hours. The reaction mixture was cooled to ambient temperature and filtered, and the filtrate was concentrated *in vacuo.* The residue was dissolved in ethanol and acidified with 1N HCl to pH 3-4, and the volatile component was removed *in vacuo.* The resulting crude material was purified by a reverse phase HPLC (water/methanol/TFA) to provide the TFA salt of *Cap*-5 as a semi-viscous white foam (1.0 g). ¹H NMR (DMSO-d₆, δ = 2.5, 500 MHz) δ 10.68 (br s, 1H), 7.51 (m, 5H), 5.23 (s, 1H), 3.34 (app br s, 2H), 3.05 (app br s, 2H), 1.95 (app br s, 4H); RT = 0.30 min (Cond. 1); >98% homogeneity index; LC/MS: Anal. Calcd. for [M+H]⁺ C₁₂H₁₆NO₂: 206.12; found 206.25.

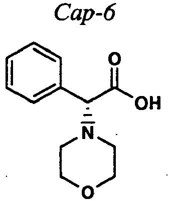

The TFA salt of *Cap*-6 was synthesized from (R)-2-phenylglycine and 1-bromo-2-(2-bromoethoxy)ethane by using the method of preparation of *Cap*-5. ¹H NMR (DMSO-d₆, δ = 2.5, 500 MHz) δ 12.20 (br s, 1H), 7.50 (m, 5H), 4.92 (s, 1H), 3.78 (app br s, 4H), 3.08 (app br s, 2H), 2.81 (app br s, 2H); RT = 0.32 min (Cond. 1); >98%; LC/MS: Anal. Calcd. for [M+H]⁺ C₁₂H₁₆NO₃: 222.11; found 222.20; HRMS: Anal. Calcd. for [M+H]⁺ C₁₂H₁₆NO₃: 222.1130; found 222.1121.

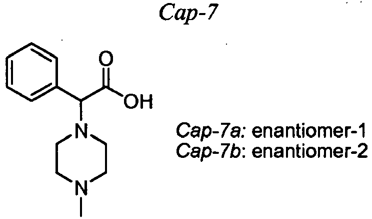

A CH₂Cl₂ (200 mL) solution of p-toluenesulfonyl chloride (8.65 g, 45.4 mmol) was added dropwise to a cooled (-5 °C) CH₂Cl₂ (200 mL) solution of (S)-benzyl 2-hydroxy-2-phenylacetate (10.0 g, 41.3 mmol), triethylamine (5.75 mL, 41.3 mmol) and 4-dimethylaminopyridine (0.504 g, 4.13 mmol), while maintaining the temperature between -5 °C and 0 °C. The reaction was stirred at 0 °C for 9 hours, and then stored in a freezer (-25 °C) for 14 hours. It was allowed to thaw to ambient temperature and washed with water (200 mL), 1N HCl (100 mL) and brine (100 mL), dried (MgSO₄), filtered, and concentrated *in vacuo* to provide benzyl 2-phenyl-2-(tosyloxy)acetate as a viscous oil which solidified upon standing (16.5 g). The chiral integrity of the product was not checked and that product was used for the next step without further purification. ¹H NMR (DMSO-d₆, δ = 2.5, 500 MHz) δ 7.78 (d, *J* = 8.6, 2H), 7.43-7.29 (m, 10H), 7.20 (m, 2H), 6.12 (s, 1H), 5.16 (d, *J* = 12.5, 1H), 5.10 (d, *J* = 12.5, 1H), 2.39 (s, 3H). RT = 3.00 (Cond. 3); >90% homogeneity index; LC/MS: Anal. Calcd. for [M+H]⁺ C₂₂H₂₀NaO₅S: 419.09; found 419.04.

A THF (75 mL) solution of benzyl 2-phenyl-2-(tosyloxy)acetate (6.0 g, 15.1 mmol), 1-methylpiperazine (3.36 mL, 30.3 mmol) and N,N-diisopropylethylamine (13.2 mL, 75.8 mmol) was heated at 65 °C for 7 hours. The reaction was allowed to cool to ambient temperature and the volatile component was removed *in vacuo.* The residue was partitioned between ethylacetate and water, and the organic layer was washed with water and brine, dried (MgSO₄), filtered, and concentrated *in vacuo.* The resulting crude material was purified by flash chromatography (silica gel, ethyl acetate) to provide benzyl 2-(4-methylpiperazin-1-yl)-2-phenylacetate as an orangish-brown viscous oil (4.56 g). Chiral HPLC analysis (Chiralcel OD-H) indicated that the sample is a mixture of enantiomers in a 38.2 to 58.7 ratio. The separation of the enantiomers were effected as follow: the product was dissolved in 120 mL of ethanol/heptane (1:1) and injected (5 mL/injection) on chiral HPLC column (Chiracel OJ, 5 cm ID x 50 cm L, 20 µm) eluting with 85:15 Heptane/ethanol at 75 mL/min, and monitored at 220 nm. Enantiomer-1 (1.474 g) and enantiomer-2 (2.2149 g) were retrieved as viscous oil. ¹H NMR (CDCl₃, δ = 7.26, 500 MHz) 7.44-7.40 (m, 2H), 7.33-7.24 (m, 6H), 7.21-7.16 (m, 2H), 5.13 (d, *J*= 12.5, 1H), 5.08 (d, *J*= 12.5, 1H), 4.02 (s, 1H), 2.65-2.38 (app br s, 8H), 2.25 (s, 3H). RT = 2.10 (Cond. 3); >98% homogeneity index; LC/MS: Anal. Calcd. for [M+H]⁺ C₂₀H₂₅N₂O₂: 325.19; found 325.20.

A methanol (10 mL) solution of either enantiomer of benzyl 2-(4-methylpiperazin-1-yl)-2-phenylacetate (1.0 g, 3.1 mmol) was added to a suspension of 10% Pd/C (120 mg) in methanol (5.0 mL). The reaction mixture was exposed to a balloon of hydrogen, under a careful monitoring, for <50 min. Immediately after the completion of the reaction, the catalyst was filtered through diatomaceous earth (Celite^{®}) and the filtrate was concentrated *in vacuo* to provide *Cap*-7*,* contaminated with phenylacetic acid as a tan foam (867.6 mg; mass is above the theoretical yield). The product was used for the next step without further purification. ¹H NMR DMSO-d₆, δ = 2.5, 500 MHz) 7:44-7.37 (m, 2H), 7.37-7.24(m, 3H), 3.92 (s, 1H), 2.63-2.48 (app. bs, 2H), 2.48-2.32 (m, 6H), 2.19 (s, 3H); RT = 0.31 (Cond. 2); >90% homogeneity index; LC/MS: Anal. Calcd. for [M+H]⁺ C₁₃H₁₉N₂O₂: 235.14; found 235.15; HRMS: Anal. Calcd. for [M+H]⁺ C₁₃H₁₉N₂O₂: 235.1447; found 235.1440.

The synthesis of *Cap*-8 and *Cap*-9 was conducted according to the synthesis: of *Cap*-7 by using appropriate amines for the SN₂ displacement step (i.e., 4-hydroxypiperidine for *Cap*-8 and (S)-3-fluoropyrrolidine for *Cap*-9) and modified conditions for the separation of the respective stereoisomeric intermedites, as described below.

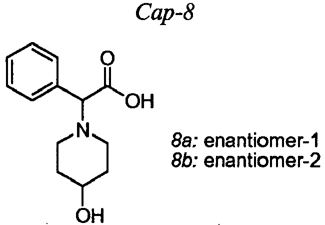

The enantiomeric separation of the intermediate benzyl 2-(4-hydroxypiperidin-1-yl)-2-phenyl acetate was effected by employing the following conditions: the compound (500 mg) was dissolved in ethanol/heptane (5 mL/45 mL). The resulting solution was injected (5 mL/injection) on a chiral HPLC column (Chiracel OJ, 2 cm ID x 25 cm L, 10 µm) eluting with 80:20 heptane/ethanol at 10 mL/min, monitored at 220 nm, to provide 186.3 mg of enantiomer-1 and 209.1 mg of enantiomer-2 as light-yellow viscous oils. These benzyl ester was hydrogenolysed according to the preparation of *Cap*-7 to provide *Cap*-8: ¹H NMR (DMSO-d₆, δ = 2.5, 500 MHz) 7.40 (d, *J* = 7, 2H), 7.28-7.20 (m, 3H), 3.78 (s 1H), 3.46 (m, 1H), 2.93 (m, 1H), 2.62 (m, 1H), 2.20 (m, 2H), 1.70 (m, 2H), 1.42 (m, 2H). RT = 0.28 (Cond. 2); >98% homogeneity index; LC/MS: Anal. Calcd. for [M+H]⁺ C₁₃H₁₈NO₃; 236.13; found 236.07; HRMS: Calcd. for [M+H]⁺ C₁₃H₁₈NO₃: 236.1287; found 236.1283.

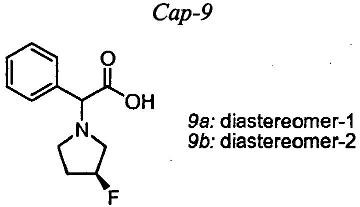

The diastereomeric separation of the intermediate benzyl 2-((S)-3-fluoropyrrolidin-1-yl)-2-phenylacetate was effected by employing the following conditions: the ester (220 mg) was separated on a chiral HPLC column (Chiracel OJ-H, 0.46 cm ID x 25 cm L, 5 µm) eluting with 95% CO₂/ 5% methanol with 0.1% TFA, at 10 bar pressure, 70 mL/min flow rate, and a temperature of 35 °C. The HPLC elute for the respective stereiosmers was concentrated, and the residue was dissolved in CH₂Cl₂ (20 mL) and washed with an aqueous medium (10 mL water + 1 mL saturated NaHCO₃ solution). The organic phase was dried (MgSO₄), filtered, and concentrated *in vacuo* to provide 92.5 mg of fraction-1 and 59.6 mg of fraction-2. These benzyl esters were hydrogenolysed according to the preparation of *Cap*-7 to prepare Caps 9a and 9b. *Cap*-9a (diastereomer-1; the sample is a TFA salt as a result of purification on a reverse phase HPLC using H₂O/methanol/TFA solvent): ¹H NMR (DMSO-d₆, δ = 2.5, 400 MHz) 7.55-7.48 (m, 5H), 5.38 (d of m, *J* = 53.7, 1H), 5.09 (br s, 1H), 3.84-2.82 (br m, 4H), 2.31-2.09 (m, 2H). RT = 0.42 (Cond. 1); >95% homogeneity index; LC/MS: Anal. Calcd. for [M+H]⁺ C₁₂H₁₅FNO₂: 224.11; found 224.14; *Cap*-9b (diastereomer-2): ¹H NMR (DMSO-d₆, δ = 2.5, 400 MHz) 7.43-7.21 (m, 5H), 5.19 (d of m, *J* = 55.9, 1H), 3.97 (s, 1H), 2.95-2.43 (m, 4H), 2.19-1.78 (m, 2H). RT = 0.44 (Cond. 1); LC/MS: Anal. Calcd. for [M+H]⁺ C₁₂H₁₅FNO₂: 224.11; found 224.14.

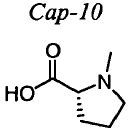

To a solution of D-proline (2.0 g, 17 mmol) and formaldehyde (2.0 mL of 37% wt. in H₂O) in methanol (15 mL) was added a suspension of 10% Pd/C (500 mg) in methanol (5 mL). The mixture was stirred under a balloon of hydrogen for 23 hours. The reaction mixture was filtered through diatomaceous earth (Celite^{®}) and concentrated *in vacuo* to provide *Cap*-10 as an off-white solid (2.15 g). ¹H NMR (DMSO-d₆, δ = 2.5, 500 MHz) 3.42 (m, 1H), 3.37 (dd, *J* = 9.4, 6.1, 1H), 2.85-2.78 (m, 1H), 2.66 (s, 3H), 2.21-2.13 (m, 1H), 1.93-1.84 (m, 2H), 1.75-1.66 (m, 1H). RT = 0.28 (Cond. 2); >98% homogeneity index; LC/MS: Anal. Calcd. for [M+H]⁺ C₆H₁₂NO₂:130.09; found 129.96.

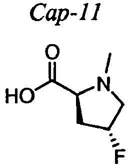

A mixture of (2S,4R)-4-fluotopyrrolidine-2-carboxylic acid (0.50 g, 3.8 mmol), formaldehyde (0.5 mL of 37% wt. in H₂O), 12 N HCl (0.25 mL) and 10% Pd/C (50 mg) in methanol (20 mL) was stirred under a balloon of hydrogen for 19 hours. The reaction mixture was filtered through diatomaceous earth (Celite^{®}) and the filtrate was concentrated *in vacuo.* The residue was recrystallized from isopropanol to provide the HCl salt of *Cap*-11 as a white solid (337.7 mg). ¹H NMR (DMSO-d₆, δ = 2.5, 500 MHz) 5.39 (d m, *J =* 53.7, 1H), 4.30 (m, 1H), 3.90 (ddd, *J =* 31.5, 13.5, 4.5, 1H), 3.33 (dd, *J* = 25.6, 13.4, 1H), 2.85 (s, 3H), 2.60-2.51 (m, 1H), 2.39-2.26 (m, 1H). RT = 0.28 (Cond. 2); >98% homogeneity index; LC/MS: Anal. Calcd. for [M+H]⁺ C₆H₁₁FNO₂: 148.08; found 148.06.

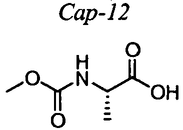

L-Alanine (2.0 g, 22.5 mmol) was dissolved in 10% aqueous sodium carbonate solution (50 mL), and a THF (50 mL) solution of methyl chloroformate (4.0 mL) was added to it. The reaction mixture was stirred under ambient conditions for 4.5 hours and concentrated *in vacuo.* The resulting white solid was dissolved in water and acidified with 1N HCl to a pH ~ 2-3. The resulting solutions was extracted with ethyl acetate (3 x 100 mL), and the combined organic phase was dried (Na₂SO₄), filtered, and concentrated *in vacuo* to provide a colorless oil (2.58 g). 500 mg of this material was purified by a reverse phase HPLC (H₂O/methanol/TFA) to provide 150 mg of *Cap*-12 as a colorless oil. ¹H NMR (DMSO-d₆, δ = 2.5, 500 MHz) 7.44 (d, *J* = 7.3, 0.8H), 7.10 (br s, 0.2H), 3.97 (m, 1H), 3.53 (s, 3H), 1.25 (d, *J* = 7.3, 3H).

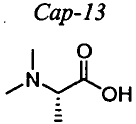

A mixture of L-alanine (2.5 g, 28 mmol), formaldehyde (8.4 g, 37 wt. %), 1N HCl (30 mL) and 10% Pd/C (500 mg) in methanol (30 mL) was stirred under a hydrogen atmosphere (50 psi) for 5 hours. The reaction mixture was filtered through diatomaceous earth (Celite^{®}) and the filtrate was concentrated *in vacuo* to provide the HCl salt of *Cap*-13 as an oil which solidified upon standing under vacuum (4.4 g; the mass is above theoretical yield). The product was used without further purification. ¹H NMR (DMSO-d₆, δ = 2.5, 500 MHz) δ 12.1 (br s, 1H), 4.06 (q, *J* = 7.4, 1H), 2.76 (s, 6H), 1.46 (d, *J* = 7.3, 3H).

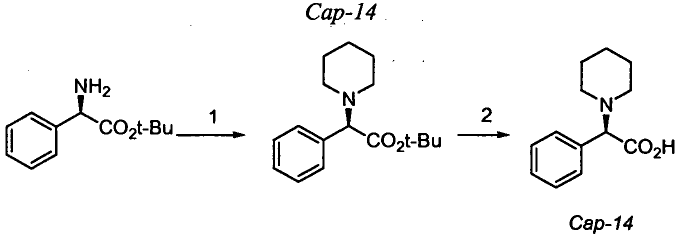

Step 1: A mixture of (R)-(-)-D-phenylglycine tert-butyl ester (3.00 g, 12.3 mmol), NaBH₃CN (0.773 g, 12.3 mmol), KOH (0.690 g, 12.3 mmol) and acetic acid (0.352 mL, 6.15 mmol) were stirred in methanol at 0 °C. To this mixture was added glutaric dialdehyde (2.23 mL, 12.3 mmol) dropwise over 5 minutes. The reaction mixture was stirred as it was allowed to warm to ambient temperature and stirring was continued at the same temperature for 16 hours. The solvent was subsequently removed and the residue was partitioned with 10% aqueous NaOH and ethyl acetate. The organic phase was separated, dried (MgSO₄), filtered and concentrated to dryness to provide a clear oil. This material was purified by reverse-phase preparative HPLC (Primesphere C-18, 30 x 100mm; CH₃CN-H₂O-0.1% TFA) to give the intermediate ester (2.70 g, 56%) as a clear oil. ¹HNMR (400 MHz, CDCl₃) δ 7.53-7.44 (m, 3H), 7.40-7.37 (m, 2H), 3.87 (d, *J* = 10.9 Hz, 1H), 3.59 (d, *J* = 10.9 Hz, 1H), 2.99 (t, *J* = 11.2 Hz, 1H), 2.59 (t, *J* = 11.4 Hz, 1H), 2.07-2.02 (m, 2H), 1.82 (d, *J* = 1.82 Hz, 3H), 1.40 (s, 9H). LC/MS: Anal. Calcd. for C₁₇H₂₅NO₂: 275; found: 276 (M+H)⁺.

Step 2: To a stirred solution of the intermediate ester (1.12g, 2.88mmol) in dichloromethane (10 mL) was added TFA (3 mL). The reaction mixture was stirred at ambient temperature for 4 hours and then it was concentrated to dryness to give a light yellow oil. The oil was purified using reverse-phase preparative HPLC (Primesphere C-18, 30 x 100mm; CH₃CN-H₂O-0.1% TFA). The appropriate fractions were combined and concentrated to dryness *in vacuo.* The residue was then dissolved in a minimum amount of methanol and applied to applied to MCX LP extraction cartridges (2 x 6 g). The cartridges were rinsed with methanol (40 mL) and then the desired compound was eluted using 2M ammonia in methanol (50 mL). Product-containing fractions were combined and concentrated and the residue was taken up in water. Lyophilization of this solution provided the title compound (0.492 g, 78%) as a light yellow solid. ¹HNMR (DMSO-d₆) δ 7.50 (s, 5H), 5.13 (s, 1H), 3.09 (br s, 2H), 2.92-2.89 (m, 2H), 1.74 (m, 4H), 1.48 (br s, 2H). LC/MS: Anal. Calcd. for C₁₃H₁₇NO₂: 219; found: 220 (M+H)⁺.

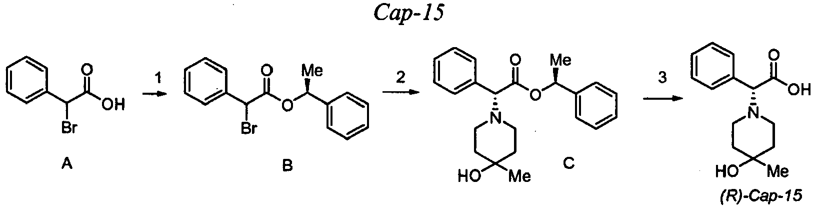

Step 1; (S)-1-Phenylethyl 2-bromo-2-phenylacetate: To a mixture of α-bromophenylacetic acid (10.75 g, 0.050 mol), (S)-(-)-1-phenylethanol (7.94 g, 0.065 mol) and DMAP (0.61 g, 5.0 mmol) in dry dichloromethane (100 mL) was added solid EDCI (12.46 g, 0.065 mol) all at once. The resulting solution was stirred at room temperature under Ar for 18 hours and then it was diluted with ethyl acetate, washed (H₂O x 2, brine), dried (Na₂SO₄), filtered, and concentrated to give a pale yellow oil. Flash chromatography (SiO₂/ hexane-ethyl acetate, 4:1) of this oil provided the title compound (11.64 g, 73%) as a white solid. ¹HNMR (400 MHz, CDCl₃) δ 7.53-7.17 (m, 10H), 5.95 (q, *J* = 6.6 Hz, 0.5H), 5.94 (q, *J* = 6.6 Hz, 0.5H), 5.41 (s, 0.5H), 5.39 (s, 0.5H), 1.58 (d, *J* = 6.6 Hz, 1.5H), 1.51 (d, *J* = 6.6 Hz, 1.5H).

Step 2; (S)-1-Phenylethyl (R)-2-(4-hydroxy-4-methylpiperidin-1-yl)- 2-phenylacetate: To a solution of (S)-1-phenylethyl 2-bromo-2-phenylacetate (0.464 g, 1.45 mmol) in THF (8 mL) was added triethylamine (0.61 mL, 4.35 mmol), followed by tetrabutylammonium iodide (0.215 g, 0.58 mmol). The reaction mixture was stirred at room temperature for 5 minutes and then a solution of 4-methyl-4-hydroxypiperidine (0.251 g, 2.18 mmol) in THF (2 mL) was added. The mixture was stirred for 1 hour at room temperature and then it was heated at 55-60°C (oil bath temperature) for 4 hours. The cooled reaction mixture was then diluted with ethyl acetate (30 mL), washed (H₂O x2, brine), dried (MgSO₄), filtered and concentrated. The residue was purified by silica gel chromatography (0-60% ethyl acetate-hexane) to provide first the (S,R)-isomer of the title compound (0.306 g, 60%) as a white solid and then the corresponding (S,S)-isomer (0.120 g, 23%), also as a white solid. (S,R)-isomer: ¹HNMR (CD₃OD) δ 7.51-7.45 (m, 2H), 7.41-7.25 (m, 8H), 5.85 (q, *J* = 6.6 Hz, 1H), 4.05 (s, 1H), 2.56-2.45 (m, 2H), 2.41-2.29 (m, 2H), 1.71-1.49 (m, 4H), 1.38 (d, *J* = 6.6 Hz, 3H), 1.18 (s, 3H). LCMS: Anal. Calcd. for C₂₂H₂₇NO₃: 353; found: 354 (M+H)⁺. (S,S)-isomer: ¹HNMR (CD₃OD) δ 7.41-7.30 (m, 5H), 7.20-7.14 (m, 3H), 7.06-7.00 (m, 2H), 5.85 (q, *J* = 6.6 Hz, 1H), 4.06 (s, 1H), 2.70-2.60 (m, 1H), 2.51 (dt, *J* = 6.6, 3.3 Hz, 1H), 2.44-2.31 (m, 2H), 1.75-1.65 (m, 1H), 1.65-1.54 (m, 3H), 1:50 (d, *J* = 6.8 Hz, 3H), 1.20 (s, 3H). LCMS: Anal. Calcd. for C₂₂H₂₇NO₃: 353; found: 354 (M+H)⁺.

Step 3; (R)-2-(4-Hydroxy-4-methylpiperidin-1-yl)-2-phenylacetic acid: To a solution of (S)-1-phenylethyl (R)-2-(4-hydroxy-4-methylpiperidin-1-yl)-2-phenylacetate (0.185 g, 0.52 mmol) in dichloromethane (3 mL) was added trifluoroacetic acid (1 mL) and the mixture was stirred at room temperature for 2 hours. The volatiles were subsequently removed *in vacuo* and the residue was purified by reverse-phase preparative HPLC (Primesphere C-18, 20 x 100mm; CH₃CN-H₂O-0.1% TFA) to give the title compound (as TFA salt) as a pale bluish solid (0,128 g, 98%). LCMS: Anal. Calcd. for C₁₄H₁₉NO₃: 249; found: 250 (M+H)⁺.

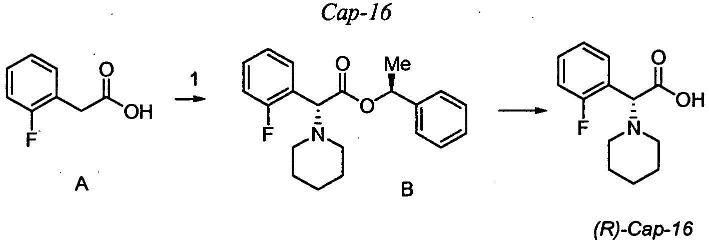

Step 1; (S)-1-Phenylethyl 2-(2-fluorophenyl)acetate: A mixture of 2-fluorophenylacetic acid (5.45 g, 35.4 mmol), (S)-1-phenylethanol (5.62 g, 46.0 mmol), EDCI (8.82 g, 46.0 mmol) and DMAP (0.561 g, 4.60 mmol) in CH₂Cl₂ (100 mL) was stirred at room temperature for 12 hours. The solvent was then concentrated and the residue partitioned with H₂O-ethy acetate. The phases were separated and the aqueous layer back-extracted with ethyl acetate (2x). The combined organic phases were washed (H₂O, brine), dried (Na₂SO₄), filtered, and concentrated *in vacuo.* The residue was purified by silica gel chromatography (Biotage/ 0-20% ethyl acetate-hexane) to provide the title compound as a colorless oil (8.38 g, 92%). ¹HNMR (400 MHz, CD₃OD) δ 7.32 - 7.23 (m, 7H), 7.10-7.04 (m, 2), 5.85 (q, *J* = 6.5 Hz, 1H), 3.71 (s, 2H), 1.48 (d, *J* = 6.5 Hz, 3H).

Step 2; (R)-((S)-1-Phenylethyl) 2-(2-fluorophenyl)-2-(piperidin-1-yl)acetate: To a solution of (S)-1-phenylethyl 2-(2-fluorophenyl)acetate (5.00 g, 19.4 mmol) in THF (1200 mL) at 0°C was added DBU (6.19 g, 40.7 mmol) and the solution was allowed to warm to room temperature while stirring for 30 minutes. The solution was then cooled to -78 °C and a solution of CBr₄ (13.5 g, 40.7 mmol) in THF (100 mL) was added and the mixture was allowed to warm to -10 °C and stirred at this temperature for 2 hours. The reaction mixture was quenched with saturated aq. NH₄Cl and the layers were separated. The aqueous layer was back-extracted with ethyl acetate (2x) and the combined organic phases were washed (H₂O, brine), dried (Na₂SO₄), filtered, and concentrated *in vacuo.* To the residue was added piperidine (5.73 mL, 58.1 mmol) and the solution was stirred at room temperature for 24 hours. The volatiles were then concentrated *in vacuo* and the residue was purified by silica gel chromatography (Biotage/ 0-30% diethyl ether-hexane) to provide a pure mixture of diastereomers (2:1 ratio by ¹HNMR) as a yellow oil (2.07 g, 31%), along with unreacted starting material (2.53 g, 51%). Further chromatography of the diastereomeric mixture (Biotage/ 0-10% diethyl ether-toluene) provided the title compound as a colorless oil (0.737 g, 11%). ¹HNMR (400 MHz, CD₃OD) δ 7.52 (ddd, *J* = 9.4, 7.6, 1.8 Hz, 1H), 7.33 - 7.40 (m, 1), 7.23 - 7.23 (m, 4H), 7.02 - 7.23 (m, 4H), 5.86 (q, *J* = 6.6 Hz, 1H), 4.45 (s, 1H), 2.39 - 2.45 (m, 4H), 1.52 - 1.58 (m, 4H), 1.40 - 1.42 (m, 1H), 1.38 (d, *J* = 6.6 Hz, 3H). LCMS: Anal. Calcd. for C₂₁H₂₄FNO₂: 341; found: 342 (M+H)⁺.

Step 3; (R)-2-(2-fluorophenyl)-2-(piperidin-1-yl)acetic acid: A mixture of (R)-((S)-1-phenylethyl) 2-(2-fluorophenyl)-2-(piperidin-1-yl)acetate (0.737 g, 2.16 mmol) and 20% Pd(OH)₂/C (0.070 g) in ethanol (30 mL) was hydrogenated at room temperature and atmospheric pressure (H₂ balloon) for 2 hours. The solution was then purged with Ar, filtered through diatomaceous earth (Celite^{®}), and concentrated *in vacuo.* This provided the title compound as a colorless solid (0.503 g, 98%). ¹HNMR (400 MHz, CD₃OD) δ 7.65 (ddd, *J* = 9.1, 7.6, 1.5 Hz, 1H), 7.47-7.53 (m, 1H), 7.21-7.30 (m, 2H), 3.07-3.13 (m, 4H), 1.84 (br s, 4H), 1.62 (br s, 2H). LCMS: Anal. Calcd. for C₁₃H₁₆FNO_{2:} 237; found: 238 (M+H)⁺.

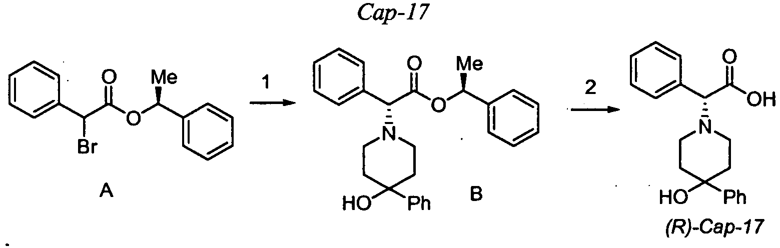

Step 1; (S)-1-Phenylethyl (R)-2-(4-hydroxy-4-phenylpiperidin-1-yl)- 2-phenylacetate: To a solution of (S)-1-phenylethyl 2-bromo-2-phenylacetate (1.50 g, 4.70 mmol) in THF (25 mL) was added triethylamine (1.31 mL, 9.42 mmol), followed by tetrabutylammonium iodide (0.347 g, 0.94 mmol). The reaction mixture was stirred at room temperature for 5 minutes and then a solution of 4-phenyl-4-hydroxypiperidine (1.00 g, 5.64 mmol) in THF (5 mL) was added. The mixture was stirred for 16 hours and then it was diluted with ethyl acetate (100 mL), washed (H₂O x2, brine), dried (MgSO₄), filtered and concentrated. The residue was purified on a silica gel column (0-60% ethyl acetate-hexane) to provide an approximately 2:1 mixture of diastereomers, as judged by ¹HNMR. Separation of these isomers was performed using supercritical fluid chromatography (Chiralcel OJ-H, 30 x 250mm; 20% ethanol in CO₂ at 35 °C), to give first the (R)-isomer of the title compound (0.534 g, 27%) as a yellow oil and then the corresponding (S)-isomer (0.271 g, 14%), also as a yellow oil. (S,R)-isomer: ¹HNMR (400 MHz, CD₃OD) δ 7.55-7.47 (m, 4H), 7.44-7.25 (m, 10H), 7.25-7.17 (m, 1H), 5.88 (q, *J* = 6.6 Hz, 1H), 4.12 (s, 1H), 2.82-2.72 (m, 1H), 2.64 (dt, *J* = 11.1, 2.5 Hz, 1H), 2.58-2.52 (m, 1H), 2.40 (dt, *J* = 11.1,2.5 Hz, 1H), 2.20 (dt, *J* = 12.1, 4.6 Hz, 1H), 2.10 (dt, *J* = 12.1, 4.6 Hz, 1H), 1.72-1.57 (m, 2H), 1.53 (d, *J* = 6.5 Hz, 3H). LCMS: Anal. Calcd. for C₂₇H₂₉NO₃: 415; found: 416 (M+H)⁺; (S,S)-isomer: ¹HNMR (400 MHz, CD₃OD) δ 7.55-7.48 (m, 2H), 7.45-7.39 (m, 2H), 7.38-7.30 (m, 5H), 7.25-7.13 (m, 4H), 7.08-7.00 (m, 2H), 5.88 (q, *J* = 6.6 Hz, 1H), 4.12 (s, 1H), 2.95-2.85 (m, 1H), 2.68 (dt, *J* = 11.1, 2.5 Hz, 1H), 2.57-2.52 (m, 1H), 2.42 (dt, *J* = 11.1, 2.5 Hz, 1H), 2.25 (dt, *J* = 12.1, 4.6 Hz, 1H), 2.12 (dt, *J* = 12.1, 4.6 Hz, 1H), 1.73 (dd, *J* = 13.6, 3.0 Hz, 1H), 1.64 (dd, *J* = 13.6, 3.0 Hz, 1H), 1.40 (d, *J* = 6.6 Hz, 3H). LCMS: Anal. Calcd. for C₂₇H₂₉NO₃: 415; found: 416 (M+H)⁺.

The following esters were prepared in similar fashion employing step 1 in the synthesis of *Cap*-17.

| | | |
|---|---|---|
| *Intermediate-17a* | 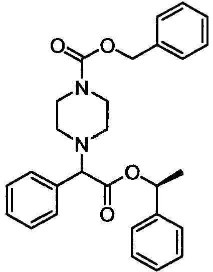 | Diastereomer 1: ¹H NMR (500 MHz, DMSO-d₆) δ ppm 1.36 (d, *J*=6.41 Hz, 3H) 2.23 - 2.51 (m, 4H) 3.35 (s, 4H) 4.25 (s, 1H) 5.05 (s, 2H) 5.82 (d, *J*=6.71 Hz, 1H) 7.15 - 7.52 (m, 15H). |
| | | LCMS: Anal. Calcd. for: C₂₈H₃₀N₂O₄ 458.55; Found: 459.44 (M+H)⁺, |
| | | Diastereomer 2: ¹H NMR (500 MHz, DMSO-d₆) δ ppm 1.45 (d, *J*=6.71 Hz, 3H) 2.27 - 2.44 (m, 4H) 3.39 (s, 4H) 4.23 (s, 1H) 5.06 (s, 2H) 5.83 (d, *J*=6.71 Hz, 1H) 7.12 (dd, *J*=6.41, 3.05 Hz, 2H) 7.19 - 7.27 (m, 3H) 7.27 - 7.44 (m, 10H). |
| | | LCMS: Anal. Calcd. for: C₂₈H₃₀N₂O₄ 458.55; Found: 459.44 (M+H)⁺. |
| *Intermediate -17b* | 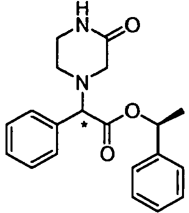 | Diasteromer 1: RT = 11.76 min (Cond'n II); LCMS: Anal. Calcd. for: C₂₀H₂₂N₂O₃ 338.4 Found: 339.39 (M+H)⁺; Diastereomer 2: RT = 10.05 min (Cond'n II); LCMS: Anal. Calcd. for: C₂₀H₂₂N₂O₃ 338.4; Found: 339.39 (M+H)⁺. |
| *Intermediate -17c* | 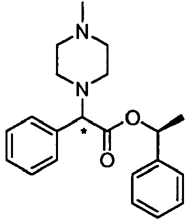 | Diastereomer 1: T_{R}= 4.55 min (Cond'n I); LCMS: Anal. Calcd. for: C₂₁H₂₆N₂O₂ 338.44 Found: 339.45 (M+H)⁺; Diastereomer 2: T_{R} = 6.00 min (Cond'n I); LCMS: Anal. Calcd. for: C₂₁H₂₆N₂O₂ 338.44 Found: 339.45 (M+H)⁺. |
| *Intermediate -17d* | 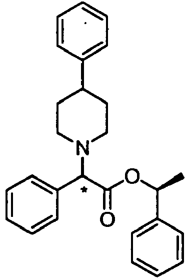 | Diastereomer 1: RT = 7.19 min (Cond'n I); LCMS: Anal. Calcd. for: C₂₇H₂₉NO₂ 399.52 Found: 400.48 (M+H)⁺-; Diastereomer 2: RT = 9.76 min (Cond'n I); LCMS: Anal. Calcd. for: C₂₇H₂₉NO₂ 399.52 Found: 400.48 (M+H)⁺. |

*Chiral SFC Conditions for determining retention time for intermediates 17b-17d*

### Condition 1

Column: Chiralpak AD-H Column, 4.6X250 mm, 5µm
Solvents: 90% CO2 - 10% methanol with 0.1%DEA
Temp: 35 °C
Pressure: 150 bar
Flow rate: 2.0 mL/min.
UV monitored @ 220 nm
Injection: 1.0 mg/3mL methanol

### Condition 2

Column: Chiralcel OD-H Column, 4.6X250 mm, 5µm
Solvents: 90% CO2 - 10% methanol with 0.1%DEA
Temp: 35 °C
Pressure: 150 bar
Flow rate: 2.0 mL/min.
UV monitored @ 220 nm
Injection: 1.0 mg/mL methanol

*Cap-17, Step 2*; (R)-2-(4-Hydroxy-4-phenylpiperidin-1-yl)-2-phenylacetic acid: To a solution of (S)-1-phenylethyl (R)-2-(4-hydroxy-4-phenylpiperidin-1-yl)-2-phenylacetate (0.350 g, 0.84 mmol) in dichloromethane (5 mL) was added trifluoroacetic acid (1 mL) and the mixture was stirred at room temperature for 2 hours. The volatiles were subsequently removed *in vacuo* and the residue was purified by reverse-phase preparative HPLC (Primesphere C-18, 20 x 100mm; CH₃CN-H₂O-0.1% TFA) to give the title compound (as TFA salt) as a white solid (0.230 g, 88%). LCMS: Anal. Calcd. for C₁₉H₂₁NO₃: 311; found: 312 (M+H)⁺.

The following carboxylic acids were prepared in a similar fashion:

| | | |
|---|---|---|
| *Cap-17a* | 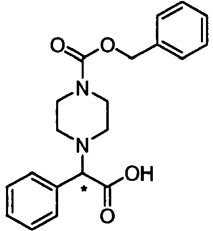 | RT= 2.21 (Cond'n 11); ¹H NMR (500 MHz, DMSO-d₆) δ ppm 2.20 - 2.35 (m, 2H) 2.34 - 2.47 (m, 2H) 3.37 (s, 4H) 3.71 (s, 1H) 5.06 (s, 2H) 7.06 - 7.53 (m, 10H). LCMS: Anal. Calcd. for: C₂₀H₂₂N₂O₄ 354.40; Found: 355.38 (M+H)⁺. |
| *Cap-17b* | 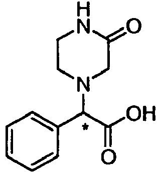 | RT= 0.27 (Cond'n III); LCMS: Anal. Calcd. for: C₁₂H₁₄N₂O₃ 234.25; Found: 235.22 (M+H)⁺. |
| *Cap-17c* | 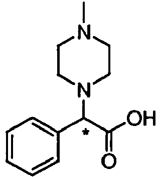 | RT= 0.48 (Cond'n II); LCMS: Anal. Calcd. for: C₁₃H₁₈N₂O₂ 234.29; Found: 235.31 (M+H)⁺. |
| *Cap-17d* | 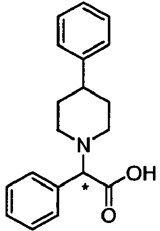 | RT= 2.21 (Cond'n I); LCMS: Anal. Calcd. for: C₁₉H₂₁NO₂ 295.38; Found: 296.33 (M+H)⁺. |

*LCMS Conditions for determining retention time for Caps 17a-17d*

### Condition 1

Column: Phenomenex-Luna 4.6 X 50 mm S10
Start%B=0
Fianl % B = 100
Gradient Time = 4 min
Flow Rate = 4 mL/min
Wavelength = 220
Solvent A = 10% methanol - 90% H₂O- 0.1% TFA
Solvent B = 90% methanol-10% H₂O - 0.1% TFA

### Condition 2

Column: Waters-Sunfire 4.6 X 50 mm S5
Start % B = 0
Fianl % B = 100
Gradient Time = 2 min
Flow Rate = 4 mL/min
Wavelength = 220
Solvent A = 10% methanol - 90% H₂O - 0.1% TFA
Solvent B = 90% methanol - 10% H₂O - 0.1 % TFA

### Condition 3

Column: Phenomenex 10µ 3.0 X 50 mm
Start%B=0
Fianl % B = 100
Gradient Time = 2 min
Flow Rate = 4 mL/min
Wavelength = 220
Solvent A = 10% methanol - 90% H₂O - 0.1% TFA
Solvent B = 90% methanol - 10% H₂O - 0.1 % TFA

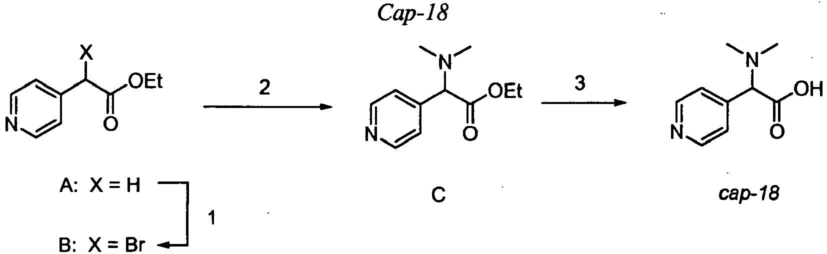

Step 1; (R,S)-Ethyl 2-(4-pyridyl)-2-bromoacetate: To a solution of ethyl 4-pyridylacetate (1.00 g, 6.05 mmol) in dry THF (150 mL) at 0 °C under argon was added DBU (0.99 mL, 6.66 mmol). The reaction mixture was allowed to warm to room temperature over 30 minutes and then it was cooled to -78 °C. To this mixture was added CBr₄ (2.21 g, 6.66 mmol) and stirring was continued at -78 °C for 2 hours. The reaction mixture was then quenched with sat. aq. NH₄Cl and the phases were separated. The organic phase was washed (brine), dried (Na₂SO₄), filtered, and concentrated *in vacuo.* The resulting yellow oil was immediately purified by flash chromatography (SiO₂/ hexane-ethyl acetate, 1:1) to provide the title compound (1.40 g, 95%) as a somewhat unstable yellow oil. ¹HNMR (400 MHz, CDCl₃) δ 8.62 (dd, *J* = 4.6, 1.8 Hz, 2H), 7.45 (dd, *J* = 4.6, 1.8 Hz, 2H), 5.24 (s, 1H), 4.21-4.29 (m, 2H), 1.28 (t, *J* = 7.1 Hz, 3H). LCMS: Anal. Calcd. for C₉H₁₀BrNO₂: 242, 244; found: 243, 245 (M+H)⁺.

Step 2; (R,S)-Ethyl 2-(4-pyridyl)-2-(N,N-dimethylamino)acetate: To a solution of (R,S)-ethyl 2-(4-pyridyl)-2-bromoacetate (1.40 g, 8.48 mmol) in DMF (10 mL) at room temperature was added dimethylamine (2M in THF, 8.5 mL, 17.0 mmol). After completion of the reaction (as judged by tlc) the volatiles were removed *in vacuo* and the residue was purified by flash chromatography (Biotage, 40+M SiO₂ column; 50%-100% ethyl acetate-hexane) to provide the title compound (0.539 g, 31%) as a light yellow oil. ¹HNMR (400 MHz, CDCl₃) δ 8.58 (d, *J* = 6.0 Hz, 2H), 7.36 (d, *J* = 6.0 Hz, 2H), 4.17 (m, 2H), 3.92 (s, 1H), 2.27 (s, 6H), 1.22 (t, *J* = 7.0 Hz). LCMS: Anal. Calcd. for C₁₁H₁₆N₂O₂: 208; found: 209 (M+H)⁺.

Step 3; (R,S)-2-(4-Pyridyl)-2-(N,N-dimethylamino)acetic acid: To a solution of (R,S)-ethyl 2-(4-pyridyl)-2-(N,N-dimethylamino)acetate (0.200 g, 0.960 mmol) in a mixture of THF-methanol-H₂O (1:1:1, 6 mL) was added powdered LiOH (0.120 g, 4.99 mmol) at room temperature. The solution was stirred for 3 hours and then it was acidified to pH 6 using 1N HCl. The aqueous phase was washed with ethyl acetate and then it was lyophilized to give the dihydrochloride of the title compound as a yellow solid (containing LiCl). The product was used as such in subsequent steps. ¹HNMR (400 MHz, DMSO-d₆) δ 8.49 (d, *J* = 5.7 Hz, 2H), 7.34 (d, *J* = 5.7 Hz, 2H), 3.56 (s, 1H), 2.21 (s, 6H).

The following examples were prepared in similar fashion using the method described in Example 4;

| | | |
|---|---|---|
| *Cap-19* | 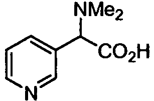 | LCMS: Anal. Calcd. for C₉H₁₂N₂O₂: 180; found: 181 (M+H)⁺. |
| *Cap-20* | 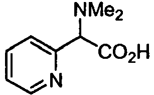 | LCMS: no ionization. ¹HNMR (400 MHz, CD₃OD) δ 8.55 (d, *J* = 4.3 Hz, 1H), 7.84 (app t, *J* = 5.3 Hz, 1H), 7.61 (d, *J* = 7.8 Hz, 1H), 7.37 (app t, *J* = 5.3 Hz, 1H), 4.35 (s, 1H), 2.60 (s, 6H). |
| *Cap-21* | 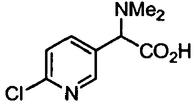 | LCMS: Anal. Calcd. for C₉H_{11CI}N₂O₂: 214, 216; found: 215, 217 (M+H)⁺. |
| *Cap-22* | 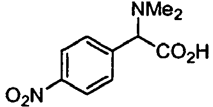 | LCMS: Anal. Calcd. for C₁₀H₁₂N₂O₄: 224; found: 225 (M+H)⁺. |
| *Cap-23* | 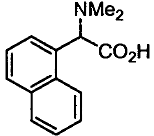 | LCMS: Anal. Calcd. for C₁₄H₁₅NO₂: 247; found: 248 (M+H)⁺. |
| *Cap-24* | 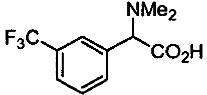 | LCMS: Anal. Calcd. for C₁₁H₁₂F₃NO₂: 247; found: 248 (M+H)⁺. |
| *Cap-25* | 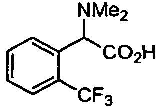 | LCMS:Anal. Calcd. for C₁₁H₁₂F₃NO₂: 247; found: 248 (M+H)⁺. |
| *Cap-26* | 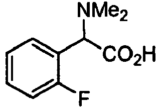 | LCMS: Anal. Calcd. for C₁₀H₁₂FNO₂: 247; found: 248 (M+H)⁺. |
| *Cap-27* | 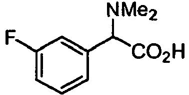 | LCMS: Anal. Calcd. for C₁₀H₁₂FNO₂: 247; found: 248 (M+H)⁺. |
| *Cap-28* | 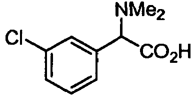 | LCMS: Anal. Calcd. for C₁₀H₁₂ClNO₂: 213, 215; found: 214, 217 (M+H)⁺. |
| *Cap-29* | 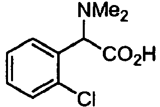 | LCMS: Anal. Calcd. for C₁₀H₁₂ClNO₂: 213, 215; found: 214, 217 (M+H)⁺. |
| *Cap-30* | 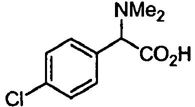 | LCMS: Anal. Calcd. for C₁₀H₁₂ClNO₂: 213, 215; found: 214, 217 (M+H)⁺. |
| *Cap-31* | 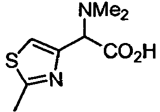 | LCMS:Anal. Calcd. for C₈H₁₁N₂O₂S: 200; found: 201 (M+H)⁺. |
| *Cap-32* | 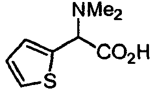 | LCMS: Anal. Calcd. for C₈H₁₁NO₂S: 185; found: 186 (M+H)⁺. |
| *Cap-33* | 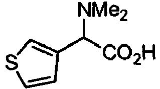 | LCMS: Anal. Calcd. for C₈H₁₁NO₂S: 185; found: 186 (M+H)⁺. |
| *Cap-34* | 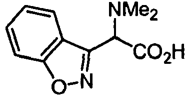 | LCMS: Anal. Calcd. for C₁₁H₁₂N₂O₃: 220; found: 221 (M+H)⁺. |
| *Cap-35* | 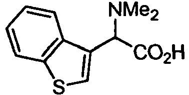 | LCMS: Anal. Calcd. for C₁₂H₁₃NO₂S: 235; found: 236 (M+H)⁺. |
| *Cap-36* | 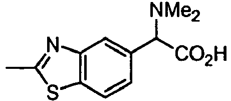 | LCMS: Anal. Calcd. for C₁₂H₁₄N₂O₂S: 250; found: 251 (M+H)⁺. |

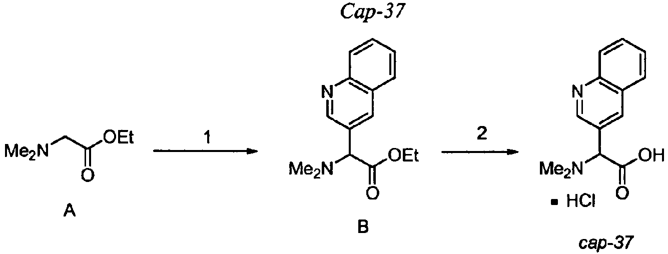

Step 1; (R,S)-Ethyl 2-(quinolin-3-yl)-2-(N,N-dimethylamino)-acetate: A mixture of ethyl N,N-dimethylaminoacetate (0.462 g, 3.54 mmol), K₃PO₄ (1.90 g, 8.95 mmol), Pd(t-Bu₃P)₂ (0.090 g, 0.176 mmol) and toluene (10 mL) was degassed with a stream of Ar bubbles for 15 minutes. The reaction mixture was then heated at 100 °C for 12 hours, after which it was cooled to room temperature and poured into H₂O. The mixture was extracted with ethyl acetate (2x) and the combined organic phases were washed (H₂O, brine), dried (Na₂SO₄), filtered, and concentrated *in vacuo.* The residue was purified first by reverse-phase preparative HPLC (Primesphere C-18, 30 x 100mm; CH₃CN-H₂O-5 mM NH₄OAc) and then by flash chromatography (SiO₂/ hexane-ethyl acetate, 1:1) to provide the title compound (0.128 g, 17%) as an orange oil. ¹HNMR (400 MHz, CDCl₃) δ 8.90 (d, *J* = 2.0 Hz, 1H), 8.32 (d, *J* = 2.0 Hz, 1H), 8.03-8.01 (m, 2H), 7.77 (ddd, *J* = 8.3, 6.8, 1.5 Hz, 1H), 7.62 (ddd, *J* = 8.3, 6.8, 1.5 Hz, 1H), 4.35 (s, 1H), 4.13 (m, 2H), 2.22 (s, 6H), 1.15 (t, *J* = 7.0 Hz, 3H). LCMS: Anal. Calcd. for C₁₅H₁₈N₂O₂: 258; found: 259 (M+H)⁺.

Step 2; (R,S) 2-(Quinolin-3-yl)-2-(N,N-dimethylamino)acetic acid: A mixture of (R,S)-ethyl 2-(quinolin-3-yl)-2-(N,N-dimethylamino)acetate (0.122 g, 0.472 mmol) and 6M HCl (3 mL) was heated at 100 °C for 12 hours. The solvent was removed *in vacuo* to provide the dihydrochloride of the title compound (0.169 g, > 100%) as a light yellow foam. The unpurified material was used in subsequent steps without further purification. LCMS: Anal. Calcd. for C₁₃H₁₄N₂O₂: 230; found: 231 (M+H)⁺.

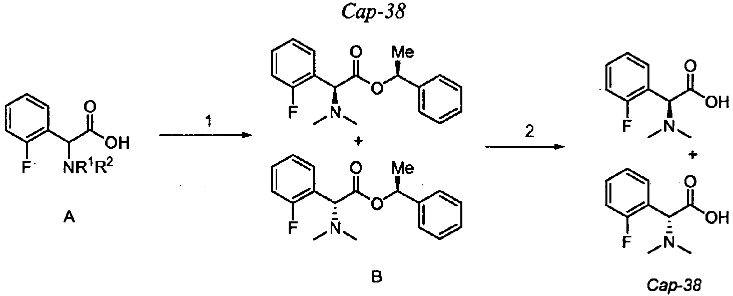

Step 1; (R)-((S)-1-phenylethyl) 2-(dimethylamino)-2-(2-fluorophenyl)acetate and (S)-((S)-1-phenylethyl) 2-(dimethylamino)-2-(2-fluorophenyl)acetate: To a mixture of (RS)-2-(dimethylamino)-2-(2-fluorophenyl)acetic acid (2.60 g, 13.19 mmol), DMAP (0.209 g, 1.71 mmol) and (S)-1-phenylethanol (2.09 g, 17.15 mmol) in CH₂Cl₂ (40 mL) was added EDCI (3.29 g, 17.1 mmol) and the mixture was allowed to stir at room temperature for 12 hours. The solvent was then removed *in vacuo* and the residue partitioned with ethyl acetate-H₂O. The layers were separated, the aqueous layer was back-extracted with ethyl acetate (2x) and the combined organic phases were washed (H₂O, brine), dried (Na₂SO₄), filtered, and concentrated *in vacuo.* The residue was purified by silica gel chromatography (Biotage/ 0-50% diethyl ether-hexane). The resulting pure diastereomeric mixture was then separated by reverse-phase preparative HPLC (Primesphere C-18, 30 x 100mm; CH₃CN-H₂O-0.1% TFA) to give first (S)-1-phenethyl (R)-2-(dimethylamino)-2-(2-fluorophenyl)acetate (0.501 g, 13%) and then (S)-1-phenethyl (S)-2-(dimethylamino)-2-(2-fluorophenyl)-acetate (0.727 g. 18%), both as their TFA salts. (S,R)-isomer: ¹HNMR (400 MHz, CD₃OD) δ 7.65 - 7.70 (m, 1H), 7.55-7.60 (ddd, *J* = 9.4, 8.1, 1.5 Hz, 1H), 7.36-7.41 (m, 2H), 7.28-7.34 (m, 5H), 6.04 (q, *J* = 6.5 Hz, 1H), 5.60 (s, 1H), 2.84 (s, 6H), 1.43 (d, *J* = 6.5 Hz, 3H). LCMS: Anal. Calcd. for C₁₈H₂₀FNO₂: 301; found: 302 (M+H)⁺; (S,S)-isomer: ¹HNMR (400 MHz, CD₃OD) δ 7.58-7.63 (m, 1H), 7.18-7.31 (m, 6H), 7.00 (dd, *J* = 8.5, 1.5 Hz, 2H), 6.02 (q, *J* = 6.5 Hz, 1H), 5.60 (s, 1H), 2.88 (s, 6H), 1.54 (d, *J* = 6.5 Hz, 3H). LCMS: Anal. Calcd. for C₁₈H₂₀FNO₂: 301; found: 302 (M+H)⁺.

Step 2; (R)-2-(dimethylamino)-2-(2-fluorophenyl)acetic acid: A mixture of (R)-((S)-1-phenylethyl) 2-(dimethylamino)-2-(2-fluorophenyl)acetate TFA salt (1.25 g, 3.01 mmol) and 20% Pd(OH)₂/C (0.125 g) in ethanol (30 mL) was hydrogenated at room temperature and atmospheric pressure (H₂ balloon) for 4 hours. The solution was then purged with Ar, filtered through diatomaceous earth (Celite^{®}), and concentrated *in vacuo.* This gave the title compound as a colorless solid (0.503 g, 98%). ¹HNMR (400 MHz, CD₃OD) δ 7.53-7.63 (m, 2H), 7.33-7.38 (m, 2H), 5.36 (s, 1H), 2.86 (s, 6H). LCMS: Anal. Calcd. for C₁₀H₁₂FNO₂: 197; found: 198 (M+H)⁺.

The S-isomer could be obtained from (S)-((S)-1-phenylethyl) 2-(dimethylamino)-2-(2-fluorophenyl)acetate TFA salt in similar fashion.

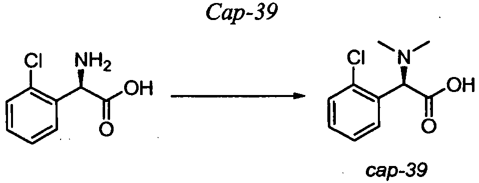

A mixture of (R)-(2-chlorophenyl)glycine (0.300 g, 1.62 mmol), formaldehyde (35% aqueous solution, 0.80 mL, 3.23 mmol) and 20% Pd(OH)₂/C (0.050 g) was hydrogenated at room temperature and atmospheric pressure (H₂ balloon) for 4 hours. The solution was then purged with Ar, filtered through diatomaceous earth (Celite®) and concentrated *in vacuo.* The residue was purified by reverse-phase preparative HPLC (Primesphere C-18, 30 x 100mm; CH₃CN-H₂O-0.1% TFA) to give theTFA salt of the title compound (R)-2-(dimethylamino)-2-(2-chlorophenyl)acetic acid as a colorless oil (0.290 g, 55%). ¹H NMR (400 MHz, CD₃OD) δ 7.59-7.65 (m, 2H), 7.45-7.53 (m, 2H), 5.40 (s, 1H), 2.87 (s, 6H). LCMS: Anal. Calcd. for C₁₀H₁₂ClNO₂: 213, 215; found: 214, 216 (M+H)⁺.

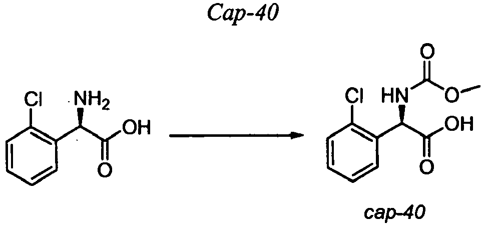

To an ice-cold solution of (R)-(2-chlorophenyl)glycine (1.00 g, 5.38 mmol) and NaOH (0.862 g, 21.6 mmol) in H₂O (5.5 mL) was added methyl chloroformate (1.00 mL, 13.5 mmol) dropwise. The mixture was allowed to stir at 0 °C for 1 hour and then it was acidified by the addition of conc. HCl (2.5 mL). The mixture was extracted with ethyl, acetate (2x) and the combined organic phase was washed (H₂O, brine), dried (Na₂SO₄), filtered, and concentrated *in vacuo* to give the title compound (R)-2-(methoxycarbonylamino)-2-(2-chlorophenyl)acetic acid as a yellow-orange foam (1.31 g, 96%). ¹H NMR (400 MHz, CD₃OD) δ 7.39 - 7.43 (m, 2H), 7.29 - 7.31 (m, 2H), 5.69 (s, 1H), 3.65 (s, 3H). LCMS: Anal. Calcd. for C₁₀H₁₀ClNO₄: 243, 245; found: 244, 246 (M+H)⁺.

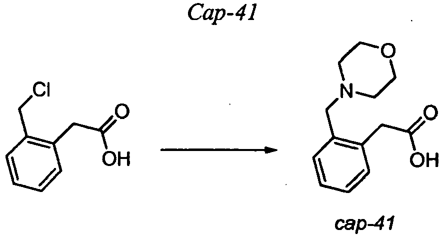

To a suspension of 2-(2-(chloromethyl)phenyl)acetic acid (2.00 g, 10.8 mmol) in THF (20 mL) was added morpholine (1.89 g, 21.7 mmol) and the solution was stirred at room temperature for 3 hours. The reaction mixture was then diluted with ethyl acetate and extracted with H₂O (2x). The aqueous phase was lyophilized and the residue was purified by silica gel chromatography (Biotage/ 0-10% methanol-CH₂Cl₂) to give the title compound 2-(2-(Morpholinomethyl)phenyl)acetic acid as a colorless solid (2.22 g, 87%). ¹HNMR (400 MHz, CD₃OD) δ 7.37-7.44 (m, 3H), 7.29-7.33 (m, 1H), 4.24 (s, 2H), 3.83 (br s, 4H), 3.68 (s, 2H), 3.14 (br s, 4H). LCMS: Anal. Calcd. for C₁₃H₁₇NO₃: 235; found: 236 (M+H)⁺.

The following examples were similarly prepared using the method described for *Cap*-41:

| | | |
|---|---|---|
| *Cap-42* | 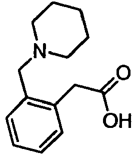 | LCMS: Anal. Calcd. for C₁₄H₁₉NO₂: 233; found: 234 (M+H)⁺. |
| *Cap-43* | 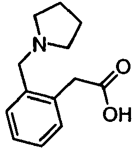 | LCMS: Anal. Calcd. for C₁₃H₁₇NO₂: 219; found: 220 (M+H)⁺. |
| *Cap-44* | 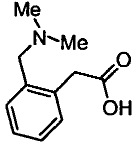 | LCMS: Anal. Calcd. for C₁₁H₁₅NO₂: 193; found: 194 (M+H)⁺. |
| *Cap-45* | 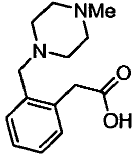 | LCMS: Anal. Calcd. for C₁₄H₂₀N₂O₂: 248; found: 249 (M+H)⁺. |

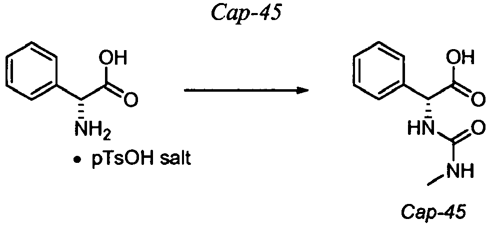

HMDS (1.85 mL, 8.77 mmol) was added to a suspension of (R)-2-amino-2-phenylacetic acid p-toluenesulfonate (2.83 g, 8.77 mmol) in CH₂Cl₂ (10 mL) and the mixture was stirred at room temperature for 30 minutes. Methyl isocyanate (0.5 g, 8.77 mmol) was added in one portion stirring continued for 30 minutes. The reaction was quenched by addition of H₂O (5 mL) and the resulting precipitate was filtered, washed with H₂O and n-hexanes, and dried under vacuum. (R)-2-(3-methylureido)-2-phenylacetic acid (1.5 g; 82 %).was recovered as a white solid and it was used without further purification. ¹H NMR (500 MHz, DMSO-d₆) δ ppm 2.54 (d, *J*=4.88 Hz, 3H) 5.17 (d, *J*=7.93 Hz, 1H) 5.95 (q, *J*=4.48 Hz, 1H) 6.66 (d, *J*=7.93 Hz, 1H) 7.26 - 7.38 (m, 5H) 12.67 (s, 1H). LCMS: Anal. Calcd. for C₁₀H₁₂N₂O₃ 208.08 found 209.121 (M+H)⁺; HPLC Phenomenex C-18 3.0 × 46 mm, 0 to 100% B over 2 minutes, 1 minute hold time, A = 90% water, 10% methanol, 0.1% TFA, B = 10% water, 90% methanol, 0.1% TFA, RT = 1.38 min, 90% homogeneity index.

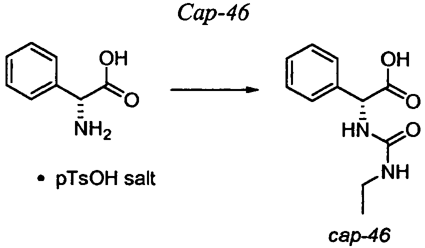

The desired product was prepared according to the method described for *Cap-*45. ¹H NMR (500 MHz, DMSO-d₆) δ ppm 0.96 (t, *J*=7.17 Hz, 3H) 2.94 - 3.05 (m, 2H) 5.17 (d, *J*=7.93 Hz, 1H) 6.05 (t, *J*=5.19 Hz, 1H) 6.60 (d, *J*=7.63 Hz, 1H) 7.26 - 7.38 (m, 5H) 12.68 (s, 1H). LCMS: Anal. Calcd. for C₁₁H₁₄N₂O₃ 222.10 found 209.121 (M+H)⁺.
HPLC XTERRA C-18 3.0 × 506 mm, 0 to 100% B over 2 minutes, 1 minutes hold time, A = 90% water, 10% methanol, 0.2% H₃PO₄, B = 10% water, 90% methanol, 0.2% H₃PO₄, RT = 0.87 min, 90% homogeneity index.

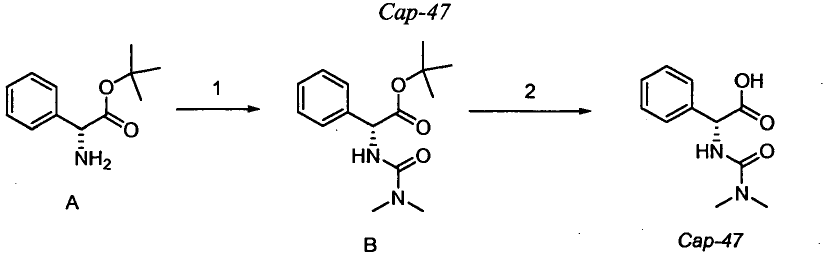

Step 1; (R)-tert-butyl 2-(3,3-dimethylureido)-2-phenylacetate: To a stirred solution of (R)-tert-butyl-2-amino-2-phenylacetate (1.0 g, 4.10 mmol) and Hunig's base (1.79 mL, 10.25 mmol) in DMF (40 mL) was added dimethylcarbamoyl chloride (0.38 mL, 4.18 mmol) dropwise over 10 minutes. After stirring at room temperature for 3 hours, the reaction was concentrated under reduced pressure and the resulting residue was dissolved in ethyl acetate. The organic layer was washed with H₂O, 1N aq. HCl and brine, dried (MgSO₄), filtered and concentrated under reduced pressure. (R)-tert-butyl 2-(3,3-dimethylureido)-2-phenylacetate was obtained as a white solid (0.86 g; 75%) and used without further purification. ¹H NMR (500 MHz, DMSO-d₆) δ ppm 1.33 (s, 9H) 2.82 (s, 6H) 5.17 (d, *J*=7.63 Hz, 1H) 6.55 (d, *J*=7.32 Hz, 1H) 7.24 - 7.41 (m, 5H). LCMS: Anal. Calcd. for C₁₅H₂₂N₂O₃ 278.16 found 279.23 (M+H)⁺; HPLC Phenomenex LUNA C-18 4.6 × 50 mm, 0 to 100% B over 4 minutes, 1 minutes hold time, A = 90% water, 10% methanol, 0.1% TFA, B = 10% water, 90% methanol, 0.1% TFA, RT = 2.26 min, 97% homogeneity index.

Step 2; (R)-2-(3,3-dimethylureido)-2-phenylacetic acid: To a stirred solution of ((R)-tert-butyl 2-(3,3-dimethylureido)-2-phenylacetate ( 0.86 g, 3.10 mmol) in CH₂Cl₂ (250 mL) was added TFA (15 mL) dropwise and the resulting solution was stirred at rt for 3 h. The desired compound was then precipitated out of solution with a mixture of EtOAC:Hexanes (5:20), filtered off and dried under reduced pressure. (R)-2-(3,3-dimethylureido)-2-phenylacetic acid was isolated as a white solid (0.59g, 86%) and used without further purification. ¹H NMR (500 MHz, DMSO-d₆) δ ppm 2.82 (s, 6H) 5.22 (d, *J*=7.32 Hz, 1H) 6.58 (d, *J*=7.32 Hz, 1H) 7.28 (t, *J*=7.17 Hz, 1H) 7.33 (t, *J*=7.32 Hz, 2H) 7.38 - 7.43 (m, 2H) 12.65 (s, 1H). LCMS: Anal. Calcd. for C₁₁H₁₄N₂O₃: 222.24; found: 223.21 (M+H)⁺. HPLC XTERRA C-18 3.0 × 50 mm, 0 to 100% B over 2 minutes, 1 minutes hold time, A = 90% water, 10% methanol, 0.2% H₃PO₄, B = 10% water, 90% methanol, 0.2% H₃PO₄, RT = 0.75 min, 93% homogeneity index.

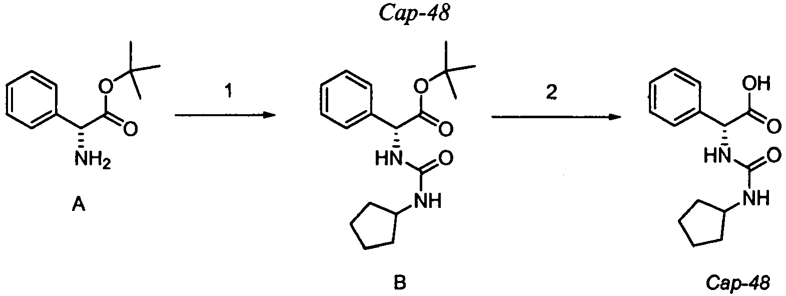

Step 1; (R)-tert-butyl 2-(3-cyclopentylureido)-2-phenylacetate: To a stirred solution of (R)-2-amino-2-phenylacetic acid hydrochloride (1.0 g, 4.10 mmol) and Hunig's base (1.0 mL, 6.15 mmol) in DMF (15 mL) was added cyclopentyl isocyanate (0.46 mL, 4.10 mmol) dropwise and over 10 minutes. After stirring at room temperature for 3 hours, the reaction was concentrated under reduced pressure and the resulting residue was traken up in ethyl acetate. The organic layer was washed with H₂O and brine, dried (MgSO₄) filtered, and concentrated under reduced pressure. (R)-tert-butyl 2-(3-cyclopentylureido)-2-phenylacetate was obtained as an opaque oil (1.32 g; 100 %) and used without further purification. ¹H NMR (500 MHz, CD₃Cl-D) δ ppm 1.50 - 1.57 (m, 2H) 1.58 - 1.66 (m, 2H) 1.87 - 1.97 (m, 2H) 3.89 - 3.98 (m, 1H) 5.37 (s, 1H) 7.26 - 7.38 (m, 5H). LCMS: Anal. Calcd. for C₁₈H₂₆N₂O₃ 318.19 found 319.21 (M+H)⁺; HPLC XTERRA C-18 3.0 × 50 mm, 0 to 100% B over 4 minutes, 1 minutes hold time, A = 90% water, 10% methanol, 0.1% TFA, B = 10% water, 90% methanol, 0.1% TFA, RT = 2.82 min, 96% homogeneity index.

Step 2; (R)-2-(3-cyclopentylureido)-2-phenylacetic acid: To a stirred solution of (R)-tert-butyl 2-(3-cyclopentylureido)-2-phenylacetate (1.31 g, 4.10 mmol) in CH₂Cl₂ (25 mL) was added TFA (4 mL) and trietheylsilane (1.64 mL; 10.3 mmol) dropwise, and the resulting solution was stirred at room temperature for 6 hours. The volatile components were removed under reduced pressure and the crude product was recrystallized in ethyl acetate/pentanes to yield (R)-2-(3-cyclopentylureido)-2-phenylacetic acid as a white solid (0.69 g, 64%). ¹H NMR (500 MHz, DMSO-d₆) δ ppm 1.17 - 1.35 (m, 2H) 1.42 - 1.52 (m, 2H) 1.53 - 1.64 (m, 2H) 1.67 - 1.80 (m, 2H) 3.75 - 3.89 (m, 1H) 5.17 (d, *J*=7.93 Hz, 1H) 6.12 (d, *J*=7.32 Hz, 1H) 6.48 (d, *J*=7.93 Hz, 1H) 7.24 - 7.40 (m, 5H) 12.73 (s, 1H). LCMS: Anal. Calcd. for C₁₄H₁₈N₂O₃: 262.31; found: 263.15 (M+H)⁺. HPLC XTERRA C-18 3.0 × 50 mm, 0 to 100% B over 2 minutes, 1 minutes hold time, A = 90% water, 10% methanol, 0.2% H₃PO₄, B = 10% water, 90% methanol, 0.2% H₃PO₄, RT = 1.24 min, 100% homogeneity index.

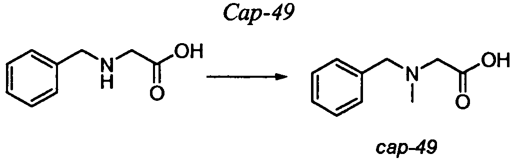

To a stirred solution of 2-(benzylamiino)acetic acid (2.0 g, 12.1 mmol) in formic acid (91 mL) was added formaldehyde (6.94 mL, 93.2 mmol). After five hours at 70 °C, the reaction mixture was concentrated under reduced pressure to 20 mL and a white solid precipitated. Following filtration, the mother liquors were collected and further concentrated under reduced pressure providing the crude product. Purification by reverse-phase preparative HPLC (Xterra 30 X 100 mm, detection at 220 nm, flow rate 35 mL/min, 0 to 35% B over 8 min; A = 90% water, 10 % methanol, 0.1% TFA, B = 10% water, 90 % methanol, 0.1% TFA) provided the title compound 2-(benzyl(methyl)-amino)acetic acid as its TFA salt (723 mg, 33%) as a colorless wax. ¹H NMR (300 MHz, DMSO-d₆) δ ppm 2.75 (s, 3H) 4.04 (s, 2H) 4.34 (s, 2H) 7.29 - 7.68 (m, 5H). LCMS: Anal. Calcd. for: C₁₀H₁₃NO₂ 179.22; Found: 180.20 (M+H)⁺.

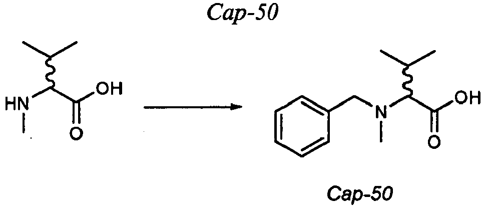

To a stirred solution of 3-methyl-2-(methylamino)butanoic acid (0.50 g, 3.81 mmol) in water (30 mL) was added K₂CO₃ (2.63 g, 19.1 mmol) and benzyl chloride (1.32 g, 11.4 mmol). The reaction mixture was stirred at ambient temperature for 18 hours. The reaction mixture was extracted with ethyl acetate (30 mL x 2) and the aqueous layer was concentrated under reduced pressure providing the crude product which was purified by reverse-phase preparative HPLC (Xterra 30 x 100mm, detection at 220 nm, flow rate 40 mL/min, 20 to 80% B over 6 min; A = 90% water, 10 % methanol, 0.1% TFA, B = 10% water, 90 % methanol, 0.1% TFA) to provide 2-(benzyl(methyl)amino)-3-methylbutanoic acid, TFA salt (126 mg, 19%) as a colorless wax. ¹H NMR (500 MHz, DMSO-d₆) δ ppm 0.98 (d, 3H) 1.07 (d, 3H) 2.33 - 2.48 (m, 1H) 2.54 - 2.78 (m, 3H) 3.69 (s, 1H) 4.24 (s, 2H) 7.29 - 7.65 (m, 5H). LCMS: Anal. Calcd. for: C₁₃H₁₉NO₂ 221.30; Found: 222.28 (M+H)⁺.

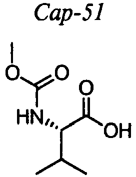

Na₂CO₃ (1.83g, 17.2 mmol) was added to NaOH (33 mL of 1M/H₂O, 33 mmol) solution of L-valine (3.9 g, 33.29 mmol) and the resulting solution was cooled with ice-water bath. Methyl chloroformate (2.8 mL, 36.1 mmol) was added drop-wise over 15 min, the cooling bath was removed and the reaction mixture was stirred at ambient temperature for 3.25 hr. The reaction mixture was washed with ether (50 mL, 3x), and the aqueous phase was cooled with ice-water bath and acidified with concentrated HCl to a pH region of 1-2, and extracted with CH₂Cl₂ (50 mL, 3x). The organic phase was dried (MgSO₄), filtered, and concentrated *in vacuo* to afford *Cap-*51 as a white solid (6 g). ¹H NMR for the dominant rotamer (DMSO-d₆, δ= 2.5 ppm, 500 MHz): 12.54 (s, 1H), 7.3 (d, *J =* 8.6, 1H), 3.84 (dd, *J =* 8.4, 6.0, 1H), 3.54 (s, 3H), 2.03 (m, 1H), 0.87 (m, 6H). HRMS: Anal. Calcd. for [M+H]⁺ C₇H₁₄NO₄: 176.0923; found 176.0922

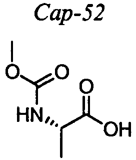

*Cap-52* was synthesized from L-alanine according to the procedure described for the synthesis of *Cap*-51. For characterization purposes, a portion of the crude material was purified by a reverse phase HPLC (H₂O/MeOH/TFA) to afford *Cap-52* as a colorless viscous oil. ¹H NMR (DMSO-d₆, δ= 2.5 ppm, 500 MHz): 12.49 (br s, 1H), 7.43 (d, *J* = 7.3, 0.88H), 7.09 (app br s, 0.12H), 3.97 (m, 1H), 3.53 (s, 3H), 1.25 (d, *J* = 7.3, 3H).

*Cap-53* to -64 were prepared from appropriate starting materials according to the procedure described for the synthesis of *Cap*-51, with noted modifications if any.

| Cap | Structure | Data |
|---|---|---|
| *Cap-53a:* (R) | 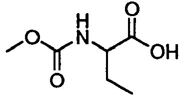 | ¹H NMR (DMSO-d₆, δ = 2.5 ppm, 500 MHz): δ 12.51 (br s, 1H), 7.4 (d, *J* = 7.9, 0.9H), 7.06 (app s, 0.1H), 3.86-3.82 (m, 1H), 3.53 (s, 3H), 1.75-1.67 (m, 1H), 1.62-1.54 (m, 1H), 0.88 (d, *J* = 7.3, 3H). RT = 0.77 minutes (Cond. 2); LC/MS: Anal. Calcd. for [M+Na]⁺ C₆H₁₁NNaO₄: 184.06; found 184.07. HRMS Calcd. for [M+Na]⁺C₆H₁₁NNaO₄: 184.0586; found 184.0592. |
| *Cap-53b:* (S) | | |
| *Cap-54a:* (R) | 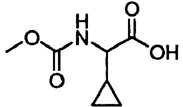 | ¹H NMR (DMSO-d₆, δ = 2.5 ppm, 500 MHz): δ 12.48 (s, 1H), 7.58 (d,*J* = 7.6, 0.9H), 7.25 (app s, 0.1H), 3.52 (s, 3H), 3.36-3.33 (m, 1H), 1.10-1.01 (m, 1H), 0.54-0.49 (m, 1H), 0.46-0.40 (m, 1H), 0.39-0.35 (m, 1H), 0.31-0.21 (m, 1H). HRMS Calcd. for [M+H]⁺C₇H₁₂NO₄: 174.0766; found 174.0771 |
| *Cap-54b:* (S) | | |
| *Cap-55* | 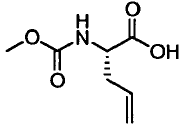 | ¹H NMR (DMSO-d₆, δ = 2.5 ppm, 500 MHz): δ 12.62 (s, 1H), 7.42 (d, *J* = 8.2, 0.9H), 7.07 (app s, 0.1H), 5.80-5.72 (m, 1H), 5.10 (d, *J* = 17.1, 1 H), 5.04 (d, *J* = 10.4, 1H), 4.01-3.96 (m, 1H), 3.53 (s, 3H), 2.47-2.42 (m, 1H), 2.35-2.29 (m, 1H). |
| *Cap-56* | 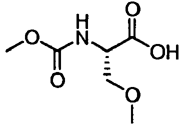 | 1H NMR (DMSO-d₆, δ = 2.5 ppm, 500 MHz): δ 12.75 (s, 1H), 7.38 (d, *J* = 8.3, 0.9H), 6.96 (app s, 0.1H), 4.20-4.16 (m, 1H), 3.60-3.55 (m, 2H), 3.54 (s, 3H), 3.24 (s, 3H). |
| *Cap- 57* | 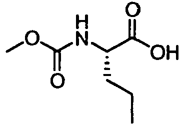 | ¹H NMR (DMSO-d₆, δ = 2.5 ppm, 500 MHz): δ 12.50 (s, 1H), 8.02 (d, *J* = 7.7, 0.08H), 7.40 (d, *J* = 7.9, 0.76H), 7.19 (d, *J* = 8.2, 0.07H), 7.07 (d, *J* = 6.7, 0.09H), 4.21-4.12 (m, 0.08H), 4.06-3.97 (m, 0.07H), 3.96-3.80 (m, 0.85H), 3.53 (s, 3H), 1.69-1.51 (m, 2H), 1.39-1.26 (m, 2H), 0.85 (t, *J* = 7.4, 3H). LC (Cond. 2): RT = 1.39 LC/MS: Anal. Calcd. for [M+H]⁺ C₇H₁₄NO₄: 176.09; found 176.06. |
| *Cap-58* | 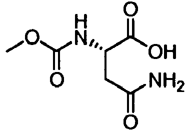 | ¹H NMR (DMSO-d₆, δ = 2.5 ppm, 500 MHz): δ 12.63 (bs, 1H), 7.35 (s,1H), 7.31 (d, *J* = 8.2, 1H), 6.92 (s, 1H), 4.33-4.29 (m, 1H), 3.54 (s, 3H), 2.54(dd, *J* = 15.5, 5.4, 1H), 2.43 (dd, *J* = 15.6, 8.0, 1H). RT = 0.16 min (Cond. 2); LC/MS: Anal. Calcd. for [M+H]⁺ C₆H₁₁N₂O₅: 191.07; found 191.14. |
| *Cap-59a*: (R) | | 1H NMR (DMSO-d₆, δ = 2.5 ppm, 400 MHz): δ 12.49 (br s, 1H), 7.40 (d, *J* = 7.3, 0.89H), 7.04 (br s, 0.11H), 4.00-3.95 (m, 3H), 1.24 (d, *J* = 7.3, 3H), 1.15 (t, *J* = 7.2, 3H). HRMS: Anal. Calcd. for [M+H]⁺ C₆H₁₂NO₄: 162.0766; found 162.0771. |
| *Cap-59b*:(S) | 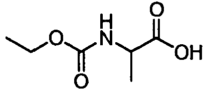 | |
| *Cap-60* | 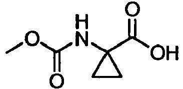 | The crude material was purified with a reverse phase HPLC (H₂O/MeOH/TFA) to afford a colorless viscous oil that crystallized to a white solid upon exposure to high vacuum. ¹H NMR (DMSO-d₆, δ = 2.5 ppm, 400 MHz): δ 12.38 (br s, 1H), 7.74(s, 0.82H), 7.48 (s, 0.18H), 3.54/3.51 (two s, 3H), 1.30 (m, 2H), 0.98 (m, 2H). HRMS: Anal. Calcd. for [M+H]⁺ C₆H₁₀NO₄: 160.0610; found 160.0604. |
| *Cap-61* | 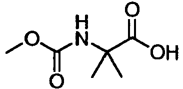 | ¹H NMR (DMSO-d₆, δ = 2.5 ppm, 400 MHz): δ 12.27 (br s, 1H), 7.40 (br s, 1H), 3.50 (s, 3H), 1.32 (s, 6H). HRMS: Anal. Calcd. for [M+H]⁺ C₆H₁₂NO₄: 162.0766; found 162.0765. |
| *Cap-62* | 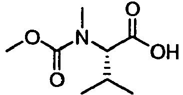 | ¹H NMR (DMSO-d₆, δ = 2.5 ppm, 400 MHz): δ 12.74 (br s, 1H), 4.21 (d, *J* = 10.3, 0.6H), 4.05 (d, *J* = 10.0, 0.4H), 3.62/3.60 (two singlets, 3H), 3.0 (s, 3H), 2.14-2.05 (m, 1H), 0.95 (d, *J* = 6.3, 3H), 0.81 (d, *J* = 6.6, 3H). LC/MS: Anal. Calcd. for [M-H]⁻ C₈H₁₄NO₄: 188.09; found 188.05. |
| *Cap-63* | 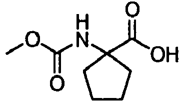 | [Note: the reaction was allowed to run for longer than what was noted for the general procedure.] ¹H NMR (DMSO-d₆, δ = 2.5 ppm, 400 MHz): 12.21 (br s, 1H), 7.42 (br s, 1 H), 3.50 (s, 3H), 2.02-1.85 (m, 4H), 1.66-1.58 (m, 4H). LC/MS: Anal. Calcd. for [M+H]⁺ C₈H₁₄NO₄: 188.09; found 188.19. |
| *Cap-64* | 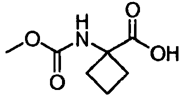 | [Note: the reaction was allowed to run for longer than what was noted for the general procedure.] ¹H NMR (DMSO-d₆, δ = 2.5 ppm, 400 MHz): 12.35 (br s, 1H), 7.77 (s, 0.82H), 7.56/7.52 (overlapping br s, 0.18H), 3.50 (s, 3H), 2.47-2.40 (m, 2H), 2.14-2.07 (m, 2H), 1.93-1.82 (m, 2H). |

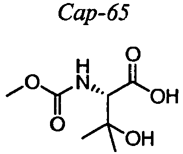

Methyl chloroformate (0.65 mL, 8.39 mmol) was added dropwise over 5 min to a cooled (ice-water) mixture of Na₂CO₃ (0.449 g, 4.23 mmol), NaOH (8.2 mL of 1M/H₂O, 8.2 mmol) and (S)-3-hydroxy-2-(methoxycarbonylamino)-3-methylbutanoic acid (1.04 g, 7.81 mmol). The reaction mixture was stirred for 45 min, and then the cooling bath was removed and stirring was continued for an additional 3.75 hr. The reaction mixture was washed with CH₂Cl₂, and the aqueous phase was cooled with ice-water bath and acidified with concentrated HCl to a pH region of 1-2. The volatile component was removed *in vacuo* and the residue was taken up in a 2:1 mixture of MeOH/CH₂Cl₂ (15 mL) and filtered, and the filterate was rotervaped to afford *Cap-65* as a white semi-viscous foam (1.236 g). ¹H NMR (DMSO-d₆, δ = 2.5 ppm, 400 MHz): δ 6.94 (d, *J* = 8.5, 0.9 H), 6.53 (br s, 0.1H), 3.89 (d, *J* = 8.8, 1H), 2.94 (s, 3H), 1.15 (s, 3H), 1.13 (s, 3H).

*Cap-66* and -67 were prepared from appropriate commercially available starting materials by employing the procedure described for the synthesis of *Cap-65.*

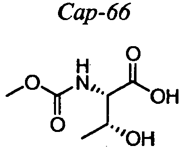

¹H NMR (DMSO-d₆, δ = 2.5 ppm, 400 MHz): δ 12.58 (br s, 1H), 7.07 (d, *J* = 8.3, 0.13H), 6.81 (d, *J* = 8.8, 0.67H), 4.10-4.02 (m, 1.15H), 3.91 (dd, *J* = 9.1, 3.5, 0.85H), 3.56 (s, 3H), 1.09 (d, *J* = 6.2, 3H). [Note: only the dominant signals ofNH were noted].

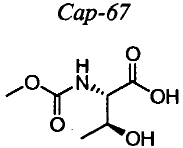

¹H NMR (DMSO-d₆, δ = 2.5 ppm, 400 MHz): 12.51 (br s, 1H), 7.25 (d, *J* = 8.4, 0.75H), 7.12 (br d, *J* = 0.4, 0.05H), 6.86 (br s, 0.08H), 3.95-3.85 (m, 2H), 3.54 (s, 3H), 1.08 (d, *J* = 6.3, 3H). [Note: only the dominant signals of NH were noted]

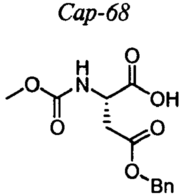

Methyl chloroformate (0.38 ml, 4.9 mmol) was added drop-wise to a mixture of 1N NaOH (aq) (9.0 ml, 9.0 mmol), 1M NaHCO₃ (aq) (9.0 ml, 9.0 mol), L-aspartic acid β-benzyl ester (1.0 g, 4.5 mmol) and Dioxane (9 ml). The reaction mixture was stirred at ambient conditions for 3 hr, and then washed with Ethyl acetate (50 ml, 3x). The aqueous layer was acidified with 12N HCl to a pH ~ 1-2, and extracted with ethyl acetate (3 x 50 ml). The combined organic layers were washed with brine, dried (Na₂SO₄), filtered, and concentrated *in vacuo* to afford *Cap-68* as a light yellow oil (1.37g; mass is above theoretical yield, and the product was used without further purification). ¹H NMR (DMSO-d₆, δ = 2.5 ppm, 500 MHz): δ 12.88 (br s, 1H), 7.55 (d, *J* = 8.5, 1H), 7.40-7.32 (m, 5H), 5.13 *(d, J =* 12.8, *1H), 5.10 (d, J =* 12.9, 1H), 4.42-4.38 (m, 1H), 3.55 (s, 3H), 2.87 (dd, *J =* 16.2, 5.5, 1H), 2.71 (dd, *J =16.2, 8.3,* 1H). LC (Cond. 2): RT = 1.90 min; LC/MS: Anal. Calcd. For [M+H]⁺ C₁₃H₁₆NO₆: 282.10; found 282.12.

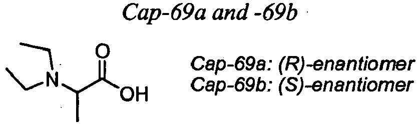

NaCNBH₃ (2.416 g, 36.5 mmol) was added in batches to a chilled (~15 °C) water (17 mL)/MeOH (10 mL) solution of alanine (1.338 g, 15.0 mmol). A few minutes later acetaldehyde (4.0 mL, 71.3 mmol) was added drop-wise over 4 min, the cooling bath was removed, and the reaction mixture was stirred at ambient condition for 6 hr. An additional acetaldehyde (4.0 mL) was added and the reaction was stirred for 2 hr. Concentrated HCl was added slowly to the reaction mixture until the pH reached ~ 1.5, and the resulting mixture was heated for 1 hr at 40 °C. Most of the volatile component was removed *in vacuo* and the residue was purified with a Dowex ® 50WX8-100 ion-exchange resin (column was washed with water, and the compound was eluted with dilute NH₄OH, prepared by mixing 18 ml of NH₄OH and 282 ml of water) to afford *Cap-69* (2.0 g) as an off-white soft hygroscopic solid. ¹H NMR (DMSO-d₆, δ = 2.5 ppm, 400 MHz): δ 3.44 (q, *J* = 7.1, 1H), 2.99-2.90 (m, 2H), 2.89-2.80 (m, 2H), 1.23 (d, *J* = 7.1, 3H), 1.13 (t, *J* = 7.3, 6H).

*Cap-70* to -74 were prepared according to the procedure described for the synthesis of *Cap-69* by employing appropriate starting materials.

| | | |
|---|---|---|
| *Cap-70a:* (R) | 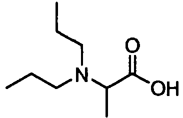 | ¹H NMR (DMSO-d₆, δ = 2.5 ppm, 400 MHz): δ 3.42 (q, *J* = 7.1, 1H), 2.68-2.60 (m, 4H), 1.53-1.44 (m, 4H), 1.19 (d, *J* = 7.3, 3H), 0.85 (t, *J* = 7.5, 6H). LC/MS: Anal. Calcd. for [M+H]⁺ C₉H₂₀NO₂: 174.15; found 174.13. |
| *Cap-70b*: (S) | | |
| *Cap-71a:* (R) | 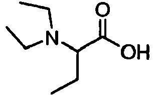 | ¹H NMR (DMSO-d₆, δ = 2.5 ppm, 500 MHz): δ 3.18-3.14 (m, 1H), 2.84-2.77 (m, 2H), 2.76-2.68 (m, 2H), 1.69-1.54 (m, 2H), 1.05 (t, *J* = 7.2, 6H), 0.91 (t, *J* = 7.3, 3H). LC/MS: Anal. Calcd. for [M+H]⁺ C₈H₁₈NO₂: 160.13; found 160.06. |
| *Cap-71b*: (S) | | |
| *Cap-72* | 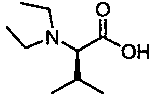 | ¹H NMR (DMSO-d₆, δ = 2.5 ppm, 400 MHz): δ 2.77-2.66 (m, 3H), 2.39-2.31 (m, 2H), 1.94-1.85 (m, 1H), 0.98 (t, *J* = 7.1, 6H), 0.91 (d, *J* = 6.5, 3H), 0.85 (d, *J* = 6.5, 3H). LC/MS: Anal. Calcd. for [M+H]⁺ C₉H₂₀NO₂: 174.15; found 174.15. |
| *Cap-73* | 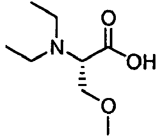 | ¹H NMR (DMSO-d₆, δ = 2.5 ppm, 500 MHz): δ 9.5 (br s, 1H), 3.77 (dd, *J* = 10.8, 4.1, 1H), 3.69-3.61 (m, 2H), 3.26 (s, 3H), 2.99-2.88 (m, 4H), 1.13 (t, *J* = 7.2, 6H). |
| *Cap-74* | 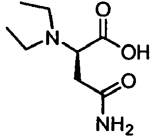 | ¹H NMR (DMSO-d₆, δ = 2.5 ppm, 500 MHz): δ 7.54 (s, 1H), 6.89 (s, 1H), 3.81 (t, *J* = 6.6, k, 1H), 2.82-2.71 (m, 4H), 2.63 (dd, *J* = 15.6, 7.0, 1H), 2.36 (dd, *J* = 15.4, 6.3, 1H), 1.09 (t, *J* = 7.2, 6H). RT = 0.125 minutes (Cond. 2); LC/MS: Anal. Calcd. for [M+H]⁺ C₈H₁₇N₂O₃ : 189.12; found 189.13. |
| *Cap-74x* | 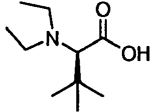 | LC/MS: Anal. Calcd. for [M+H]⁺ C₁₀H₂₂NO₂: 188.17; found 188.21 |

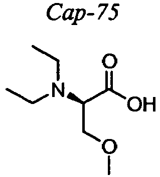

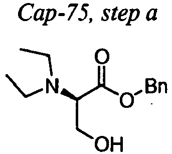

NaBH₃CN (1.6 g, 25.5 mmol) was added to a cooled (ice/water bath) water (25 ml)/methanol (15 ml) solution of H-D-Ser-OBzl HCl (2.0 g, 8.6 mmol). Acetaldehyde (1.5 ml, 12.5 mmol) was added drop-wise over 5 min, the cooling bath was removed, and the reaction mixture was stirred at ambient condition for 2 hr. The reaction was carefully quenched with 12N HCl and concentrated *in vacuo.* The residue was dissolved in water and purified with a reverse phase HPLC (MeOH/H₂O/TFA to afford the TFA salt of (R)-benzyl 2-(diethylamino)-3-hydroxypropanoate as a colorless viscous oil (1.9g). ¹H NMR (DMSO-d₆, δ = 2.5 ppm, 500 MHz): δ 9.73 (br s, 1H), 7.52-7.36 (m, 5H), 5.32 (d, *J* = 12.2, 1H), 5.27 (d, *J* = 12.5, 1H), 4.54-4.32 (m, 1H), 4.05-3.97 (m, 2H), 3.43-3.21 (m, 4H), 1.23 (t, *J* = 7.2, 6H). LC/MS (Cond. 2): RT = 1.38 min; LC/MS: Anal. Calcd. for [M+H]⁺ C₁₄H₂₂NO₃: 252.16; found 252.19.

### Cap-75

NaH (0.0727 g, 1.82 mmol, 60%) was added to a cooled (ice-water) THF (3.0 mL) solution of the TFA salt (R)-benzyl 2-(diethylamino)-3-hydroxypropanoate (0.3019 g, 0.8264 mmol) prepared above, and the mixture was stirred for 15 min. Methyl iodide (56 µL, 0.90 mmol) was added and stirring was continued for 18 hr while allowing the bath to thaw to ambient condition. The reaction was quenched with water and loaded onto a MeOH pre-conditioned MCX (6 g) cartridge, and washed with methanol followed by compound elution with 2N NH₃/Methanol. Removal of the volatile component *in vacuo* afforded *Cap-75,* contaminated with (R)-2-(diethylamino)-3-hydroxypropanoic acid, as a yellow semi-solid (100 mg). The product was used as is without further purification.

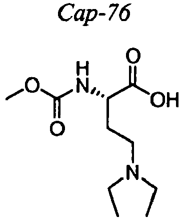

NaCNBH₃ (1.60 g, 24.2 mmol) was added in batches to a chilled (~15 °C) water/MeOH (12 mL each) solution of (S)-4-amino-2-(tert-butoxycarbonylamino) butanoic acid (2.17 g, 9.94 mmol). A few minutes later acetaldehyde (2.7 mL, 48.1 mmol) was added drop-wise over 2 min, the cooling bath was removed, and the reaction mixture was stirred at ambient condition for 3.5 hr. An additional acetaldehyde (2.7 mL, 48.1 mmol) was added and the reaction was stirred for 20.5 hr. Most of the MeOH component was removed *in vacuo,* and the remaining mixture was treated with concentrated HCl until its pH reached ~ 1.0 and then heated for 2 hr at 40 °C. The volatile component was removed *in vacuo,* and the residue was treated with 4 M HCl/dioxane (20 mL) and stirred at ambient condition for 7.5 hr. The volatile component was removed *in vacuo* and the residue was purified with Dowex ® 50WX8-100 ion-exchange resin (column was washed with water and the compound was eluted with dilute NH₄OH, prepared from 18 ml of NH₄OH and 282 ml of water) to afford intermediate (S)-2-amino-4-(diethylamino)butanoic acid as an off-white solid (1.73 g).

Methyl chloroformate (0.36 mL, 4.65 mmol) was added drop-wise over 11 min to a cooled (ice-water) mixture of Na₂CO₃ (0.243 g, 2.29 mmol), NaOH (4.6 mL of 1M/H₂O, 4.6 mmol) and the above product (802.4 mg). The reaction mixture was stirred for 55 min, and then the cooling bath was removed and stirring was continued for an additional 5.25 hr. The reaction mixture was diluted with equal volume of water and washed with CH₂Cl₂ (30 mL, 2x), and the aqueous phase was cooled with ice-water bath and acidified with concentrated HCl to a pH region of 2. The volatile component was then removed *in vacuo* and the crude material was free-based with MCX resin (6.0g; column was washed with water, and sample was eluted with 2.0 M NH₃/MeOH) to afford impure *Cap-76* as an off-white solid (704 mg). ¹H NMR (MeOH-d₄, δ = 3.29 ppm, 400 MHz): δ 3.99 (dd, *J* = 7.5, 4.7, 1H), 3.62 (s, 3H), 3.25-3.06 (m, 6H), 2.18-2.09 (m, 1H), 2.04-1.96 (m, 1H), 1.28 (t, *J* = 7.3, 6H). LC/MS: Anal. Calcd. for [M+H]⁺ C₁₀H₂₁N₂O₄: 233.15; found 233.24.

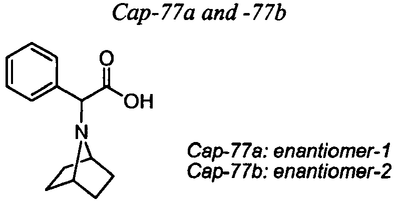

The synthesis of *Cap*-77 was conducted according to the procedure described for *Cap-7* by using 7-azabicyclo[2.2.1]heptane for the SN₂ displacement step, and by effecting the enantiomeric separation of the intermediate benzyl 2-(7-azabicyclo[2.2.1]heptan-7-yl)-2-phenylacetate using the following condition: the intermediate (303.7 mg) was dissolved in ethanol, and the resulting solution was injected on a chiral HPLC column (Chiracel AD-H column, 30 x 250 mm, 5 um) eluting with 90% CO₂-10% EtOH at 70 mL/min, and a temperature of 35°C to provide 124.5 mg of enantiomer-1 and 133.8 mg of enantiomer-2. These benzyl esters were hydrogenolysed according to the preparation of *Cap-7* to provide *Cap*-77: ¹H NMR (DMSO-d₆, δ = 2.5 ppm, 400 MHz): δ 7.55 (m, 2H), 7.38-7.30 (m, 3H), 4.16 (s, 1H), 3.54 (app br s, 2H), 2.08-1.88 (m, 4 H), 1.57-1.46 (m, 4H). LC (Cond. 1): RT = 0.67 min; LC/MS: Anal. Calcd. for [M+H]⁺ C₁₄H₁₈BrNO₂: 232.13; found 232.18. HRMS: Anal. Calcd. for [M+H]⁺ C₁₄H₁₈BrNO₂: 232.1338; found 232.1340.

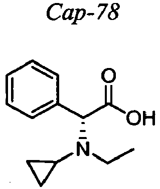

NaCNBH₃ (0.5828 g, 9.27 mmol) was added to a mixture of the HCl salt of (*R*)-2-(ethylamino)-2-phenylaletic acid (an intermediate in the synthesis of *Cap-3;* 0.9923 mg, 4.60 mmol) and (1-ethoxycyclopropoxy)trimethylsilane (1.640 g, 9.40 mmol) in MeOH (10 mL), and the semi-heterogeneous mixture was heated at 50 °C with an oil bath for 20 hr. More (1-ethoxycyclopropoxy)trimethylsilane (150 mg, 0.86 mmol) and NaCNBH₃ (52 mg, 0.827 mmol) were added and the reaction mixture was heated for an additional 3.5 hr. It was then allowed to cool to ambient temperature and acidified to a ~ pH region of 2 with concentrated HCl, and the mixture was filtered and the filtrate was rotervaped. The resulting crude material was taken up in *i*-PrOH (6 mL) and heated to effect dissolution, and the non-dissolved part was filtered off and the filtrate concentrated *in vacuo.* About 1/3 of the resultant crude material was purified with a reverse phase HPLC (H₂O/MeOH/TFA) to afford the TFA salt of *Cap-78* as a colorless viscous oil (353 mg). ¹H NMR (DMSO-d₆, δ = 2.5 ppm, 400 MHz; after D₂O exchange): δ 7.56-7.49 (m, 5H), 5.35 (S, 1H), 3.35 (m, 1H), 3.06 (app br s, 1H), 2.66 (m, 1H), 1.26 (t, *J* = 7.3, 3H), 0.92 (m, 1H), 0.83-0.44 (m, 3H). LC (Cond. 1): RT = 0.64 min; LC/MS: Anal. Calcd. for [M+H]⁺ C₁₃H₁₈NO₂: 220.13; found 220.21. HRMS: Anal. Calcd. for [M+H]⁺ C₁₃H₁₈NO₂: 220.1338; found 220.1343.

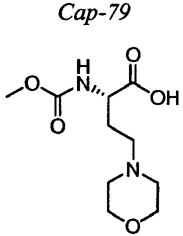

Ozone was bubbled through a cooled (-78 °C) CH₂Cl₂ (5.0 mL) solution *Cap-*55 (369 mg, 2.13 mmol) for about 50 min until the reaction mixture attained a tint of blue color. Me₂S (10 pipet drops) was added, and the reaction mixture was stirred for 35 min. The -78 °C bath was replaced with a -10 °C bath and stirring continued for an additional 30 min, and then the volatile component was removed *in vacuo* to afford a colorless viscous oil.

NaBH₃CN (149 mg, 2.25 mmol) was added to a MeOH (5.0 mL) solution of the above crude material and morpholine (500 µL, 5.72 mmol) and the mixture was stirred at ambient condition for 4 hr. It was cooled to ice-water temperature and treated with concentrated HCl to bring its pH to ~2.0, and then stirred for 2.5 hr. The volatile component was removed *in vacuo,* and the residue was purified with a combination of MCX resin (MeOH wash; 2.0 N NH₃/MeOH elution) and a reverse phase HPLC (H₂O/MeOH/TFA) to afford *Cap-79* containing unknown amount of morpholine.

In order to consume the morpholine contaminant, the above material was dissolved in CH₂Cl₂ (1.5 mL) and treated with Et₃N (0.27 mL, 1.94 mmol) followed by acetic anhydride (0.10 mL, 1.06 mmol) and stirred at ambient condition for 18 hr. THF (1.0 mL) and H₂O (0.5 mL) were added and stirring continued for 1.5 hr. The volatile component was removed *in vacuo,* and the resultant residue was passed through MCX resin (MeOH wash; 2.0 N NH₃/MeOH elution) to afford impure *Cap-*79 as a brown viscous oil, which was used for the next step without further purification.

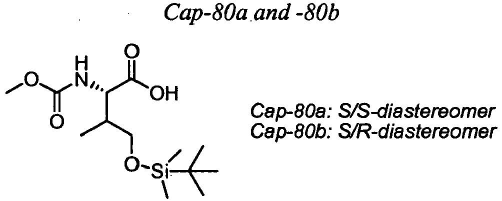

SOCl₂ (6.60 mL, 90.5 mmol) was added drop-wise over 15 min to a cooled (ice-water) mixture of (S)-3-amino-4-(benzyloxy)-4-oxobutanoic acid (10.04g, 44.98 mmol) and MeOH (300 mL), the cooling bath was removed and the reaction mixture was stirred at ambient condition for 29 hr. Most of the volatile component was removed *in vacuo* and the residue was carefully partitioned between EtOAc (150 mL) and saturated NaHCO₃ solution. The aqueous phase was extracted with EtOAc (150 mL, 2x), and the combined organic phase was dried (MgSO₄), filtered, and concentrated *in vacuo* to afford (S)-1-benzyl 4-methyl 2-aminosuccinate as a colorless oil (9.706g). ¹H NMR (DMSO-d₆, δ = 2.5 ppm, 400 MHz): δ 7.40-7.32 (m, 5H), 5.11 (s, 2H), 3.72 (app t, *J* = 6.6, 1H), 3.55 (s, 3H), 2.68 (dd, *J* = 15.9, 6.3, 1H), 2.58 (dd, *J* = 15.9, 6.8, 1H), 1.96 (s, 2H). LC (Cond. 1): RT = 0.90 min; LC/MS: Anal. Calcd. for [M+H]⁺ C₁₂H₁₆NO₄: 238.11; found 238.22.

Pb(NO₃)₂ (6.06 g, 18.3 mmol) was added over 1 min to a CH₂Cl₂ (80 mL) solution of (S)-1-benzyl 4-methyl 2-aminosuccinate (4.50 g, 19.0 mmol), 9-bromo-9-phenyl-9*H*-fluorene (6.44 g, 20.0 mmol) and Et₃N (3.0 mL, 21.5 mmol), and the heterogeneous mixture was stirred at ambient condition for 48 hr. The mixture was filtered and the filtrate was treated with MgSO₄ and filtered again, and the final filtrate was concentrated. The resulting crude material was submitted to a Biotage purification (350 g silica gel, CH₂Cl₂ elution) to afford (S)-1-benzyl 4-methyl 2-(9-phenyl-9H-fluoren-9-ylamino)succinate as highly viscous colorless oil (7.93 g). ¹H NMR (DMSO-d₆, δ = 2.5 ppm, 400 MHz): δ 7.82 (m, 2H), 7.39-7.13 (m, 16H), 4.71 (d, *J* = 12.4, 1H), 4.51 (d, *J* = 12.6, 1H), 3.78 (d, *J* = 9.1, NH), 3.50 (s, 3H), 2.99 (m, 1H), 2.50-2.41 (m, 2H, partially overlapped with solvent). LC (Cond. 1): RT = 2.16 min; LC/MS: Anal. Calcd. for [M+H]⁺ C₃₁H₂₈NO₄: 478.20; found 478.19.

LiHMDS (9.2 mL of 1.0 M/THF, 9.2 mmol) was added drop-wise over 10 min to a cooled (-78 °C) THF (50 mL) solution of (S)-1-benzyl 4-methyl 2-(9-phenyl-9H-fluoren-9-ylamino)succinate (3.907 g, 8.18 mmol) and stirred for ~1 hr. MeI (0.57 mL, 9.2 mmol) was added drop-wise over 8 min to the mixture, and stirring was continued for 16.5 hr while allowing the cooling bath to thaw to room temperature. After quenching with saturated NH₄Cl solution (5 mL), most of the organic component was removed *in vacuo* and the residue was partitioned between CH₂Cl₂ (100 mL) and water (40 mL). The organic layer was dried (MgSO₄), filtered, and concentrated *in vacuo,* and the resulting crude material was purified with a Biotage (350 g silica gel; 25% EtOAc/hexanes) to afford 3.65 g of a 2S/3S and 2S/3R diastereomeric mixtures of 1-benzyl 4-methyl 3-methyl-2-(9-phenyl-9H-fluoren-9-ylamino)succinate in ~1.0:0.65 ratio (¹H NMR). The stereochemistry of the dominant isomer was not determined at this juncture, and the mixture was submitted to the next step without separation. Partial ¹H NMR data (DMSO-d₆, δ = 2.5 ppm, 400 MHz): major diastereomer, δ 4.39 (d, *J* = 12.3, 1H of CH₂), 3.33 (s, 3H, overlapped with H₂O signal), 3.50 (d, *J* = 10.9, NH), 1.13 (d, *J* = 7.1, 3H); minor diastereomer, δ 4.27 (d, *J* =12.3, 1H of CH₂), 3.76 (d, *J* = 10.9, NH), 3.64 (s, 3H), 0.77 (d, *J* = 7.0, 3H). LC (Cond. 1): RT = 2.19 min; LC/MS: Anal. Calcd. for [M+H]⁺ C₃₂H₃₀NO₄: 492.22; found 492.15.

Diisobutylaluminum hydride (20.57 ml of 1.0 M in hexanes, 20.57 mmol) was added drop-wise over 10 min to a cooled (-78 °C) THF (120 mL) solution of (2S)-1-benzyl 4-methyl 3-methyl-2-(9-phenyl-9H-fluoren-9-ylamino)succinate (3.37 g, 6.86 mmol) prepared above, and stirred at -78 °C for 20 hr. The reaction mixture was removed from the cooling bath and rapidly poured into ~1M H₃PO₄/H₂O (250 mL) with stirring, and the mixture was extracted with ether (100 mL, 2x). The combined organic phase was washed with brine, dried (MgSO₄), filtered and concentrated *in vacuo.* A silica gel mesh of the crude material was prepared and submitted to chromatography (25% EtOAc/hexanes; gravity elution) to afford 1.1g of (2S,3S)-benzyl 4-hydroxy-3-methyl-2-(9-phenyl-9*H*-fluoren-9-ylamino)butanoate; contaminated with benzyl alcohol, as a colorless viscous oil and (2S,3R)-benzyl 4-hydroxy-3-methyl-2-(9-phenyl-9*H*-fluoren-9-ylamino)butanoate containing the (2S,3R) stereoisomer as an impurity. The later sample was resubmitted to the same column chromatography purification conditions to afford 750 mg of purified material as a white foam. [Note: the (2S, 3S) isomer elutes before the (2S,3R) isomer under the above condition]. (2S, 3S) isomer: ¹H NMR (DMSO-d₆, δ = 2.5 ppm, 400 MHz): 7.81 (m, 2H), 7.39-7.08 (m, 16H), 4.67 (d, *J* = 12.3, 1H), 4.43 (d, *J* = 12.4, 1H), 4.21 (app t, *J* = 5.2, OH), 3.22 (d, *J* = 10.1, NH), 3.17 (m, 1H), 3.08 (m, 1H), ~2.5 (m, 1H, overlapped with the solvent signal), 1.58 (m, 1H), 0.88 (d, *J* = 6.8, 3H). LC (Cond. 1): RT = 2.00 min; LC/MS: Anal. Calcd. for [M+H]⁺ C₃₁H₃₀NO₃: 464.45; found 464.22. (2S, 3R) isomer: ¹H NMR (DMSO-d₆, δ = 2.5 ppm, 400 MHz): 7.81 (d, *J* = 7.5, 2H), 7.39-7.10 (m, 16H), 4.63 (d, *J* = 12.1, 1H), 4.50 (app t, *J* = 4.9, 1H), 4.32 (d, *J* = 12.1, 1H), 3.59-3.53 (m, 2H), 3.23 (m, 1H), 2.44 (dd, *J* = 9.0, 8.3, 1H), 1.70 (m, 1H), 0.57 (d, *J* = 6.8, 3H). LC (Cond. 1): RT = 1.92 min; LC/MS: Anal. Calcd. for [M+H]⁺ C₃₁H₃₀NO₃: 464.45; found 464.52.

The relative stereochemical assignments of the DIBAL-reduction products were made based on NOE studies conducted on lactone derivatives prepared from each isomer by employing the following protocol: LiHMDS (50 µL of 1.0 M/THF, 0.05 mmol) was added to a cooled (ice-water) THF (2.0 mL) solution of (2S,3S)-benzyl 4-hydroxy-3-methyl-2-(9-phenyl-9*H*-fluoren-9-ylamino)butanoate (62.7 mg, 0.135 mmol), and the reaction mixture was stirred at similar temperature for ~2 hr. The volatile component was removed *in vacuo* and the residue was partitioned between CH₂Cl₂ (30 mL), water (20 mL) and saturated aqueous NH₄Cl solution (1 mL). The organic layer was dried (MgSO₄), filtered, and concentrated *in vacuo,* and the resulting crude material was submitted to a Biotage purification (40 g silica gel; 10-15% EtOAc/hexanes) to afford (3S,4S)-4-methyl-3-(9-phenyl-9*H*-fluoren-9-ylamino)dihydrofuran-2(3*H*)-one as a colorless film of solid (28.1 mg). (2S,3R)-benzyl 4-hydroxy-3-methyl-2-(9-phenyl-9*H*-fluoren-9-ylamino)butanoate was elaborated similarly to (3S,4R)-4-methyl-3-(9-phenyl-9*H*-fluoren-9-ylamino)dihydrofuran-2(3*H*)-one. (3S,4S)-lactone isomer: ¹H NMR (DMSO-d₆, δ = 2.5 ppm, 400 MHz), 7.83 (d, *J* = 7.5, 2H), 7.46-7.17 (m, 11H), 4.14 (app t, *J* = 8.3, 1H), 3.60 (d, *J* = 5.8, NH), 3.45 (app t, *J* = 9.2, 1H), ~2.47 (m, 1H, partially overlapped with solvent signal), 2.16 (m, 1H), 0.27 (d, *J* = 6.6, 3H). LC (Cond. 1): RT = 1.98 min; LC/MS: Anal. Calcd. for [M+Na]⁺ C₂₄H₂₁NNaO₂: 378.15; found 378.42. (3S,4R)-lactone isomer: ¹H NMR (DMSO-d₆, δ = 2.5 ppm, 400 MHz), 7.89 (d, *J* = 7.6, 1H), 7.85 (d, *J* = 7.3, 1H), 7.46-7.20 (m, 11H), 3.95 (dd, *J* = 9.1, 4.8, 1H), 3.76 (d, *J* = 8.8, 1H), 2.96 (d, *J* = 3.0, NH), 2.92 (dd, *J* = 6.8, 3, NCH), 1.55 (m, 1H), 0.97 (d, *J* = 7.0, 3H). LC (Cond. 1): RT = 2.03 min; LC/MS: Anal. Calcd. for [M+Na]⁺ C₂₄H₂₁NNaO₂: 378.15; found 378.49.

TBDMS-Cl (48 mg, 0.312 mmol) followed by imidazole (28.8 mg, 0.423 mmol) were added to a CH₂Cl₂ (3 ml) solution of (2S,3S)-benzyl 4-hydroxy-3-methyl-2-(9-phenyl-9H-fluoren-9-ylamino)butanoate (119.5 mg, 0.258 mmol), and the mixture was stirred at ambient condition for 14.25 hr. The reaction mixture was then diluted with CH₂Cl₂ (30 mL) and washed with water (15 mL), and the organic layer was dried (MgSO₄), filtered, and concentrated *in vacuo.* The resultant crude material was purified with a Biotage (40 g silica gel; 5% EtOAc/hexanes) to afford (2S,3S)-benzyl 4-(tert-butyldimethylsilyloxy)-3-methyl-2-(9-phenyl-9H-fluoren-9-ylamino)butanoate, contaminated with TBDMS based impurities, as a colorless viscous oil (124.4 mg). (2S,3R)-benzyl 4-hydroxy-3-methyl-2-(9-phenyl-9H-fluoren-9-ylamino)butanoate was elaborated similarly to (2S,3R)-benzyl 4-(tert-butyldimethylsilyloxy)-3-methyl-2-(9-phenyl-9H-fluoren-9-ylamino)butanoate. (2S,3S)-silyl ether isomer: ¹H NMR (DMSO-d₆, δ = 2.5 ppm, 400 MHz), 7.82 (d, *J* = 4.1, 1H), 7.80 (d, *J* = 4.0, 1H), 7.38-7.07 (m, 16 H), 4.70 (d, *J* = 12.4, 1H), 4.42 (d, *J* = 12.3, 1H), 3.28-3.19 (m, 3H), 2.56 (dd, *J* = 10.1, 5.5, 1H), 1.61 (m, 1H), 0.90 (d, *J* = 6.8, 3H), 0.70 (s, 9H), -0.13 (s, 3H), -0.16 (s, 3H). LC (Cond. 1, where the run time was extended to 4 min): RT = 3.26 min; LC/MS: Anal. Calcd. for [M+H]⁺ C₃₇H₄₄NO₃Si: 578.31; found 578.40. (2S,3R)-silyl ether isomer: ¹H NMR (DMSO-d₆, δ = 2.5 ppm, 400 MHz), 7.82 (d, *J* = 3.0, 1H), 7.80 (d, *J* = 3.1, 1H), 7.39-7.10 (m, 16H), 4.66 (d, *J* = 12.4, 1H), 4.39 (d, *J* = 12.4, 1H), 3.61 (dd, *J* = 9.9, 5.6, 1H), 3.45 (d, *J* = 9.5, 1H), 3.41 (dd, *J* = 10, 6.2, 1H), 2.55 (dd, *J* = 9.5, 7.3, 1H), 1.74 (m, 1H), 0.77 (s, 9H), 0.61 (d, *J* = 7.1, 3H), -0.06 (s, 3H), -0.08 (s, 3H).

A balloon of hydrogen was attached to a mixture of (2S,3S)-benzyl 4-(tert-butyldimethylsilyloxy)-3-methyl-2-(9-phenyl-9*H*-fluoren-9-ylamino)butanoate (836 mg, 1.447 mmol) and 10% Pd/C (213 mg) in EtOAc (16 mL) and the mixture was stirred at room temperature for ~ 21 hr, where the balloon was recharged with H₂ as necessary. The reaction mixture was diluted with CH₂Cl₂ and filtered through a pad of diatomaceous earth (Celite-545^{®}), and the pad was washed with EtOAc (200 mL), EtOAc/MeOH (1:1 mixture, 200 mL) and MeOH (750 mL). The combined organic phase was concentrated, and a silica gel mesh was prepared from the resulting crude material and submitted to a flash chromatography (8:2:1 mixture of EtOAc/i-PrOH/H₂O) to afford (2S,3S)-2-amino-4-(tert-butyldimethylsilyloxy)-3-methylbutanoic acid as a white fluffy solid (325 mg). (2S,3R)-benzyl 4-(tert-butyldimethylsilyloxy)-3-methyl-2-(9-phenyl-9H-fluoren-9-ylamino)butanoate was similarly elaborated to (2S,3R)-2-amino-4-(tert-butyldimethylsilyloxy)-3-methylbutanoic acid. (2S,3S)-amino acid isomer: ¹H NMR (Methanol-d₄, δ = 3.29 ppm, 400 MHz), 3.76 (dd, *J* = 10.5, 5.2, 1H), 3.73 (d, *J* = 3.0, 1H), 3.67 (dd, *J* = 10.5, 7.0, 1H), 2.37 (m, 1H), 0.97 (d, *J* = 7.0, 3H), 0.92 (s, 9H), 0.10 (s, 6H). LC/MS: Anal. Calcd. for [M+H]⁺ C₁₁H₂₆NO₃Si: 248.17; found 248.44. (2S,3R)-amino acid isomer: ¹H NMR (Methanol-d₄, δ = 3.29 ppm, 400 MHz), 3.76-3.75 (m, 2H), 3.60 (d, *J* = 4.1, 1H), 2.16 (m, 1H), 1.06 (d, *J* = 7.3, 3H), 0.91 (s, 9H), 0.09 (s, 6H). Anal. Calcd. for [M+H]⁺ C₁₁H₂₆NO₃Si: 248.17; found 248.44.

Water (1mL) and NaOH (0.18 mL of 1.0 M/H₂O, 0.18 mmol) were added to a mixture of (2S,3S)-2-amino-4-(tert-butyldimethylsilyloxy)-3-methylbutanoic acid (41.9 mg, 0.169 mmol) and Na₂CO₃ (11.9 mg, 0.112 mmol), and sonicated for about 1 min to effect dissolution of reactants. The mixture was then cooled with an ice-water bath, methyl chloroformate (0.02 mL, 0.259 mmol) was added over 30 s, and vigorous stirring was continued at similar temperature for 40 min and then at ambient temperature for 2.7 hr. The reaction mixture was diluted with water (5 mL), cooled with ice-water bath and treated drop-wise with 1.0 N HCl aqueous solution (~0.23 mL). The mixture was further diluted with water (10 mL) and extracted with CH₂Cl₂ (15 mL, 2x). The combined organic phase was dried (MgSO₄), filtered, and *concentrated in vacuo* to afford *Cap-80a* as an off-white solid. (2S,3R)-2-amino-4-(tert-butyldimethylsilyloxy)-3-methylbutanoic acid was similarly elaborated to *Cap-*80b. *Cap-80a:* ¹H NMR (DMSO-d₆, δ = 2.5 ppm, 400 MHz), 12.57 (br s, 1H), 7.64 (d, *J* = 8.3, 0.3H), 7.19 (d, *J* = 8.8, 0.7H), 4.44 (dd, *J* = 8.1, 4.6, 0.3H), 4.23 (dd, *J* = 8.7, 4.4, 0.7H), 3.56/3.53 (two singlets, 3H), 3.48-3.40 (m, 2H), 2.22-2.10 (m, 1H), 0.85 (s, 9H), ~0.84 (d, 0.9H, overlapped with t-Bu signal), 0.79 (d, *J* = 7, 2.1H), 0.02/0.01/0.00 (three overlapping singlets, 6H). LC/MS: Anal. Calcd. for [M+Na]⁺ C₁₃H₂₇NNaO₅Si: 328.16; found 328.46. *Cap*-80b: ¹H NMR (CDCl₃, δ = 7.24 ppm, 400 MHz), 6.00 (br d, *J* = 6.8, 1H), 4.36 (dd, *J* = 7.1, 3.1, 1H), 3.87 (dd, *J* = 10.5, 3.0, 1H), 3.67 (s, 3H), 3.58 (dd, *J* = 10.6,4.8, 1H), 2.35 (m, 1H), 1.03 (d, *J* = 7.1, 3H), 0.90 (s, 9H), 0.08 (s, 6H). LC/MS: Anal. Calcd. for [M+Na]⁺ C₁₃H₂₇NNaO₅Si: 328.16; found 328.53. The crude products were utilized without further purification.

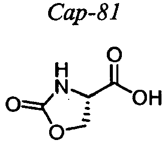

Prepared according to the protocol described by Falb et al. *Synthetic Communications* 1993, 23, 2839.

### Cap-82 to Cap-85

*Cap-82* to *Cap-85* were synthesized from appropriate starting materials according to the procedure described for *Cap-51.* The samples exhibited similar spectral profiles as that of their enantiomers (i.e., *Cap-4, Cap-13, Cap-51* and *Cap-*52, respectively)

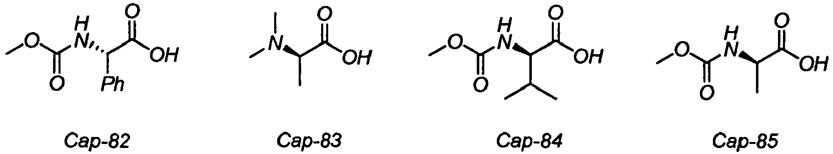

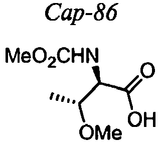

To a mixture of O-methyl-L-threonine (3.0 g, 22.55 mmol), NaOH (0.902 g, 22.55 mmol) in H₂O (15 mL) was added ClCO₂Me (1.74 mL, 22.55 mmol) dropwise at 0°C. The mixture was allowed to stir for 12 h and acidified to pH 1 using 1N HCl. The aqueous phase was extracted with EtOAc and (2x250 mL) and 10% MeOH in CH₂Cl₂ (250 mL) and the combined organic phases were concentrated under *in vacuo* to afford a colorless oil (4.18 g, 97%) which was of sufficient purity for use in subsequent steps. ¹HNMR (400 MHz, CDCl₃) δ 4.19 (s, 1H), 3.92-3.97 (m, 1H), 3.66 (s, 3H), 1.17 (d, *J* = 7.7 Hz, 3H). LCMS: Anal. Calcd. for C₇H₁₃NO₅: 191; found: 190 (M-H)⁻.

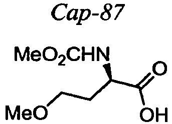

To a mixture of L-homoserine (2.0 g, 9.79 mmol), Na₂CO₃ (2.08 g, 19.59 mmol) in H₂O (15 mL) was added ClCO₂Me (0.76 mL, 9.79 mmol) dropwise at 0°C. The mixture was allowed to stir for 48 h and acidified to pH 1 using 1N HCl. The aqueous phase was extracted with EtOAc and (2X250 mL) and the combined organic phases were concentrated under *in vacuo* to afford a colorless solid (0.719 g, 28%) which was of sufficient purity for use in subsequent steps. ¹HNMR (400 MHz, CDCl₃) δ 4.23 (dd, *J* = 4.5, 9.1 Hz, 1H), 3.66 (s, 3H), 3.43-3.49 (m, 2H), 2.08 - 2.14 (m, 1H), 1.82 - 1.89 (m, 1H). LCMS: Anal. Calcd. for C₇H₁₃NO₅: 191; found: 192 (M+H)⁺.

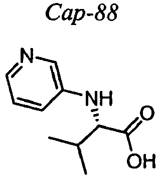

A mixture of L-valine (1.0 g, 8.54 mmol), 3-bromopyridine (1.8 mL, 18.7 mmol), K₂CO₃ (2.45 g, 17.7 mmol) and CuI (169 mg, 0.887 mmol) in DMSO (10 mL) was heated at 100°C for 12h. The reaction mixture was cooled to rt, poured into H₂O (ca. 150 mL) and washed with EtOAc (x2). The organic layers were extracted with a small amount of H₂O and the combined aq phases were acidified to ca. pH 2 with 6N HCl. The volume was reduced to about one-third and 20g of cation exchange resin (Strata) was added. The slurry was allowed to stand for 20 min and loaded onto a pad of cation exchange resin (Strata) (ca. 25g). The pad was washed with H₂O (200 mL), MeOH (200 mL), and then NH₃ (3M in MeOH, 2X200 mL). The appropriate fractions was concentrated *in vacuo* and the residue (ca. 1.1 g) was dissolved in H₂O, frozen and lyophyllized. The title compound was obtained as a foam (1.02 g, 62%). ¹HNMR (400 MHz, DMSO-d₆) δ 8.00 (s, br, 1H), 7.68 - 7.71 (m, 1H), 7.01 (s, br, 1H), 6.88 (d, *J* = 7.5 Hz, 1H), 5.75 (s, br, 1H), 3.54 (s, 1H), 2.04 - 2.06 (m, 1H), 0.95 (d, *J* = 6.0 Hz, 3H), 0.91 (d, *J* = 6.6 Hz, 3H). LCMS: Anal. Calcd. for C₁₀H₁₄N₂O₂: 194; found: 195 (M+H)⁺.

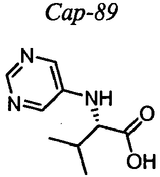

A mixture of L-valine (1.0 g, 8.54 mmol), 5-bromopyrimidine (4.03 g, 17.0 mmol), K₂CO₃ (2.40 g, 17.4 mmol) and CuI (179 mg, 0.94 mmol) in DMSO (10 mL) was heated at 100°C for 12h. The reaction mixture was cooled to RT, poured into H₂O (ca. 150 mL) and washed with EtOAc (x2). The organic layers were extracted with a small amount of H₂O and the combined aq phases were acidified to ca. pH 2 with 6N HCl. The volume was reduced to about one-third and 20g of cation exchange resin (Strata) was added. The slurry was allowed to stand for 20 min and loaded onto a pad of cation exchange resin (Strata) (ca. 25g). The pad was washed with H₂O (200 mL), MeOH (200 mL), and then NH₃ (3M in MeOH, 2x200 mL). The appropriate fractions was concentrated *in vacuo* and the residue (ca. 1.1 g) was dissolved in H₂O, frozen and lyophyllized. The title compound was obtained as a foam (1.02 g, 62%). ¹HNMR (400 MHz, CD₃OD) showed the mixture to contain valine and the purity could not be estimated. The material was used as is in subsequent reactions. LCMS: Anal. Calcd. for C₉H₁₃N₃O₂: 195; found: 196 (M+H)⁺.

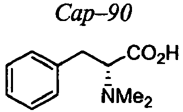

*Cap-90* was prepared according to the method described for the preparation of *Cap-1.* The crude material was used as is in subsequent steps. LCMS: Anal. Calcd. for C₁₁H₁₅NO₂: 193; found: 192 (M-H)⁻.

The following caps were prepared according to the method of example 51:

| Cap | Structure | LCMS |
|---|---|---|
| *Cap-91* | 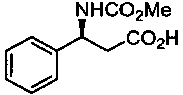 | LCMS: Anal. Calcd. for C₁₁H₁₃NO₄: 223; found: 222 (M-H)⁻. |
| *Cap-92* | 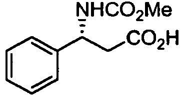 | LCMS: Anal. Calcd. for C₁₁H₁₃NO₄: 223; found: 222 (M-H)⁻. |
| *Cap-93* | 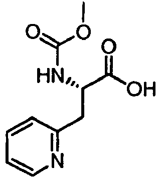 | LCMS: Anal. Calcd. for C₁₀H₁₂N₂O₄: 224; found: 225 (M+H)⁺. |
| *Cap-94* | 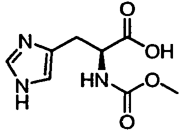 | LCMS: Anal. Calcd. for C₈H₁₁N₃O₄: 213; found: 214 (M+H)⁺. |
| *Cap-95* | 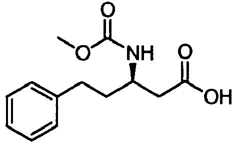 | LCMS: Anal. Calcd. for C₁₃H₁₇NO₄: 251; found: 250 (M-H)⁻. |
| *Cap-96* | 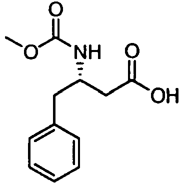 | LCMS: Anal. Calcd. for C₁₂H₁₅NO₄: 237; found: 236 (M-H)⁻. |
| *Cap-97* | 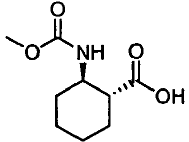 | LCMS: Anal. Calcd. for C₉H₁₅NO₄: 201; found: 200 (M-H)⁻. |
| *Cap-98* | 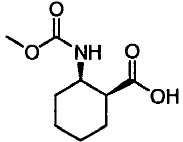 | LCMS: Anal. Calcd. for C₉H₁₅NO₄: 201; found: 202 (M+H)⁺. |
| *Cap-99* | 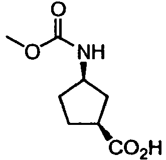 | ¹HNMR (400 MHz, CD₃OD) δ 3.88 - 3.94 (m, 1H), 3.60, 3.61 (s, 3H), 2.80 (m, 1H), 2.20 (m 1H), 1.82 -1.94 (m, 3H), 1.45 - 1.71 (m, 2H). |
| *Cap-99a* | 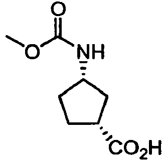 | ¹HNMR (400 MHz, CD₃OD) δ 3.88 - 3.94 (m, 1H), 3.60, 3.61 (s, 3H), 2.80 (m, 1H), 2.20 (m 1H), 1.82 -1.94 (m, 3H), 1.45 - 1.71 (m, 2H). |
| *Cap-100* | 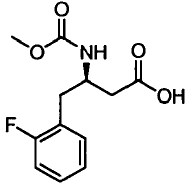 | LCMS: Anal. Calcd. for C₁₂H₁₄NO₄F: 255; found: 256 (M+H)⁺. |
| *Cap-101* | 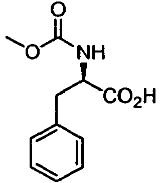 | LCMS: Anal. Calcd. for C₁₁H₁₃NO₄: 223; found: 222 (M-H)⁻. |
| *Cap-102* | 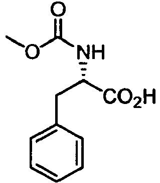 | LCMS: Anal. Calcd. for C₁₁H₁₃NO₄: 223; found: 222 (M-H)⁻ |
| *Cap-103* | 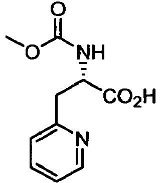 | LCMS: Anal. Calcd. for C₁₀H₁₂N₂O₄: 224; found: 225 (M+H)⁺. |
| *Cap-104* | 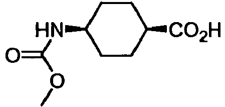 | ¹HNMR (400 MHz, CD₃OD) δ 3.60 (s, 3H), 3.50 - 3.53 (m, 1H), 2.66 - 2.69 and 2.44 -2.49 (m, 1H), 1.91 - 2.01 (m, 2H), 1.62 - 1.74 (m, 4H), 1.51 -1.62 (m, 2H). |
| *Cap-105* | 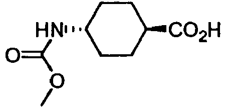 | ¹HNMR (400 MHz, CD₃OD) δ 3.60 (s, 3H), 3.33 - 3.35 (m, 1H, partially obscured by solvent), 2.37 - 2.41 and 2.16 - 2.23 (m, 1H), 1.94 -2.01 (m, 4H), 1.43 - 1.53 (m, 2H), 1.17 - 1.29 (m, 2H). |
| *Cap-106* | 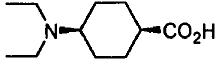 | ¹HNMR (400 MHz, CD₃OD) δ 3.16 (q, J = 7.3 Hz, 4H), 2.38 - 2.41 (m, 1H), 2.28 -2.31 (m, 2H), 1.79 - 1.89 (m, 2H), 1.74 (app, ddd J = 3.5, 12.5, 15.9 Hz, 2H), 1.46 (appdt *J* = 4.0, 12.9 Hz, 2H), 1.26 (t, *J* = 7.3 Hz, 6H). |
| *Cap-107* | 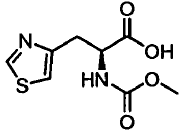 | LCMS: Anal. Calcd. for C₈H₁₀N₂O₄S: 230; found: 231 (M+H)⁺. |
| *Cap-108* | 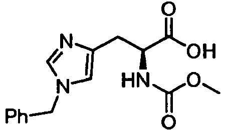 | LCMS: Anal. Calcd. for C₁₅H₁₇N₃O₄: 303; found: 304 (M+H)⁺. |
| *Cap-109* | 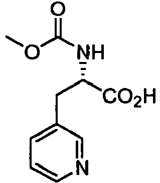 | LCMS: Anal. Calcd. for C₁₀H₁₂N₂O₄: 224; found: 225 (M+H)⁺. |
| *Cap-110* | 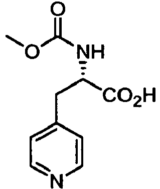 | LCMS: Anal. Calcd. for C₁₀H₁₂N₂O₄: 224; found: 225 (M+H)⁺. |
| *Cap-111* | 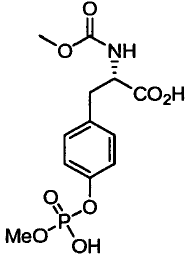 | LCMS: Anal. Calcd. for C₁₂H₁₆NO₈P: 333; found: 334 (M+H)⁺. |
| *Cap-112* | 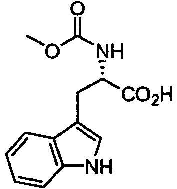 | LCMS: Anal. Calcd. for C₁₃H₁₄N₂O₄: 262; found: 263 (M+H)⁺. |
| *Cap-113* | 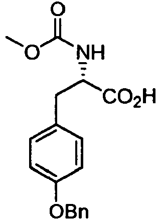 | LCMS: Anal. Calcd. for C₁₈H₁₉NO₅: 329; found: 330 (M+H)⁺. |
| *Cap-114* | 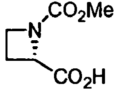 | ¹HNMR (400 MHz, CDCl₃) δ 4.82 - 4.84 (m, 1H), 4.00 -4.05 (m, 2H), 3.77 (s, 3H), 2.56 (s, br, 2H) |
| *Cap-115* | 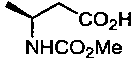 | ¹HNMR (400 MHz, CDCl₃) δ 5.13 (s, br, 1H), 4.13 (s, br, 1H), 3.69 (s, 3H), 2.61 (d, *J* = 5.0 Hz, 2H), 1.28 (d, *J* = 9.1 Hz, 3H). |
| *Cap-116* | 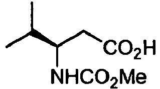 | ¹HNMR (400 MHz, CDCl₃) δ 5.10 (d, *J* = 8.6 Hz, 1H), 3.74 - 3.83 (m, 1H), 3.69 (s, 3H), 2.54 - 2.61 (m, 2H), 1.88 (sept, *J* = 7.0 Hz, 1H), 0.95 (d, *J* = 7.0 Hz, 6H). |

### Cap-117 to Cap-123

For the preparation of caps *Cap-117* to *Cap-123* the the Boc amino acids were commercially available and were deprotected by treatment with 25% TFA in CH₂Cl₂. After complete reaction as judged by LCMS the solvents were removed *in vacuo* and the corresponding TFA salt of the amino acid was carbamoylated with methyl chloroformate according to the procedure for *Cap-51.*

| Cap | Structure | LCMS |
|---|---|---|
| *Cap-117* | 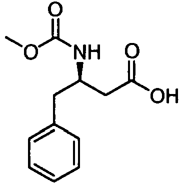 | LCMS: Anal. Calcd. for C₁₂H₁₅NO₄S: 237; found: 238 (M+H)⁺. |
| *Cap-118* | 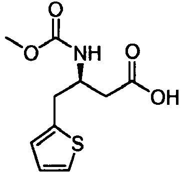 | LCMS: Anal. Calcd. for C₁₀H₁₃NO₄S: 243; found: 244 (M+H)⁺. |
| *Cap-119* | 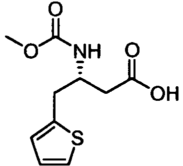 | LCMS: Anal. Calcd. for C₁₀H₁₃NO₄S: 243; found: 244 (M+H)⁺. |
| *Cap-120* | 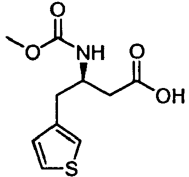 | LCMS: Anal. Calcd. for C₁₀H₁₃NO₄S: 243; found: 244 (M+H)⁺. |
| *Cap-121* | 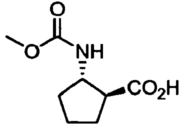 | ¹HNMR (400 MHz, CDCl₃) δ 4.06 - 4.16 (m, 1H), 3.63 (s, 3H), 3.43 (s, 1H), 2.82 and 2.66 (s, br, 1H), 1.86-2.10 (m, 3H), 1.64 - 1.76 (m, 2H), 1.44 -1.53 (m, 1H). |
| *Cap-122* | 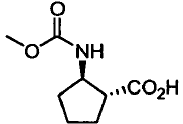 | ¹HNMR (400 MHz, CDCl₃) δ 5.28 and 5.12 (s, br, 1H), 3.66 (s, 3H), 2.64 - 2.74 (m, 1H), 1.86 - 2.12 (m, 3H), 1.67 -1.74 (m, 2H), 1.39 - 1.54 (m, 1H). |
| *Cap-123* | 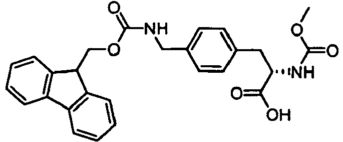 | LCMS: Anal. Calcd. for C₂₇H₂₆N₂O₆: 474; found: 475 (M+H)⁺. |

### Preparation of Cap-124 . (4S,5R)-5-methyl-2-oxooxazolidine-4-carboxylic acid

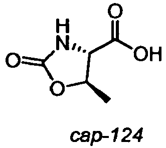

The hydrochloride salt of L-threonine tert-butyl ester was carbamoylated according to the procedure for *Cap-51.* The crude reaction mixture was acidified with 1N HCl to pH~1 and the mixture was extracted with EtOAc (2X50 mL). The combined organic phases were concentrated *in vacuo* to give a colorless which solidified on standing. The aqueous layer was concentrated *in vacuo* and the resulting mixture of product and inorganic salts was triturated with EtOAc-CH₂Cl₂-MeOH (1:1:0.1) and then the organic phase concentrated *in vacuo* to give a colorless oil which was shown by LCMS to be the desired product. Both crops were combined to give 0.52 g of a solid. ¹HNMR (400 MHz, CD₃OD) δ 4.60 (m, 1H), 4.04 (d, *J* = 5.0 Hz, 1H), 1.49 (d, *J* = 6.3 Hz, 3H). LCMS: Anal. Calcd. for C₅H₇NO₄: 145; found: 146 (M+H)⁺.

### Preparation of Cap-125. (S)-2-(tert-butoxycarbonylamino)-4-(dimethylamino)butanoic acid.

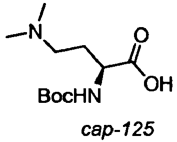

*Cap-125* was prepared according to the procedure for the preparation of *Cap-1.* The crude product was used as is in subsequent reactions. LCMS: Anal. Calcd. for C₁₁H₂₂N₂O₄: 246; found: 247 (M+H)⁺.

### Preparation of (S)-2-(methoxycarbonylamino)-3-(1-methyl-1H-imidazol-2-yl)propanoic acid (Cap-126).

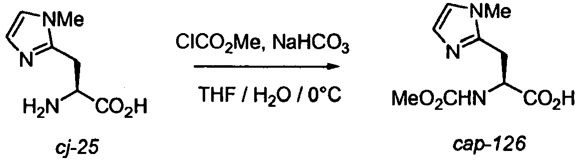

This procedure is a modification of that used to prepare *Cap-51.* To a suspension of (S)-2-amino-3-(1-methyl-1H-imidazol-2-yl)propanoic acid (0.80 g, 4.70 mmol) in THF (10mL) and H₂O (10 mL) at 0°C was added NaHCO₃ (0.88 g, 10.5 mmol). The resulting mixture was treated with ClCO₂Me (0.40 mL, 5.20 mmol) and the mixture allowed to stir at 0°C. After stirring for ca. 2h LCMS showed no starting material remaining. The reaction was acidified to pH 2 with 6 N HCl.

The solvents were removed *in vacuo* and the residue was suspended in 20 mL of 20% MeOH in CH₂Cl₂. The mixture was filtered and concentrated to give a light yellow foam (1.21 g,). LCMS and ¹H NMR showed the material to be a 9:1 mixture of the methyl ester and the desired product. This material was taken up in THF (10mL) and H₂O (10mL), cooled to 0°C and LiOH (249.1 mg, 10.4 mmol) was added. After stirring ca. 1h LCMS showed no ester remaining. Therefore the mixture was acidified with 6N HCl and the solvents removed *in vacuo.* LCMS and ¹H NMR confirm the absence of the ester. The title compound was obtained as its HCl salt contaminated with inorganic salts (1.91 g, >100%). The compound was used as is in subsequent steps without further purification.
¹HNMR (400 MHz, CD₃OD) δ 8.84, (s, 1H), 7.35 (s, 1H), 4.52 (dd, *J* = 5.0, 9.1 Hz, 1H), 3.89 (s, 3H), 3.62 (s, 3H), 3.35 (dd, *J* = 4.5, 15.6 Hz, 1H, partially obscured by solvent), 3.12 (dd, *J* = 9.0, 15.6 Hz, 1H).
LCMS: Anal. Calcd. for C₁₇H₁₅NO₂: 392; found: 393 (M+H)⁺.

### Preparation of (S)-2-(methoxycarbonylamino)-3-(1-methyl-1H-imidazol-4-yl)propanoic acid (Cap-127).

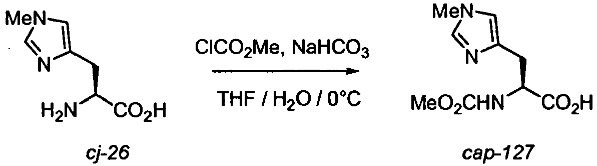

*Cap*-127 was prepared according to the method for *Cap-*126 above starting from (S)-2-amino-3-(1-methyl-1H-imidazol-4-yl)propanoic acid (1.11 g, 6.56 mmol), NaHCO₃ (1.21 g, 14.4 mmol) and ClCO₂Me (0.56 mL, 7.28 mmol). The title compound was obtained as its HCl salt (1.79 g, >100%) contaminated with inorganic salts. LCMS and ¹H NMR showed the presence of ca. 5% of the methyl ester. The crude mixture was used as is without further purification.
¹HNMR (400 MHz, CD₃OD) δ 8.90 (s, 1H), 7.35 (s, 1H), 4.48 (dd, *J* = 5.0, 8.6 Hz, 1H), 3.89 (s, 3H), 3.62 (s, 3H), 3.35 (m, 1H), 3.08 (m, 1H).
LCMS: Anal. Calcd. for C₁₇H₁₅NO₂: 392; found: 393 (M+H)⁺.

### Preparation of (S)-2-(methoxycarbonylamino)-3-(1H-1,2,3-triazol-4-yl)propanoic acid (Cap-128).

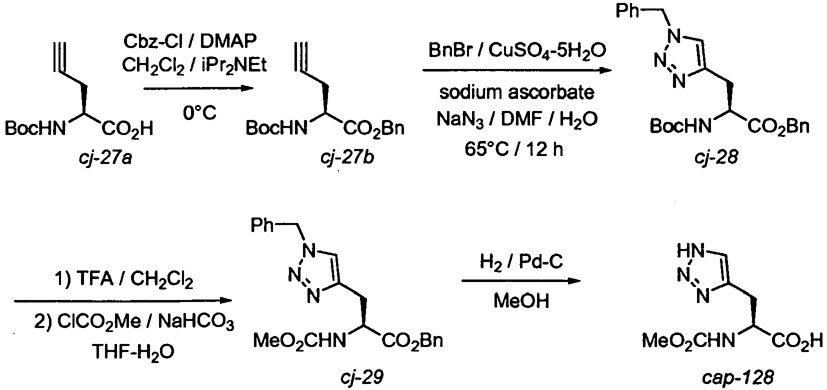

### Step 1. Preparation of (S)-benzyl 2-(tert-butoxycarbonylamino)pent-4-ynoate (cj-27b).

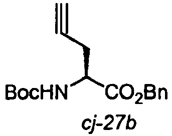

To a solution of cj-27a (1.01 g, 4.74 mmol), DMAP (58 mg, 0.475 mmol) and iPr₂NEt (1.7 mL, 9.8 mmol) in CH₂Cl₂ (100 mL) at 0°C was added Cbz-Cl (0.68 mL, 4.83 mmol). The solution was allowed to stir for 4 h at 0°C, washed (IN KHSO₄, brine), dried (Na₂SO₄) filtered, and concentrated *in vacuo.* The residue was purified by flash column chromatography (TLC 6:1 hex:EtOAc) to give the title compound (1.30 g, 91%) as a colorless oil. ¹HNMR (400 MHz, CDCl₃) δ 7.35 (s, 5H), 5.35 (d, br, *J* = 8.1 Hz, 1H), 5.23 (d, *J* = 12.2 Hz, 1H), 5.17 (d, *J* = 12.2 Hz, 1H), 4.48 - 4.53 (m, 1H), 2.68 - 2.81 (m, 2H), 2.00 (t, *J* = 2.5 Hz, 1H), 1.44 (s, 9H). LCMS: Anal. Calcd. for C₁₇H₂₁NO₄: 303; found: 304 (M+H)⁺.

### Step 2. Preparation of (S)-benzyl 3-(1-benzyl-1H-1,2,3-triazol-4-yl)-2-(tert-butoxycarbonylamino)propanoate (cj-28).

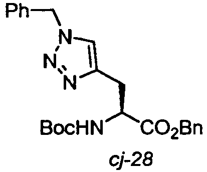

To a mixture of (S)-benzyl 2-(tert-butoxycarbonylamino)pent-4-ynoate (0.50 g, 1.65 mmol), sodium ascorbate (0.036 g, 0.18 mmol), CuSO₄-5H₂O (0.022 g, 0.09 mmol) and NaN₃ (0.13 g, 2.1 mmol) in DMF-H₂O (5 mL, 4:1) at rt was added BnBr (0.24 mL, 2.02 mmol) and the mixture was warmed to 65°C . After 5h LCMS indicated low conversion. A further portion of NaN₃ (100 mg) was added and heating was continued for 12h. The reaction was poured into EtOAc and H₂O and shaken. The layers were separated and the aqueous layer extracted 3x with EtOAc and the combined organic phases washed (H₂O x3, brine), dried (Na₂SO₄), filtered, and concentrated. The residue was purified by flash (Biotage, 40+M 0-5% MeOH in CH₂Cl₂; TLC 3% MeOH in CH₂Cl₂) to afford a light yellow oil which solidified on standing (748.3 mg, 104%). The NMR was consistent with the desired product but suggests the presence of DMF. The material was used as is without further purification. ¹HNMR (400 MHz, DMSO-d₆) δ 7.84 (s, 1H), 7.27 -7.32 (m, 10H), 5.54 (s, 2H), 5.07 (s, 2H), 4.25 (m, 1H), 3.16 (dd, *J* = 1.0, 5.3 Hz, 1H), 3.06 (dd, *J =* 5.3, 14.7 Hz), 2.96 (dd, *J* = 9.1, 14.7 Hz, 1H), 1.31 (s, 9H).
LCMS: Anal. Calcd. for C₂₄H₂₈N₄O₄: 436; found: 437 (M+H)⁺.

### Step 2. Preparation of (S)-benzyl 3-(1-benzyl-1H-1,2,3-triazol-4-yl)-2-(methoxycarbonylamino)propanoate (cj-29).

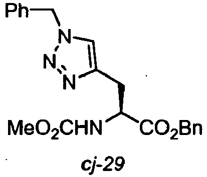

A solution of (S)-benzyl 3-(1-benzyl-1H-1,2,3-triazol-4-yl)-2-(tert-butoxycarbonylamino)propanoate (0.52 g, 1.15 mmol) in CH₂Cl₂ was added TFA (4 mL). The mixture was allowed to stir at room temperature for 2h. The mixture was concentrated *in vacuo* to give a colorless oil which solidified on standing. This material was dissolved in THF-H₂O and cooled to 0°C. Solid NaHCO₃ (0.25 g, 3.00 mmol) was added followed by ClCO₂Me (0.25 mL, 3.25 mmol). After stirring for 1.5h the mixture was acidified to pH~2 with 6N HCl and then poured into H₂O-EtOAc. The layers were separated and the aq phase extracted 2x with EtOAc. The combined org layers were washed (H₂O, brine), dried (Na₂SO₄), filtered, and concentrated *in vacuo* to give a colorless oil (505.8 mg, 111%, NMR suggested the presence of an unidentified impurity) which solidified while standing on the pump. The material was used as is without further purification. ¹HNMR (400 MHz, DMSO-d₆) δ 7.87 (s, 1H), 7.70 (d, *J* = 8.1 Hz, 1H), 7.27 - 7.32 (m, 10H), 5.54 (s, 2H), 5.10 (d, *J* = 12.7 Hz, 1H), 5.06 (d, *J* = 12.7 Hz, 1H), 4.32 - 4.37 (m, 1H), 3.49 (s, 3H), 3.09 (dd, *J* = 5.6, 14.7 Hz, 1H), 2.98 (dd, *J* = 9.6, 14.7 Hz, 1H). LCMS: Anal. Calcd. for C₂₁H₂₂N₄O₄: 394; found: 395 (M+H)⁺.

### Step 3. Preparation of (S)-2-(methoxycarbonylamino)-3-(1H-1,2,3-triazol-4-yl)propanoic acid (Cap-128).

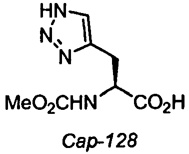

(S)-benzyl 3-(1-benzyl-1H-1,2,3-triazol-4-yl)-2-(methoxycarbonylamino)propanoate (502 mg, 1.11 mmol) was hydrogenated in the presence of Pd-C (82 mg) in MeOH (5 mL) at atmospheric pressure for 12h. The mixture was filtered through diatomaceous earth (Celite^{®}) and concentrated *in vacuo.* (S)-2-(methoxycarbonylamino)-3-(1H-1,2,3-triazol-4-yl)propanoic acid was obtained as a colorless gum (266 mg, 111%) which was contaminated with ca. 10% of the methyl ester. The material was used as is without further purification.
¹HNMR (400 MHz, DMSO-d₆) δ 12.78 (s, br, 1H), 7.59 9s, 1H), 7.50 (d, *J* = 8.0 Hz, 1H), 4.19 - 4.24 (m, 1H), 3.49 (s, 3H), 3.12 (dd, *J* = 4.8 Hz, 14.9 Hz, 1H), 2.96 (dd, *J* = 9.9, 15.0 Hz, 1H). LCMS: Anal. Calcd. for C₇H₁₀N₄O₄: 214; found: 215 (M+H)⁺.

### Preparation of (S)-2-(methoxycarbonylamino)-3-(1H-pyrazol-1-yl)propanoic acid (Cap-129).

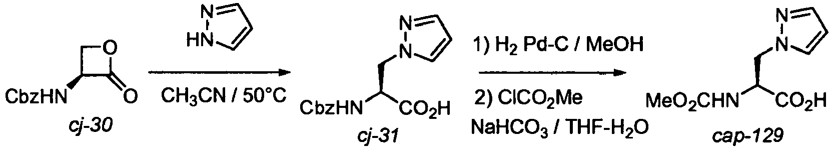

### Step 1. Preparation of (S)-2-(benzyloxycarbonylamino)-3-(1H-pyrazol-1-yl)propanoic acid (cj-31).

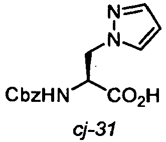

A suspension of (S)-benzyl 2-oxooxetan-3-ylcarbamate (0.67 g, 3.03 mmol), and pyrazole (0.22 g, 3.29 mmol) in CH₃CN (12 mL) was heated at 50°C for 24h. The mixture was cooled to rt overnight and the solid filtered to afford (S)-2-(benzyloxycarbonylamino)-3-(1H-pyrazol-1-yl)propanoic acid (330.1 mg). The filtrate was concentrated *in vacuo* and then triturated with a small amount of CH₃CN (ca. 4 mL) to afford a second crop (43.5 mg). Total yield 370.4 mg (44%).
m.p. 165.5 - 168°C. lit m.p. 168.5 - 169.5 Vederas et al. J. Am. Chem. Soc. 1985,107, 7105.
¹HNMR (400 MHz, CD₃OD) δ 7.51 1 (d, *J* = 2.0, 1H), 7.48 (s, *J* = 1.5 Hz, 1H), 7.24 - 7.34 (m, 5H), 6.23 m, 1H), 5.05 (d, 12.7 H, 1H), 5.03 (d, *J* = 12.7 Hz, 1H), 4.59 - 4.66 (m, 2H), 4.42 - 4.49 (m, 1H). LCMS: Anal. Calcd. for C₁₄H₁₅N₃O₄: 289; found: 290 (M+H)⁺.

### Step 2. Preparation of (S)-2-(methoxycarbonylamino)-3-(1H-pyrazol-1-yl)propanoic acid (Cap-129).

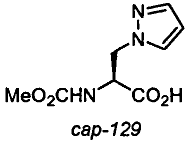

(S)-2-(benzyloxycarbonylamino)-3-(1H-pyrazol-1-yl)propanoic acid (0.20 g, 0.70 mmol) was hydrogenated in the presence of Pd-C (45 mg) in MeOH (5 mL) at atmospheric pressure for 2h. The product appeared to be insoluble in MeOH, therefore the rxn mixture was diluted with 5mL H₂O and a few drops of 6N HCl. The homogeneous solution was filtered through diatomaceous earth (Celite^{®}), and the MeOH removed *in vacuo.* The remaining solution was frozen and lyophyllized to give a yellow foam (188.9 mg). This material was suspended in THF-H₂O (1:1, 10mL) and then cooled to 0°C. To the cold mixture was added NaHCO₃ (146.0 mg, 1.74 mmol) carefully (evolution of CO₂). After gas evolution had ceased (ca. 15 min) ClCO₂Me (0.06 mL, 0.78 mmol) was added dropwise. The mixture was allowed to stir for 2h and was acidified to pH-2 with 6N HCl and poured into EtOAc. The layers were separated and the aqueous phase extract with EtOAC (x5).

The combined organic layers were washed (brine), dried (Na₂SO₄), filtered, and concentrated to give the title compound as a colorless solid (117.8 mg, 79%). ¹HNMR (400 MHz, DMSO-d₆) δ 13.04 (s, 1H), 7.63 (d, *J* = 2.6 Hz, 1H), 7.48 (d, *J* = 8.1 Hz, I H), 7.44 (d, *J =* 1.5 Hz, 1H), 6.19 (app t, *J* = 2.0 Hz, 1H), 4.47 (dd, *J* = 3.0, 12.9 Hz, 1H), 4.29 - 4.41 (m, 2H), 3.48 (s, 3H). LCMS: Anal. Calcd. for C₈H₁₁N₃O₄: 213; found: 214 (M+H)⁺.

### Cap-130. N-Acetyl -(R)-Phenylglycine

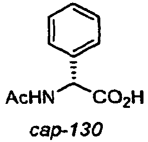

*Cap-130* was prepared by acylation of commercially available (R)-phenylglycine analgous to the procedure given in: Calmes, M.; Daunis, J.; Jacquier, R.; Verducci, J. Tetrahedron, 1987, 43(10), 2285.

### EXAMPLES

The present disclosure will now be described in connection with certain embodiments which are not intended to limit its scope. Thus, the following examples, which include specific embodiments, will illustrate one practice of the present disclosure, it being understood that the examples are for the purposes of illustration of certain embodiments and are presented to provide what is believed to be the most useful and readily understood description of its procedures and conceptual aspects.

Solution percentages express a weight to volume relationship, and solution ratios express a volume to volume relationship, unless stated otherwise. Nuclear magnetic resonance (NMR) spectra were recorded either on a Bruker 300, 400, or 500 MHz spectrometer; the chemical shifts (δ) are reported in parts per million. Flash chromatography was carried out on silica gel (SiO₂) according to Still's flash chromatography technique (J. Org. Chem. 1978, 43, 2923*).*

Purity assessment and low resolution mass analysis were conducted on a Shimadzu LC system coupled with Waters Micromass ZQ MS system. It should be noted that retention times may vary slightly between machines. The LC conditions employed in determining the retention time (RT) were:

| *Condition 1* | |
|---|---|
| Column | = Phenomenex-Luna 3.0X 50 mm S 10 |
| Start %B | = 0 |
| Final %B | = 100 |
| Gradient time | = 2 min |
| Stop time | = 3 min |
| Flow Rate | = 4 mL/min |
| Wavelength | = 220 nm |
| Solvent A | = 0.1% TFA in 10% methanol/90%H₂O |
| Solvent B | = 0.1% TFA in 90% methanol/10% H₂O |

| *Condition 2* | |
|---|---|
| Column | = Phenomenex-Luna 4.6X50 mm S 10 |
| Start %B | = 0 |
| Final %B | = 100 |
| Gradient time | = 2 min |
| Stop time | = 3 min |
| Flow Rate | = 5 mL/min |
| Wavelength | = 220 nm |
| Solvent A | = 0.1% TFA in 10% methanol/90%H₂O |
| Solvent B | = 0.1% TFA in 90% methanol/10% H₂O |

| *Condition 3* | |
|---|---|
| Column | = HPLC XTERRA C18 3.0 x 50mm S7 |
| Start %B | = 0 |
| Final %B | = 100 |
| Gradient time | = 3 min |
| Stop time | = 4 min |
| Flow Rate | = 4 mL/min |
| Wavelength | = 220 nm |
| Solvent A | = 0.1% TFA in 10% methanol/90%H₂O |
| Solvent B | = 0.1% TFA in 90% methanol/10% H₂O |

Method A: LCMS - Xterra MS C-18 3.0 x 50mm, 0 to 100% B over 30.0 minute gradient, 1 minute hold time, A = 5% acetonitrile, 95% water, 10mm ammonium acetate, B = 95% acetonitrile, 5% water, 10mm ammonium acetate.

Method B: HPLC - X-Terra C-18 4.6 x 50mm, 0 to 100% B over 10.0 minute gradient, 1 minute hold time, A = 10% methanol 90% water 0.1% TFA, B = 90% methanol 10% water 0.1% TFA

Method C: HPLC - YMC C-18 4.6 x 50mm, 0 to 100% B over 10.0 minute gradient, 1 minute hold time, A = 10% methanol 90% water 0.2% H₃PO₄, B = 90% methanol 10% water 0.2% H₃PO₄.

Method D: HPLC - Phenomenex C-18 4.6 x 150mm, 0 to 100% B over 10.0 minute gradient, 1 minute hold time, A = 10% methanol 90% water 0.2% H₃PO₄, B = 90% methanol 10% water 0.2% H₃PO₄

Method E: LCMS - Gemini C-18 4.6 x 50mm, 0 to 100% B over 10.0 minute gradient, 1 minute hold time, A = 5% acetonitrile, 95% water, 10mm ammonium acetate, B = 95% acetonitrile, 5% water, 10mm ammonium acetate.

Method F: LCMS-Luna C-18 3.0 x 50mm, 0 to 100% B over 7.0 minute gradient, 1 minute hold time, A = 5% acetonitrile, 95% water, 10mm ammonium acetate, B = 95% acetonitrile, 5% water, 10mm ammonium acetate.

### Example M132

### methyl ((1S)-2-((1R,3S,5R)-3-(4-(4'-(2-((1R,3S,SR)-2-(N(methoxycarbonyl)-L-alanyl)-2-azabicyclo[3.1.0]hex-3-yl)-1H-imidazol-4-yl)-4-biphenyl)-1H-imidazol-2-yl)-2-azabicyclo[3.1.0]hex-2-yl)-1-methyl -2-oxoethyl)carbamate

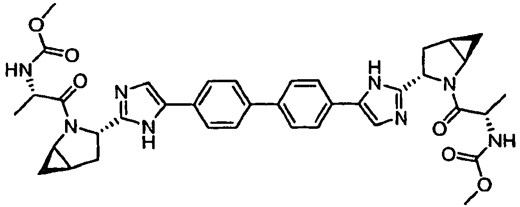

### Example M132, Step a

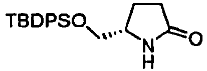

To a solution of (S)-5-(hydroxymethyl)pyrrolidin-2-one (10g, 87 mmol) in CH₂Cl₂ (50 mL) was added tert-butylchlorodiphenylsilane (25.6 g, 93 mmol), triethylamine (12.1 mL, 87 mmol) and DMAP (1.06 g, 8.7 mmol). The mixture was stirred at room temperature for 5 hours, treated with CH₂Cl₂ (50 mL) and washed with water (50 mL). The organic layer was dried (Na₂SO₄), filtered, and concentrated *in vacuo.* The crude product was purified with a flash chromatography (30 to 100% of EtOAc/hexanes) to afford ether M 132a as colorless oil (22.7 g, 74% yield). ¹H NMR (DMSO-d₆, δ = 2.50, 400 MHz): 7.69 (br s, 1H), 7.64-7.61 (m, 4H), 7.50-7.42 (m, 6H), 3.67-3.62 (m, 1H), 3.58-3.51 (m, 2H), 2.24-2.04 (m, 3H), 1.89-1.78 (m, 1H), 1.00 (s, 9H).

### Example M132, Step b

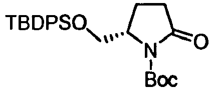

Di-*tert*-butyl dicarbonate (28.0 g, 128 mmol) was added slowly to a cooled (ice/water) CH₂Cl₂ (120 mL) solution of ether M132a (22.7 g, 64.2 mmol), triethylamine (8.95 mL, 64.2 mmol), and DMAP (7.84 g, 64.2 mmol). At the end of addition, the cooling bath was removed and stirring continued at ambient condition for 20 hours. The volatile component was removed *in vacuo,* and the crude material was submitted to a flash chromatography (20 to 50 % EtOAc/hexanes) to afford carbamate M132b as off-white solid (29 g, 99% yield). ¹H NMR (DMSO-d₆, δ = 2.50, 400 MHz): 7.61-7.54 (m, 4H), 7.50-7.38 (m, 6H), 4.19-4.16 (m, 1H), 3.90 (dd, J = 10.4, 3.6, 1H), 3.68 (dd, J = 10.4, 2.1, 1H), 2.68-2.58 (m, 1H), 2.40-2.33 (m, 1H), 2.22-2.12 (m, 1H), 2.01-1.96 (m, 1H), 1.35 (s, 9H), 0.97 (s, 9H).

### Example M132, Step c

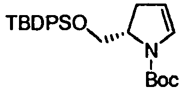

A three-necked flask equipped with a thermometer and a nitrogen inlet was charged with carbamate M132b (10.054 g, 22.16 mmol) and toluene (36 mL), and lowered into -55 °C cooling bath. When the internal temperature of the mixture reached ∼ -50 °C, Superhydride (23 mL of 1.0 M in THF, 23.00 mmol) was added dropwise over 30 minutes while maintaining the internal temperature between -50 and -45 °C, and stirred for 35 minutes while maintaining the temperature between -50 and -45 °C. Hunig's Base (16.5 mL, 94 mmol) was added dropwise over 10 minutes. Then DMAP (34 mg, 0.278 mmol) was added in one batch, followed by the addition of trifluoroacetic anhydride (3.6 mL, 25.5 mmol) over 15 minutes, while maintaining the internal temperature between -50 and -45 °C. The bath was removed 10 minutes later, and the reaction mixture was stirred for 14 hours while allowing it to thaw to ambient temperature. It was then diluted with toluene (15 mL), cooled with ice-water bath, and treated slowly with water (55 mL) over 5 minutes. At the end of addition, the phases were separated, and the organic layer was washed with water (50 mL, 2x) and then concentrated *in vacuo.* The crude material was purified with a flash chromatography (5% EtOAc/hexanes) to afford carbamate M132c as a colorless viscous oil (7.947 g, 82%). ¹H NMR (DMSO-d₆, δ = 2.50, 400 MHz): 7.62-7.58 (m, 4H), 7.49-7.40 (m, 6H), 6.47 (br m, 1H), 5.07-5.01 (br m, 1H), 4.18 (br m, 1H), 3.89 (br m, 0.48H), 3.69 (br m, 1.52 H), 2.90-2.60 (br m, 2H), 1.40/1.26 (two overlapping s, 9H), 0.98 (s, 9H).

### Example M132, Step d

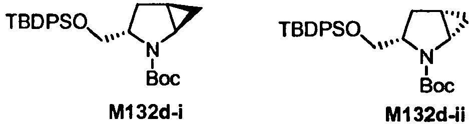

Diethylzinc (19 mL of∼1.1 M in toluene, 20.90 mmol) was added dropwise over 15 minutes to a cooled (-30 °C) toluene (27 mL) solution of carbamate M132c (3.94 g, 9.0 mmol). Then chloroiodomethane (97%, stabilized over copper; 3 mL, 41.2 mmol) was added dropwise over 10 minutes, and stirred while maintaining the bath temperature around -25 °C for 1 hour and around -21 °C for 18.5 hours. The reaction was then opened to air and quenched by a slow addition of 50% saturated NaHCO₃ solution (40 mL), and then removed from the cooling bath and stirred at ambient condition for 20 minutes. It was filtered through a filter paper and the white cake was washed with 50 mL of toluene. The organic phase of the filtrate was separated, and washed with water (40 mL, 2x), dried (MgSO₄), filtered, and concentrated *in vacuo.* The resulting crude material was purified with a Biotage system (350 g silica gel; sample was loaded with 7% EtOAc/hexanes; eluted with 7-20% EtOAc/hexanes) to afford methanopyrrolidine M132d as a colorless viscous oil, mainly as the trans isomer (3.691 g, 90.7%). [Note: the exact trans/cis ratio has not been determined yet at this stage]. ¹H NMR (DMSO-d₆, δ = 2.50, 400 MHz) of M132d-i: 7.62-7.60 (m, 4H), 7.49-7.40 (m, 61-17, 3.76 (br m, 1H), 3.67 (br m, 2H), 3.11-3.07 (m, 1H), 2.23 (br m, 1H), 2.03 (br m, 1H), 1.56-1.50 (m, 1H), 1.33 (br s, 9H), 1.00 (s, 9H), 0.80-0.75 (m, 1H), 0.30 (br m, 1H).

### Example M132, Step e

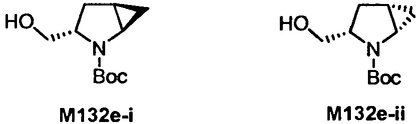

TBAF (7.27 mL of 1.0 M in THF, 7.27 mmol) was added dropwise over 5 minutes to a THF (30 mL) solution of the ether M132d (3.13 g, 6.93 mmol) and the mixture was stirred at ambient condition for 4.75 hours. After it was treated with saturated NH₄Cl solution (5 mL), most of the volatile component was removed *in vacuo,* and the residue was partitioned between CH₂Cl₂ (70 mL) and 50% saturated NH₄Cl solution (30 mL). The aqueous phase was extracted with CH₂Cl₂ (30 mL), and the combined organic phase was dried (MgSO₄), filtered, concentrated *in vacuo,* and then exposed to high vacuum overnight. The resulting crude material was purified with a Biotage (40-50% EtOAc/hexanes) to afford alcohol M132e as colorless oil, mainly as the trans isomer, contaminated with traces of lower Rf impurities (1.39 g, 94%). [Note: the exact trans/cis ratio has not been determined yet at this stage]. ¹H NMR (DMSO-d₆, δ = 2.50, 400 MHz) of M132e-i: 4.70 (app t, J = 5.7, 1H), 3.62-3.56 (m, 1H), 3.49-3.44 (m, 1H), 3.33-3.27 (m, 1H), 3.08-3.04 (m, 1H), 2.07 (br m, 1H), 1.93-1.87 (m, 1H), 1.51-144 (m, 1H), 1.40 (s, 9H), 0.76-0.71 (m, 1H), 0.26 (br m, 1H).

### Example M132, Step f

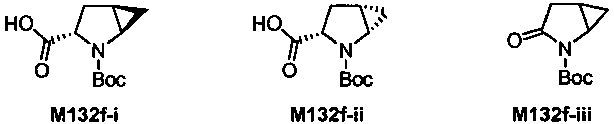

A semi-solution of NaIO₄ (6.46 g, 30.2 mmol) in H₂O (31 mL) was added to CH₃CN (20 mL) and CCl₄ (20 mL) solution of alcohol M132e (2.15 g, 10.08 mmol) prepared above, and RuCl₃ (0.044 g, 0.212 mmol) was added immediately and the heterogeneous reaction mixture was vigorously stirred for 75 minutes. The reaction mixture was diluted H₂O (60 mL) and extracted with CH₂Cl₂ (50 mL, 3x). The combined organic phase was treated with 1 mL CH₃OH, allowed to stand for about 5 minutes, and then filtered through a pad of diatomaceous earth (Celite^{®}). The pad was washed with CH₂Cl₂ (50 mL), and the filtrate was rotervaped to afford a light charcoal-colored solid. ¹H NMR of the crude material indicated a 1.00: 0.04 : 0.18 mole ratio among *trans* acid M132f-i: presumed *cis* acid M132f-ii: side product M132f-iii. The crude material was dissolved in EtOAc (∼10 mL) with heating, and allowed to stand at ambient condition with seeding. About 15 minutes into the cooling phase, a rapid crystal formation was observed. About 1 hour later, hexanes (∼6 mL) was added and the mixture was refrigerated overnight (it does not appear that additional compound has precipitated out). The mixture was filtered and washed with ice/water cooled hexanes/EtOAc (2:1 ratio; 20 mL) and dried under high vacuum to afford the first crop of acid M132f-i (off-white crystals, 1.222 g). The mother liquor was rotervaped, and the residue was dissolved in ∼3 ml of EtOAc (with heating), allowed to stand at ambient condition for 1 hour, and then 3 mL hexanes was added and stored in a refrigerator for ∼15 hours. A second crop of acid M132f-i (grey crystals, 0.133 g) was retrieved similarly. ¹H NMR (DMSO-d₆, δ = 2.50, 400 MHz): 12.46 (br s, 1H), 3.88 (br m, 1H), 3.27 (br m, 1H; partially overlapped with the signal of water), 2.28 (br m, 1H), 2.08 (br m, 1H), 1.56 (br m, 1H), 1.40/1.34 (two overlapped br s, 9H), 0.73-0.68 (m, 1H), 0.46-0.43 (m, 1H). Optical rotation (10 mg/mL of CHCl₃): [α]_{D} = -216 for first crop & -212 for the second crop.

### Example M132, Step g

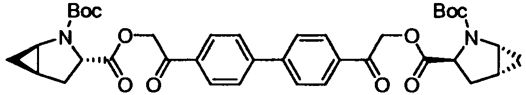

Ketoester M132g was prepared from acid M132f-i and 1,1'-(biphenyl-4,4'-diyl)bis(2-bromoethanone) by employing the procedure described for the preparation of ketoester M122a. LC (Cond. I): RT = 2.09 minutes. LC/MS: Anal. Calcd. for [M+H-Boc]⁺ C₃₃H₃₇N₂O₈: 589.26; found 589.29.

### Example M132, Step h

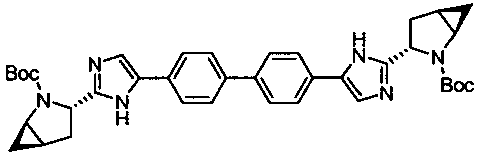

Carbamate M132h was prepared from ketoester M132g according to the procedure described for the preparation of imidazole 1b from ketoamide 1a, with the exception that 20 mol equiv of NH₄OAc was employed for the thermal cyclization, and that CH₂Cl₂ was employed during the work up step. LC (Cond. I): RT = 1.48 minutes. LC/MS: Anal. Calcd. for [M+H]⁺ C₃₈H₄₅N₆O₄: 649.35; found 649.40.

### Example M132, Step i

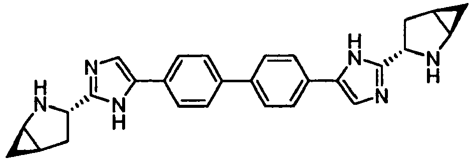

Pyrrolidine M132i (.4HCl) was prepared from carbamate M132h according to the procedure described for the preparation of pyrrolidine M122c. The crude material was submitted to subsequent acylation step without purification. ¹H NMR (DMSO-d₆, δ = 2.50, 400 MHz): 10.5-10.0 (br signal, not integratable), 8.02 (s, 2H), 7.95 (d, J = 8.6, 4H), 7.85 (d, J = 8.3, 4H), 4.75 (m, 2H), 3.43 (m, 2H), 2.67-2.50 (m, 4H), 1.95 (m, 2H), 1.11 (m, 2H), 0.86 (m, 2H). RT = 1.00 minutes. LC/MS: Anal. Calcd. for [M+H]⁺ C28H20N6: 449.25 ; found 49.27.

### Example M132

Example M132, along with its analogs Example M133-M137 highlighted in the table below, were prepared as TFA salts from pyrrolidine M132i (.4HCl) by employing the procedure described for the synthesis of Example M122 and appropriate acids. Example M132: LC (Cond. I): RT = 1.14 min; >95% homogeneity index. LC/MS: Anal. Calcd. for [M+H]⁺ C₃₈H₄₃N₈O₆: 707.33; found 707.43.

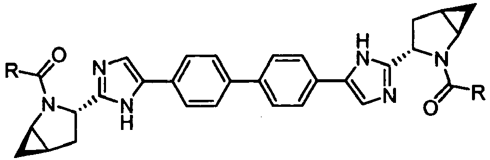

| *Example* | *Compound Name* | 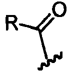 | *RT (LC-Cond.); % homogeneity index; MS data* |
|---|---|---|---|
| *M133* | dimethyl (4,4'-biphenyldiylbis(1H-imidazole-4,2-diyl(1R,3S,5R)-2-azabicyclo[3.1.0]hexane-3,2-diyl((2S)-1-oxo-1,2-butanediyl)))biscarbamate | 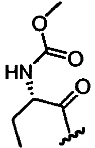 | 1.22 minutes (Cond. I); >98%; LC/MS: Anal. Calcd. for [M+H]⁺ C₄₀H₄₇N₈O6: 735.36; found 735.48 |
| *M134* | methyl (2-((1R,3S,SR)-3-(4-(4'-(2-((1R,3S,SR)-2-(((methoxycarbonyl)amino)acetyl)-2-azabicyclo[3.1.0]hex-3-yl)-1H-imidazol-4-yl)-4-biphenylyl)-1H-imidazol-2-yl)-2-azabicyclo[3.1.0]hex-2-yl)-2-oxoethyl) carbamate | 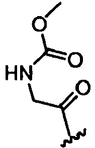 | 1.12 minutes (Cond. I); >98%; LC/MS: Anal. Calcd. for [M+H]⁺ C₃₆H₃₉N₈O₆: 679.30; found 679.38 |
| *M135* | dimethyl (4,4'-biphenyldiylbis(1H-imidazole-4,2-diyl(1R,3S,5R)-2-azabicyclo[3.1.0]hexane-3,2-diyl((1S)-1-cyclopropyl-2-oxo-2,1-ethanediyl)))biscarbamate | 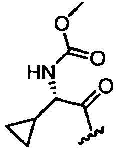 | 1.25 minutes (Cond. I); >98%; LC/MS: Anal. Calcd. for [M+H]⁺ C₄₂H₄₇N₈O₆: 759.36; found 759.45 |
| *M136* | methyl ((1R)-1-(((1R,3S,5R)-3-(4-(4'-(2-((1R,3S,5R)-2-((2R)-2-((methoxycarbonyl)amino)-3-methylbutanoyl)-2-azabicyclo[3.1.0]hex-3-yl)-1H-imidazol-4-yl)-4-biphenylyl)-1H-imidazol-2-yl)-2-azabicyclo[3.1.0]hex-2-yl)carbonyl)-2-methylpropyl)carbamate | 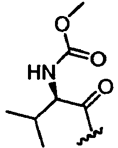 | 1.34 minutes (Cond. I); >98%; LC/MS: Anal. Calcd. for [M+H]⁺ C₄₂H₅₀N₈O₆: 763.39; found 763.46 |
| *M137* | dimethyl (4,4'-biphenyldiylbis(1H-imidazole-4,2-diyl(1R,3S,SR)-2-azabicyclo[3.1.0]hexane-3,2-diyl((1R)-2-oxo-1-phenyl-2,1-ethanediyl)))biscarbamate | 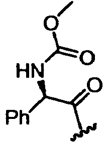 | 1.38 minutes (Cond. I); >98%; LC/MS:Anal. Calcd. for [M+H]⁺ C₄₈H₄₇N₈O₆: 831.36; found 831.37 |

### Example M138

### methyl ((1S)-1-(((1R,3S,5R)-3-(4-(4'-(2-((1R,3S,SR)-2-acetyl-2-azabicyclo[3.1.0]hex-3yl)-1Himidazol-4yl)-4-biphenylyl)-1Himidazol-2-yl)-2-azabicyclo[3.1.0]hex-2-yl)carbonyl)-2-methylpropyl)carbamate

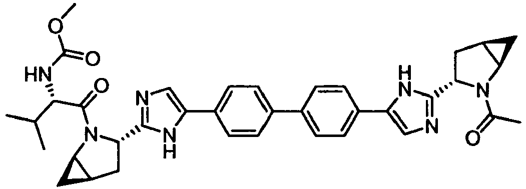

### Example M138, Step a

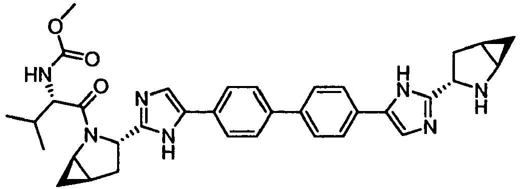

To a slurry of pyrrolidine M132i (.4HCl) (0.3 g, 0.32 mmol) in DMF (4 mL), (*S*)-2-(methoxycarbonylamino)-3-methylbutanoic acid (97 mg, 0.55 mmol) and *i-*Pr₂EtN (0.26 mL, 1.5 mmol) were added. After the mixture became a clear solution, HATU (0.2 g, 0.53 mmol) was added, and it was stirred at room temperature for 3 hours. The volatile component was removed *in vacuo,* and the resulting residue (which was a mixture of starting material, and mono- and bis-acylated products) was dissolved in methanol and purified with a reverse phase HPLC (CH₃OH/H₂O/TFA) to isolate the TFA salt of pyrrolidine M138a as white foam (80 mg). LC (Cond. I): RT = 1.17 minutes. LC/MS: Anal. Calcd. for [M+H]⁺ C₃₅H₄₀N₇O₃: 606.32; found 606.36.

### Example M138

Acetic acid (7.6 mg, 0.13 mmol), i-Pr₂EtN (32 mg, 0.25 mmol), and HATU (53 mg, 0.14 mmol) were sequentially added to a DMF (2 mL) solution of the TFA salt of pyrrolidine M138a (40 mg, 0.04 mmol), and the reaction mixture was stirred at room temperature for 3 hours. The volatile component was removed *in vacuo,* and the residue was dissolved in methanol and purified with a reverse phase HPLC (CH₃OH/H₂O/TFA) to afford the TFA salt of Example M138 as white foam (8 mg). LC (Cond. I): RT = 1.20 min; >95% homogeneity index. LC/MS: Anal. Calcd. for [M+H]⁺ C₃₇H₄₂N₇O₄: 648.33; found 648.36.

### Example M139

### methyl ((1S)-2-methyl-1-(((1R,3S,5R)-3-(4-(4'-(2-((1R,3S,SR)-2-(5-((3aS,4S,6aR)-2-oxohexahydro-1H-thieno[3,4-d]imidazol-4-yl)pentanoyl)-2-azabicyclo[3.1.0]hex-3-yl)-1H-imidazol-4-yl)-4-biphenylyl)-1H-imidazol-2-yl)-2-azabicyclo[3.1.0]hex-2-yl)carbonyl- 2-methylpropyl)carbamate

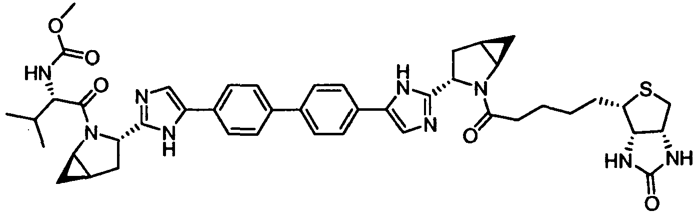

HATU (53 mg) was added to a mixture of the TFA salt of pyrrolidine M138a (40 mg, 0.042 mmol), *d*-Biotin (10.3 mg, 0.042 mmol) and i-Pr₂EtN (0.044 mL, 0.253 mmol), and the reaction mixture was stirred at room temperature for 3 hours. The volatile component was removed *in vacuo,* and the residue was dissolved in methanol and purified with a reverse phase HPLC (CH₃OH/H₂O/TFA) to afford the TFA salt of Example M139 as a light yellow solid (7 mg). LC (Cond. I): RT = 1.28 min; >95% homogeneity index. LC/MS: Anal. Calcd. for [M+H]⁺ C₄₅H₅₄N₉O₅S: 832.40; found 832.34.

### Example M140

### N((1S)-1-(((1R,3S,SR)-3-(4-(4'-(2-((1R,3S,SR)-2-((2S)-2-acetamido-3-methylbutanoyl)-2-azabicyclo[3.1.0]hex-3-yl)-1H-imidazol-4-yl)-4-biphenylyl)-1H imidazol-2-yl)-2-azabicyclo[3.1.0]hex-2-yl)carbonyl)-2-methylpropyl)acetamide

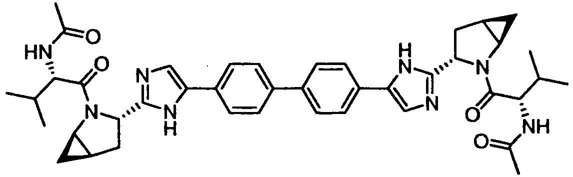

### Example M140, Step a

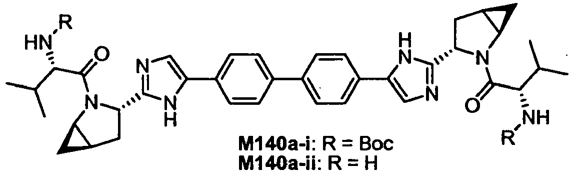

HATU (852 mg, 2.24 mmol) was added to a DMF (20 mL) solution of pyrrolidine M132i (.4HCL) (650 mg, 1.09 mmol), Boc-*L*-Valine (523 mg, 2.41 mmol), *i*-Pr₂EtN (1.15 mL, 6.56 mmol), and the reaction mixture was stirred at room temperature for 1 hour. The volatile component was removed *in vacuo,* and the crude was dissolved in CH₃OH and purified with a reverse phase HPLC (CH₃OH/H₂O/TFA) to afford 0.96 g of acylated product. A portion of the product (0.72 g) was dissolved in CH₂Cl₂ (4 mL), treated with TFA (0.26 mL, 3.35 mmol), and the resulting mixture was stirred at ambient condition for 4 hours. The volatile component was removed *in vacuo* to afford the TFA salt of M140a-ii, *tert-butyl ((18)-1-(((1R,3S,SR)-3-(4-(4'-(2-((1R,3S,SR)-2-((2S)-2-((tert-butoxycarbonyl)amino)-3-methylbutanoyl)-2-azabicyclo[3.1.0]hex-3-yl)-1H-imidazol-4-yl)-4-biphenylyl)-1H imidazol-2 yl)-2-azabicyclo[3.1.0]hex-2-yl)carbonyl)-2-methylpropyl)carbamate,* which was used in the next step without purification. LC (Cond. I): RT = 0.93 minutes. LC/MS: Anal. Calcd. for [M+H]⁺ C₃₈H₄₇N₈O₂: 647.38; found 647.26.

The free base form of carbamate M140a-i could be isolated at the coupling stage as follows: the HPLC fraction was neutralized with excess 2.0 N NH₃/CH₃OH, the volatile component was removed *in vacuo* and the residue was partitioned between CH₂Cl₂ and ∼5% saturated NaHCO₃ solution. The organic layer was dried (Na₂SO4) filtered, and concentrated *in vacuo* to afford M140a-i, *(2S)-1-((1R,3S,5R)-3-(4-(4'-(2-((1R,3S,5R)-2-((2S)-2-amino-3-methylbutanoyl)-2-azabicyclo[3.1.0]hex-3-yl)-1H-imidazol-5-yl)-4-biphenylyl)-1H-imidazol-2-yl)-2-azabicyclo[3.1.0]hex-2-yl)-3-methyl-1-oxo-2-butanamine,* as a light yellow foam. LC (Cond. I): RT = 1.64 min; >95% homogeneity index. LC/MS: Anal. Calcd. for [M+H]⁺ C₄₈H₆₃N₈O₆: 847.49; found 847.54.

### Example M140

Acetic anhydride (21 mg, 0.204 mmol) and *i*-Pr₂EtN (0.083 mL, 0.476 mmol) were sequentially added to a DMF (3 mL) solution of the TFA salt of M140a-ii (75 mg, 0.068 mmol), and the reaction mixture was stirred at ambient condition until completion, as determined by LC/MS analysis. The volatile component was removed *in vacuo,* and the residue was dissolved in CH₃OH and submitted to a reverse phase HPLC (CH₃OH/H₂O/TFA) to afford the TFA salt of Example M140 as white foam (35 mg). LC (Cond. I): RT = 1.18 min; >98% homogeneity index. LC/MS: Anal. Calcd. for [M+H]⁺ C₄₂H₅₁N₈O4: 731.40; found 731.34.

### Example M141

### N((1S)-1-(((1R,3S,SR)-3-(4-(4'-(2-((1R,3S,SR)-2-((2S)-2-((cyclopropylcarbonyl)amino)-3-methylbutanoyl)-2-azabicyclo[3.1.0]hex-3-yl)-1H imidazol-4-y/)-4-biphenyly/)-1H-imidazol-2-yl)-2-azabicyclo[3.1. 0]hex-2-yl)carbonyl)-2-methylpropyl)cyclopropanecarbamate

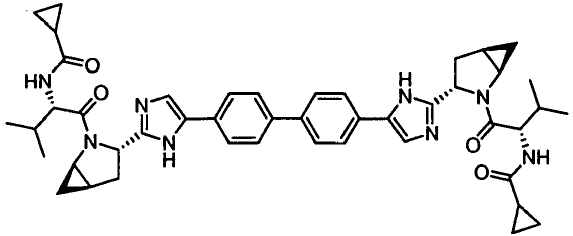

Example M141 was prepared as a TFA salt from amine M140a-ii (TFA salt) and cyclopropanecarboxylic acid according to the coupling procedure described for the synthesis of carbamate M140a-i. LC (Cond. I): RT = 1.33 min; >95% homogeneity index. LC/MS: Anal. Calcd. for [M+H]⁺ C₄₆H₅₅N₈O₄: 783.42; found 783.36.

### Example M142-M143

Example M142 (free base) and Example M143 (TFA salt) were prepared by employing the procedures described for the synthesis of Example M140 and appropriate materials.

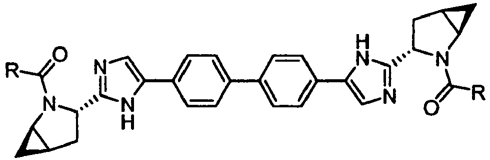

| *Example* | *Compound Name* | 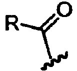 | *RT (LC-Cond.); % homogeneity index; MS data* |
|---|---|---|---|
| M142 | tert-butyl ((1R)-1-(((1R,3S,5R)-3-(4-(4'-(2-((1R,3S,5R)-2-((2R)-2-((tert-butoxycarbonyl)amino)-3-methylbutanoyl)-2-azabicyclo[3.1.0]hex-3-yl)-1H-imidazol-4-yl)-4-biphenylyl)-1H-imidazol-2-yl)-2-azabic yclo[3.1.0]hex-2-yl)carbonyl)-2-methylpropyl)carbamate | 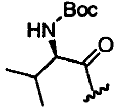 | 1.65 minutes (Cond. I); >98%; LC/MS: Anal. Calcd. for [M+H]⁺ C₄₈H₆₃N₈O₆: 847.49; found 847.54 |
| M143 | N-((1R)-1-(((1R,3S,5R)-3-(4-(4'-(2-((1R,3S,SR)-2-((2R)-2-acetamido-3-methylbutanoyl)-2-azabicyclo[3.1.0]hex-3-yl)-1H-imidazol-4-yl)-4-biphenylyl)-1H-imidazol-2-yl)-2-azabicyclo[3.1.0]hex-2-yl)carbonyl)-2-methylpropyl)acetamide | 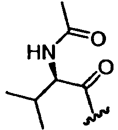 | 1.26 minutes (Cond. I); >98%; LCMS: Anal. Calcd. for [M+H]⁺ C₄₂H₅₁N₈O₄: 731.40; found 731.34 |

### Example M144

### N,N'-(4,4'-biphenyldiylbis(1H-imidazole-4,2-diyl(1R,3S,5R)-2-azabicyclo[3.1.0]hexane-3,2-diyl((2S)-3-methyl-1-oxo-1, -butanediyl)))di(2-pyrimidinamine)

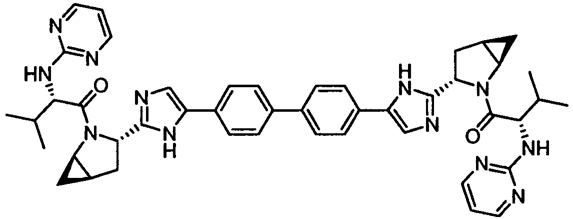

A mixture of the TFA salt of amine M140a-ii (75 mg, 0.068 mmol), 2-bromopyrimidine (32.4 mg, 0.204 mmol), and *i*-Pr₂EtN (0.048 mL, 0.272 mmol) in DMSO (0.2 mL)/toluene (1.2 mL) was heated at 90 °C for 20 hours. Additional 2-bromopyrimidine (32.4 mg, 0.204 mmol) was added and heating continued for 8 hours. Most of the volatile component was removed *in vacuo* and the residue was purified twice by a reverse phase HPLC system, (CH₃OH/H₂O/TFA) followed by (ACN/H₂O/TFA) to afford the TFA salt of Example M144, contaminated with unknown minor impurities, as a yellow foam (10 mg). RT = 20.9 minutes (see the LC method detail below); >95% homogeneity index. LC/MS: Anal. Calcd. for [N4+H]⁺ C₄₆H₅₁N₁₂O: 803.43; found 803.70.

### Method details for analysis of Example M144:

Instrument: Waters Acquity HPLC with Waters PDA UV-Vis detection and Waters SQ MS (ESCI probe)
Column: Waters Acquity BEH C18; 1.7um; 150 X 2.1 mm ID; (at 35C)
Mobile phase A: Water/ acetonitrile (97.5/2.5) with 5 mM ammonium formate; 0.05% formic acid at pH 3.3
Mobile phase B: : Acetonitrile/Water (97.5/2.5) with 5 mM ammonium formate; 0.05% formic acid
Flow: 0.35 ml/min
Hold 10%B 0-1min
10-35%B 1-20 min
35-98%B 20-32 min
hold 98% 32-35 min
98-10%B 35- 35.5 min
hold 10%B 35.5-40.0 min
UV detection: @ 260 nm

### Example M145

### methyl ((1S)-1-(((6S)-6-(4-(4'-(2-((6S)-5-((2S)-2-((methoxycarbonyl)amino)-3-methylbutanoyl)-5-azaspiro[2.4]hept-6-yl)-1H-imidazol-4-yl)-4-biphenylyl)-1H-imidazol-2-yl)-5-azaspiro[2.4]hept-5-yl)carbonyl)-2-methylpropyl)carbamate

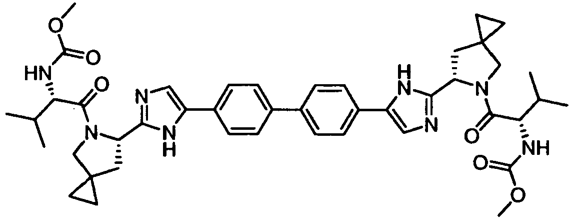

### Example M145, Step a

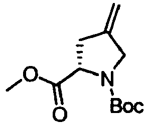

TMSCHN₂ (3.63 mL of 2.0 M/ether, 7.26 mmol) was added dropwise to a CH₃OH (33 mL) / benzene (33 mL) solution of (*S*)-1-*tert*-butyl 4-methylenepyrrolidine-1,2-dicarboxylate (1.5 g, 6.60 mmol), and the reaction mixture was stirred at ambient condition for 3.5 hours. Removal of the volatile component *in vacuo* afforded ester M145a as a tan oil (1.57 g). ¹H NMR (CDCl₃, δ = 7.26 ppm, 500 MHz): 5.01-4.98 (m, 2H), 4.50-4.48 (m, 0.5H), 4.39-4.37 (m, 0.5H), 4.07-4.04 (m, 2H), 3.71 (s, 3H), 3.01-2.87 (m, 1H), 2.66-2.55 (m, 1H), 1.46/1.41 (two overlapping s, 9H).

### Example M145, Step b

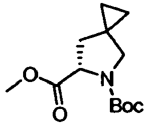

Diethylzinc (5.65 mL of 1.1 M in toluene, 6.22 mmol) was added dropwise over 20 minutes to a cooled (-22 °C) toluene (4 mL) solution of ester M145a (0.50 g, 2.07 mmol). Cloroiodomethane (0.90 mL, 12.4 mmol) was added dropwise over 10 minutes, and the reaction mixture was stirred at -22 °C for 18 hours. The reaction was quenched with saturated solution of NaHCO₃ (aq.) (5 mL) at similar temperature and was then allowed to thaw to ambient temperature. The mixture was filtered, and the precipitate was washed with EtOAc (100 mL). The layers of the filtrate were separated, and the organic phase was dried (MgSO₄), filtered, and concentrated *in vacuo.* The resulting crude material was purified with a Biotage (10-20% EtOAc/hexanes) to afford ester M145b as colorless oil (0.139 g). ¹H NMR (CDCl₃, δ = 7.26 ppm, 500 MHz): 4.47-4.44 (m, 0.5H), 4.47-4.34 (m, 0.5H), 3.73 (s, 3H), 3.39-3.27 (m, 2H), 2.25-2.18 (m, 1H), 1.86-1.84 (m, 0.5H), 1.78-1.75 (m, 0.5H), 1.44/1.40 (two overlapping s, 9H), 0.63-0.48 (m, 4H). LC (Cond. II): RT = 2.26 minutes. LC/MS: Anal. Calcd. for [M+Na]⁺ C₁₂H₁₉NNaO₄: 264.12; found 264.22.

### Example M145, Step c

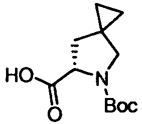

A water (0.61 mL) solution of LiOH (0.016 g, 0.65 mmol) was added to an ethanol (1.2 mL) solution of ester M145b (0.139 g, 0.544 mmol), and the reaction mixture was stirred at ambient condition for 19 hours. The volatile component was removed *in vacuo,* and the residue was dissolved in water (5 mL), cooled (ice/water), acidified to a pH region of 3.0 with 1N HCl (aq.), and then extracted with EtOAc (50 mL, 3x). The combined organic layer was dried (MgSO₄), filtered, and concentrated *in vacuo* to afford acid M145c as viscous oil which solidified upon standing (0.129 g). ¹H NMR (CDCl₃, δ = 7.26 ppm, 500 MHz): 4.48-4.40 (m, 1H), 3.55-3.05 (m, 2H), 2.37-1.87 (m, 2H), 1.47/1.44 (two overlapping s, 9H), 0.78-0.50 (m, 4H). LC (Cond. II): RT = 2.26 minutes. LC/MS: Anal. Calcd. for [M+Na]⁺ C₁₂H₁₉NNaO₄: 264.12; found 264.22.

### Example M145, Step d

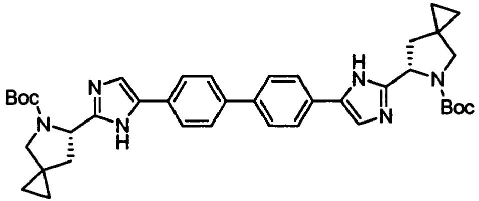

Carbamate M145d was prepared starting from acid M145c and 1,1'-(biphenyl-4,4'-diyl)bis(2-bromoethanone) and employing the general procedure described for the synthesis of M122b with the exception that the ketoester-cyclization step was conducted under microwave conditions (140 °C; 90 min). LC (Cond. II): RT = 2.54 minutes. LC/MS: Anal. Calcd. for [M+H]⁺ C₄₀H₄₉N₆O₄: 677.38; found 677.45.

### Example M145, Step e

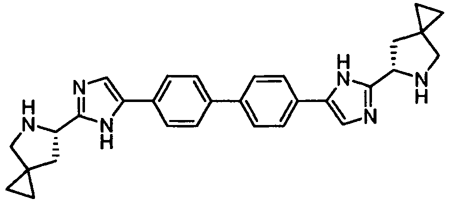

25% TFA/CH₂Cl₂ (5.3 mL) was added to carbamate M145d (0.718 g, 1.06 mmol) and the resultant mixture was stirred at ambient condition for 5 hours. The volatile component was removed *in vacuo,* and the residue was free-based with MCX (6 g, CH₃OH washing; 1:1 CHCl₃ / 2 N NH₃/CH₃OH elution) to afford pyrrolidine M145e as a light yellow solid (406 mg). ¹H NMR (DMSO-d₆, d = 2.5 ppm, 500 MHz): 11.89 (br s, 2H), 7.82 (d, J = 7.9, 4H), 7.67 (d, J = 7.9, 4H), 7.50 (br s, 2H), 4.37 (m, 2H), 2.92 (app d, J = 9.8, 2H), 2.81 (app d, J = 10.1, 2H), 2.09-2.05 (m, 2H), 1.98-1.94 (m, 2H), 0.62-0.49 (m, 8H). LC (Cond. II): RT = 1.75 minutes. LC/MS: Anal. Calcd. for [M+H]⁺ C₃₀H₃₃N₆: 477.28; found 477.35.

### Example M145

Example M 145, along with its analogs Example M 146-M 147 highlighted in the table below, were prepared as TFA salts starting from pyrrolidine M145 and appropriate acids, by employing the general HATU coupling condition outlined for Example-1, with the exception that the reaction mixture was diluted with CH₃OH and directly submitted to a reverse phase HPLC (CH₃OH/H₂O/TFA) purification. Example M145: LC (Cond. II), RT = 2.27 min); >95% homogeneity index. LC/MS: Anal. Calcd. for [M+H]⁺ = C₄₄H₅₅N₈O₆: 791.42; found 791.44.

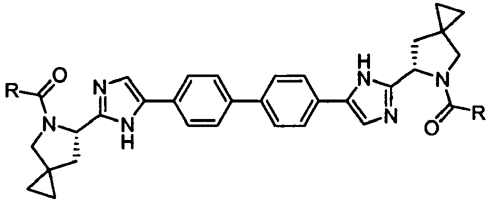

| *Example* | *Compound Name* | 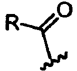 | *RT (LC-Cond.); %homogeneity index; MS data* |
|---|---|---|---|
| *M146* | methyl (2-((6S)-6-(4-(4'-(2-((6S)-5-(((methoxycarbonyl)amino)acetyl)-5-azaspiro[2.4]hept-6-yl)-1H-imidazol-4-yl)-4-biphenylyl)-1H-imidazol-2-yl)-5-azaspiro[2.4]hept-5-yl)-2-oxoethyl)carbamate | 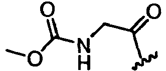 | 1.97 minutes (Cond. II); >95%; LC/MS: Anal. Calcd. for [M+H]⁺ C₃₈H₄₃N₈O₆: 707.33; found 707.36 |
| *M147* | methyl ((1S)-2-((6S)-6-(4-(4'-(2-((6S)-5-(N-(methoxycarbonyl)-L-alanyl)-5-azaspiro[2.4]hept-6-yl)-1H-imidazol-4-yl)-4-biphenylyl)-1H-imidazol-2-yl)-5-azaspiro[2.4]hept-5-yl)-1-methyl-2-oxoethyl)carbam ate | 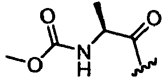 | 2.01 minutes (Cond. II); >95%; LC/MS: Anal. Calcd. for [M+H]⁺ C₄₀H₄₇N₈O₆: 735.36; found 735.41 |
| *M148* | dimethyl (4,4'-biphenyldiylbis(1H-imidazole-4,2-diyl(6S)-5-azaspiro[2.4]heptane-6,5-diyl((2S)-1-oxo-1,2-butanediyl)))biscarbamate | 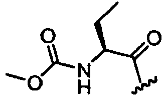 | 2.14 minutes (Cond. II); >95%; LC/MS: Anal. Calcd. for [M+H]⁺ C₄₂H₅₁N₈O₆: 763.39; found 763.46 |
| *M149* | methyl ((1R)-1-(((6S)-6-(4-(4'-(2-((6S)-5-((2R)-2-((methoxycarbonyl)amino)-3-methylbutanoyl)-5-azaspiro[2.4]hept-6-yl)-1H-imidazol-4-yl)-4-biphenylyl)-1H-imidazol-2-yl)-5-azaspiro[2.4]hept-5-yl)carbonyl)-2-methylpropyl)carbamate | 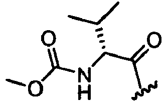 | 2.44 minutes (Cond. II); >95%; LC/MS: Anal. Calcd. for [M+H]⁺ C₄₄H₅₅N₈O₆: 791.42; found 791.44 |
| *M150* | dimethyl (4,4'-biphenyldiylbis(1H-imidazole-4,2-diyl(6S)-5-azaspiro[2.4]heptane-6,5-diyl((1R)-2-oxo-1-phenyl-2,1-ethanediyl)))biscarbamate | 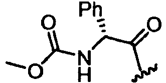 | 2.52 minutes (Cond. II); >95%; LC/MS: Anal. Calcd. for [M+H]⁺ C₅₀H₅₁N₈O₆: 859.39; found 859.42 |

### Example M151

### methyl ((1S)-1-(((2S,5S)-2-(4-(4'-(2-((2S,5S)-1-((2S)-2-((methoxycarbonyl)amino)-3-methylbutanoyl)-5-methyl-2-pyrrolidinyl)-1H-imidazol-4-yl)-4-biphenylyl)-1H-imidazol-2-yl)-5-methyl-1-pyrrolidinyl)carbonyl)-2-methylpropyl)carbamate

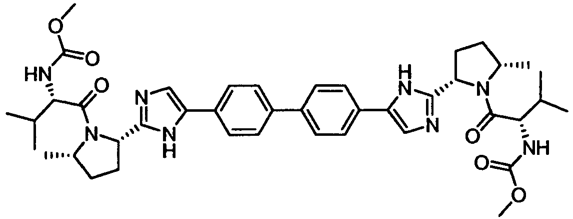

### Example M151, Step a

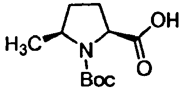

The title compound was synthesized according to a literature protocol (J. Med. Chem., 2006, 49, 3520) with the following purification modifications: the crude material (2.7 g) was recrystallized from EtOAc/hexanes at ambient temperature to afford acid M151a as a white crystal (2.2 g). ¹H NMR (CDCl₃, δ = 7.24 ppm, 400 MHz): 4.32 (br m, 1H), 3.89 (br m, 1H), 2.40 (brm, 1H), 2.00 (m, 2H), 1.65 (m, 1H), 1.45 (s, 9H), 1.20 (d, J = 5.6, 3H). LC (Cond. I): RT = 1.40 minutes. LC/MS: Anal. Calcd. for [M+Na]⁺ C₁₁H₂₀NO₄Na: 252.12; found 252.21.

### Example M151, Step b

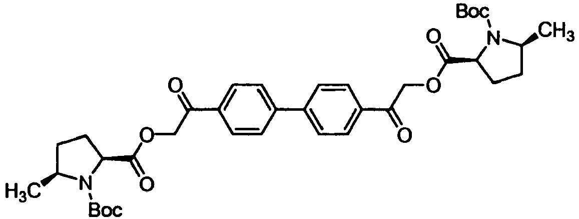

To a mixture of acid M151a and 1,1'-(biphenyl-4,4'-diyl)bis(2-bromoethanone) (1.85 g, 4.66 mmol) in acetonitrile (50 mL), *i*-Pr₂EtN (1.24 g, 9.6 mmol) was added dropwise, and the reaction was stirred at room temperature for 7 hr. The volatile component was removed *in vacuo* and the residue was partitioned between ethyl acetate and water (1:1, 100 mL). The organic layer was separated and washed with saturated NaHCO₃ and brine, dried (Na₂SO₄), filtered, and concentrated *in vacuo* to afford ketoester M151b as white foam (3.19 g), which was used in the next step without purification. ¹H NMR (DMSO-d₆, δ = 2.5 ppm, 400 MHz): 8.10 (d, J = 8.5, 4H), 7.95 (d, J= 8.5, 4H), 5.70-5.50 (br m, 4H), 4.40 (br m, 2H), 3.90 (br m, 2H), 2.25 (m, 2H), 2.15 (m, 4H), 1.60 (m, 2H), 1.41 (s, 8H), 1.39 (s, 10H), 1.20 (m, 6H). LC (Cond. I): RT = 2.15 minutes. LC/MS: parent ion was not observed.

### Example M151, Step c

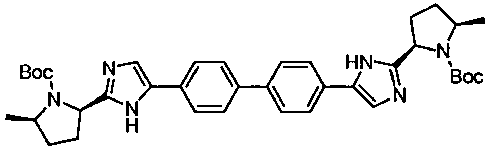

A mixture of ketoester M151b (2.94 g, 4.24 mmol) and ammonium acetate (6.54 g, 85 mmol) in xylene (40 mL) was heated in a sealed tube at 140°C for 2 hours. The volatile component was removed *in vacuo* and the residue was partitioned between CH₂Cl₂ (100 mL) and water (50 mL). The organic layer was washed with saturated NaHCO₃ (20 mL), dried (Na₂SO₄), concentrated *in vacuo.* The resultant crude material was purified with a Biotage (0-100% EtOAc/hexanes) to afford imidazole M151c as light brown solid (1.72 g). LC (Cond. n: RT = 1.03 minutes. LC/MS: Anal. Calcd. for [M+H]⁺ C₃₈H₄₉N₆O₄: 653.37; found 653.40.

### Example M151, Step d

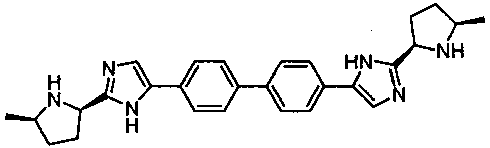

4 N HCl in dioxane (14 mL, 56 mmol) was added dropwise to a dioxane (70 mL) solution of carbamate M151c (1.72 g, 2.63 mmol), and the reaction mixture was stirred at room temperature for 4 hours. Removal of the volatile component *in vacuo* afforded the HCl salt of pyrrolidine M151d as a yellow solid (1.58 g). ¹H NMR (DMSO-d₆, δ= 2.5 ppm, 400 MHz): δ 9.85 (br s, 1H), 8.80 (br s, 1H), 7.89 (d, J = 8.3, 4H), 7.77 (s, 2H), 7.75 (d, J = 8.6, 4H), 4.70 (br m, 2H), 3.75 (br m, 2H), 2.45-2.35 (m, 4H), 2.25 (m, 2H), 1.75 (m, 2H), 1.50 (d, J = 6.6, 6H). LC (Cond. I): RT = 1.03 minutes. LC/MS: Anal. Calcd. for [M+H]⁺ C₂₈H₃₃N₆: 453.28 ; found 453.28.

### Example M151

Example M151, along with its analogs Example M152-M161 highlighted in the table below, were prepared as TFA salts starting from pyrrolidine M151d and appropriate acids, by employing the general HATU coupling condition outlined for Example-1, with the exception that the reaction mixture was diluted with CH₃OH and directly submitted to a reverse phase HPLC purification (CH₃OH/H₂O/TFA). Example M151: LC (Cond. I): RT = 1.41 min; >95% homogeneity index. LC/MS: Anal. Calcd. for [M+H]⁺ C₄₂H₅₅N₈O₆: 767.42; found 767.40.

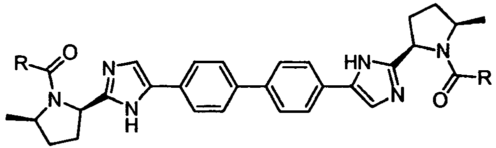

| *Example* | *Compound Name* | 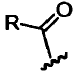 | *RT (LC-Cond); % homogeneity index; MS data* |
|---|---|---|---|
| *M152* | methyl ((1R)-1-(((2S,5S)-2-(4-(4'-(2-((2S,5S)-1-((2R)-2-((methoxycarbonyl)amino)-3-methylbutanoyl)-5-methyl-2-pyrrolidinyl)-1H-imidazol-4-yl)-4-biphenylyl)-1H-imidazol-2-yl)-5-methyl-1-pyrrolidinyl)carbonyl)-2-methylpropyl)carbamate | 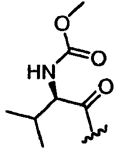 | 1.38 minutes (Cond. I); >98%; LC/MS: Anal. Calcd. for [M+H]⁺ C₄₂H₅₅N₈O₆: 767.42; found 767.33 |
| *M153* | dimethyl (4,4'-biphenyldiylbis(1H-imidazole-4,2-diyl((2S,5S)-5-methyl-2,1-pyrrolidinediyl)((1R)-2-oxo-1-phenyl-2,1-ethanediyl)))biscarbamate | 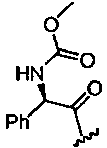 | 1.46 minutes (Cond. I); >98%; LC/MS: Anal. Calcd. for [M+H]⁺ C₄₈H₅₁N₈O₆: 835.39; found 835.31 |
| *M154* | dimethyl (4,4'-biphenyldiylbis(1H-imidazole-4,2-diyl((2S,5S)-5-methyl-2,1-pyrrolidinediyl)((2S)-1-oxo-1,2-butanediyl)))biscarbamate | 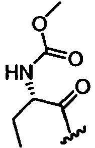 | 1.27 minutes (Cond. I); >98%; LC/MS: Anal. Calcd. for [M+H]⁺ C₄₀H₅₁N₈O₆: 739.39; found 739.28 |
| *M155* | methyl (2-((2S,5S)-2-(4-(4'-(2-((2S,5S)-1-(((methoxycarbonyl)amino)acetyl)-5-methyl-2-pyrrolidinyl)-1H-imidazol-4-yl)-4-biphenylyl)-1H-imidazol-2-yl)-5-methyl-1-pyrrolidinyl)-2-oxoethyl)carbamate | 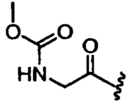 | 1.19 minutes (Cond. I); >98%; LC/MS: Anal. Calcd. for [M+H]⁺ C₃₆H₄₃N₈O₆: 683.33; found 683.32 |
| *M156* | methyl (2-((2S,5S)-2-(4-(4'-(2-((2S,5S)-1-(2-((methoxycarbonyl)amino)-2-methylpropanoyl)-5-methyl-2-pyrrolidinyl)-1H-imidazol-4-yl)-4-biphenylyl)-1H-imidazol-2-yl)-5-methyl-1-pyrrolidinyl)-1,1-dimethy 1-2-oxoethyl)carbamate | 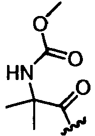 | 1.33 minutes (Cond. I); >98%; LC/MS: Anal. Calcd. for [N4+H]⁺ C₄₀H₅₁N₈O₆: 739.39; found 739.28 |
| *M157* | methyl ((1S)-2-((2S,5S)-2-(4-(4'-(2-((2S,5S)-1-(N-(methoxycarbonyl)-L-alanyl)-5-methyl-2-pyrrolidinyl)-1H-imidazol-4-yl)-4-biphenylyl)-1H-imidazol-2-yl)-5-methyl-1-pyrrolidinyl)-1-methyl-2-oxoethyl)carbamate | 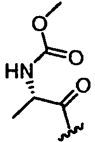 | 1.21 minutes (Cond. I); >98%; LC/MS: Anal. Calcd. for [M+H]⁺ C₃₈H₄₇N₈O₆: 711.36; found 711.23 |
| *M158* | 4,4'-(4,4'-biphenyldiyl)bis(2-((2S,5S)-5-methyl-1-(3-methylbutanoyl)-2-pyrrolidinyl)-1H-imidazole) | 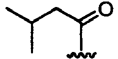 | 1.42 minutes (Cond. I); >98%; LC/MS: Anal. Calcd. for [M+H]⁺ C₃₈H₄₉N₆O₂: 621.39; found 621.28 |
| *M159* | 4,4'-(4,4'-biphenyldiyl)bis(2-((2S,5S)-5-methyl-1-(phenylacetyl)-2-pyrrolidinyl)-1H-imidazole) | 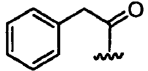 | 1.46 minutes (Cond. I); >98%; LC/MS: Anal. Calcd. for [M+H]⁺ C₄₄H₄₅N₆O₂: 689.36; found 689.26 |
| *M160* | (2R,2'R)-1,1'-(4,4'-biphenyldiylbis(1H-imidazole-4,2-diyl((2S,5S)-5-methyl-2,1-pyrrolidinediyl)))bis(3-methyl-1-oxo-2-butanol) | 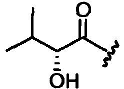 | 1.27 minutes (Cond. I); >98%; LC/MS: Anal. Calcd. for [M+H]⁺ C₃₈H₄₉N₆O₄: 653.38; found 653.27 |
| *M161* | (2S,2'S)-1,1'-(4,4'-biphenyldiylbis(1H-imidazole-4,2-diyl((2S,5S)-5-methyl-2,1-pyrrolidinediyl)))bis(3-methyl-1-oxo-2-butanol) | 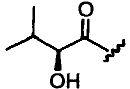 | 1.267 minutes (Cond. I); >98%; LC/MS: Anal. Calcd. for [M+H]⁺ C₃₈H₄₉N₆O₄: 653.38; found 653.34 |

### Example M162

### 2-((2S,5S)-1-acetyl-5-methyl-2-pyrrolidinyl)-4-(4'-(2-((2S,5S)-1-acetyl-5-methyl-2-pyrrolidinyl)-1H-imidazol-5-yl)-4-biphenylyl)-1H-imidazole

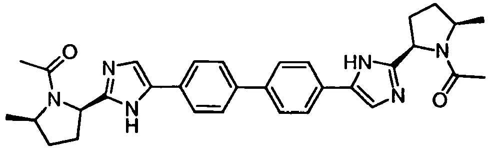

Acetic anhydride (38.4 mg, 0.376 mmol) and i-Pr₂EtN (0.153 mL, 0.877 mmol) were added to a DMF (3 mL) solution of the HCl salt of pyrrolidine M151d (75 mg, 0.125 mmol), and the mixture was stirred at room temperature for 2 hours. The volatile component was removed *in vacuo,* and the residue was dissolved in CH₃OH and purified with a reverse phase HPLC (CH₃OH/H₂O/TFA) to afford the TFA salt of M 162 as a white foam (55 mg). LC (Cond. I): RT = 1.13 min; >95% homogeneity index. LC/MS: Anal. Calcd. for [M+H]⁺ C₃₂H₃₇N₆O₂: 537.30 ; found 537.21.

### Examples J1-J14.f and E1-E5m

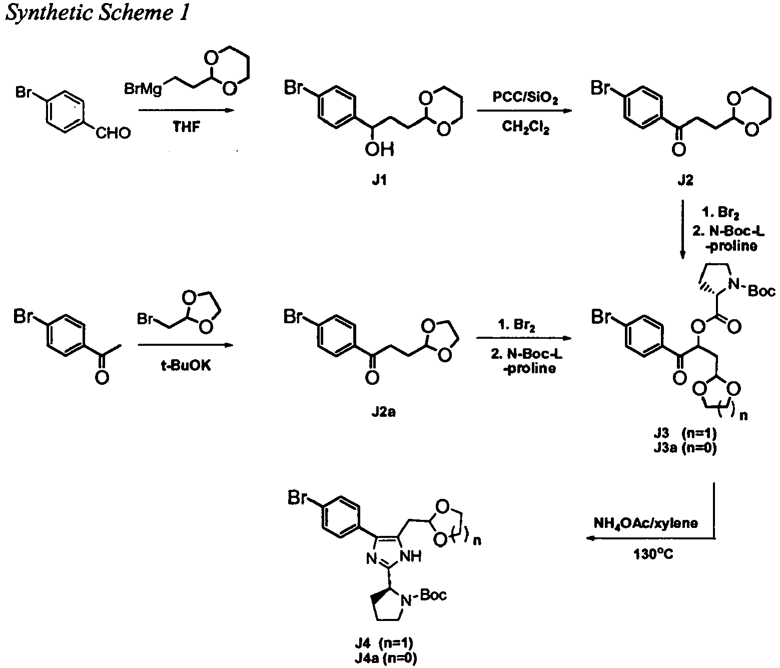

LCMS conditions 1: Phenomenex-Luna 4.6 x 50 mm S10, 0 to 100%B over 2 min, 3 min stop time, 4 mL/min, 220nm, A: 10% CH₃OH-90%H2O - 0.1% TFA; B: 90% CH₃OH-10%H2O-0.1% TFA.

LCMS conditions 2: Phenomenex-Luna 4.6 x 50 mm S 10,0 to 100%B over 3 min, 4 min stop time, 4 mL/min, 220nm, A: 10% CH₃OH-90%H2O - 0.1% TFA; B: 90% CH₃OH-10%H2O-0.1% TFA.

LCMS conditions 3: Luna 4.6 x 30 mm C18, 0 to 100%B over 2 min, 3 min stop time, 5 mL/min, 220nm, A: 5% Acetonitrile-90%H2O - 10 Mm NH₄OAc; B: 90% Acetonitrile -10%H2O-0.1% 10 Mm NH₄OAc.

### Reference: J.Org. Chem. (1992) 57,1784.

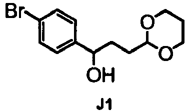

108 mL of (1,3-dioxan-2-ylethyl) magnesium bromide (0.5M) was added to a solution of 4-bromobenzaldehyde (10g, 54.0 mmol) in THF (350 mL) at -78 °C under nitrogen and stirred for 2 hours before warming to 0 °C. The reaction was quenched with sat NH₄Cl soln, diluted with diethyl ether and washed with brine. The crude product was charged (CH₂Cl₂) to a 40M Biotage silica gel cartridge; Gradient elution 15 - 100% B over 750 mL (A = Hexanes; B = ethyl acetate) to give Example J1, 1-(4-bromophenyl)-3-(1,3-dioxan-2-yl)propan-1-ol (quantitative yield). 1H NMR (500 MHz, CDCl₃) δ 7.44 (d, *J* = 8.5 Hz, 2H), 7.22 (d, *J* = 8.5 Hz, 2H), 4.70-4.66 (m, 1H), 4.57 (t, *J* = 4.9 Hz, 1H), 4.12-4.19 (m, 2H), 3.76 (tt, *J* = 11.9, 2.7 Hz, 2H), 2.87 (d, *J* = 3.7 Hz, 1H), 2.12-2.03 (m, 1H), 1.86-1.87 (m, 2H), 1.74-1.68 (m, 2H), 1.36-1.32 (m, 1H). RT = 1.8 minutes (condition 1); LRMS: No parent ion evident.

### Reference: JOC (1989) 54 5387.

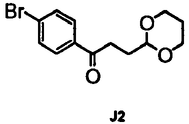

PCC (8.16g, 59.8 mmol) was admixed with 9 g SiO₂ and ground (mortar & pestle) and suspended in Dichloromethane (360 mL). To the suspension was added in one portion Example J1, 1-(4-bromophenyl)-3-(1,3-dioxan-2-yl)propan-1-ol (9g, 29.9 mmol) dissolved in 5 mL of the same solvent. The reaction mixture was stirred for 2 hours an filtered through celite (rinse with (CH₂Cl₂). After being czoncentrated the residue was charged (CH₂Cl₂) to a 40 M Biotage silica gel cartridge. Gradient elution 15-70% B over 750 mL mL (A = Hexanes; B = ethyl acetate) gave Example J2, 1-(4-bromophenyl)-3-(1,3-dioxan-2-yl)propan-1-one 7.7 g (86 %). ¹H NMR (500 MHz, CDCl₃) δ 7.84 (d, *J* = 8.7 Hz, 2H), 7.59 (d, *J* = 8.5 Hz, 2H), 4.65 (t, *J* = 4.9 Hz, 1H), 4.09-4.06 (m, 2H), 3.74 (dt, *J* = 11.0, 2.4 Hz, 2H), 3.06 (t, *J* = 7.3 Hz, 2H), 2.07-2.01 (m, 3H), 1.35-1.31 (m, 1H). LCMS: RT = 1.9 minutes (condition 1); C13H15BrO3 Calcd.: 299; found: 299 (M+H)⁺.

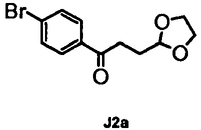

The potassium *tert*-butoxide (15 mL, 1M in THF) was added dropwise to a solution of 1-(4-bromophenyl)ethanone (3 g, 15.07 mmol) in DMSO (60 mL) at 0 °C and stirred for 30 min under nitrogen. The enolate was cannulated into a solution of 2-(bromomethyl)-1,3-dioxolane (2.52 g, 15.07 mmol) in DMSO (10 mL) at 0 °C and the reaction allowed to warm to 24°C and stirred 6 hours. Concentrate to remove solvent (high vacuum rotory evaporation) and charge (CH₂Cl₂) of residue to a 40 (M) Biotage silica gel cartridge and gradient elution 5- 35% B over 1 L (A = Hexanes; B = ethyl acetate) gave Example J2a, 1-(4-bromophenyl)-3-(1,3-dioxolan-2-yl)propan-1-one 327 mg (7.6%) and 571 mg of a bis addition product. ¹H NMR (500 MHz, CDCl₃) δ 7.83 (d, *J* = 8.5 Hz, 2H), 7.58 (d, *J* = 8.5 Hz, 2H), 4.97 (t, *J* = 4.3 Hz, 1H), 3.96-3.94 (m, 2H), 3.87-3.84 (m, 2H), 3.08-3.05 (m, 2H), 2.15-2.11 (m, 2H).

### Reference: Bromination JACS (1952) 74 6263. Displacement/Cyclization J.Med.Chem. (2001) 44 2990.

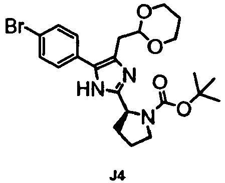

Bromine (1.3 mL, 25.2 mmol) was added to a solution of Example J2, 1-(4-bromophenyl)-3-(1,3-dioxan-2-yl)propan-1-one (7.7g, 25.7 mmol) in diethyl ether (60 mL) and 1,4-dioxane (40 mL) and the solution stirred 30 min at 24 °C (Until TLC indicated reaction complete). The solvent was removed by rotory evaporation and the residue was taken up in acetonitrile (350 mL). (S)-N-Boc-Proline (5.54g, 25.7 mmol) was added followed by dropwise addition of Hunig's base (8.5 mL, 51.5 mmol) and the reaction was stirred 6 hours before being concentrated. The crude product was taken up in CH₂Cl₂ and charged to a 40 (M) Biotage silica gel cartridge. Gradient elution 15 - 100% B over 1 L (A = hexanes; B = ethyl acetate) gave Example J3, 2-(1-(4-bromophenyl)-3-(1,3-dioxan-2-yl)-1-oxopropan-2-yl) 1-*rert-*butyl pyrrolidine-1,2-dicarboxylate 13 g (100%).). RT = 2.2 minutes (condition 1); LCMS: Anal. C23H30BrNO7 Calcd. 534; found: 534 (M+Na)⁺.

Ammonium acetate (6.45 g, 107 mmol) was added to a solution of Example J3, 2-(1-(4-bromophenyl)-3-(1,3-dioxan-2-yl)-1-oxopropan-2-yl) 1-*tert*-butyl pyrrolidine-1,2-dicarboxylate (5.5 g, 10.7 mmol) in xylene (120 mL) and stirred for 3 hours at 130 °C in a screw capped 500 mL pressure vessel. After being cooled, the reaction mixture was diluted with ethyl acetate (600 mL) and washed with sat NaHCO₃ and brine before being concentrated by rotory evaporation under high vacuum. The crude product was taken up in CH₂Cl₂ and charged to a 40 (M) Biotage silica gel cartridge. Gradient elution 15 - 100% B over 2 L (A = CH₂Cl₂; B = ethyl acetate) gave Example J4, (S)-*tert*-butyl 2-(4-((1,3-dioxan-2-yl)methyl)-5-(4-bromophenyl)-1H-imidazol-2-yl)pyrrolidine-1-carboxylate 2.22 g (40%). 1H NMR (500 MHz, CDCl₃) δ 7.46 (s, 4H), 4.94-4.92 (m, 1H), 4.77 (t, *J* = 4.9 Hz, 1H), 4.15-4.12 (m, 2H), 3.81-3.75 (m, 2H), 3.3-3.37 (m, 2H), 3.0-2.90 (m, 2H), 2.10-2.05 (m, 4H), 1.94-1.91 (m, 1H), 1.49 (s, 9H) 1.36-1.34 (m, 1H). RT = 1.8 minutes (condition 1); HRMS: Anal. C23H30BrN3O4 Calcd. 492.1492; found: 492.1505 (M+H)⁺.

| | | |
|---|---|---|
| *J4a* | 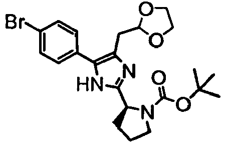 | RT = 1.68 min, (Cond 1) |
| Derived from example J2a | | HRMS: Anal. Calcd. for C₂₂H₂₉BrN₃O₄ 478.1336; found: 478.1356 (M+H)⁺. |

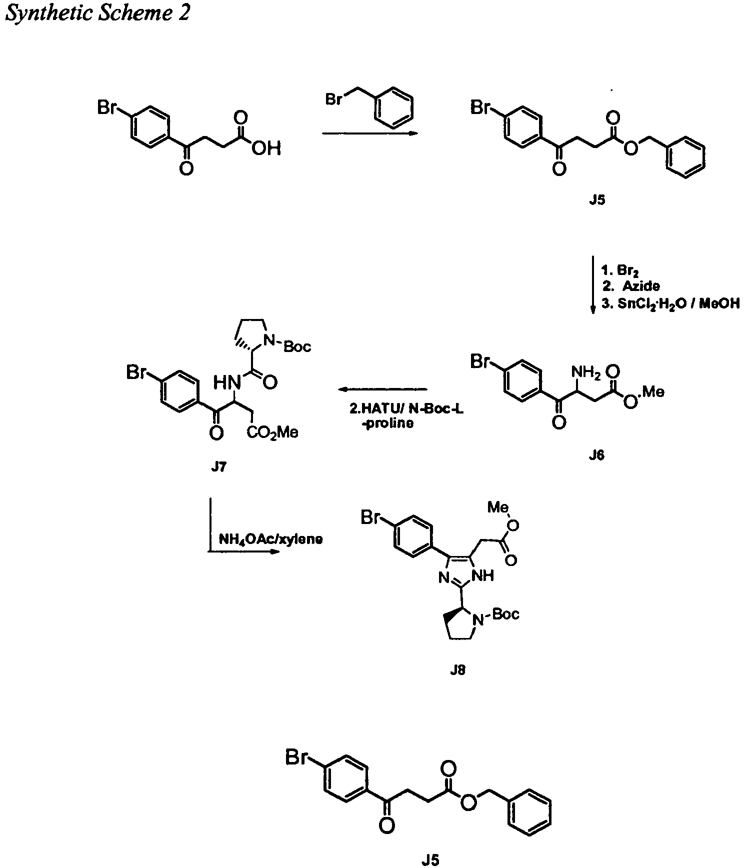

Benzyl bromide (9.98 g, 58.3 mmol) was added to a solution of 4-(4-bromophenyl)-4-oxobutanoic acid (15.0 g, 58.3 mmol) and K₂CO₃ (3.5 g, 58.3 mmol) in DMF (300 mL) and stirred for 18 hours. The reaction mixture was partitioned between water (200 mL) and ethyl acetate (500 mL). Sat'd NaHCO₃ soln (20 mL) was added and the aqueous layer extracted with ethyl acetate (2x) and the combined organic layers were washed with brine and dried and filtered. Concentration gave Example J5, benzyl 4-(4-bromophenyl)-4-oxobutanoate 16 g (79%) which was used without further purification. ¹H NMR (500 MHz, CDCl₃) δ 7.83 (d, *J* = 8.6 Hz, 2H), 7.61 (d, *J* = 8.5 Hz, 2H), 7.35-7.34 (m, 5H), 5.14 (s, 2H), 3.27 (t, *J* = 6.7 Hz, 2H), 2.81 (t, *J* = 6.7 Hz, 2H). HRMS: Anal. C17H16BrNO3 Calcd. 347.0277; found: 347.0283 (M+H)⁺.

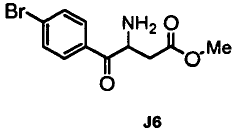

Bromine (2.5 mL, 46.1 mmol) was added to a solution of Example J5, benzyl 4-(4-bromophenyl)-4-oxobutanoate (16 g, 46.1 mmol) in ether (200 mL) and 1,4-Dioxane (50 mL) and the solution was stirred for 6 hours before being concentrated by rotory evaporation and taken up in acetonitrile (450 mL). Sodium azide (3.0 g, 46.1 mmol) was added and the reaction mixture stirred 18 hours. The solvent was removed upon concentration and the residue taken up in ethyl acetate and wash with water, brine, dried Na₂SO₄, and filtered. Concentration gave benzyl 3-azido-4-(4-bromophenyl)-4-oxobutanoate 17g (95%) which was carried forward without further purification. Tin (II) chloride dehydrate (24.9 g, 131 mmol) was added to a solution of benzyl 3-azido-4-(4-bromophenyl)-4-oxobutanoate (17 g, 43.8 mmol) in CH₃OH (550 mL) and stirred for 14 hours at 65 °C. The reaction was concentrated by rotory evaporation and dried under high vacuum for 18 hours to give a mixture of benzyl and Example J6, methyl 3-amino-4-(4-bromophenyl)-4-oxobutanoate, (transesterifcation had occurred) and carried forward with purification. RT = 1.3 minutes (condition 1); LCMS: Anal. C11H12BrNO3 Calcd. 286.0; found: 286.14 (M+H)⁺.

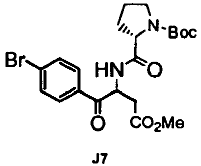

HATU (10.5 g, 27.6 mmol) was added to a solution of Example J6, methyl 3-amino-4-(4-bromophenyl)-4-oxobutanoate (10 g, 27.6 mmol), (S)-N-Boc-proline (7.13 g, 33.1 mmol), and Hunig'sBase (45 mL, 276 mmol) in DMF (150 mL) and stirred for 18 hours at 24 °C. The reaction was diluted with ethyl acetate (2 vol) and H2O (1/4 vol) and sat NaHCO₃ (1/8 vol). Filter through diatomaceous earth (Celite^{®}) to remove tin salts. Extract aqueous layer (2x) with ethyl acetate, and concentrate combined organic to remove solvents (high vacuum on rotory evaporator). The residue was subject to a short silica gel chromatography to remove by-products, and the resultant crude product was charged (CH₂Cl₂) a 65 (M) Biotage silica gel cartridge. Gradient elution 15 - 70% B over 2 L (A = Hexanes; B = ethyl acetate) gave a less polar band of (2S)-*tert*-butyl 2-(4-(benzyloxy)-1-(4-bromophenyl)-1,4-dioxobutan-2-ylcarbamoyl)pyrrolidine-1-carboxylate 1.35 g (8.7%) and more polar Example J7, (2S)-*tert*-butyl 2-(4-(methoxy)-1-(4-bromophenyl)-1,4-dioxobutan-2-ylcarbamoyl)pyrrolidine-1-carboxylate 7.6 g (49%). RT = 2.0 minutes (condition 1); LCMS: Anal. C21H27BrN2O6 Calcd. 383; found: 383 (M-Boc).

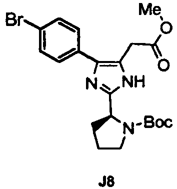

Ammonium acetate (9.4 g, 157 mmol) was added to a solution of Example J7, (2S)-*tert*-butyl 2-(4-(methoxy)-1-(4-bromophenyl)-1,4-dioxobutan-2-ylcarbamoyl)pyrrolidine-1-carboxylate (7.6 g, 15.7 mmol) in xylene (80 mL) and stirred for 4 hours at 140 °C in a screw capped 150 mL pressure vessel. After being cooled, the reaction mixture was diluted with ethyl acetate and washed with sat NaHCO₃ and brine before being concentrated by rotory evaporation under high vacuum. The crude product was taken up in CH₂Cl₂ and charged to a 40 (M) Biotage silica gel cartridge. Gradient elution 15 - 100% B over 2 L (A = CH₂Cl₂; B = ethyl acetate) gave Example J8, (S)-*tert*-butyl 2-(4-(4-bromophenyl)-5-(2-methoxy-2-oxoethyl)-1H-imidazol-2-yl)pyrrolidine-1-carboxylate 3.38 g (46%). ¹H NMR (500 MHz, DMSO-d₆) δ 7.54 (s, 4H), 4.83-4.73 (m, 1H), 3.81 (br. s, 1H), 3.62 (s, 3H), 3.53-3.51 (m, 1H), 3.38-3.31 (m, 2H), 2.25-2.15 (m, 1H), 1.99-1.83 (m, 3H), 1.41/1.16 (s, 9H). RT = 1.6 minutes (Condition 1). LCMS: Anal. Calcd. for C21H26BrN3O4 464.11; found: 464.40 (M+H)⁺.

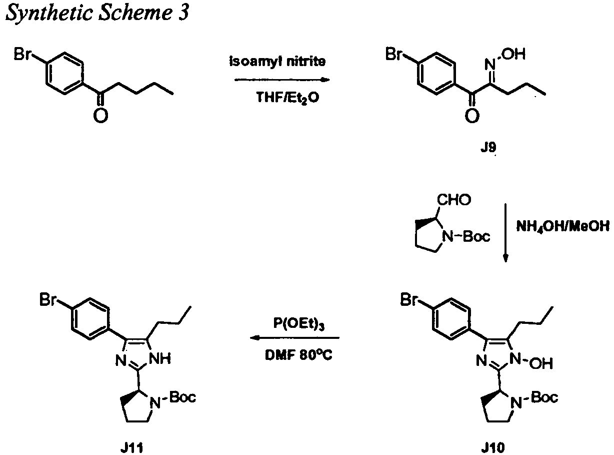

### Reference: J. Med.Chem.(84), 27, 20. Syn. Lett. (2004) 2315.

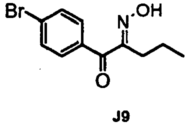

The conc. HCl (40 mL) was added dropwise to a solution of 1-(4-bromophenyl)pentan-1-one (4.68g, 19.41 mmol) and sodium nitrite (4.02 g, 58.2 mmol) in THF (80 ml) at 0 °C and allowed to warm to room temperature and stirred 18 hours. The reaction mixture was diluted with diethyl ether and the organic phase washed with sat'd NaHCO₃ and brine. Concentration gave (Z)-1-(4-bromophenyl)-2-(hydroxyimino)pentan-1-one 3.1g (33%) as an oil which was used without further purification. ). RT = 2.1 min, (Condition 1) LCMS: Anal. Calcd. for C11H12BrN1O2 270.01; found: 270.15 (M+H)⁺.

### Reference: Bioorg. Med. Chem.Lett (2002) 1009.

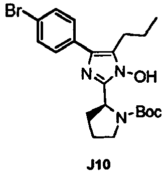

The 28% ammonium hydroxide (15 mL) was added to a solution of (Z)-1-(4-bromophenyl)-2-(hydroxyimino)pentan-1-one (1.5g, 5.55 mmol) and (S)-N-BOC-prolinal (1.1g, 5.55 mmol) in methanol (60 mL) and stirred for 18 hours at 24 °C. The reaction mixture was partitioned between CH₂Cl₂ and water and the organic phase concentrated and applied (CH₂Cl₂) to a 40 (M) Biotage silica gel column. Gradient elution, 5-100%, over 1L (A = Hexanes; B = ethyl acetate) gave (S)-*tert-*butyl 2-(4-(4-bromophenyl)hydroxy-5-propyl-1H-imidazol-2-yl)pyrrolidine-1-carboxylate (863 mg, 34.5 % yield). ¹H NMR (500 MHz, DMSO-d₆) δ 7.54 (m, 4H), 5.0-4.88 (m, 1H), 3.53-3.41 (m, 1H), 3.41-3.36 (m, 1H), 2.72 (t, *J* = 7.0 Hz, 2H), 2.24-2.03 (m, 2H), 1.96-1.91 (m, 1H), 1.89-1.81 (m, 1H), 1.60 (h, *J* = 7.6 Hz, 2H), 1.39/1.17 (s, 9H). 0.92 (t, *J* = 7.0 Hz, 3H). RT = 1.9 min, (Condition 1) LCMS: Anal. Calcd. for C21H28BrN3O3 450.13; found: 450.33 (M+H)⁺.

### Reference: Chem. Pharm. Bull. (1994) 42, 560.

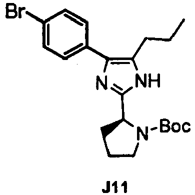

The triethyl phosphite (0.9 mL, 5.33 mmol) was added to a solution of (S)-*tert*-butyl 2-(4-(4-bromophenyl)hydroxy-5-propyl-1 H-imidazol-2-yl)pyrrolidine-1-carboxylate (800 mg, 1.776 mmol) in DMF (2 mL) and stirred for 18 hours at 80 °C. Add second 0.8 mL and the reaction was continued an additional 8 hours, cooled, and taken up in ethyl acetate (400 mL) and washed with water and brine. Apply in CH₂Cl₂ to a 25 (M) Biotage silica gel column. Gradient elution, 15-100%, over 750 mL (A = Hexanes; B = ethyl acetate) gave (S)-*tert*-butyl 2-(4-(4-bromophenyl)-5-propyl-1H-imidazol-2-yl)pyrrolidine-1-carboxylate 585 mg (76 %). ¹H NMR (500 MHz, DMSO-d₆) δ 7.55-7.53 (m, 4H), 4.80-4.69 (m, 1H), 3.53-3.51 (m, 1H), 3.38-3.32 (m, 1H), 2.71 (t, *J* = 7.0 Hz, 2H), 2.24-2.11 (m, 1H), 2.0-1.79 (m, 1H), 1.89-1.79 (m, 2H), 1.63-1.59 (m, 2H), 1.41/1.17 (s, 9H). 0.91 (t, *J* = 7.6 Hz, 3H). RT = 1.8 min, (Condition 1) LRMS: Anal. Calcd. for C21H28BrN3O2 434.14; found: 434.0 (M+H)⁺.

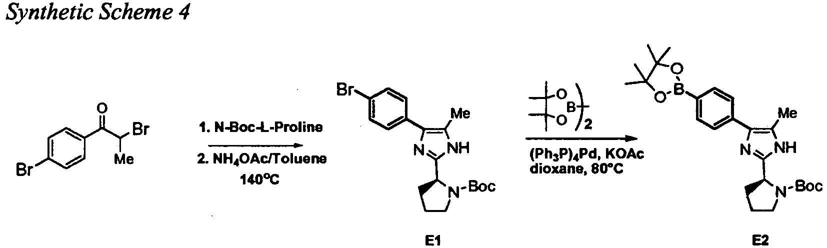

To a mixture of 2,4'-dibromopropiophenone (4.96 g, 0.017 mol) and N-Boc-L-proline (4.09 g, 0.019 mol) in dry CH₃CN (75 mL) was added DIEA (3.30 mL, 0.019 mol) and the mixture was stirred at room temperature under Ar for 16 hours. The mixture was then concentrated under reduced pressure and the concentrate was partitioned with CH₂Cl₂-10% saturated NaHCO₃. The organic phase was washed (brine), dried (Na₂SO₄), filtered, and concentrated to give the proline ester (7.28 g, >100%) as a colorless gum which was used as such in the next step. LCMS: Anal. Calcd. for C19H24BrNO5 426; found: 426 (M+H)⁺.

A mixture of the proline ester (0.435 g, 1.0 mmol) and ammonium acetate (0.308 g, 4.0 mmol) in toluene (5 mL) was heated at 140°C (bath temperature) in a sealed tube for 5 hours. The cooled reaction mixture was evaporated and the residue was chromatographed (SiO₂/ ethyl acetate-hexane, 3:2) to give Example E1 (0.320 g, 79%) as a nearly colorless foam. ¹HNMR (400 MHz, CDCl₃) δ 7.53 (s, 4H), 4.98 (m, 0.3H), 4.87 (m, 0.7H), 3.65 (m, 1H), 3.4-3.6 (m, 1H), 2.50 (s, 3H), 2.32 (m, 1H), 2.13 (m, 2H), 1.94 (m, 1H), 1.47 (s, 3H), 1.31 (s, 6H). LCMS: Anal. Calcd. for C19H24BrN3O2: 405, 407; found: 406, 408 (M+H)⁺.

| | | |
|---|---|---|
| | 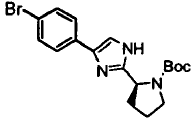 | RT = 1.71 min (condition 1); |
| *E1a* | | LCMS: Anal. Calcd. for C₁₈H₂₃BrN₃O₂: 392.10; found 391.96 [M+H]+ |
| | 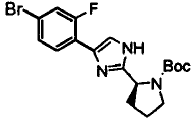 | RT = 2.27 min, (Cond 2) |
| *E1b* | | LRMS: Anal. Calcd. for C₁₈H₂₂BrFN₃O₂ 410.09 and 412.09; found: 410.08 and 412.08 (M+H)⁺. |
| | 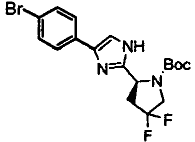 | RT = 2.19 min, (Cond 2) |
| *E1c* | | LCMS: Anal. Calcd. for C₁₈H₂₁BrF₂N₃O₂ 428.08 and 430.08; found: 428.07 and 430.07 (M+H)⁺. |

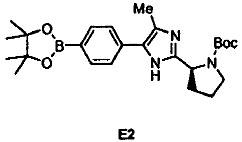

A mixture of Example E1, (S)-*tert*-butyl 2-(4-(4-bromophenyl)-5-methyl-1H-imidazol-2-yl)-pyrrolidine-1-carboxylate (1.568 g, 3.86 mmol), bis(pinacolato)diboron (2.058 g, 8.10 mmol) and potassium acetate (0.947 g, 9.65 mmol) in dioxane (25 mL) was purged with a stream of Ar bubbles for 10 min and then (Ph₃P)₄Pd (0.223 g, 0.19 mmol) was added and purging with Ar was continued for another 10 min. The reaction vessel was then sealed and heated at 80°C (bath temperature) for 18 hours. The cooled mixture was diluted with dichloromethane and then it was washed (H₂O, brine), dried (Na₂SO₄), filtered, and concentrated. The residue was triturated with ethyl acetate and the resulting solid was filtered, washed with a little ethyl acetate and dried in vacuo to give the title compound (quantitative) as a solid. It was used as such in the next step without further purification. ¹HNMR (400 MHz, DMSO-d₆) δ 11.93 (br s, 0.3H), 11.71 (br s, 0.7H), 7.65 (br s, 4H), 4.79 (m, 0.4H), 4.69 (m, 0.6H), 3.52 (m, 1H), 3.36 (m, 2H), 2.38 (s, 3H), 1.78-2.26 (m, 4H), 1.41 (s, 4H), 1.30 (s, 10H), 1.17 (m, 6H). LCMS: Anal. Calcd. for C25H36BN3O4:453; found: 454 (M+H)⁺.

| | | |
|---|---|---|
| *E2a* | 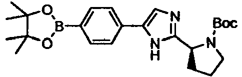 | RT = 1.64 min (condi 1); LCMS: Anal. Calcd. for C24H35BN3O4 440.27; found 440.23. [M+H]+ |
| *E2b* | 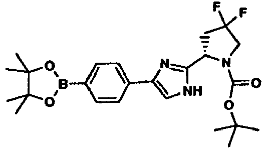 | RT = 2.44 min, (Cond 2) |
| | | LCMS: Anal. Calcd. for C24H33BF2N3O4 476.25; found: 476.51 (M+H)⁺. |

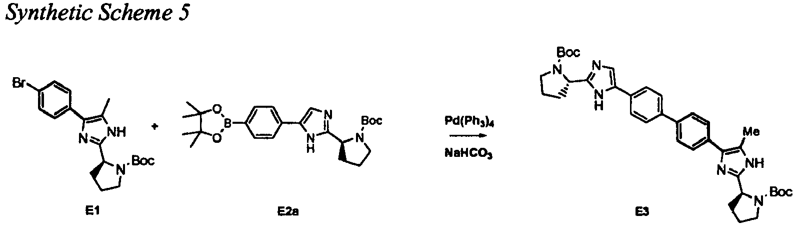

A mixture of Example E1, (S)-*tert*-butyl 2-(4-(4-bromophenyl)-5-methyl-1H-imidazol-2-yl)-pyrrolidine-1-carboxylate (0.682 g, 1.68 mmol), Example E2a, (S)-*tert*-butyl 2-(4-(4-(4,4,5,5-tetramethyl-1,3,2-dioxaborolan-2-yl)phenyl)-1H-imidazol-2-yl)pyrrolidine-1-carboxylate (0.764 g, 1.74 mmol) and NaHCO₃ (0.465 g, 5.53 mmol) in a mixture of DME (20 mL) and H₂O (5 mL) was purged with a stream of Ar bubbles for 10 min. To this mixture was added (Ph₃P)₄Pd (0.091 g, 0.08 mmol) and purging with Ar was continued for another 10 min. The reaction vessel was then sealed and heated at 80 °C (bath temperature) for 18 hours. The cooled mixture was concentrated under reduced pressure and the concentrate was diluted with ethyl acetate and washed with H₂O. The aqueous phase was back-extracted with ethyl acetate and the combined organic phase was washed (brine), dried (Na₂SO₄), filtered, and concentrated to give a gum. The residue was chromatographed (SiO₂/ ethyl acetate-hexane, 7:3) to give Example E3 (0.592 g, 59%) as a foam. ¹HNMR (400 MHz, CDCl₃) δ 7.37-7.88 (m, 8H), 7.27 (s, 1H), 5.10 (t, J = 7.65 Hz, 2H), 3.45 (m, 4H), 3.00 (m, 2H), 2.44 (s, 3H), 2.20 (br s, 4H), 1.99 (m, 2H), 1.53 (s, 18H). LCMS: Anal. Calcd. for C37H46N6O4: 638; found: 639 (M+H)⁺.

| | | | |
|---|---|---|---|
| *E3a* | | 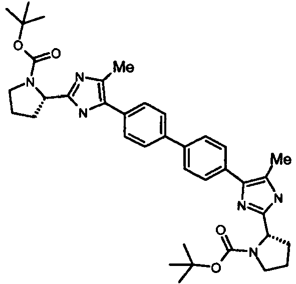 | LCMS: Anal. Calcd. for C38H4 8N6O4: 652; found: 653 (M+H)⁺ |
| Derived from example E2 and E3 | | | |
| *J12* | tert-butyl (2S)-2-(4-(4'-(2-((2S)-1-(tert-butoxycarbonyl)-2-pyrrolidinyl)-4-(1,3-dioxan-2-ylmethyl)-1H-imidazol-5-yl)-4-biphenylyl)-1H-imidazol-2-yl)-1-pyrrolidinecarboxylate | 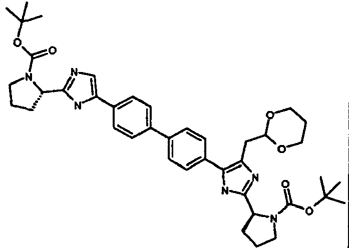 | RT = 1.65 min, (cond 3) HRMS: Anal. Calcd. for C₄₁H₅₃ N₆O₆ 725.40 21; found: 725.40 26 (M+H)⁺ |
| Derived from example E2a and J4 | | | |
| *J12a* | tert-butyl (2S)-2-(4-(4'-(2-((2S)-1-(tert-butoxycarbonyl)-2-pyrrolidinyl)-4-(1,3-dioxolan-2-ylmethyl)-1H-imidazol-5-yl)-4-biphenylyl)-1H-imidazol-2-yl)-1-pyrrolidinecarboxylate | 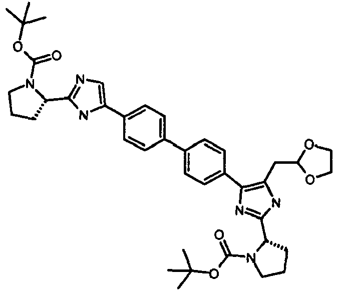 | RT = 1.62 min, (cond 1) HRMS: Anal. Calcd. for C₄₀H₅₁ N₆O₆ 711.38 65; found: 711.38 74 (M+H)⁺ |
| Derived from example E2a and J4a | | | |
| *J12b* | tert-butyl (2S)-2-(4-(4'-(2-((2S)-1-(tert-butoxycarbonyl)-2-pyrrolidinyl)-1H-imidazol-4-yl)-4-biphenylyl)-5-(2-methoxy-2-oxoethyl)-1H-imidazol-2-yl)-1-pyrrolidinecarboxylate | 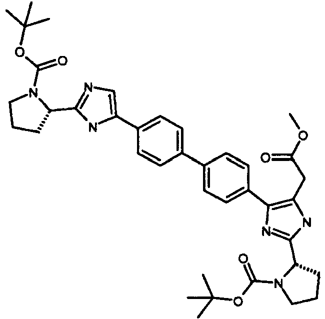 | RT = 1.72 min, (cond 1) HRMS: Anal. Calcd. for C₃₉H₄₈ N₆O₆ 697.30 87; found: 697.37 21 (M+H)⁺ |
| Derived from example E2a and J8 | | | |
| *J12c* | tert-butyl (2S)-2-(4-(4'-(2-((2S)-1-(tert-butoxycarbonyl)-2-pyrrolidinyl)-1H-imidazol-4-yl)-4-biphenylyl)-5-propyl-1H-imidazol-2-yl)-1-pyrrolidinecarboxylate | 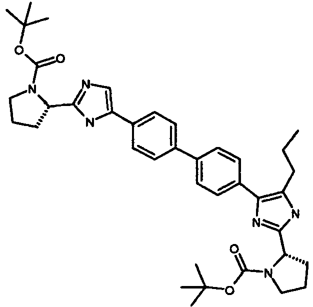 | RT = 1.72 min, (cond 1) LCMS: Anal. Calcd. for C₃₉H₅₀ N₆O₄ 667.40; found: 667.30 (M+H)⁺ |
| Derived from example E2a and J11 | | | |
| *J12d* | ethyl 2-((2S)-1-(tert-butoxycarbonyl)-2-pyrrolidinyl)-5-(4'-(2-((2S)-1-(tert-butoxycarbonyl)-2-pyrrolidinyl)-1H-imidazol-5-yl)-4-biphenylyl)-1H-imidazole-4-carboxylate | 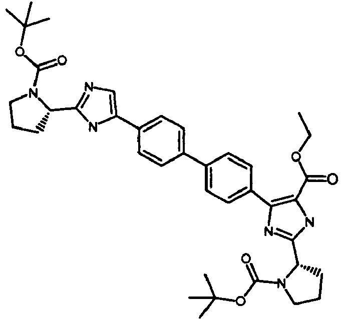 | RT = 1.70 min, (95%) (Cond 2); LRMS: Anal. Calcd. for C38H4 7N6O6 683.36; found: 683.42 (M+H)⁺ |
| *J12e* | tert-butyl (2S)-2-(4-(4'-(2-((2S)-1-(tert-butoxycarbonyl)-2-pyrrolidinyl)-1H-imidazol-4-yl)-3'-fluoro-4-biphenylyl)-1H-imidazol-2-yl)-4,4-difluoro-1-pyrrolidinecarboxylate | 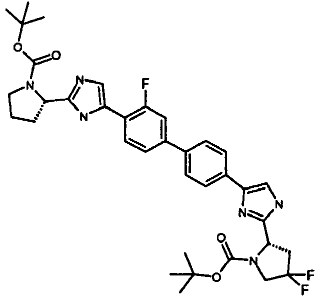 | RT = 2.24 min, (cond 2) LCMS: Anal. Calcd. for C₃₆H₄₂ F₃N₆O₄ 679.32; found: 679.57 (M+H)⁺ |
| Derived from example E2b and E1b | | | |

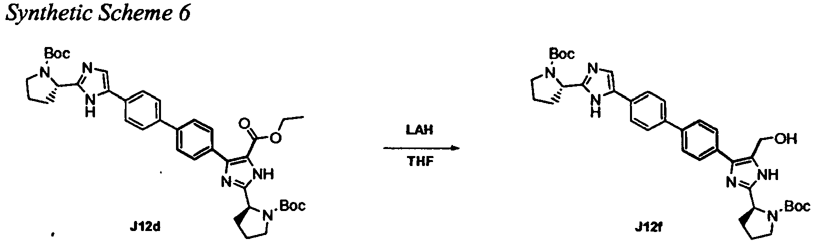

The LAH (0.7 mL, 1M in THF) was added to a solution of Example J12d, (466 mg, 0.669 mmol) in THF (50 mL) and stirred at 0°C for 1.5 hours before slowly allowing to warm to room temperature. After 3 hours the reaction was quenched with water (0.4 mL), 15% NaOH (0.4 mL) and water (0.4 mL) and the aluminum salts removed by filtration. The salts were rinsed with THF, the combined filtrates were concentrated, and the residue charged (CH₂Cl₂) to a 25 (S) Biotage silica gel cartridge and gradient eluted 15 - 100% over 1L solvent (A = CH₂Cl₂; B = 10% CH₃OH/ ethyl acetate) to give Example J12f, tert-butyl (2S)-2-(4-(4'-(2-((2S)-1-(tert-butoxycarbonyl)-2-pyrrolidinyl)-4-(hydroxymethyl)-1 H-imidazol-5-yl)-4-biphenylyl)-1H-imidazol-2-yl)-1-pyrrolidinecarboxylate, 88 mg (20%) in addition to recovered J12d and over reduction. RT = 1.7 minutes (Condition 1); LCMS Anal. Calcd. for C37H46N6O5 665.36; found: 665.46 (M+H)⁺.

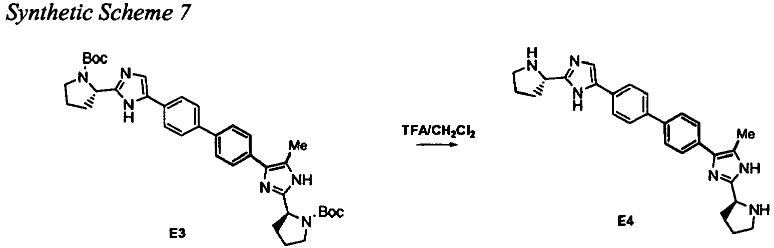

A solution of Example E3 (0.240 g, 0.376 mmol) in 5 mL of TFA-CH₂Cl₂ (4:1) was stirred at room temperature for 2 hours and then the volatiles were removed under reduced pressure. The resulting gum was taken up in a minimum volume of CH₃OH and adsorbed on an MCX LP cartridge (6 g, pre-conditioned with CH₃OH). The cartridge was washed with CH₃OH and then eluted with 2M NH₃ in CH₃OH. The product-containing fractions were combined and evaporated to give E4 (quantitative) as a gum which was used as such in the next step. LCMS: Anal. Calcd. for C27H3ON6:438; found: 439 (M+H)⁺.

| | | | | |
|---|---|---|---|---|
| *E4a* | | 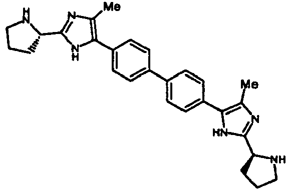 | LCMS: Anal. Calcd. for C₂₈H₃₂N₆: 452; found: 453 (M+H)⁺. | |
| *J13* Derived from example J12 | | 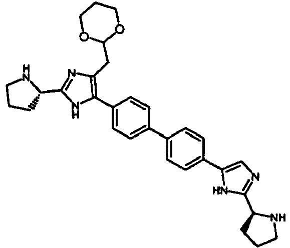 | RT=1.18 min, (cond 1) LCMS: Anal. Calcd. for C₃₁H₃₆N₆O 2 525.29; found: 525.31 (M+H)⁺. | Prepared using experim ental conditio ns from example 152k-1. |
| *J13a Derived from example J12a* | | 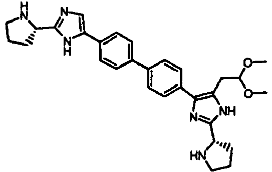 | RT = 1.14 min, (cond 1) LCMS: Anal. Calcd. for C₃₀H₃₆N₆O 2 513.29; found: 513.42 (M+H)⁺. | Prepared using experim ental conditio ns in example 152k-1. |
| *J13b Derived from example J12b* | | 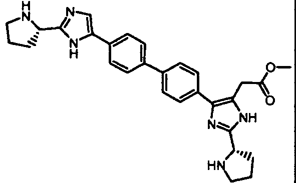 | RT = 1.19 min, (cond 1) LCMS: Anal. Calcd. for C₂₉H₃₂N₆O 2 497.26; found: 497.48 (M+H)⁺. | Prepared using experim ental conditio ns as outlined in example 152k-1. |
| *J13c Derived from example J12c* | | 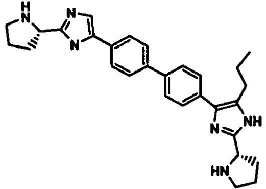 | RT = 1.26 min, (cond 1) LCMS: Anal. Calcd. for C₃₉H₅₀N₆O 4 467.28; found: 467.55 (M+H)⁺. | Prepared using experim ental conditio ns from example 152k-1. |
| *J13e Derived from example J12e* | 2-((2S)-4,4-difluoro-2-pyrrolidinyl)-4-(3'-fluoro-4'-(2-((2S)-2-pyrrolidinyl)-1H-imidazol-4-yl)-4-biphenylyl)-1H-imidazole | 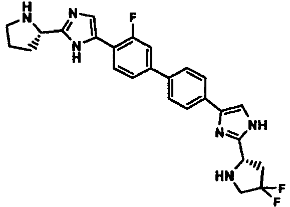 | RT = 1.77 min, (Cond 2) LCMS: Anal. Calcd. for C₂₆H₂₆F₃N 68 479.22; found: 479.39 (M+H)⁺. | Prepared using experim ental conditions from example 152k-1. |
| *J13f Derived from example J12f* | | 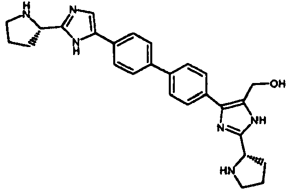 | RT = 1.02 min, (cond 1) LCMS: Anal. Calcd. for C₂₇H₃₁N₆O 455.25; found: 455.47 (M+H)⁺. | Prepared using experim ental conditio ns from example 152k-1. |

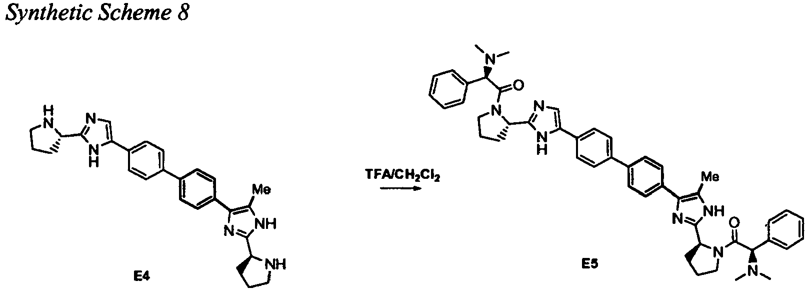

A solution of (R)-2-(dimethylamino)-2-phenylacetic acid hydrochloride (0.047 g, 0.220 mmol), HATU (0.084 g, 0.220 mmol) and DIEA (0.17 mL, 0.70 mmol) in dry DMF (1 mL) was stirred at room temperature for 5 min and then a solution of E4 (0.041 g, 0.094 mmol) in dry DMF (0.5 mL) was added. The mixture was stirred at room temperature for 18 hours and then it was quenched with AcOH (0.2 mL) and a few drops of TFA. This solution was submitted directly to preparative HPLC (C-18/ CH₃CN-H2O+0.1% TFA) to give the TFA salt of Example E5, (1R)-2-((2S)-2-(4-(4'-(2-((2S)-1-((2R)-2-(dimethylamino)-2-phenylacetyl)-2-pyrrolidinyl)-1H-imidazol-4-yl)-4-biphenylyl)-5-methyl-1 H-imidazol-2-yl)-1-pyrrolidinyl)-N,N-dimethyl-2-oxo-1-phenylethanamine (0.011 g, 10%) as a white solid. ¹HNMR (400 MHz, CH₃OH-d₄) δ 7.80-7.93 (m, 6H), 7.71-7.74 (m, 2H), 7.50-7.67 (m, 10H), 5.37-5.51 (m, 2H), 5.30 (m, 2H), 4.04 (br s, 3H), 3.00-3.13 (m, 4H), 2.81 (br s, 8H), 2.55 (s, 2H), 2.49 (s, 1H), 2.36 (m, 2H), 2.12-2.25 (m, 5H), 1.96 (br s, 2H). LCMS: Anal. Calcd. for C47H52N8O2: 760; found: 761 (M+H)⁺.

| | | | |
|---|---|---|---|
| *E5a* | (1R)-2-((2S)-2-(4-(4'-(2-((2S)-1-((2R)-2-(diethylamino)-2-phenylacetyl)-2-pyrrolidinyl)-1H-imidazol-4-yl)-4-biphenylyl)-5-methyl-1H-imidazol-2-yl)-1-pyrrolidinyl)-N,N-diethyl-2-oxo-1-phenylethanamine | 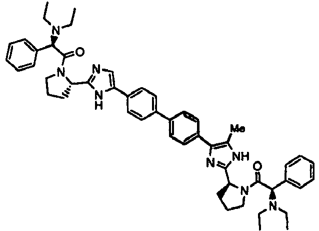 | LCMS: Anal. Calcd. for C₅₁H₆₀N₈O₂ : 816; found: 817 (M+H)⁺. |
| Derived from example E4 | | | |
| *E5b* | 1-((1R)-2-((2S)-2-(4-(4'-(4-methyl-2-((2S)-1-((2R)-2-phenyl-2-(1-piperidinyl)acetyl)-2-pyrrolidinyl)-1H-imidazol-5-yl)-4-biphenylyl)-1H-imidazol-2-yl)-1-pyrrolidinyl)-2-oxo-1-phenylethyl)piperidine | 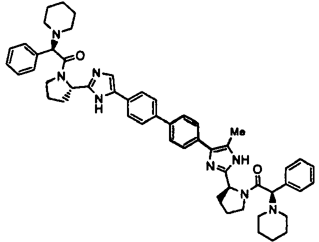 | LCMS: Anal. Calcd. for C₅₃H₆₀N₈O₂ : 840; found: 841 (M+H)⁺. |
| Derived from example E4 | | | |
| *E5c* | methyl ((1R)-2-((2S)-2-(4-(4'-(2-((2S)-1-((2R)-2-((methoxycarbonyl) amino)-2-phenylacetyl)-2-pyrrolidinyl)-1H-imidazol-4-yl)-4-biphenylyl)-5-methyl-1H-imidazol-2-yl)-1-pyrrolidinyl)-2-oxo-1-phenylethyl)carbam ate | 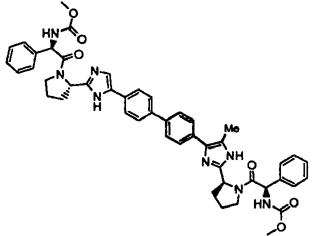 | LCMS: Anal. Calcd. for C₄₇H₄₈N₈O₆ : 820; found: 821 (M+H)⁺. |
| Derived from example E4 | | | |
| *E5d* | methyl ((1S)-2-((2S)-2-(4-(4'-(2-((2S)-1-(N-(methoxycarbonyl)-L-alanyl)-2-pyrrolidinyl)-4-methyl-1H-imidazol-5-yl)-4-biphenylyl)-1H-imidazol-2-yl)-1-pyrrolidinyl)-1-methyl-2-oxoethyl)carbamate | 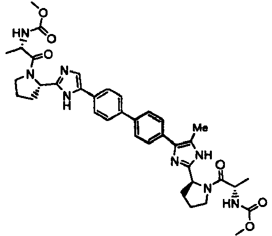 | LCMS: Anal. Calcd. for C₃₇H₄₄N₈O₆ : 696; found: 697 (M+H)⁺. |
| Derived from example E4 | | | |
| *E5e* | methyl ((1S)-1-(((2S)-2-(4-(4'-(2-((2S)-1-((2S)-2-((methoxycarbonyl) amino)-3-methylbutanoyl)-2-pyrrolidinyl)-1H-imidazol-4-yl)-4-biphenylyl)-5-methyl-1H-imidazol-2-yl)-1-pyrrolidinyl)carbon yl)-2-methylpropyl)carba mate | 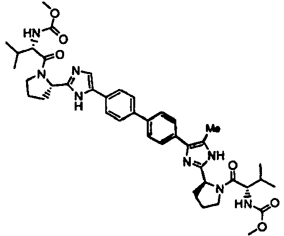 | LCMS: Anal. Calcd. for C₄₁H₅₂N₈O₆ : 752; found: 753 (M+H) . |
| Derived from example E4 | | | |
| *E5f* | methyl ((1S,2R)-2-methoxy-1-(((2S)-2-(4-(4'-(2-((2S)-1-(N-(methoxycarbonyl)-O-methyl-L-threonyl)-2-pyrrolidinyl)-4-methyl-1H-imidazol-5-yl)-4-biphenylyl)-1H-imidazol-2-yl)-1-pyrrolidinyl)carbon yl)propyl)carbamat e | 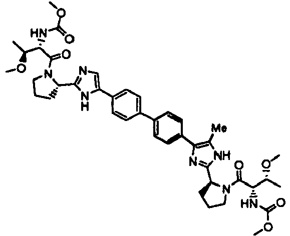 | LCMS: Anal. Calcd. for C₄₁H₅₂N₈O₈ : 784; found: 785 (M+H)⁺. |
| Derived from example E4 | | | |
| *E5g* | (1R,1'R)-2,2'-(4,4'-biphenyldiylbis((4-methyl-1H-imidazole-5,2-diyl)(2S)-2,1-pyrrolidinediyl))bis (N,N-dimethyl-2-oxo-1-phenylethanamine) | 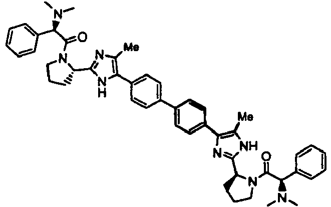 | LCMS: Anal. Calcd. for C₄₈H₅₄N₈O₂ : 774; found: 775 (M+H)⁺. |
| Derived from example E4a | | | |
| *E5h* | (1R,1'R)-2,2'-(4,4'-biphenyldiylbis((4-methyl-1H-imidazole-5,2-diyl)(2S)-2,1-pyrrolidinediyl))bis (N,N-diethyl-2-oxo-1-phenylethanamine) | 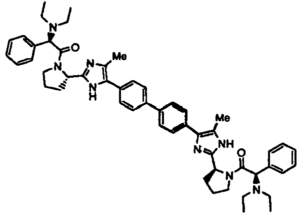 | LCMS: Anal. Calcd. for C₅₂H₆₂N₈O₂ : 830; found: 831 (M+H)⁺. |
| Derived from example E4a | | | |
| *E5i* | 1,1'-(4,4'-biphenyldiylbis((4-methyl-1H-imidazole-5,2-diyl)(2S)-2,1-pyrrolidinediyl((1R )-2-oxo-1-phenyl-2,1-ethanediyl)))dipiper idine | 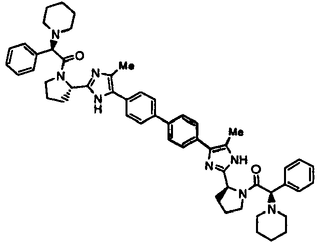 | LCMS: Anal. Calcd. for C54H62N8 O2:854; found: 855 (M+H)⁺. |
| Derived from example E4a | | | |
| *E5j* | dimethyl (4,4'-biphenyldiylbis((4-methyl-1H-imidazole-5,2- | 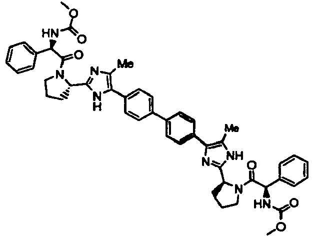 | LCMS: Anal. Calcd. for C48H50N8 O6: 834; found: 835 (M+H)⁺. |
| Derived from example E4a | diyl)(2S)-2,1-pyrrolidinediyl((1R )-2-oxo-1-phenyl-2,1-ethanediyl)))biscarb amate | | |
| | | | |
| *E5k* | methyl ((1S)-2-((2S)-2-(4-(4'-(2-((2S)-1-(N-(methoxycarbonyl)-L-alanyl)-2-pyrrolidinyl)-5-methyl-1H-imidazol-4-yl)-4-biphenylyl)-5-methyl-1H-imidazol-2-yl)-1-pyrrolidinyl)-1-methyl-2-oxoethyl)carbamate | 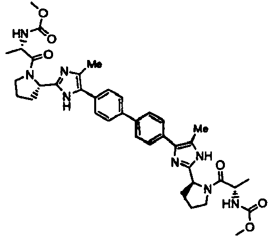 | LCMS: Anal. Calcd. for C38H46N8 O6:710; found:711 (M+H)⁺. |
| Derived from example E4a | | | |
| *E5l* | methyl ((1S)-1-(((2S)-2-(4-(4'-(2- | 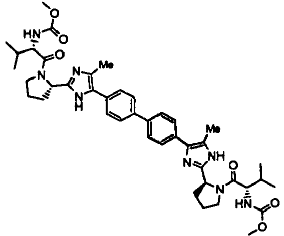 | LCMS: Anal. Calcd. for C42H54N8 O6:766; found: 767 (M+H)⁺. |
| Derived from example E4a | ((2S)-1-((2S)-2-((methoxycarbonyl) amino)-3-methylbutanoyl)-2-pyrrolidinyl)-4-methyl-1H-imidazol-5-yl)-4-biphenylyl)-5-methyl-1H-imidazol-2-yl)-1-pyrrolidinyl)carbon yl)-2-methylpropyl)carba mate | | |
| *E5m* | methyl ((1S,2R)-2-methoxy-1-(((2S)-2-(4-(4'-(2-((2S)-1-(N-(methoxycarbonyl)-O-methyl-L-threonyl)-2-pyrrolidinyl)-5-methyl-1H-imidazol-4-yl)-4-biphenylyl)-5-methyl-1H-imidazol-2-yl)-1-pyrrolidinyl)carbon yl)propyl)carbamat e | 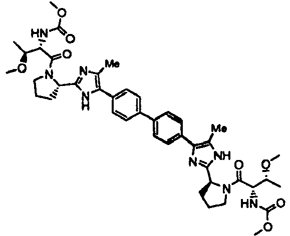 | LCMS: Anal. Calcd. for C42H54N8 O8: 798; found: 799 (M+H)⁺. |
| Derived from example E4a | | | |
| | | | |
| *J14* | methyl ((1S)-1-(((2S)-2-(4-(1,3- | 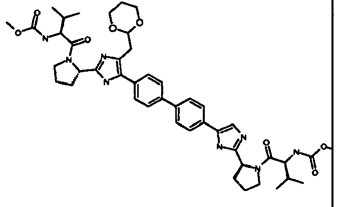 | RT = 1.37 min, (Cond |
| Derived from example J13 | dioxan-2-ylmethyl)-5-(4'-(2-((2S)-1-((2S)-2-((methoxycarbonyl) amino)-3-methylbutanoyl)-2-pyrrolidinyl)-1H-imidazol-4-yl)-4-biphenylyl)-1H-imidazol-2-yl)-1-pyrrolidinyl)carbon yl)-2-methylpropyl)carba mate | | 1); HRMS: Anal. Calcd. for C₄₅H₅₈N₈O₈ 839.4450; found: 839.4456 (M+H)⁺. |
| *J14a* | methyl ((1S,2R)-1-(((2S)-2-(4-(2,2-dimethoxyethyl)-5-(4'-(2-((2S)-1-(N-(methoxycarbonyl)-O-methyl-L- | 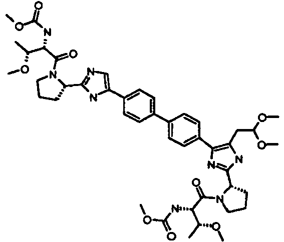 | RT = 1.44 min, (Cond 1); HRMS: Anal. Calcd. for C₄₄H₅₈N₈O_{1 0} 859.4349; found: 859.4352 (M+H)⁺. |
| Derived from example J13a | threonyl)-2-pyrrolidinyl)-1H-imidazol-4-yl)-4-biphenylyl)-1H-imidazol-2-yl)-1-pyrrolidinyl)carbon yl)-2-methoxypropyl)car bamate | | |
| *J14a.1* | methyl ((1S)-1-(((2S)-2-(4-(2,2- | 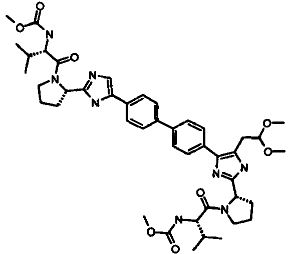 | RT = 1.44 min, (Cond 1); HRMS: Anal. Calcd. for C₄₄H₅₈N₈O₈ 827.4450; found: 827.4449 (M+H)⁺. |
| Derived from example J13a | dimethoxyethyl)-5-(4'-(2-((2S)-1-((2S)-2-((methoxycarbonyl) amino)-3-methylbutanoyl)-2-pyrrolidinyl)-1H-imidazol-4-yl)-4-biphenylyl)-1H-imidazol-2-yl)-1-pyrrolidinyl)carbon yl)-2-methylpropyl)carba mate | | |
| *J14b* | methyl (2-((2S)-1-(N-(methoxycarbonyl)-O-methyl-L-threonyl)-2-pyrrolidinyl)-4-(4'-(2-((2S)-1-(N-(methoxycarbonyl)-O-methyl-L-threonyl)-2-pyrrolidinyl)-1H-imidazol-4-yl)4-biphenylyl)-1H-imidazol-5-yl)acetate | 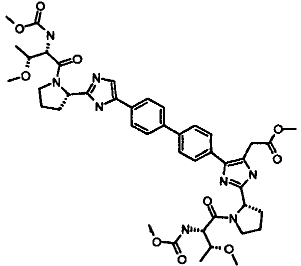 | HRMS: Anal. Calcd. for C₄₃H₅₄N₈O_{1 0} 843.4036; found: 843.4046 (M+H) ⁺. |
| Derived from example J13b | | | |
| *J14b.1* | methyl (2-((2S)-1-(N-(methoxycarbonyl)-L-valyl)-2-pyrrolidinyl)-4-(4'-(2-((2S)-1-(N-(methoxycarbonyl)-L-valyl)-2-pyrrolidinyl)-1H-imidazol-4-yl)-4-biphenylyl)-1H-imidazol-5-yl)acetate | 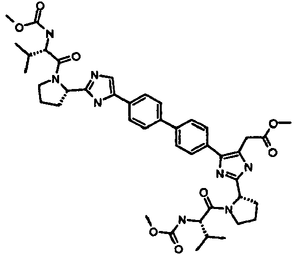 | RT = 1.42 min, (Cond 1); HRMS: Anal. Calcd. for C₄₃H₅₄N₈O₈ 811.4137; found: 811.4154 (M+H) ⁺. |
| Derived from example J13b | | | |
| *J14c* | methyl ((1S)-2-((2S)-2-(4-(4'-(2-((2S)-1-(N-(methoxycarbonyl)-L-alanyl)-2-pyrrolidinyl)-4-propyl-1H-imidazol-5-yl)-4-biphenylyl)-1H-imidazol-2-yl)-1-pyrrolidinyl)-1-methyl-2-oxoethyl)carbamate | 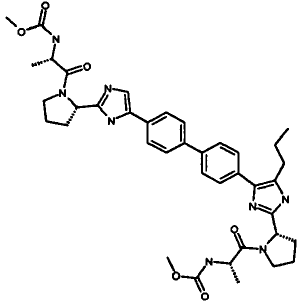 | RT=1.42 min, (Cond 1); HRMS: Anal. Calcd. for C₃₉H₄₉N₈O₆ 725.3775; found: 725.3758 (M+H) ⁺. |
| Derived from example J13c | | | |
| *J14c.1* | (1R)-2-((2S)-2-(4-(4'-(2-((2S)-1-((2R)-2-(diethylamino)-2-phenylacetyl)-2-pyrrolidinyl)-1H-imidazol-4-yl)-4-biphenylyl)-5-propyl-1H-imidazol-2-yl)-1-pyrrolidinyl)-N,N-diethyl-2-oxo-1-phenylethanamine | 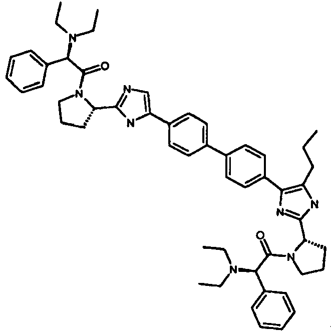 | RT=1.39 min, (Cond 1); HRMS: Anal. Calcd. for C₅₃H₆₅N₈O₂ 845.5225; found: 845.5207 (M+H) ⁺. |
| Derived from example J13c | | | |
| *J14c.2* | methyl ((1S)-1-(((2S)-2-(4-(4'-(2-((2S)-1-((2S)-2-((methoxycarbonyl) amino)-3-methylbutanoyl)-2-pyrrolidinyl)-1H-imidazol-4-yl)-4-biphenylyl)-5-propyl-1H-imidazol-2-yl)-1-pyrrolidinyl)carbon yl)-2-methylpropyl)carba mate | 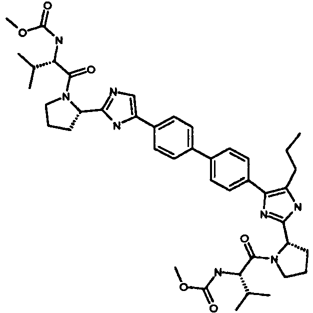 | RT = 1.59 min, (Cond 1); HRMS: Anal. Calcd. for C₄₃H₅₇N₈O₆ 781.4317; found: 781.4377 (M+H) ⁺. |
| Derived from example J13c | | | |
| *J14e* | methyl ((1S)-1-(((2S)-4,4-difluoro-2-(4-(3'-fluoro-4'-(2-((2S)-1-((2S)-2-((methoxycarbonyl) amino)-3-methylbutanoyl)-2-pyrrolidinyl)-1H-imidazol-4-yl)-4-biphenylyl)-1H-imidazol-2-yl)-1-pyrrolidinyl)carbon yl)-2-methylpropyl)carba mate | 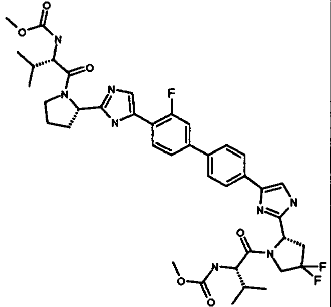 | RT = 1.99 min, (Cond 2) HRMS: Anal. Calcd. for C₄₀H₄₈F₃N₈ O₆ 793.3643; found: 793.3653 (M+H)⁺. |
| Derived from example J13e | | | |
| *J14e.1* | (1R)-2-((2S)-2-(4-(4'-(2-((2S)-1-((2R)-2-(diethylamino)-2-phenylacetyl)-4,4-difluoro-2-pyrrolidinyl)-1H-imidazol-4-yl)-3-fluoro-4-biphenylyl)-1H-imidazol-2-yl)-1-pyrrolidinyl)-N,N-diethyl-2-oxo-1-phenylethanamine | 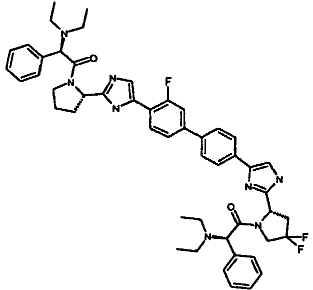 | RT = 1.79 min, (Cond 2) |
| Derived from example J13e | | | HRMS: Anal. Calcd. for C₅₀H₅₆F₃N₈ O₂ 857.4473; found: 857.4478 (M+H)⁺. |
| | | | |
| *J14f* | methyl ((1S)-1-(((2S)-2-(4-(hydroxymethyl)-5-(4'-(2-((2S)-1-((2S)-2-((methoxycarbonyl) amino)-3-methylbutanoyl)-2-pyrrolidinyl)-1H-imidazol-4-yl)-4-biphenylyl)-1H-imidazol-2-yl)-1-pyrrolidinyl)carbon yl)-2-methylpropyl)carba mate | 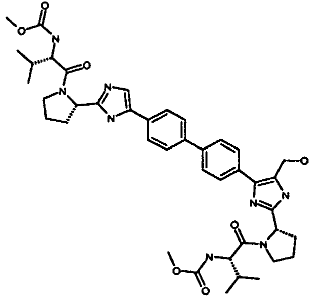 | RT = 1.40 min, (Cond 1); |
| Derived from example J13f | | | HRMS: Anal. Calcd. for C₄₁H₅₃N₈ O₇ 769.4037; found: 769.4020 (M+H)⁺. |
| *J14f.1* | methyl ((1S)-2-((2S)-2-(4-(4'-(4-(hydroxymethyl)-2-((2S)-1-(N-(methoxycarbonyl)- | 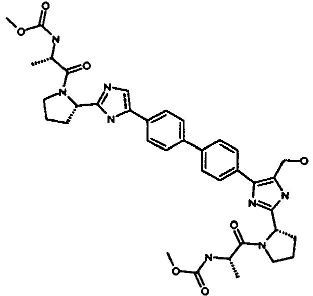 | RT=1.21 min, (Cond 1); |
| Derived from example J13f | L-alanyl)-2-pyrrolidinyl)-1H-imidazol-5-yl)-4-biphenylyl)-1H-imidazol-2-yl)-1-pyrrolidinyl)-1-methyl-2-oxoethyl)carbamate | | HRMS: Anal. Calcd. for C₃₇H₄₅N₈ O₇ 713.3411; found: 713.3391 (M+H)⁺. |

### Section PY

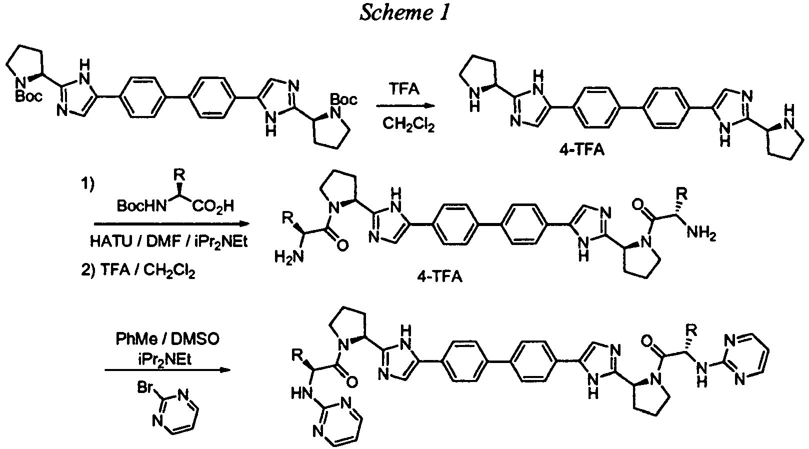

### Example PY1. 4,4'-bis(2-((S)-pyrrolidin-2-yl)-1H-imidazol-5-yl)biphenyl trifluoroacetic acid salt

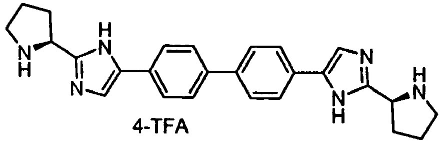

To a solution of (2S,2'S)-tert-butyl 2,2'-(5,5'-(biphenyl-4,4'-diyl)bis(1H-imidazole-5,2-diyl))dipyrrolidine-1-carboxylate (2.61 g, 4.18 mmol) in CH₂Cl₂ (25 mL) was added TFA (12 mL) and the mixture was allowed to stir at room temperature. After allowing the reaction to stir for 2 hours it was concentrated to dryness *in vacuo.* The material was used withouth further purification in subsequent steps. LCMS: Anal. Calcd. for C₂₆H₂₈N₆: 424; found: 425 (M+H)⁺.

### Example P Y2. (28, 2'S)-1,1'-((2S,2'S)-2,2'-(5,5'-(biphenyl-4,4'-diyl)bis(1H-imidazole-5,2-diyl))bis(pyrrolidine-2,1-diyl))bis(2-amino-3-methylbutan-1-one).

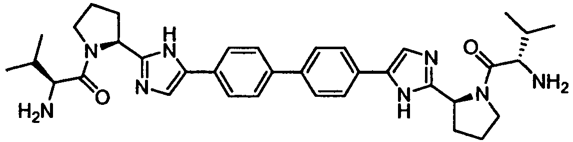

To a solution of 4,4'-bis(2-((S)-pyrrolidin-2-yl)-1H-imidazol-5-yl)biphenyl tetrakis(2,2,2-trifluoroacetate) (2.695 g, 3.06 mmol), (S)-2-(tert-butoxycarbonylamino)-3-methylbutanoic acid (1.60 g, 7.34 mmol) and DIEA (5.3 mL, 30.6 mmol) in DMF (15 mL) was added HATU (2.39 g, 6.27 mmol) and the mixture was allowed to stir at room temperature for 14 h. MeOH (5 mL) was added and the mixture was allowed to stir for 4h. It was then poured into ca. 150 mL of cold water and allowed to stand for 20 min. The solid was filtered and dried under vacuum overnight and then purified by biotage (40+M, 0 to 25% MeOH in EtOAc) to give a yellow brown foam (1.76 g, 70%). ¹HNMR (300 MHz, DMSO-d₆) δ 12.11 - 12.17 (m, 1H), 11.76 (s, 1H), 7.73 - 7.94 (m, 4H), 7.62 -7.70 (m, 4H), 7.49 (d, J = 1.8 Hz, 1H), 6.76 (d, J = 8.5 Hz, 1H), 5.06 (dd, J = 2.7, 6.3 Hz, 2H), 4.00 - 4.07 (m, 2H), 3.77 (s, br, 3H), 2.07 - 2.15 (m, 4H), 1.88 - 2.00 (m, 6H), 1.37 (s, 18H), 0.88 (d, J = 6.6 Hz, 6H), 0.82 (d, J = 6.6 Hz, 6H). LCMS: Anal. Calcd. for C₄₆H₆₂N₈O₆: 822; found: 823 (M+H)⁺. The material was suspended in CH₂Cl₂ (15 mL) and TFA (6 mL) was added. After stirring for 2h the solvents were removed *in vacuo* giving a yellow orange solid. The solid was partitioned between sat NaHCO₃ and EtOAc however the material was insoluble in EtOAc. Therefore the volatiles were removed *in vacuo* and the residue was loaded on to an SCX cation exchange cartridge and eluted with MeOH and then NH₃ in MeOH (2M). The appropriate fractions were concentrated *in vacuo* to give a yellow foam (1.24 g, 65%). LCMS: Anal. Calcd. for C₃₆H₄₆N₈O₂: 622; found: 623 (M+H)⁺. The material was used as is in subseqent steps.

The following were prepared similarly. Note that in some cases the TFA salt obtained from the Boc deprotection was carried forward directly.

| *Example* | *Structure* | *Analytical Data* |
|---|---|---|
| *Example PY3* | 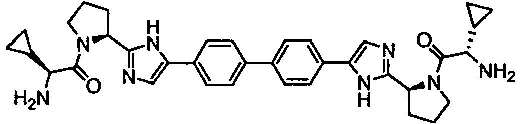 | LCMS: Anal. Calcd. for C₃₆H₄₂N₈ O₂:618; found: 619 (M+H)⁺. |
| *Example PY4* | 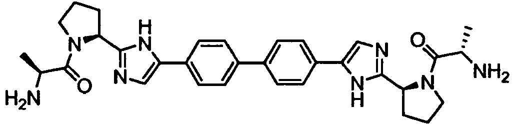 | LCMS: Anal. Calcd. for C₃₂H₃₈N₈ O₂: 566; found: 567 (M+H)⁺. |
| *Example PY5* | 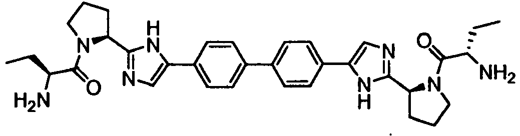 | LCMS: Anal. Calcd. for C₃₄H₄₂N₈ O₂:594; found: 595 (M+H)⁺. |
| *Example PY6* | 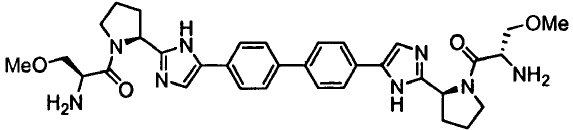 | LCMS: Anal. Calcd. for C₃₄H₄₂N₈ O₄:626; found: 627 (M+H)⁺. |
| *Example PY7* | 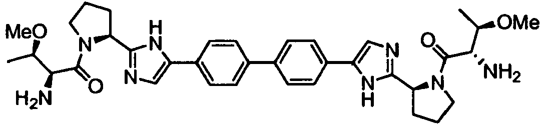 | LCMS: Anal. Calcd. for C₃₆H₄₂N₈ O₂: 618; found: 619 (M+H)⁺. |

### ExamplePY8 (N,N'-(4,4'-biphenyldiylbis(1Himidazole-4,2-diyl(2S)-2,1-pyrrolidinediyl((2S)-3-methyl-1-oxo-1,2-butanediyl)))di(2-pyrimidinamine)

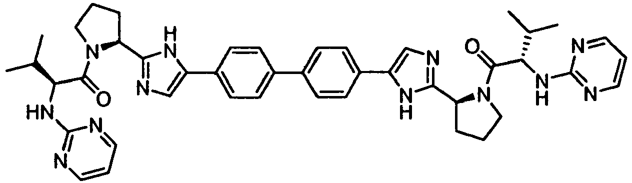

A mixture of (2S,2'S)-1,1'-((25,2'S)-2,2'-(5,5'-(biphenyl-4,4'-diyl)bis(1H-imidazole-5,2-diyl))bis(pyrrolidine-2,1-diyl))bis(2-amino-3-methylbutan-1-one) (222 mg, 0.36 mmol), 2-bromopyrimidine (0.170 g, 1.07 mmol) and iPr₂NEt (0.25 mL, 1.432 mmol) in toluene (3 mL) and DMSO (0.5 mL) was heated at 90°C overnight. LCMS indicated the reaction to be incomplete therefore heating was continued for a further 12 hours. The volatiles were removed *in vacuo* and the residue was diluted with MeOH and purified by prep HPLC (CH₃CN-H₂O-NH₄OAc). The appropriate fractions were concentrated *in vacuo* and subsequently re-purified by prep HPLC (CH₃CN-H₂O-TFA). The appropriate fractions were adsorbed onto an MCX cation exchange resin cartridge (Oasis) and the resin was washed with MeOH and eluted with 2M NH3 in MeOH. The solvent was removed *in vacuo* and the residue was lyophyllized to give a colorless solid (17.2 mg, 6%). ¹HNMR (300 MHz, DMSO-d₆) δ 12.15, 12.28 (s, 1H, rotamers, 1:1 ratio), 8.28 (d, J = 4.8 Hz, 4H), 7.79 (app d, J = 8.1 Hz, 4H), 7.62 -7.70 (m, 4H), 7.49 (d, J = 1.5 Hz, 2H), 6.85 (d, J = 8.4 Hz, 2H), 6.60 (t, J = 4.8 Hz, 2H), 5.05 (dd, J = 6.9, 4.0 Hz, 2H), 4.50 (app t, unresolved dd, J = 8.5, 8.0 Hz, 2H), 3.98 - 4.06 (m, 2H), 3.78 - 3.85 (m, 2H), 1.98 - 2.23 (m, 10H), 0.96 (d, J= 6.6 Hz, 6H), 0.92 (d, J= 6.6 Hz, 6H). LCMS: Anal. Calcd. for C₄₄H₅₀N₁₂O₂: 778; found: 779 (M+H)⁺.

The following were prepared similarly:

Note that in some cases the TFA salt obtained from the Boc deprotection was carried forward directly and an appropriate amount of iPr₂NEt was added to the reaction mixture.

| *Compound Name* | *Example* | *Structure* | *Analytical Data* |
|---|---|---|---|
| N,N'-(4,4'-biphenyldiyl bis(1H-imidazole-4,2-diyl(2S)-2,1-pyrrolidinedi yl((1S)-1-cyclopropyl-2-oxo-2,1-ethanediyl))) di(2-pyrimidinamine) | *Example PY9* | 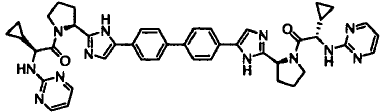 | LCMS: Anal. Calcd. for C₃₆H₄₂N₈O ₂: 618; found: 619 (M+H)⁺. |
| N,N'-(4,4'-biphenyldiyl bis(1H-imidazole-4,2-diyl(2S)-2,1-pyrrolidinedi yl((2S)-1-oxo-1,2-propanediyl)) )di(2-pyrimidinami ne) | *Example PY10* | 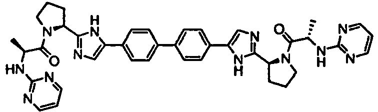 | LCMS: Anal. Calcd. for C₃₂H₃₈N₈O 2:566; found: 567 (M+H)⁺. |
| N,N'-(4,4'-biphenyldiyl bis(1H-imidazole-4,2-diyl(2S)-2,1-pyrrolidinedi yl((2S)-1-oxo-1,2-butanediyl))) di(2-pyrimidinami ne) | *Example PY11* | 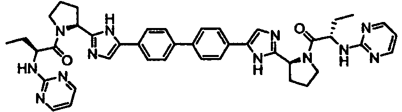 | LCMS: Anal. Calcd. for C₃₄H₄₂N₈O 2:594; found: 595 (M+H)⁺. |
| N,N'-(4,4'-biphenyldiyl bis(1H-imidazole-4,2-diyl(2S)-2,1-pyrrolidinedi yl((2S)-3-methoxy-1-oxo-1,2-propanediyl)) )di(2-pyrimidinami ne) | *Example PY12* | 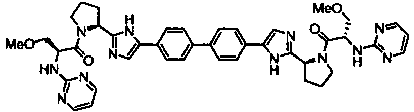 | LCMS: Anal. Calcd. for C₃₄H₄₂N₈O ₄:626; found: 627 (M+H)⁺. |
| N,N'-(4,4'-biphenyldiyl bis(1H-imidazole-4,2-diyl(2S)-2,1-pyrrolidinedi yl((2S,3R)-3-methoxy-1-oxo-1,2-butanediyl))) di(2-pyrimidinami ne) | *Example PY13* | 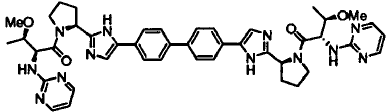 | LCMS: Anal. Calcd. for C₃₆H₄₂N₈O 2:618; found: 619 (M+H)⁺. |

### Example PY14. (Modified method)

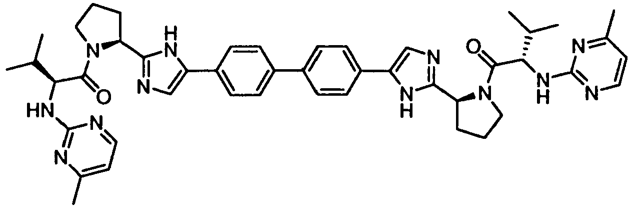

A mixture of (2S,2'S)-1,1'-((25,2'S)-2,2'-(5,5'-(biphenyl-4,4'-diyl)bis(1H-imidazole-5,2-diyl))bis(pyrrolidine-2,1-diyl))bis(2-amino-3-methylbutan-1-one) (50 mg, 0.080 mmol), 2-chloro-4-methylpyrimidine (103 mg, 0.803 mmol), and DIPEA (0.140 mL, 0.803 mmol) in NMP (3 mL) was heated in a sealed tube for 4h at 140°C using a Microwave. The volatiles were removed *in vacuo* and the residue was diluted with MeOH and filtered through a Strata XC MCX cartridge. The cartridge was washed with methanol. The compound was release from the cartridge by washing with a solution of 2M of Ammonia/Methanol. The filtrate was evaporated under reduced pressure to give an orange oil. The crude material was purified HPLC (CH₃CN-H₂O-NH₄OAc). The appropriate fractions were concentrated *in vacuo* and subsequently re-purified by prep HPLC (CH₃CN-H₂O-TFA). The appropriate fractions were concentrated *in vacuo* to give a Yellow solid (21.5 mg, 31.9%). LCMS: Anal. Calcd. for C₄₆H₅₄N₁₂O₂:807; found: 807.5 (M+H)⁺.

| Example | Structure | Analytical Data |
|---|---|---|
| *Example PY15* | 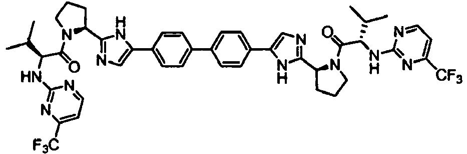 | LCMS: Anal. Calcd. for C₄₆H₄₈F₆N₆O₂:9 14; found:915.16 (M+H)⁺. |
| *Example PY16* | 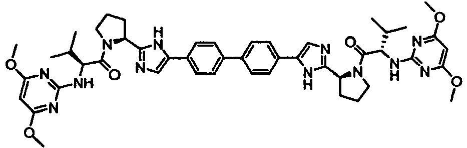 | LCMS: Anal. Calcd. for C₄₈H₅₈N₁₂O₆:89 8; found: (M+H)⁺899.21. 16 |
| *Example PY17* | 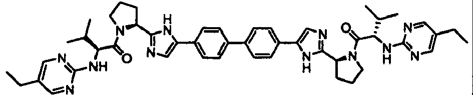 | LCMS: Anal. Calcd. for C₄₈H₅₈N₁₂O₂:83 4; found: (M+H)⁺835.45. |
| *Example PY18* | 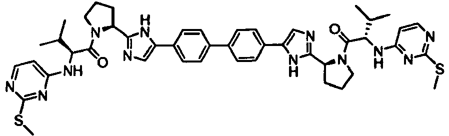 | LCMS: Anal. Calcd. for C₄₆H₅₄N₁₂O₄S₂: 870; found:(M+H)⁺ 871.24. |

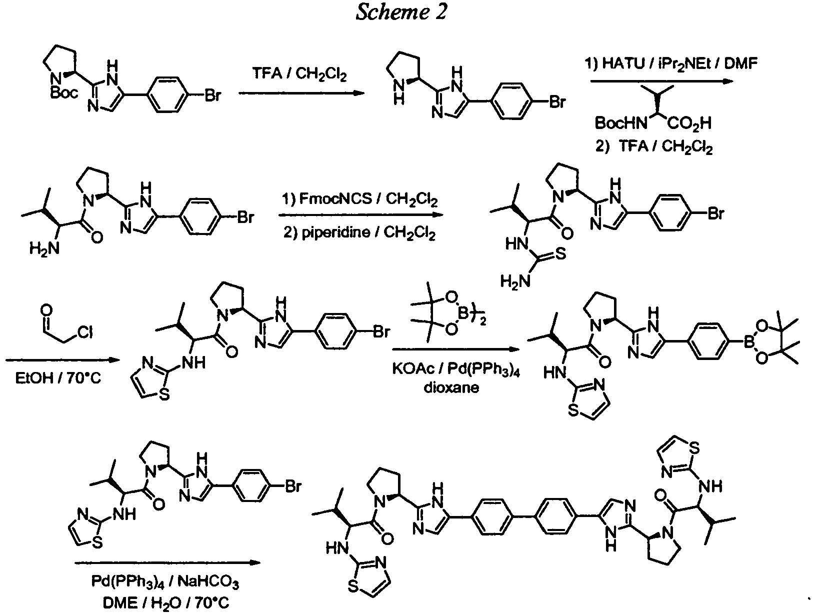

### Example PY19. (S)-2-amino-1-((S)-2-(5-(4-bromophenyl)-1H-imidazol-2-yl)pyrrolidin-1-yl)-3-methylbutan-1-one.

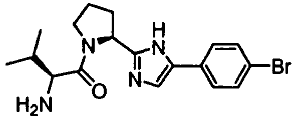

The title compound was prepared from (S)-tert-butyl 2-(5-(4-bromophenyl)-1H-imidazol-2-yl)pyrrolidine-1-carboxylate and (S)-2-amino-3-methylbutanoic acid by the procedures detailed in Examples 1 and 2. ¹HNMR (300 MHz, DMSO-d₆) δ 11.73 (s, 1H), 7.68 (d, J = 5.9 Hz, 1H), 7.65 (d, J = 5.9 Hz, 1H), 7.46 -7.58 (m, 3H), 5.14, 5.05 (dd, J = 7.0, 3.0 Hz, 1H, rotamers, 1:1 ratio), 3.66 (app t, J = 6.5 Hz, 1H), 3.53 - 3.61 and 3.38 - 3.47 (m, 1H, rotamers, 1:1 ratio), 3.28 (s, 2H), 1.70 - 2.21 (m, 6H), 0.75 - 0.88 (m, 6H). LCMS: Anal. Calcd. for C₁₈H₂₃BrN₄O: 390, 392; found: 391, 393 (M+H)⁺.

### Example PY20. 1-((S)-1-((S)-2-(5-(4-bromophenyl)-1H-imidazol-2-yl)pyrrolidin-1-yl)-3-methyl-1-oxobutan-2-yl)thiourea.

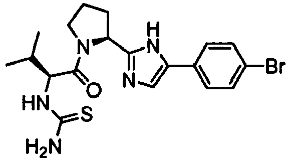

To a solution of (S)-2-amino-1-((S)-2-(5-(4-bromophenyl)-1H-imidazol-2-yl)pyrrolidin-1-yl)-3-methylbutan-1-one (1.530 g, 3.91 mmol) in CH₂Cl₂ (10 mL) was added O-(9H-fluoren-9-yl)methyl carbonisothiocyanatidate (1.100 g, 3.91 mmol) as a solid in one portion. The mixture was allowed to stir at room temperature for 12 h. Piperidine (2 mL) was added to the mixture and it was allowed to stir at room temperature for 1 h. A further portion of piperidine (2 mL) was added and the solution allowed to stir 1 h. The solution was concentrated to dryness and the residue was purified by column chromatography (biotage, eluting with 5:4.5:0.5 hex:EtOAc:MeOH, and then 5% MeOH in EtOAc). The title compound as obtained as a light yellow glass (1.30 g, 74%). ¹HNMR (300 MHz, DMSO-d₆) δ 11.81 (s, H), 7.65 (d, J = 8.8 Hz), 7.63 -7.71 (m, overlap with previous peak, 2H total), 7.42-7.56 (m, 2H), 7.10 -7.14 (m, 1H), 5.06 (dd, J = 7.0, 3.0 Hz, 1H), 4.94 (app t, J = 8.0 Hz, 1H), 4.72 - 4.76 and 4.56 - 4.62 (m, 1H, rotamers, 1:1 ratio), 3.77 - 3.90 (m, 1H), 2.07 - 2.14 (m, 2H), 1.90 - 1.98 (m, 3H), 0.93 (d, J = 7.0 Hz, 3H), 0.84 (d, J = 7.0 Hz, 3H). LCMS: Anal. Calcd. for C₁₉H₂₄BrN₅OS: 449,451; found: 450, 452 (M+H)⁺.

### Example PY21. (S)-1-((S)-2-(5-(4-bromophenyl)-1H-imidazol-2-yl)pyrrolidin-1-yl)-3-methyl-2-(thiazol-2-ylamino)butan-1-one.

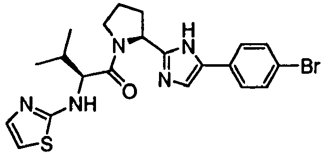

A solution of 1-((S)-1-((S)-2-(5-(4-bromophenyl)-1H-imidazol-2-yl)pyrrolidin-1-yl)-3-methyl-1-oxobutan-2-yl)thiourea (1.29 g, 2.86 mmol) was dissolved in EtOH (50 mL) and 2-chloroacetaldehyde (0.4 mL, 3.15 mmol) was added. The mixture was heated at 70°C overnight. A further portion of 2-chloroacetaldehyde (0.4 mL, 3.15 mmol) was added and heating continued for a further 12 h. The solution was concentrated *in vacuo* and the residue was purified by column chromatography (biotage), eluting with 50% EtOAc in hexanes and then 10% MeOH in EtOAc. The desired product was isolated as a orange-brown foam (557.2 mg). LCMS (NH₄OAc) shows this to be of sufficient purity. Eluting further with 100% MeOH gave a second fraction (980.8 mg) which was shown to contain the product by TLC (5:4.5:0.5 hex:EtOAc:MeOH). This second fraction from the column was re-purified to give a light orange-brown foam (426.6 mg). The two fractions were combined to afford the title compound as a orange brown foam (983.8 mg, 72%).
¹HNMR (300 MHz, CD₃OD) δ 7.57 (app d, J = 8.0 Hz, 2H), 7.47 (app d, J = 8.0 Hz, 2H), 7.32 (s, 1H), 6.98 (d, J = 3.6 Hz, 1H), 6.55 (d, J = 3.6 Hz, 1H), 5.32 - 5.35 and 5.10 - 5.15 (m, 1H, rotamers, 1:3 ratio), 4.08-4.15 (m, 1H), 3.82 - 3.90 (m, 1H), 3.03 - 3.15 (m, 1H), 2.03 - 2.33 (m, 4H), 1.06 and 0.99 (d, J = 7.0 Hz, 3H, rotamers 1:3 ratio), 1.04 and 0.94 (d, J = 7.0 Hz, 3H, rotamers 1:3 ratio). LCMS: Anal. Calcd. for C₂₁H₂₄BrN₅OS: 473, 475; found: 474, 476 (M+H)⁺.

### Example PY22. (S)-3-methyl-1-((S)-2-(5-(4-(4,4,5,5-tetramethyl-1,3,2-dioxaborolan-2-yl)phenyl)-1H-imidazol-2-yl)pyrrolidin-1-yl)-2-(thiazol-2-ylamino)butan-1-one.

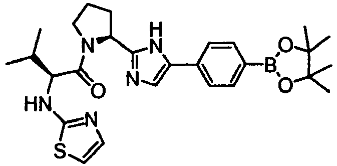

A mixture of (S)-1-((S)-2-(5-(4-bromophenyl)-1H-imidazol-2-yl)pyrrolidin-1-yl)-3-methyl-2-(thiazol-2-ylamino)butan-1-one (87.5 mg, 0.184 mmol), 4,4,4',4',5,5,5',5'-octamethyl-2,2'-bi(1,3,2-dioxaborolane) (141 mg, 0.553 mmol), potassium acetate (91 mg, 0.922 mmol) and Pd(Ph₃P)₄ (21.31 mg, 0.018 mmol) was suspended in dioxane and degassed by bubbling N₂ through the mixture. It was then heated at 85°C. After heating for 4 h the mixture was concentrated and purified by passing through a pad of silica gel eluting with 1:1 hex:EtOAc, and then EtOAc (neat) to give afforded the desired product as a light yellow film (101 mg). LCMS (CH₃CN-H₂O-NH₄OAc) showed that the product was contaminated with ca. 10% PPh₃O. The material was used as is in subsequent steps. LCMS: Anal. Calcd. for C₂₇H₃₆BN₅O₃S: 521; found: 522 (M+H)⁺.

### Example PY23. (N,N'-(4,4'-biphenyldiylbis(1H-imidazole-4,2-diyl(2S)-2,1-pyrrolidinediyl((2S)-3-methyl-1-oxo-1,2-butanediyl)))bis(1,3-thiazol-2-amine)

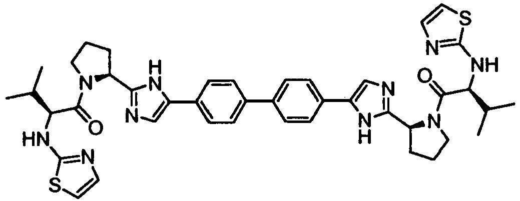

A mixture of (S)-3-methyl-1-((S)-2-(5-(4-(4,4,5,5-tetramethyl-1,3,2-dioxaborolan-2-yl)phenyl)-1H-imidazol-2-yl)pyrrolidin-1-yl)-2-(thiazol-2-ylamino)butan-1-one (275 mg, 0.527 mmol), (S)-1-((S)-2-(5-(4-bromophenyl)-1H-imidazol-2-yl)pyrrolidin-1-yl)-3-methyl-2-(thiazol-2-ylamino)butan-1-one (250 mg, 0.527 mmol), NaHCO₃ (133 mg, 1.582 mmol) and Pd(Ph₃P)₄ (60.9 mg, 0.053 mmol) in DME (3 mL) and Water (1 mL) was degassed by passing a stream of N₂ through the mixture. The vessel was sealed and the reaction was heated at 80°C overnight. The reaction mixture was diluted with H₂O and extracted with EtOAc containing ca. 5% MeOH (X3). The combined org layers were concentrated, dissolved in MeOH and loaded onto an MCX cartridge. The cartridge was washed with MeOH and then NH₃ in MeOH (2M). The appropriate fractions were concentrated *in vacuo* and the residue was purified by prep HPLC (CH₃CN-H₂O-NH₄OAc). The material was purified by column chromatography (biotage) eluting with 0 to 20% MeOH in EtOAc. Further purification by prep HPLC (CH₃CN-H₂O-NH₄OAc) followed by lyophilization afforded the title compound as a colorless solid (6.9 mg, 2%).
¹HNMR (300 MHz, DMSO-d₆) δ 13.41 (s, 1H), 11.75 (s, 2H), 7.75 - 7.80 (m, 4H), 7.68 -7.71 (m, 4H), 7.49 (s, 2H), 6.99 (d, J = 3.7 Hz, 2H), 6.57 (d, J = 3.7 Hz, 2H), 5.34 - 5.36 (m, 1H), 5.07 (app dd, J = 3.7, 7.0Hz, 2H), 4.42 (t, J = 8.4 Hz, 2H), 3.98 - 4.05 (m, 2H), 3.79 - 3.85 (m, 2H), 3.44 - 3.56 (m, 1H), 1.97 - 2.21 (m, 8H), 1.01 and 0.95 (d, J = 7.0 Hz, 6H, rotamers, 1:3 ratio), 0.99 and 0.92 (d, J = 7.0 Hz, 6H, rotamers, 1:3 ratio)_{.} LCMS: Anal. Calcd. for C₄₂H₄₈N₁₀O₂S₂: 788; found: 789 (M+H)⁺.

### Examples FY1-FY3

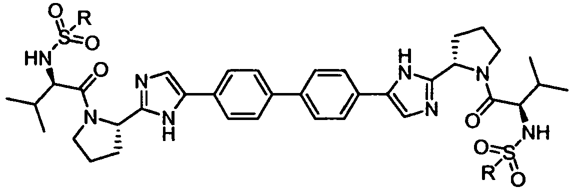

Examples FY1-FY3 were prepared according to the protocols described for the synthesis of Example F66 and by employing appropriate materials.
LC/MS Condition:
Column: Phenomenex 10u 3.0 X 50 mm
Start % B = 0
Final % B = 100
Gradient Time = 3min
Flow Rate = 4 mL/Min
Wavelength = 220
Solvent A = 10% MeOH - 90% H₂O - 0.1 % TFA
Solvent B = 90% MeOH - 10% H₂O - 0.1% TFA

| *Example* | *R* | *RT (LC-Cond. is noted above); % homogeneity index; MS data* |
|---|---|---|
| *FY1* | 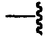 | 2.03 min; > 95%; LC/MS: Anal. Calcd. for [M+H]⁺ C₃₈H₅₁N₈O₆S₂ 779.34; found: 779.72 |
| *FY2* | 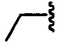 | 2.19 min; > 95%; LC/MS: Anal. Calcd. for [M+H]⁺ C₄₀H₅₅N₈O₆ S₂ 807.37; found: 807.78 |
| *FY3* | 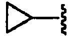 | 2.24 min; > 95%; LC/MS: Anal. Calcd. for [M+H]⁺ C₄₂H₅₅N₈O₆ S₂ 831.37; found: 832.02 |

### BIOLOGICAL ACTIVITY

An HCV Replion assay was utilized in the present disclosure, and was prepared, conducted and validated as described in commonly owned PCT/US2006/022197 and in O'Boyle et. al. Antimicrob Agents Chemother. 2005 Apr;49(4): 1346-53.

HCV 16-377-neo replicon cells were used to test the currently described compound series as well as cells resistant to compound A due to a Y2065H mutation in NS5A (described in application PCT/US2006/022197). The compounds tested were determined to have more than 10-fold less inhibitory activity on cells resistant to compound A than wild-type cells indicating a related mechanism of action between the two compound series. Thus, the compounds of the present disclosure can be effective to inhibit the function of the HCV NS5A protein and are understood to be as effective in combinations as previously described in application PCT/US2006/022197 and commonly owned WO/O4014852. Further, the compounds of the present disclosure can be effective against the HCV 1b genotype. It should also be understood that the compounds of the present disclosure can inhibit multiple genotypes of HCV. Table 2 shows the EC50 values of representative compounds of the present disclosure against the HCV 1b genotype. In one embodiment compounds of the present disclosure are active against the 1a, 1b, 2a, 2b, 3a, 4a, and 5a genotypes. EC50 ranges against HCV 1b are as follows: A = 1-10 µM; B = 100-999 nM; C = 1-99 nM; and D = 1-999 pM.

The compounds of the present disclosure may inhibit HCV by mechanisms in addition to or other than NS5A inhibition. In one embodiment the compounds of the present disclosure inhibit HCV replicon and in another embodiment the compounds of the present disclosure inhibit NS5A.

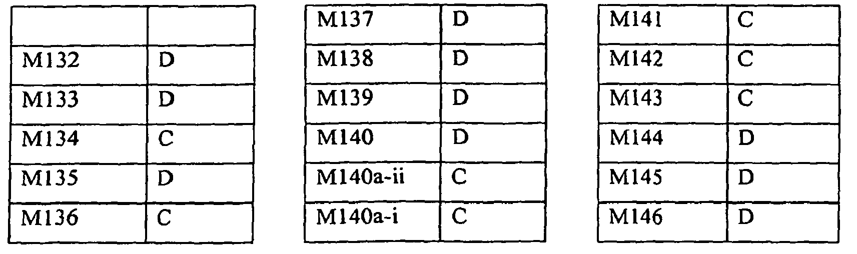

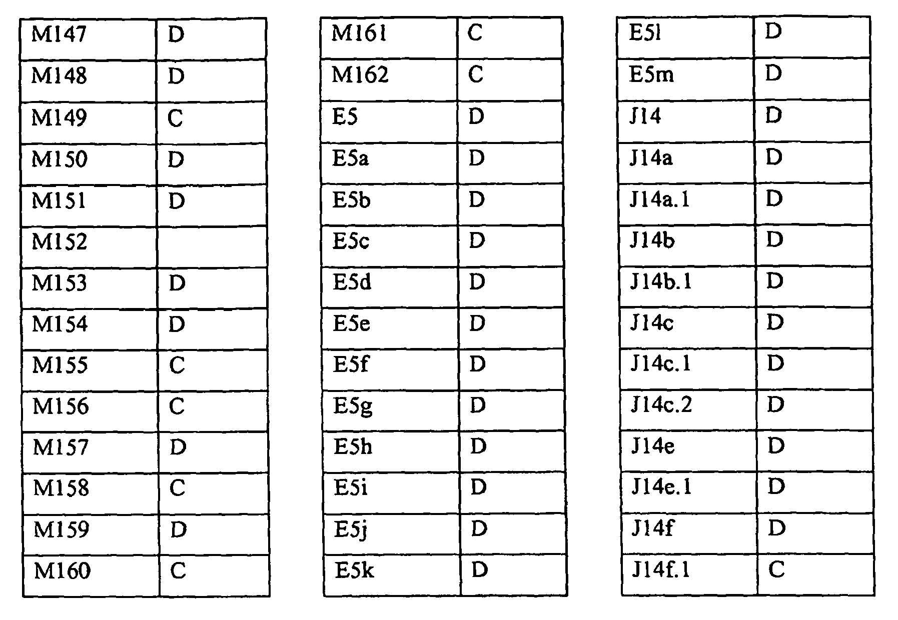

The compounds of the present disclosure may inhibit HCV by mechanisms in addition to or other than NS5A inhibition. In one embodiment the compounds of the present disclosure inhibit HCV replicon and in another embodiment the compounds of the present disclosure inhibit NS5A. Compounds of the present disclosure may inhibit multiple genotypes of HCV.

## Claims

1. A compound selected from
methyl ((1S)-2-((1R,3S,SR)-3-(4-(4'-(2-((1R,3S,SR)-2-(N-(methoxycarbonyl)-L-alanyl)-2-azabicyclo[3.1.0]hex-3-yl)-1H-imidazol-4-yl)-4-biphenylyl)-1H-imidazol-2-yl)-2-azabicyclo[3.1.0]hex-2-yl)-1-methyl-2-oxoethyl)carbamate;
dimethyl (4,4'-biphenyldiylbis(1H-imidazole-4,2-diyl(1R,3S,5R)-2-azabicyclo[3.1.0]hexane-3,2-diyl((2S)-1-oxo-1,2-butanediyl)))biscarbamate;
methyl (2-((1R,3S,5R)-3-(4-(4'-(2-((1R,3S,5R)-2-(((methoxycarbonyl)amino)acetyl)-2-azabicyclo[3.1.0]hex-3-yl)-1H-imidazol-4-yl)-4-biphenylyl)-1 H-imidazol-2-yl)-2-azabicyclo[3.1.0]hex-2-yl)-2-oxoethyl) carbamate;
dimethyl (4,4'-biphenyldiylbis(1H-imidazole-4,2-diyl(1R,3S,5R)-2-azabicyclo[3.1.0]hexane-3,2-diyl((1S)-1-cyclopropyl-2-oxo-2,1-ethanediyl)))biscarbamate;
methyl ((1R)-1-(((1R,3S,SR)-3-(4-(4'-(2-((1R,3S,SR)-2-((2R)-2-((methoxycarbonyl)amino)-3-methylbutanoyl)-2-azabicyclo[3.1.0]hex-3-yl)-1H-imidazol-4-yl)-4-biphenylyl)-1H-imidazol-2-yl)-2-azabicyclo[3.1.0]hex-2-yl)carbonyl)-2-methylpropyl)carbamate;
dimethyl (4,4'-biphenyldiylbis(1H-imidazole-4,2-diyl(1R,3S,5R)-2-azabicyclo[3.1.0]hexane-3,2-diyl((1R)-2-oxo-1-phenyl-2,1-ethanediyl)))biscarbamate;
methyl ((1S)-1-(((1R,3S,SR)-3-(4-(4'-(2-((1R,3S,SR)-2-acetyl-2-azabicyclo[3.1.0]hex-3-yl)-1 H-imidazol-4-yl)-4-biphenylyl)-1H-imidazol-2-yl)-2-azabicyclo[3.1.0]hex-2-yl)carbonyl)-2-methylpropyl)carbamate;
methyl ((1S)-2-methyl-1-(((1R,3S,SR)-3-(4-(4'-(2-((1R,3S,SR)-2-(5-((3aS,4S,6aR)-'2-oxohexahydro-1H-thieno[3,4-d]imidazol-4-yl)pentanoyl)-2-azabicyclo[3.1.0]hex-3-yl)-1H-imidazol-4-yl)-4-biphenylyl)-1H-imidazol-2-yl)-2-azabicyclo[3.1.0]hex-2-yl)carbonyl- 2-methylpropyl)carbamate;
N-((1S)-1-(((1R,3S,5R)-3-(4-(4'-(2-((1R,3S,5R)-2-((2S)-2-acetamido-3-methylbutanoyl)-2-azabicyclo[3.1.0]hex-3-yl)-1 H-imidazol-4-yl)-4-biphenylyl)-1 H-imidazol-2-yl)-2-azabicyclo[3.1.0]hex-2-yl)carbonyl)-2-methylpropyl)acetamide;
tert-butyl ((1S)-1-(((1R,3S,5S)-3-(4-(4'-(2-((1R,35,5R)-2-((2S)-2-((tert-butoxycarbonyl)amino)-3-methylbutanoyl)-2-azabicyclo[3.1.0]hex-3-yl)-1H-imidazol-4-yl)-4-biphenylyl)-1H-imidazol-2-yl)-2-azabicyclo[3.1.0]hex-2-yl)carbonyl)-2-methylpropyl)carbamate;
(2S)-1-((1R,3S,SR)-3-(4-(4'-(2-((R,3S,5R)-2-((2S)-2-amino-3-methylbutanoyl)-2-azabicyclo[3.1.0]hex-3-yl)-1H-imidazol-5-yl)-4-biphenylyl)-1H-imidazol-2-yl)-2-azabicyclo[3.1.0]hex-2-yl)-3-methyl-1-oxo-2-butanamine;
N-((1S)-1-(((1R,3S,5R)-3-(4-(4'-(2-((1R,3S,5R)-2-((2S)-2-((cyclopropylcarbonyl)amino)-3-methylbutanoyl)-2-azabicyclo[3.1.0]hex-3-yl)-1H-imidazol-4-yl)-4-biphenylyl)-1H-imidazol-2-yl)-2-azabicyclo[3.1.0]hex-2-yl)carbonyl)-2-methylpropyl)cyclopropanecarbamate;
tert-butyl ((1R)-1-(((1R,3S,SR)-3-(4-(4'-(2-((1R,3S,SR)-2-((2R)-2-((tert-butoxycarbonyl)amino)-3-methylbutanoyl)-2-azabicyclo[3.1.0]hex-3-yl)-1H-imidazol-4-yl)-4-biphenylyl)-1H-imidazol-2-yl)-2-azabicyclo[3.1.0]hex-2-yl)carbonyl)-2-methylpropyl)carbamate;
N-((1R)-1-(((1R,3S,5R)-3-(4-(4'-(2-((1R,3S,5R)-2-((2R)-2-acetamido-3-methylbutanoyl)-2-azabicyclo[3.1.0]hex-3-yl)-1H-imidazol-4-yl)-4-biphenylyl)-1H-imidazol-2-yl)-2-azabicyclo[3.1.0]hex-2-yl)carbonyl)-2-methylpropyl)acetamide;
N,N'-(4,4'-biphenyldiylbis(1H-imidazole-4,2-diyl(1R,3S,5R)-2-azabicyclo [3.1.0]hexane-3,2-diyl((2S)-3-methyl-1-oxo-1,2-butanediyl)))di(2-pyrimidinamine);
methyl ((1S)-1-(((6S)-6-(4-(4'-(2-((6S)-5-((2S)-2-((methoxycarbonyl)amino)-3-methylbutanoyl)-5-azaspiro[2.4]hept-6-yl)-1H-imidazol-4-yl)-4-biphenylyl)-1H-imidazol-2-yl)-5-azaspiro [2.4]hept-5-yl)carbonyl)-2-methylpropyl)carbamate;
methyl ((1S)-1-(((2S,5S)-2-(4-(4'-(2-((2S,5S)-1-((2S)-2-((methoxycarbonyl)amino)-3-methylbutanoyl)-5-methyl-2-pyrrolidinyl)-1H-imidazol-4-yl)-4-biphenylyl)-1H-imidazol-2-yl)-5-methyl-1-pyrrolidinyl)carbonyl)-2-methylpropyl)carbamate;
methyl (2-((6S)-6-(4-(4'-(2-((6S)-5-(((methoxycarbonyl)amino)acetyl)-5-azaspiro[2.4]hept-6-yl)-1H-imidazol-4-yl)-4-biphenylyl)-1H-imidazol-2-yl)-5-azaspiro[2.4]hept-5-yl)-2-oxoethyl)carbamate;
methyl ((1S)-2-((6S)-6-(4-(4'-(2-((6S)-5-(methoxycarbonyl)-L-alanyl)-5-azaspiro[2.4]hept-6-yl)-1H-imidazol-4-yl)-4-biphenylyl)-1H-imidazol-2-yl)-5-azaspiro[2.4]hept-5-yl)-1-methyl-2-oxoethyl)carbamate;
dimethyl (4,4'-biphenyldiylbis(1H-imidazole-4,2-diyl(6S)-5-azaspiro[2.4]heptane-6,5-diyl((2S)-1-oxo-1,2-butanediyl)))biscarbamate;
methyl ((1R)-1-(((6S)-6-(4-(4'-(2-((6S)-5-((2R)-2-((methoxycarbonyl)amino)-3-methylbutanoyl)-5-azaspiro[2.4]hept-6-yl)-1H-imidazol-4-yl)-4-biphenylyl)-1H-imidazol-2-yl)-5-azaspiro[2.4]hept-5-yl)carbonyl)-2-methylpropyl)carbamate;
dimethyl (4,4'-biphenyldiylbis(1H-imidazole-4,2-diyl(6S)-5-azaspiro[2.4]heptane-6,5-diyl((1R)-2-oxo-1-phenyl-2,1-ethanediyl)))biscarbamate;
methyl ((1R)-1-(((2S,5S)-2-(4-(4'-(2-((2S,5S)-1-((2R)-2-((methoxycarbonyl)amino)-3-methylbutanoyl)-5-methyl-2-pyrrolidinyl)-1H-imidazol-4-yl)-4-biphenylyl)-1H-imidazol-2-yl)-5-methyl-1-pyrrolidinyl)carbonyl)-2-methylpropyl)carbamate;
dimethyl (4,4'-biphenyldiylbis(1H-imidazole-4,2-diyl((2S,5S)-5-methyl-2,1-pyrrolidinediyl)((1R)-2-oxo-1-phenyl-2,1-ethanediyl)))biscarbamate;
dimethyl (4,4'-biphenyldiylbis(1H-imidazole-4,2-diyl((2S,5S)-5-methyl-2,1-pyrrolidinediyl)((2S)-1-oxo-1,2-butanediyl)))biscarbamate;
methyl (2-((2S,5S)-2-(4-(4'-(2-((2S,5S)-1-(((methoxycarbonyl)amino)acetyl)-5-methyl-2-pyrrolidinyl)-1H-imidazol-4-yl)-4-biphenylyl)-1H-imidazol-2-yl)-5-methyl-1-pyrrolidinyl)-2-oxoethyl)carbamate;
methyl (2-((2S,5S)-2-(4-(4'-(2-((2S,5S)-1-(2-((methoxycarbonyl)amino)-2-methylpropanoyl)-5-methyl-2-pyrrolidinyl)-1H-imidazol-4-yl)-4-biphenylyl)-1H-imidazol-2-yl)-5-methyl-1-pyrrolidinyl)-1,1-dimethyl-2-oxoethyl)carbamate;
methyl ((1S)-2-((2S,5S)-2-(4-(4'-(2-((2S,5S)-1-(N-(methoxycarbonyl)-L-alanyl)-5-methyl-2-pyrrolidinyl)-1H-imidazol-4-yl)-4-biphenylyl)-1H-imidazol-2-yl)-5-methyl-1-pyrrolidinyl)-1-methyl-2-oxoethyl)carbamate;
4,4'-(4,4'-biphenyldiyl)bis(2-((2S,5S)-5-methyl-1-(3-methylbutanoyl)-2-pyrrolidinyl)-1H-imidazole);
4,4'-(4,4'-biphenyldiyl)bis(2-((2S,5S)-5-methyl-1-(phenylacetyl)-2-pyrrolidinyl)-1H-imidazole);
(2R,2'R)-1,1'-(4,4'-biphenyldiylbis(1H-imidazole-4,2-diyl((2S,5S)-5-methyl-2,1-pyrrolidinediyl)))bis(3-methyl-1-oxo-2-butanol);
(2S,2'S)-1,1'-(4,4'-biphenyldiylbis(1H-imidazole-4,2-diyl((2S,5S)-5-methyl-2,1-pyrrolidinediyl)))bis(3-methyl-1-oxo-2-butanol);
2-((2S,5S)-1-acetyl-5-methyl-2-pyrrolidinyl)-4-(4'-(2-((2S,5S)-1-acetyl-5-methyl-2-pyrrolidinyl)-1H-imidazol-5-yl)-4-biphenylyl)-1H-imidazole;
tert-butyl (2S)-2-(4-(4'-(2-((2S)-1-(tert-butoxycarbonyl)-2-pyrrolidinyl)-4-(1,3-dioxan-2-ylmethyl)-1H-imidazol-5-yl)-4-biphenylyl)-1H-imidazol-2-yl)-1-pyrrolidinecarboxylate;
tert-butyl (2S)-2-(4-(4'-(2-((2S)-1-(tert-butoxycarbonyl)-2-pyrrolidinyl)-4-(1,3-dioxolan-2-ylmethyl)-1H-imidazol-5-yl)-4-biphenylyl)-1H-imidazol-2-yl)-1-pyrrolidinecarboxylate;
tert-butyl (2S)-2-(4-(4'-(2-((2S)-1-(tert-butoxycarbonyl)-2-pyrrolidinyl)-1H-imidazol-4-yl)-4-biphenylyl)-5-(2-methoxy-2-oxoethyl)-1H-imidazol-2-yl)-1-pyrrolidinecarboxylate;
tert-butyl (2S)-2-(4-(4'-(2-((2S)-1-(tert-butoxycarbonyl)-2-pyrrolidinyl)-1H-imidazol-4-yl)-4-biphenylyl)-5-propyl-1H-imidazol-2-yl)-1-pyrrolidinecarboxylate;
ethyl 2-((2S)-1-(tert-butoxycarbonyl)-2-pyrrolidinyl)-5-(4'-(2-((2S)-1-(tert-butoxycarbonyl)-2-pyrrolidinyl)-1H-imidazol-5-yl)-4-biphenylyl)-1H-imidazole-4-carboxylate;
tert-butyl (2S)-2-(4-(4'-(2-((2S)-1-(tert-butoxycarbonyl)-2-pyrrolidinyl)-1H-imidazol-4-yl)-3'-fluoro-4-biphenylyl)-1H-imidazol-2-yl)-4,4-difluoro-1-pyrrolidinecarboxylate;
2-((2S)-4,4-difluoro-2-pyrrolidinyl)-4-(3'-fluoro-4'-(2-((2S)-2-pyrrolidinyl)-1H-imidazol-4-yl)-4-biphenylyl)-1H-imidazole;
(1R)-2-((2S)-2-(4-(4'-(2-((2S)-1-((2R)-2-(diethylamino)-2-phenylacetyl)-2-pyrrolidinyl)-1H-imidazol-4-yl)-4-biphenylyl)-5-methyl-1H-imidazol-2-yl)-1-pyrrolidinyl)-N,N-diethyl-2-oxo-1-phenylethanamine;
1-((1R)-2-((2S)-2-(4-(4'-(4-methyl-2-((2S)-1-((2R)-2-phenyl-2-(1-piperidinyl)acetyl)-2-pyrrotidinyl)-1H-imidazol-5-yl)-4-biphenylyl)-1H-imidazol-2-yl)-1-pyrrolidinyl)-2-oxo-1-phenylethyl)piperidine;
methyl ((1R)-2-((2S)-2-(4-(4'-(2-((2S)-1-((2R)-2-((methoxycarbonyl)amino)-2-phenylacetyl)-2-pyrrolidinyl)-1H-imidazol-4-yl)-4-biphenylyl)-5-methyl-1H-imidazol-2-yl)-1-pyrrolidinyl)-2-oxo-1-phenylethyl)carbamate;
methyl ((1S)-2-((2S)-2-(4-(4'-(2-((2S)-1-(N-(methoxycarbonyl)-L-alanyl)-2-pyrrolidinyl)-4-methyl-1H-imidazol-5-yl)-4-biphenylyl)-1H-imidazol-2-yl)-1-pyrrolidinyl)-1-methyl-2-oxoethyl)carbamate;
methyl ((1S)-1-(((2S)-2-(4-(4'-(2-((2S)-1-((2S)-2-((methoxycarbonyl)amino)-3-methylbutanoyl)-2-pyrrolidinyl)-1H-imidazol-4-yl)-4-biphenylyl)-5-methyl-1H-imidazol-2-yl)-1-pyrrolidinyl)carbonyl)-2-methylpropyl)carbamate;
methyl ((1S,2R)-2-methoxy-1-(((2S)-2-(4-(4'-(2-((2S)-1-(N-(methoxycarbonyl)-O-methyl-L-threonyl)-2-pyrrolidinyl)-4-methyl-1H-imidazol-5-yl)-4-biphenylyl)-1H-imidazol-2-yl)-1-pyrrolidinyl)carbonyl)propyl)carbamate;
(1R,1'R)-2,2'-(4,4'-biphenyldiylbis((4-methyl-1H-imidazole-5,2-diyl)(2S)-2,1-pyrrolidinediyl))biso(N,N-dimethyl-2-oxo-1-phenylethanamine);
(1R,1'R)-2,2'-(4,4'-biphenyldiylbis((4-methyl-1H-imidazole-5,2-diyl)(2S)-2,1-pyrrolidinediyl))bis(N,N -diethyl-2-oxo-1-phenylethanamine);
1,1'-(4,4'-biphenyldiylbis((4-methyl-1H-imidazole-5,2-diyl)(2S)-2,1-pyrrolidinediyl((1R)-2-oxo-1-phenyl-2,1-ethanediyl)))dipiperidine;
dimethyl (4,4'-biphenyldiylbis((4-methyl-1H-imidazole-5,2-diyl)(2S)-2,1-pyrrolidinediyl((1R)-2-oxo-1-phenyl-2,1-ethanediyl)))biscarbamate;
methyl ((1S)-2-((2S)-2-(4-(4'-(2-((2S)-1-(N-(methoxycarbonyl)-L-alanyl)-2-pyrrolidinyl)-5-methyl-1H-imidazol-4-yl)-4-biphenylyl)-5-methyl-1H-imidazol-2-yl)-1-pyrrolidinyl)-1-methyl-2-oxoethyl)carbamate;
methyl ((1S)-1-(((2S)-2-(4-(4'-(2-((2S)-1-((2S)-2-((methoxycarbonyl)amino)-3-methylbutanoyl)-2-pyrrolidinyl)-4-methyl-1H-imidazol-5-yl)-4-biphenylyl)-5-methyl-1H-imidazol-2-yl)-1-pyrrolidinyl)carbonyl)-2-methylpropyl)carbarnate;
methyl ((1S,2R)-2-methoxy-1-(((2S)-2-(4-(4'-(2-((2S)-1-(N-(methoxycarbonyl)-O-methyl-L-threonyl)-2-pyrrolidinyl)-5-methyl-1H-imidazol-4-yl)-4-biphenylyl)-5-methyl-1H-imidazol-2-yl)-1-pyrrolidinyl)carbonyl)propyl)carbamate;
methyl ((1S)-1-(((2S)-2-(4-(1,3-dioxan-2-ylmethyl)-5-(4'-(2-((2S)-1-((2S)-2-((methoxycarbonyl)amino)-3-methylbutanoyl)-2-pyrrolidinyl)-1H-imidazol-4-yl)-4-biphenylyl)-1H-imidazol-2-yl)-1-pyrrolidinyl)carbonyl)-2-methylpropyl)carbamate;
methyl ((1S,2R)-1-(((2S)-2-(4-(2,2-dimethoxyethyl)-5-(4'-(2-((2S)-1-(N-(methoxycarbonyl)-O-methyl-L-threonyl)-2-pyrrolidinyl)-1H-imidazol-4-yl)-4-biphenylyl)-1H-imidazol-2-yl)-1-pyrrolidinyl)carbonyl)-2-methoxypropyl)carbamate;
methyl ((1S)-1-(((2S)-2-(4-(2,2-dimethoxyethyl)-5-(4'-(2-((2S)-1-((2S)-2-((methoxycarbonyl)amino)-3-methylbutanoyl)-2-pyrrolidinyl)-1H-imidazol-4-yl)-4-biphenylyl)-1H-imidazol-2-yl)-1-pyrrolidinyl)carbonyl)-2-methylpropyl)carbamate;
methyl (2-((2S)-1-(N-(methoxycarbonyl)-O-methyl-L-threonyl)-2-pyrrolidinyl)-4-(4'-(2-((2S)-1-(N-(methoxycarbonyl)-O-methyl-L-threonyl)-2-pyrrolidinyl)-1H-imidazol-4-yl)-4-biphenylyl)-1H-imidazol-5-yl)acetate;
methyl (2-((2S)-1-(N-(methoxycarbonyl)-L-valyl)-2-pyrrolidinyl)-4-(4'-(2-((2S)-1-(N-(methoxycarbonyl)-L-valyl)-2-pyrrolidinyl)-1H-imidazol-4-yl)-4-biphenylyl)-1H-imidazol-5-yl)acetate;
methyl ((1S)-2-((2S)-2-(4-(4'-(2-((2S)-1-(N-(methoxycarbonyl)-L-alanyl)-2-pyrrolidinyl)-4-propyl-1H-imidazol-5-yl)-4-biphenylyl)-1H-imidazol-2-yl)-1-pyrrolidinyl)-1-methyl-2-oxoethyl)carbamate;
(1R)-2-((2S)-2-(4-(4'-(2-((2S)-1-((2R)-2-(diethylamino)-2-phenylacetyl)-2-pyrrolidinyl)-1H-imidazol-4-yl)-4-biphenylyl)-5-propyl-1H-imidazol-2-yl)-1-pyrrolidinyl)-N,N-diethyl-2-oxo-1-phenylethanamine;
methyl ((1S)-1-(((2S)-2-(4-(4'-(2-((2S)-1-((2S)-2-((methoxycarbonyl)amino)-3-methylbutanoyl)-2-pyrrolidinyl)-1H-imidazol-4-yl)-4-biphenylyl)-5-propyl-1H-imidazol- 2-yl)-1-pyrrolidinyl)carbonyl)-2-methylpropyl)carbamate;
methyl ((1S)-1-(((2S)-4,4-difluoro-2-(4-(3'-fluoro-4'-(2-((2S)-1-((2S)-2-((methoxycarbonyl)amino)-3-methylbutanoyl)-2-pyrrolidinyl)-1H-imidazol-4-yl)-4-biphenylyl)-1H-imidazol-2-yl)-1-pyrrolidinyl)carbonyl)-2-methylpropyl)carbamate;
(1R)-2-((2S)-2-(4-(4'-(2-((2S)-1-((2R)-2-(diethylamino)-2-phenylacetyl)-4,4-difluoro-2-pyrrolidinyl)-1H-imidazol-4-yl)-3-fluoro-4-biphenylyl)-1H-imidazol-2-yl)-1-pyrrolidinyl)-N,N-diethyl-2-oxo-1-phenylethanamine;
methyl ((1S)-1-(((2S)-2-(4-(hydroxymethyl)-5-(4'-(2-((2S)-1-((2S)-2-((methoxycarbonylamino)-3-methylbutanoyl)-2-pyrrolidinyl)-1H-imidazol-4-yl)-4-biphenylyl)-1H-imidazol-2-yl)-1-pyrrolidinyl)carbonyl)-2-methylpropyl)carbamate;
methyl ((1S)-2-((2S)-2-(4-(4'-(4-(hydroxymethyl)-2-((2S)-1-(N-(methoxycarbonyl)-L-alanyl)-2-pyrrolidinyl)-1H-imidazol-5-yl)-4-biphenylyl)-1H-imidazol-2-yl)-1-pyrrolidinyl)-1-methyl-2-oxoethyl)carbamate;
(N,N'-(4,4'-biphenyldiylbis(1H-imidazole-4,2-diyl(2S)-2,1-pyrrolidinediyl((2S)-3-methyl-1-oxo-1,2-butanediyl)))di(2-pyrimidinamine);
N,N'-(4,4'-biphenyldiylbis(1H-imidazole-4,2-diyl(2S)-2,1-pyrrolidinediyl((1S)-1-cyclopropyl-2-oxo-2,1-ethanediyl)))di(2-pyrimidinamine);
N,N'-(4,4'-biphenyldiylbis(1H-imidazole-4,2-diyl(2S)-2,1-pyrrolidinediyl((2S)-1-oxo-1,2-propanediyl)))di(2-pyrimidinamine);
N,N'-(4,4'-biphenyldiylbis(1H-imidazole-4,2-diyl(2S)-2,1-pyrrolidinediyl((2S)-1-oxo-1,2-butanediyl)))di(2-pyrimidinamine);
N,N'-(4,4'-biphenyldiylbis(1H-imidazole-4,2-diyl(2S)-2,1-pyrrolidinediyl((2S)-3-methoxy-1-oxo-1,2-propanediyl)))di(2-pyrimidinamine); and
N,N'-(4,4'-biphenyldiylbis(1H-imidazole-4,2-diyl(2S)-2,1-pyrrolidinediyl((2S,3R)-3-methoxy-1-oxo-1,2-butanediyl)))di(2-pyrimidinamine); 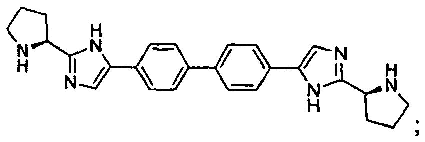 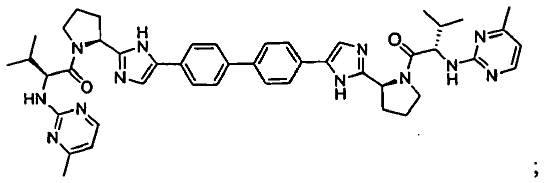 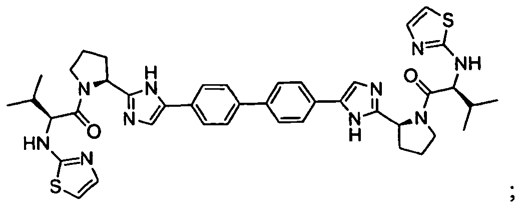 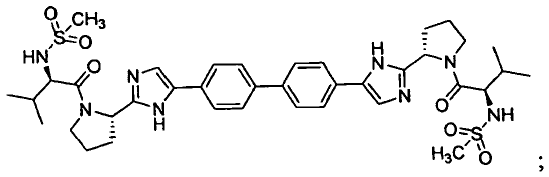 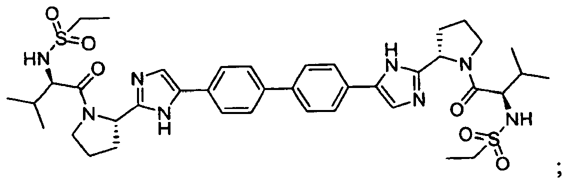 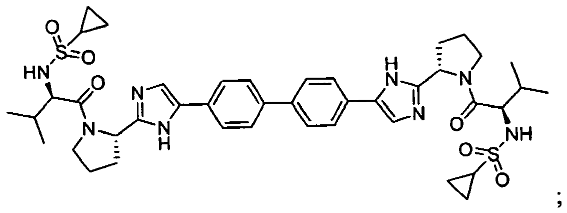 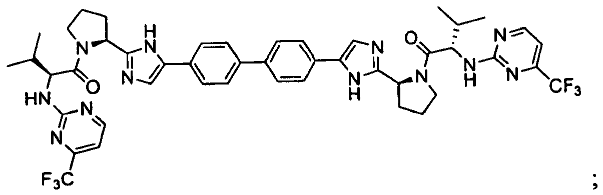 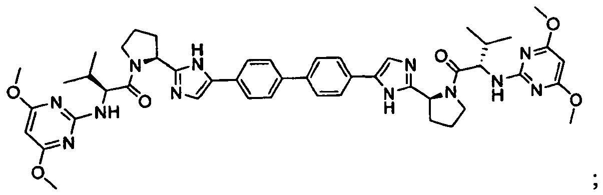 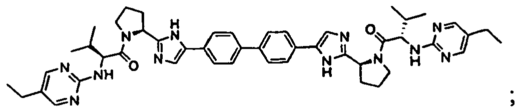 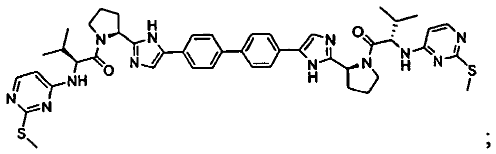 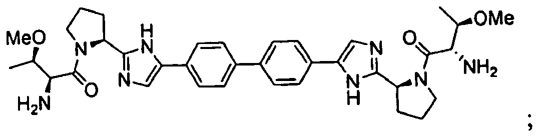 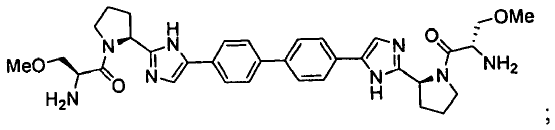 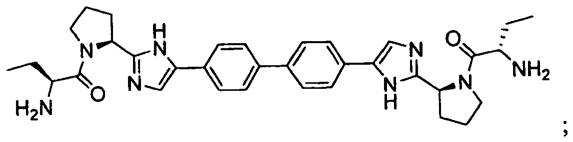 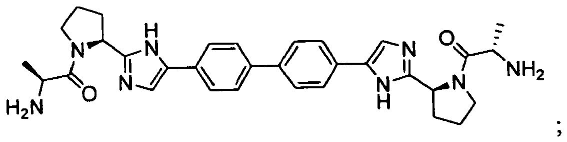 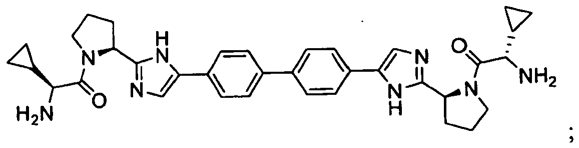 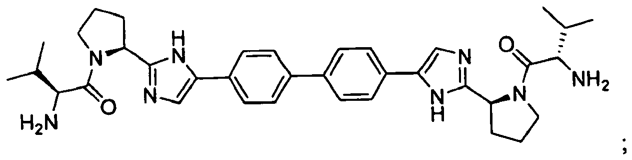 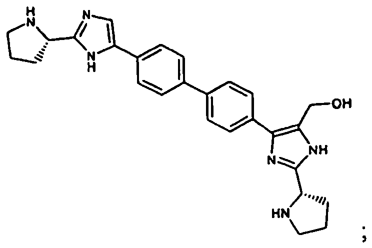 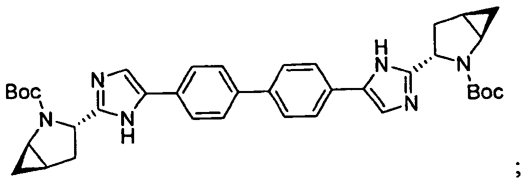 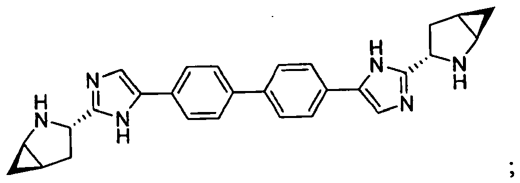 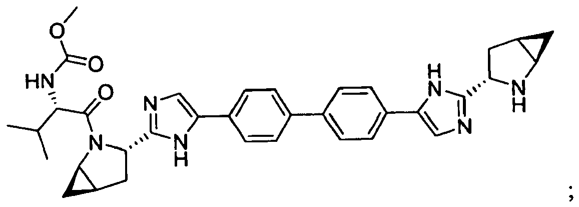 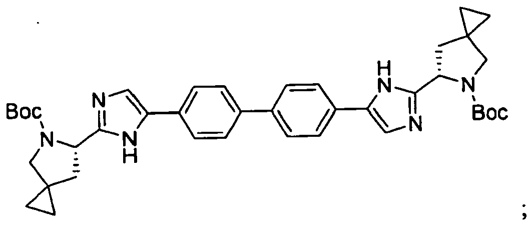 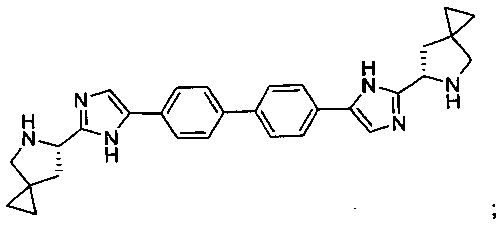 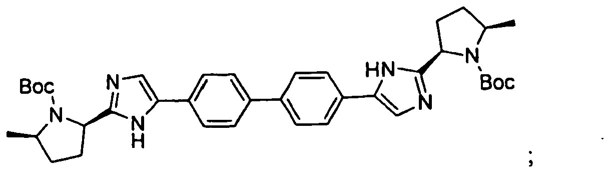 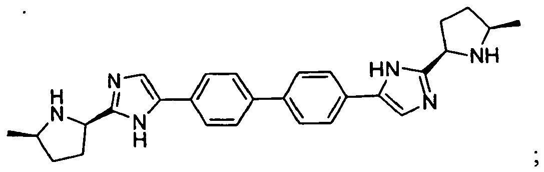 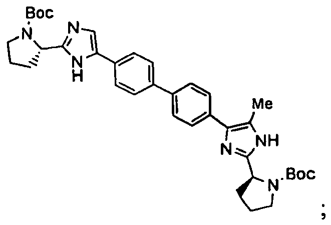 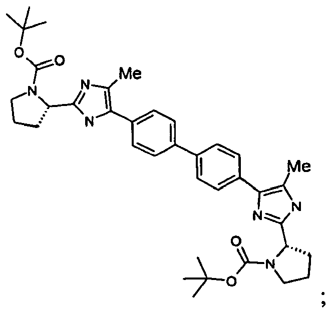 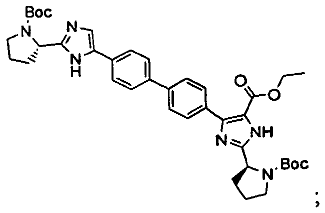 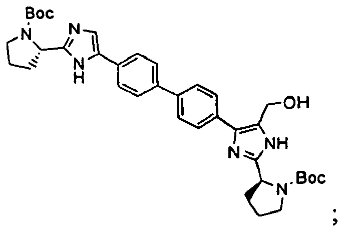 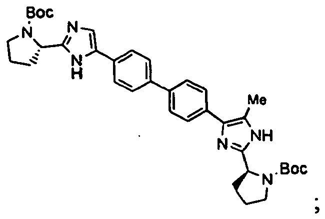 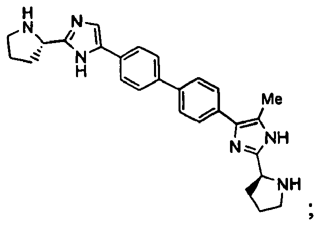 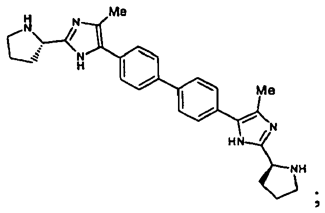 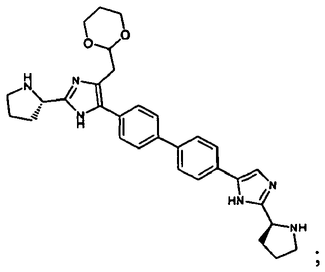 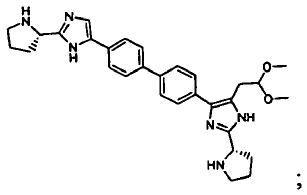 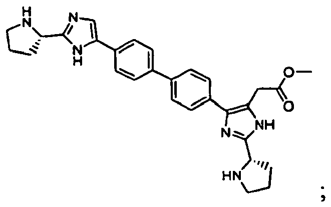 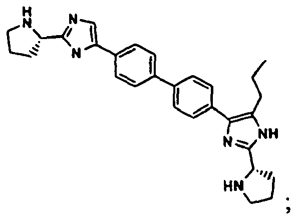 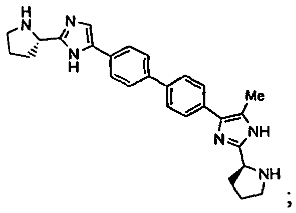 and 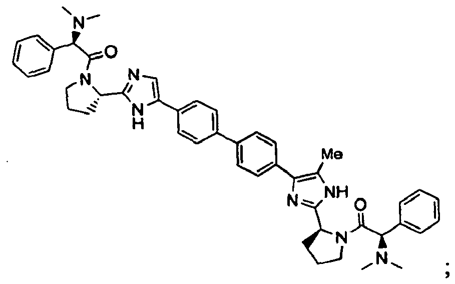 or a pharmaceutically acceptable salt thereof.

2. A composition comprising a compound of claim 1, or a pharmaceutically acceptable salt thereof, and a pharmaceutically acceptable carrier.

3. The composition of claim 2 further comprising one or two additional compounds having anti-HCV activity.

4. The composition of claim 3 wherein at least one of the additional compounds is an interferon or a ribavirin.

5. The composition of claim 4 wherein the interferon is selected from interferon alpha 2B, pegylated interferon alpha, consensus interferon, interferon alpha 2A, and lymphoblastiod interferon tau.

6. The composition of claim 3 wherein at least one of the additional compounds is selected from interleukin 2, interleukin 6, interleukin 12, a compound that enhances the development of a type 1 helper T cell response, interfering RNA, anti-sense RNA, Imiqimod, ribavirin, an inosine 5'-monophospate dehydrogenase inhibitor, amantadine, and rimantadine.

7. The composition of claim 3 wherein at least one of the additional compounds is effective to inhibit the function of a target selected from HCV metalloprotease, HCV serine protease, HCV polymerase, HCV helicase, HCV NS4B protein, HCV entry, HCV assembly, HCV egress, HCV NS5A protein, and IMPDH for the treatment of an HCV infection.

8. A compound of claim 1 or a pharmaceutically acceptable salt thereof for use in treating an HCV infection in a patient.

9. The compound for use according to claim 8 further comprising using one or two additional compounds having anti-HCV activity prior to, after or simultaneously with the compound of claim 1 or 2, or a pharmaceutically acceptable salt thereof.

10. The compound for use according to claim 9 wherein at least one of the additional compounds is an interferon or a ribavirin.

11. The compound for use according to claim 10 wherein the interferon is selected from interferon alpha 2B, pegylated interferon alpha, consensus interferon, interferon alpha 2A, and lymphoblastiod interferon tau.

12. The compound for use according to claim 9 wherein at least one of the additional compounds is selected from interleukin 2, interleukin 6, interleukin 12, a compound that enhances the development of a type 1 helper T cell response, interfering RNA, anti-sense RNA, Imiqimod, ribavirin, an inosine 5'-monophospate dehydrogenase inhibitor, amantadine, and rimantadine.

13. The compound for use according to claim 9 wherein at least one of the additional compounds is effective to inhibit the function of a target selected from HCV metalloprotease, HCV serine protease, HCV polymerase, HCV helicase, HCV NS4B portein, HCV entry, HCV assembly, HCV egress, HCV NS5A protein, and IMPDH for the treatment of an HCV infection.

## Patentansprüche

1. Eine Verbindung, ausgewählt aus
Methyl-((1S)-2-((1R,3S,5R)-3-(4-(4'-(2-((1R,3S,5R)-2-(N-(methoxycarbonyl)-L-alanyl)-2-azabicyclo[3.1.0]hex-3-yl)-1 H-imidazol-4-yl)-4-biphenylyl)-1 H-imidazol-2-yl)-2-azabicyclo[3.1.0]hex-2-yl)-1-methyl-2-oxoethyl)carbamat;
Dimethyl-(4,4'-biphenyldiylbis(1H-imidazol-4,2-diyl(1R,3S,5R)-2-azabicyclo[3.1.0]hexan-3,2-diyl((2S)-1-oxo-1,2-butanediyl)))biscarbamat;
Methyl-(2-((1R,3S,5R)-3-(4-(4'-(2-((1R,3S,5R)-2-(((methoxycarbonyl)amino)acetyl)-2-azabicyclo[3.1.0]hex-3-yl)-1H-imidazol-4-yl)-4-biphenylyl)-1H-imidazol-2-yl)-2-azabicyclo [3.1.0]hex-2-yl)-2-oxoethyl)carbamat;
Dimethyl-(4,4'-biphenyldiylbis(1H-imidazol-4,2-diyl(1R,3S,5R)-2-azabicyclo[3.1.0]hexan-3,2-diyl((1S)-1-cyclopropyl-2-oxo-2,1-ethanediyl)))biscarbamat;
Methyl-((1R)-1-(((1R,3S,5R)-3-(4-(4'-(2-((1R,3S,5R)-2-((2R)-2-((methoxycarbonyl)amino)-3-methylbutanoyl)-2-azabicyclo[3.1.0]hex-3-yl)-1H-imidazol-4-yl)-4-biphenylyl)-1H-imidazol-2-yl)-2-azabicyclo[3.1.0]hex-2-yl)carbonyl)-2-methylpropyl)carbamat;
Dimethyl-(4,4'-biphenyldiylbis(1H-imidazol-4,2-diyl(1R,3S,5R)-2-azabicyclo[3.1.0]hexan-3,2-diyl((1R)-2-oxo-1-phenyl-2,1-ethanediyl)))biscarbamat;
Methyl-((1S)-1-(((1R,3S,5R)-3-(4-(4'-(2-((1R,3S,5R)-2-acetyl-2-azabicyclo[3.1.0] hex-3-yl)-1H-imidazol-4-yl)-4-biphenylyl)-1H-imidazol-2-yl)-2-azabicyclo[3.1.0]hex-2-yl)carbonyl)-2-methylpropyl)carbamat;
Methyl-((1S)-2-methyl-1-(((1R,3S,5R)-3-(4-(4'-(2-((1R,3S,5R)-2-(5-((3aS,4S,6aR)-2-oxohexahydro-1H-thieno[3,4-d]imidazol-4-yl)pentanoyl)-2-azabicyclo[3.1.0]hex-3-yl)-1H-imidazol-4-yl)-4-biphenylyl)-1H-imidazol-2-yl)-2-azabicyclo[3.1.0]hex-2-yl)carbonyl-2-methylpropyl)carbamat;
N-((1S)-1-(((1R,3S,5R)-3-(4-(4'-(2-((1R,3S,5R)-2-((2S)-2-Acetamido-3-methylbutanoyl)-2-azabicyclo[3.1.0]hex-3-yl)-1H-imidazol-4-yl)-4-biphenylyl)-1 H-imidazol-2-yl)-2-azabicyclo[3.1.0]hex-2-yl)carbonyl)-2-methylpropyl)acetamid;
tert-Butyl-((1S)-1-(((1R,3S,5R)-3-(4-(4'-(2-((1R,3S,5R)-2-((2S)-2-((tert-butoxycarbonyl)amino)-3-methylbutanoyl)-2-azabicyclo[3.1.0]hex-3-yl)-1H-imidazol-4-yl)-4-biphenylyl)-1H-imidazol-2-yl)-2-azabicyclo[3.1.0]hex-2-yl)carbonyl)-2-methylpropyl)carbamat;
(2S)-1-((1R,3S,5R)-3-(4-(4'-(2-((1R,3S,5R)-2-((2S)-2-Amino-3-methylbutanoyl)-2-azabicyclo[3.1.0]hex-3-yl)-1H-imidazol-5-yl)-4-biphenylyl)-1H-imidazol-2-yl)-2-azabicyclo[3.1.0]hex-2-yl)-3-methyl-1-oxo-2-butanamin;
N-((1S)-1-(((1R,3S,5R)-3-(4-(4'-(2-((1R,3S,5R)-2-((2S)-2-((Cyclopropylcarbonyl)amino)-3-methylbutanoyl)-2-azabicyclo[3.1.0]hex-3-yl)-1H-imidazol-4-yl)-4-biphenylyl)-1H-imidazol-2-yl)-2-azabicyclo[3.1.0]hex-2-yl)carbonyl)-2-methylpropyl)cyclopropancarbamat;
tert-Butyl-((1R)-1-(((1R,3S,5R)-3-(4-(4'-(2-((1R,3S,5R)-2-((2R)-2-((tert-butoxycarbonyl)amino)-3-methylbutanoyl)-2-azabicyclo[3.1.0]hex-3-yl)-1H-imidazol-4-yl)-4-biphenylyl)-1H-imidazol-2-yl)-2-azabicyclo[3.1.0]hex-2-yl)carbonyl)-2-methylpropyl)carbamat;
N-((1R)-1-(((1R,3S,5R)-3-(4-(4'-(2-((1R,3S,5R)-2-((2R)-2-Acetamido-3-methylbutanoyl)-2-azabicyclo[3.1.0]hex-3-yl)-1H-imidazol-4-yl)-4-biphenylyl)-1H-imidazol-2-yl)-2-azabicyclo[3.1.0]hex-2-yl)carbonyl)-2-methylpropyl)acetamid;
N,N'-(4,4'-Biphenyldiylbis(1H-imidazol-4,2-diyl(1R,3S,5R)-2-azabicyclo[3..1.0]hexan-3,2-diyl((2S)-3-methyl-1-oxo-1,2-butandiyl)))di(2-pyrimidinamin);
Methyl-((1S)-1-(((6S)-6-(4-(4'-(2-((6S)-5-((2S)-2-((methoxycarbonyl)amino)-3-methylbutanoyl)-5-azaspiro[2.4]hept-6-yl)-1H-imidazol-4-yl)-4-biphenylyl)-1H-imidazol-2-yl)-5-azaspiro[2.4]hept-5-yl)carbonyl)-2-methylpropyl)carbamat;
Methyl-((1S)-1-(((2S,5S)-2-(4-(4'-(2-((2S,5S)-1-((2S)-2-((methoxycarbonyl)amino)-3-methylbutanoyl)-5-methyl-2-pyrrolidinyl)-1H-imidazol-4-yl)-4-biphenylyl)-1H-imidazol-2-yl)-5-methyl-1-pyrrolidinyl)carbonyl)-2-methylpropyl)carbamat;
Methyl-(2-((6S)-6-(4-(4'-(2-((6S)-5-(((methoxycarbonyl)amino)acetyl)-5-azaspiro[2.4]hept-6-yl)-1H-imidazol-4-yl)-4-biphenylyl)-1H-imidazol-2-yl)-5-azaspiro[2.4]hept-5-yl)-2-oxoethyl)carbamat;
Methyl-((1S)-2-((6S)-6-(4-(4'-(2-((6S)-5-(N-(methoxycarbonyl)-L-alanyl)-5-azaspiro[2.4]hept-6-yl)-1H-imidazol-4-yl)-4-biphenylyl)-1H-imidazol-2-yl)-5-azaspiro[2.4]hept-5-yl)-1-methyl-2-oxoethyl)carbamat;
Dimethyl-(4,4'-biphenyldiylbis(1H-imidazol-4,2-diyl(6S)-5-azaspiro[2.4]heptan-6,5-diyl((2S)-1-oxo-1,2-butandiyl)))biscarbamat;
Methyl-((1R)-1-(((6S)-6-(4-(4'-(2-((6S)-5-((2R)-2-((methoxycarbonyl)amino)-3-methylbutanoyl)-5-azaspiro[2.4]hept-6-yl)-1H-imidazol-4-yl)-4-biphenylyl)-1H-imidazol-2-yl)-5-azaspiro[2.4]hept-5-yl)carbonyl)-2-methylpropyl)carbamat;
Dimethyl-(4,4'-biphenyldiylbis(1H-imidazol-4,2-diyl(6S)-5-azaspiro[2.4]heptan-6,5-diyl((1R)-2-oxo-1-phenyl-2,1-ethandiyl)))biscarbamat;
Methyl-((1R)-1-(((2S,5S)-2-(4-(4'-(2-((2S,5S)-1-((2R)-2-((methoxycarbonyl)amino)-3-methylbutanoyl)-5-methyl-2-pyrrolidinyl)-1H-imidazol-4-yl)-4-biphenylyl)-1H-imidazol-2-yl)-5-methyl-1-pyrrolidinyl)carbonyl)-2-methylpropyl)carbamat;
Dimethyl-(4,4'-biphenyldiylbis(1H-imidazol-4,2-diyl((2S,5S)-5-methyl-2,1-pyrrolidindiyl)((1R)-2-oxo-1-phenyl-2,1-ethandiyl)))biscarbamat;
Dimethyl-(4,4'-biphenyldiylbis(1H-imidazol-4,2-diyl((2S,5S)-5-methyl-2,1-pyrrolidindiyl)((2S)-1-oxo-1,2-butandiyl)))biscarbamat;
Methyl-(2-((2S,5S)-2-(4-(4'-(2-((2S,5S)-1-(((methoxycarbonyl)amino)acetyl)-5-methyl-2-pyrrolidinyl)-1H-imidazol-4-yl)-4-biphenylyl)-1H-imidazol-2-yl)-5-methyl-1-pyrrolidinyl)-2-oxoethyl)carbamat;
Methyl-(2-((2S,5S)-2-(4-(4'-(2-((2S,5S)-1-(2-((methoxycarbonyl)amino)-2-methylpropanoyl)-5-methyl-2-pyrrolidinyl)-1H-imidazol-4-yl)-4-biphenylyl)-1H-imidazol-2-yl)-5-methyl-1-pyrrolidinyl)-1,1-dimethyl-2-oxoethyl)carbamat;
Methyl-((1S)-2-((2S,5S)-2-(4-(4'-(2-((2S,5S)-1-(N-(methoxycarbonyl)-L-alanyl)-5-methyl-2-pyrrolidinyl)-1H-imidazol-4-yl)-4-biphenylyl)-1H-imidazol-2-yl)-5-methyl-1-pyrrolidinyl)-1-methyl-2-oxoethyl)carbamat;
4,4'-(4,4'-Biphenyldiyl)bis(2-((2S,5S)-5-methyl-1-(3-methylbutanoyl)-2-pyrrolidinyl)-1H-imidazol);
4,4'-(4,4'-Biphenyldiyl)bis(2-((2S,5S)-5-methyl-1-phenylacetyl)-2-pyrrolidinyl)-1H-imidazol);
(2R,2'R)-1,1'-(4,4'-Biphenyldiylbis(1H-imidazol-4,2-diyl((2S,5S)-5-methyl-2,1-pyrrolidindiyl)))bis(3-methyl-1-oxo-2-butanol);
(2S,2'S)-1,1'-(4,4'-Biphenytdiylbis(1H-imidazol-4,2-diyl((2S,5S)-5-methyl-2,1-pyrrolidindiyl)))bis(3-methyl-1-oxo-2-butanol);
2-((2S,5S)-1-Acetyl-5-methyl-2-pyrrolidinyl)-4-(4'-(2-((2S,5S)-1-acetyl-5-methyl-2-pyrrolidinyl)-1 H-imidazol-5-yl)-4-biphenylyl)-1 H-imidazol;
tert-Butyl-(2S)-2-(4-(4'-(2-((2S)-1-(tert-butoxycarbonyl)-2-pyrrolidinyl)-4-(1,3-dioxan-2-ylmethyl)-1H-imidazol-5-yl)-4-biphenylyl)-1H-imidazol-2-yl)-1-pyrrolidincarboxylat;
tert-Butyl-(2S)-2-(4-(4'-(2-((2S)-1-(tert-butoxycarbonyl)-2-pyrrolidinyl)-4-(1,3-dioxolan-2-ylmethyl)-1H-imidazol-5-yl)-4-biphenylyl)-1H-imidazol-2-yl)-1-pyrrolidincarboxylat;
tert-Butyl-(2S)-2-(4-(4'-(2-((2S)-1-(tert-butoxycarbonyl)-2-pyrrolidinyl)-1H-imidazol-4-yl)-4-biphenylyl)-5-(2-methoxy-2-oxoethyl)-1H-imidazol-2-yl)-1-pyrrolidincarboxylat;
tert-Butyl-(2S)-2-(4-(4'-(2-((2S)-1-(tert-butoxycarbonyl)-2-pyrrolidinyl)-1H-imidazol-4-yl)-4-biphenylyl)-5-propyl-1H-imidazol-2-yl)-1-pyrrolidincarboxylat;
Ethyl-2-((2S)-1-(tert-butoxycarbonyl)-2-pyrrolidinyl)-5-(4'-(2-((2S)-1-(tert-butoxycarbonyl)-2-pyrrolidinyl)-1H-imidazol-5-yl)-4-biphenylyl)-1 H-imidazol-4-carboxylat;
tert-Butyl-(2S)-2-(4-(4'-(2-((2S)-1-(tert-butoxycarbonyl)-2-pyrrolidinyl)-1H-imidazol-4-yl)-3'-fluoro-4-biphenylyl)-1 H-imidazol-2-yl)-4,4-difluoro-1-pyrrolidincarboxylat;
2-((2S)-4,4-Difluoro-2-pyrrolidinyl)-4-(3'-fluoro-4'-(2-((2S)-2-pyrrolidinyl)-1H-imidazol-4-yl)-4-biphenylyl)-1H-imidazol;
(1R)-2-((2S)-2-(4-(4'-(2-((2S)-1-((2R)-2-(Diethylamino)-2-phenylacetyl)-2-pyrrolidinyl)-1H-imidazol-4-yl)-4-biphenylyl)-5-methyl-1H-imidazol-2-yl)-1-pyrrolidinyl)-N,N-diethyl-2-oxo-1-phenylethanamin;
1-((1R)-2-((2S)-2-(4-(4'-(4-Methyl-2-((2S)-1-((2R)-2-phenyl-2-(1-piperidinyl)acetyl)-2-pyrrolidinyl)-1H-imidazol-5-yl)-4-biphenylyl)-1H-imidazol-2-yl)-1-pyrrolidinyl)-2-oxo-1-phenylethyl)piperidin;
Methyl-((1R)-2-((2S)-2-(4-(4'-(2-((2S)-1-((2R)-2-((methoxycarbonyl)amino)-2-phenylacetyl)-2-pyrrolidinyl)-1H-imidazol-4-yl)-4-biphenylyl)-5-methyl-1H-imidazol-2-yl)-1-pyrrolidinyl)-2-oxo-1-phenylethyl)carbamat;
Methyl-((1S)-2-((2S)-2-(4-(4'-(2-((2S)-1-(N-(methoxycarbonyl)-L-alanyl)-2-pyrrolidinyl)-4-methyl-1H-imidazol-5-yl)-4-biphenylyl)-1H-imidazol-2-yl)-1-pyrrolidinyl)-1-methyl-2-oxoethyl)carbamat;
Methyl-((1S)-1-(((2S)-2-(4-(4'-(2-((2S)-1-((2S)-2-((methoxycarbonyl)amino)-3-methylbutanoyl)-2-pyrrolidinyl)-1H-imidazol-4-yl)-4-biphenylyl)-5-methyl-1H-imidazol-2-yl)-1-pyrrolidinyl)carbonyl)-2-methylpropyl)carbamat;
Methyl-((1S,2R)-2-methoxy-1-(((2S)-2-(4-(4'-(2-((2S)-1-(N-(methoxycarbonyl)-O-methyl-L-threonyl)-2-pyrrolidinyl)-4-methyl-1H-imidazol-5-yl)-4-biphenylyl)-1H-imidazol-2-yl)-1-pyrrolidinyl)carbonyl)propyl)carbamat;
(1R,1'R)-2,2'-(4,4'-Biphenyldiylbis((4-methyl-1H-imidazol-5,2-diyl)(2S)-2,1-pyrrolidinediyl))bis(N,N-dimethyl-2-oxo-1-phenylethanamin);
(1R,1'R)-2,2'-(4,4'-Biphenyldiylbis((4-methyl-1H-imidazol-5,2-diyl)(2S)-2,1-pyrrolidindiyl))bis(N,N-diethyl-2-oxo-1-phenylethanamin);
1,1'-(4,4'-Biphenyldiylbis((4-methyl-1H-imidazol-5,2-diyl)(2S)-2,1-pyrrolidindiyl((1R)-2-oxo-1-phenyl-2,1-ethandiyl)))dipiperidin;
Dimethyl-(4,4'-biphenyldiylbis((4-methyl-1H-imidazol-5,2-diyl)(2S)-2,1-pyrrolidindiyl((1R)-2-oxo-1-phenyl-2,1-ethandiyl)))biscarbamat;
Methyl-((1S)-2-((2S)-2-(4-(4'-(2-((2S)-1-(N-(methoxycarbonyl)-L-alanyl)-2-pyrrolidinyl)-5-methyl-1H-imidazol-4-yl)-4-biphenylyl)-5-methyl-1H-imidazol-2-yl)-1-pyrrolidinyl)-1-methyl-2-oxoethyl)carbamat;
Methyl-((1S)-1-(((2S)-2-(4-(4'-(2-((2S)-1-((2S)-2-((methoxycarbonyl)amino)-3-methylbutanoyl)-2-pyrrolidinyl)-4-methyl-1H-imidazol-5-yl)-4-biphenylyl)-5-methyl-1H-imidazol-2-yl)-1-pyrrolidinyl)carbonyl)-2-methylpropyl)carbamat;
Methyl-((1S,2R)-2-methoxy-1-(((2S)-2-(4-(4'-(2-((2S)-1-(N-(methoxycarbonyl)-O-methyl-L-threonyl)-2-pyrrolidinyl)-5-methyl-1H-imidazol-4-yl)-4-biphenylyl)-5-methyl-1H-imidazol-2-yl)-1-pyrrolidinyl)carbonyl)propyl)carbamat;
Methyl-((1S)-1-(((2S)-2-(4-(1,3-dioxan-2-ylmethyl)-5-(4'-(2-((2S)-1-((2S)-2-((methoxycarbonyl)amino)-3-methylbutanoyl)-2-pyrrolidinyl)-1H-imidazol-4-yl)-4-biphenylyl)-1H-imidazol-2-yl)-1-pyrrolidinyl)carbonyl)-2-methylpropyl)carbamat;
Methyl-((1S,2R)-1-(((2S)-2-(4-(2,2-dimethoxyethyl)-5-(4'-(2-((2S)-1-(N-(methoxycarbonyl)-O-methyl-L-threonyl)-2-pyrrolidinyl)-1H-imidazol-4-yl)-4-biphenylyl)-1H-imidazol-2-yl)-1-pyrrolidinyl)carbonyl)-2-methoxypropyl)carbamat;
Methyl-((1S)-1-(((2S)-2-(4-(2,2-dimethoxyethyl)-5-(4'-(2-((2S)-1-((2S)-2-((methoxycarbonyl)amino)-3-methylbutanoyl)-2-pyrrolidinyl)-1H-imidazol-4-yl)-4-biphenylyl)-1H-imidazol-2-yl)-1-pyrrolidinyl)carbonyl)-2-methylpropyl)carbamat;
Methyl-(2-((2S)-1-(N-(methoxycarbonyl)-O-methyl-L-threonyl)-2-pyrrolidinyl)-4-(4'-(2-((2S)-1-(N-(methoxycarbonyl)-O-methyl-L-threonyl)-2-pyrrolidinyl)-1H-imidazol-4-yl)-4-biphenylyl)-1H-imidazol-5-yl)acetat;
Methyl-(2-((2S)-1-(N-(methoxycarbonyl)-L-valyl)-2-pyrrolidinyl)-4-(4'-(2-((2S)-1-(N-(methoxycarbonyl)-L-valyl)-2-pyrrolidinyl)-1H-imidazol-4-yl)-4-biphenylyl)-1 H-imidazol-5-yl)acetat;
Methyl-((1S)-2-((2S)-2-(4-(4'-(2-((2S)-1-(N-(methoxycarbonyl)-L-alanyl)-2-pyrrolidinyl)-4-propyl-1H-imidazol-5-yl)-4-biphenylyl)-1H-imidazol-2-yl)-1-pyrrolidinyl)-1-methyl-2-oxoethyl)carbamat;
(1R)-2-((2S)-2-(4-(4'-(2-((2S)-1-((2R)-2-(Diethylamino)-2-phenylacetyl)-2-pyrrolidinyl)-1H-imidazol-4-yl)-4-biphenylyl)-5-propyl-1H-imidazol-2-yl)-1-pyrrolidinyl)-N,N-diethyl-2-oxo-1-phenylethanamin;
Methyl-((1S)-1-(((2S)-2-(4-(4'-(2-((2S)-1-((2S)-2-((methoxycarbonyl)amino)-3-methylbutanoyl)-2-pyrrolidinyl)-1H-imidazol-4-yl)-4-biphenylyl)-5-propyl-1H-imidazol-2-yl)-1-pyrrolidinyl)carbonyl)-2-methylpropyl)carbamat;
Methyl-((1S)-1-(((2S)-4,4-difluoro-2-(4-(3'-fluoro-4'-(2-((2S)-1-((2S)-2-((methoxycarbonyl)amino)-3-methylbutanoyl)-2-pyrrolidinyl)-1H-imidazol-4-yl)-4-biphenylyl)-1H-imidazol-2-yl)-1-pyrrolidinyl)carbonyl)-2-methylpropyl)carbamat;
(1R)-2-((2S)-2-(4-(4'-(2-((2S)-1-((2R)-2-(Diethylamino)-2-phenylacetyl)-4,4-difluoro-2-pyrrolidinyl)-1H-imidazol-4-yl)-3-fluoro-4-biphenylyl)-1 H-imidazol-2-yl)-1-pyrrolidinyl)-N,N-diethyl-2-oxo-1-phenylethanamin;
Methyl((1S)-1-(((2S)-2-(4-(hydroxymethyl)-5-(4'-(2-((2S)-1-((2S)-2-((methoxycarbonyl)amino)-3-methylbutanoyl)-2-pyrrolidinyl)-1H-imidazol-4-yl)-4-biphenylyl)-1H-imidazol-2-yl)-1-pyrrolidinyl)carbonyl)-2-methylpropyl)carbamat;
Methyl-((1S)-2-((2S)-2-(4-(4'-(4-(hydroxymethyl)-2-((2S)-1-(N-(methoxycarbonyl)-L-alanyl)-2-pyrrolidinyl)-1H-imidazol-5-yl)-4-biphenylyl)-1H-imidazol-2-yl)-1-pyrrolidinyl)-1-methyl-2-oxoethyl)carbamat;
(N,N'-(4,4'-Biphenyldiylbis(1H-imidazol-4,2-diyl(2S)-2,1-pyrrolidindiyl((2S)-3-methyl-1-oxo-1,2-butandiyl)))di(2-pyrimidinamin);
N,N'-(4,4'-Biphenyldiylbis(1H-imidazol-4,2-diyl(2S)-2,1-pyrrolidindiyl((1S)-1 - cyclopropyl-2-oxo-2,1-ethandiyl)))di(2-pyrimidinamin);
N,N'-(4,4'-Biphenyldiylbis(1H-imidazol-4,2-diyl(2S)-2,1-pyrrolidindiyl((2S)-1-oxo-1,2-propandiyl)))di(2-pyrimidinamin);
N,N'-(4,4'-Biphenyldiylbis(1H-imidazol-4,2-diyl(2S)-2,1-pyrrolidindiyl((2S)-1-oxo-1,2-butandiyl)))di(2-pyrimidinamin);
N,N'-(4,4'-Biphenyldiylbis(1H-imidazol-4,2-diyl(2S)-2,1-pyrrolidindiyl((2S)-3-methoxy-1-oxo-1,2-propandiyl)))di(2-pyrimidinamin); und
N,N'-(4,4'-Biphenyldiylbis(1H-imidazol-4,2-diyl(2S)-2,1-pyrrolidindiyl((2S,3R)-3-methoxy-1-oxo-1,2-butandiyl)))di(2-pyrimidinamin); 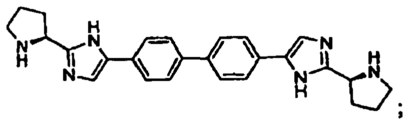 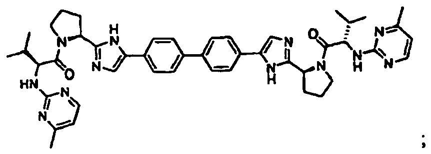 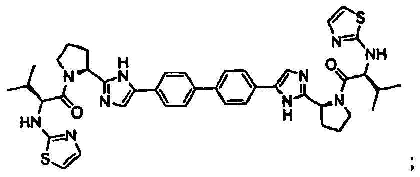 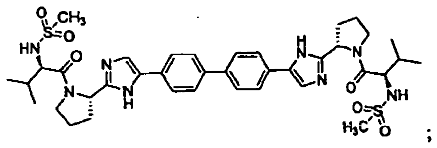 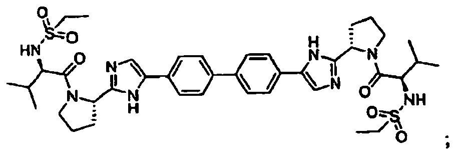 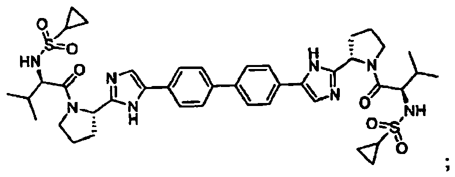 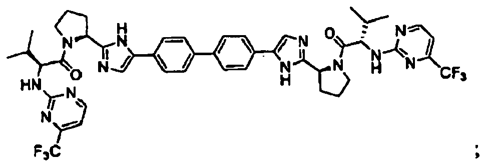 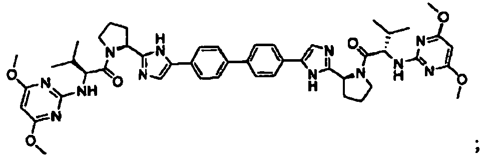 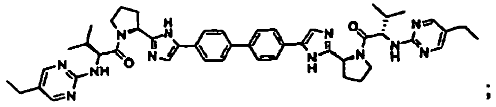 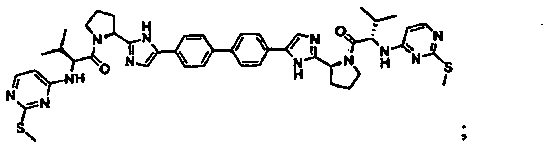 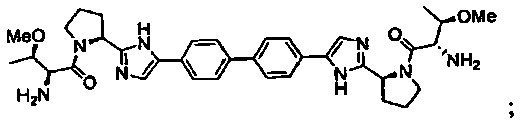 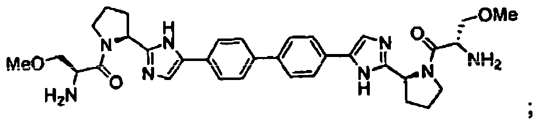 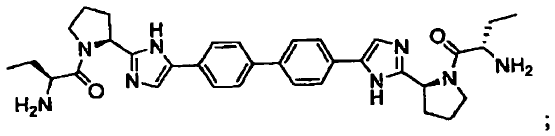 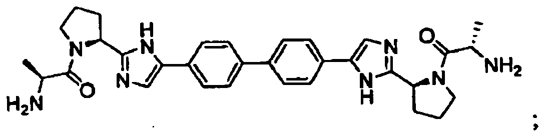 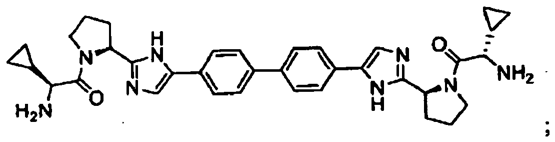 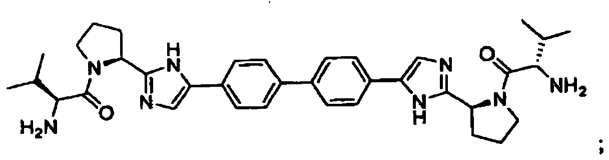 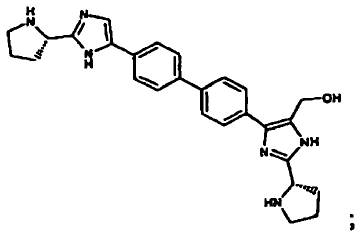 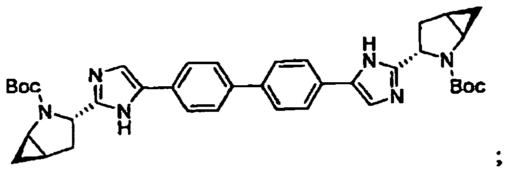 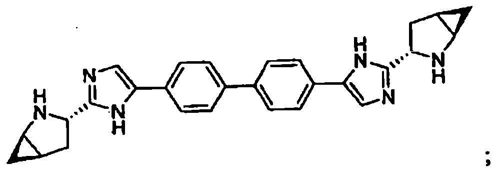 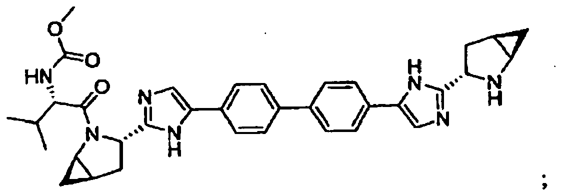 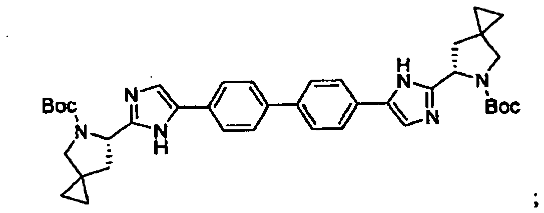 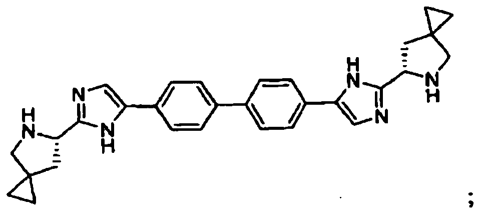 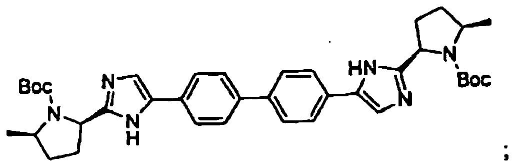 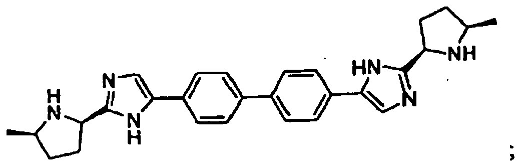 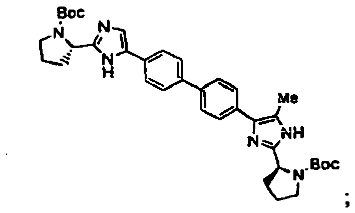 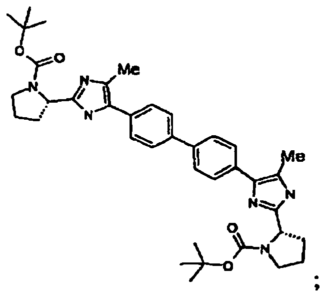 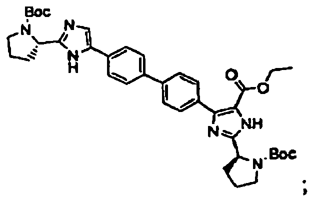 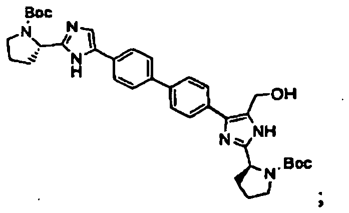 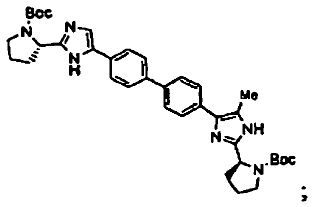 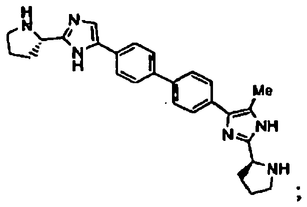 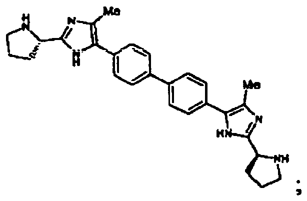 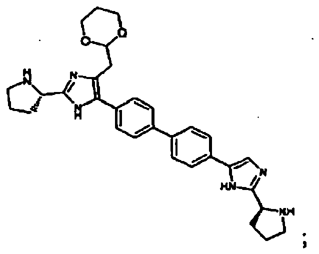 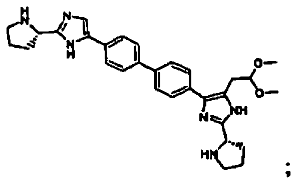 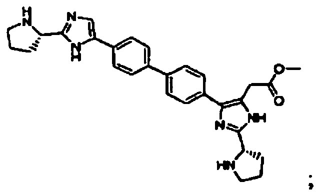 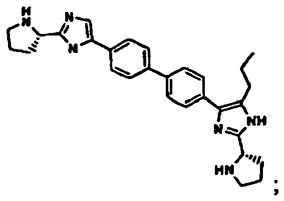 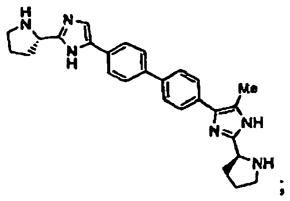 und 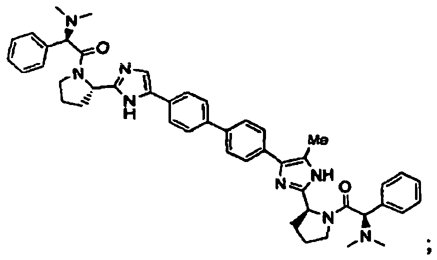 oder ein pharmazeutisch annehmbares Salz davon.

2. Eine Zusammensetzung, umfassend eine Verbindung nach Anspruch 1 oder ein pharmazeutisch annehmbares Salz davon und einen pharmazeutisch annehmbaren Träger.

3. Zusammensetzung nach Anspruch 2, weiter umfassend eine oder zwei zusätzliche Verbindungen, die eine Anti-HCV-Aktivität aufweisen.

4. Zusammensetzung nach Anspruch 3, wobei mindestens eine der zusätzlichen Verbindungen ein Interferon oder ein Ribavirin ist.

5. Zusammensetzung nach Anspruch 4, wobei das Interferon aus Interferon alpha 2B, pegyliertem Interferon alpha, consensus Interferon, Interferon alpha 2A und lymphoblastenartigem Interferon tau ausgewählt ist.

6. Zusammensetzung nach Anspruch 3, wobei mindestens eine der zusätzlichen Verbindungen aus Interleukin 2, Interleukin 6, Interleukin 12, einer Verbindung, die die Entwicklung einer Typ-1-T-Helferzellen-Antwort verbessert, interferierender RNA, Antisense-RNA, Imiqimod, Ribavirin, einem Inosin-5'-Monophospat-Dehydrogenase-Inhibitor, Amantadin und Rimantadin ausgewählt ist.

7. Zusammensetzung nach Anspruch 3, wobei mindestens eine der zusätzlichen Verbindungen wirksam ist zum Hemmen der Funktion eines Ziels, ausgewählt aus HCV-Metalloprotease, HCV-Serinprotease, HCV-Polymerase, HCV-Helicase, HCV-NS4B-Protein, HCV-Entry, HCV-Assembly, HCV-Egress, HCV-NS5A-Protein und IMPDH, für die Behandlung einer HCV-Infektion.

8. Verbindung nach Anspruch 1 oder ein pharmazeutisch annehmbares Salz davon zur Verwendung bei der Behandlung einer HCV-Infektion in einem Patienten.

9. Die Verbindung zur Verwendung nach Anspruch 8, weiter umfassend, eine oder zwei zusätzliche Verbindungen, die Anti-HCV-Aktivität aufweisen, vor der, nach der oder gleichzeitig mit der Verbindung nach Anspruch 1 oder 2 oder einem pharmazeutisch annehmbaren Salz davon zu verwenden.

10. Verbindung zur Verwendung nach Anspruch 9, wobei mindestens eine der zusätzlichen Verbindungen ein Interferon oder ein Ribavirin ist.

11. Verbindung zur Verwendung nach Anspruch 10, wobei das Interferon aus Interferon alpha 2B, pegyliertem Interferon alpha, consensus Interferon, Interferon alpha 2A und lymphoblastenartigem Interferon tau ausgewählt ist.

12. Verbindung zur Verwendung nach Anspruch 9, wobei mindestens eine der zusätzlichen Verbindungen aus Interleukin 2, Interleukin 6, Interleukin 12, einer Verbindung, die die Entwicklung einer Typ-1-T-Helferzellen-Antwort verbessert, interferierender RNA, Antisense-RNA, Imiqimod, Ribavirin, einem Inosin-5'-Monophospat-Dehydrogenase-Inhibitor, Amantadin und Rimantadin ausgewählt ist.

13. Verbindung zur Verwendung nach Anspruch 9, wobei mindestens eine der zusätzlichen Verbindungen wirksam ist zum Hemmen der Funktion eines Ziels, ausgewählt aus HCV-Metalloprotease, HCV-Serinprotease, HCV-Polymerase, HCV-Helicase, HCV-NS4B-Protein, HCV-Entry, HCV-Assembly, HCV-Egress, HCV-NS5A-Protein und IMPDH, für die Behandlung einer HCV-Infektion.

## Revendications

1. Composé sélectionné parmi
((1S)-2-((1R,3S,5R)-3-(4-(4'-(2-(1R,3S,5R)-2-(N-(méthoxycarbonyl)-L-alanyl)-2-azabicyclo[3.1.0]hex-3-yl)-1H-imidazol-4-yl)-4-biphénylyl)-1H-imidazol-2-yl)-2-azabicyclo[3.1.0]hex-2-yl)-1-méthyl-2-oxoéthyl)carbamate de méthyle;
(4,4'-biphényldiylbis(1H-imidazole-4,2-diyl(1R,3S,5R)-2-azabicyclo[3.1.0]hexane-3,2-diyl((2S)-1-oxo-1,2-butanediyl)))biscarbamate de diméthyle;
(2-((1R,3S,5R)-3-(4-(4'-(2-((1R,3S,5R)-2-(((méthoxycarbonyl)amino-acétyl)-2-azabicyclo[3.1.0]hex-3-yl)-1H-imidazol-4-yl)-4-biphénylyl)-1H-imidazol-2-yl)-2-azabicyclo[3.1.0]hex-2-yl)-2-oxoéthyl)carbamate de méthyle;
(4,4'-biphényldiylbis(1H-imidazole-4,2-diyl(1R,3S,5R)-2-azabicyclo[3.1.0]hexane-3,2-diyl((1S)-1-cyclopropyl-2-oxo-2,1-éthanediyl)))biscarbamate de diméthyle;
((1R)-1-(((1R,3S,5R)-3-(4-(4'-(2-((1R,3S,5R)-2-((2R)-2-((méthoxycarbonyl)amino)-3-méthylbutanoyl)-2-azabicyclo[3.1.0]hex-3-yl)-1H-imidazol-4-yl)-4-biphénylyl)-1 H-imidazol-2-yl)-2-azabicyclo[3.1.0]hex-2-yl)carbonyl)-2-méthylpropyl) carbamate de méthyle;
(4,4'-biphényldiylbis(1H-imidazole-4,2-diyl(1R,3S,5R)-2-azabicyclo[3.1.0]hexane-3,2-diyl((1R)-2-oxo-1-phényl-2,1-éthanediyl)))biscarbamate de diméthyle;
((1S)-1-(((1R,3S,5R)-3-(4-(4'-(2-((1R,3S,5R)-2-acétyl-2-azabicyclo[3.1.0]hex-3-yl)-1H-imidazol-4-yl)-4-biphénylyl)-1H-imidazol-2-yl)-2-azabicyclo[3.1.0]hex-2-yl)carbonyl)-2-méthylpropyl)carbamate de méthyle;
((1S)-2-méthyl-1-(((1R,3S,5R)-3-(4-(4'-(2-((1R,3S,5R)-2-(5-((3aS,4S,6aR)-2-oxohexahydro-1H-thiéno[3,4-d]-imidazol-4-yl)pentanoyl)-2-azabicyclo[3.1.0]hex-3-yl)-1H-imidazol-4-yl)-4-biphénylyl)-1H-imidazol-2-yl)-2-azabicyclo[3.1.0]hex-2-yl)carbonyl)-2-méthylpropyl)carbamate de méthyle;
N-((1S)-1-(((1R,3S,5R)-3-(4-(4'-(2-((1R,3S,5R)-2-((2S)-2-acétamido-3-méthylbutanoyl)-2-azabicyclo[3.1.0]hex-3-yl)-1H-imidazol-4-yl)-4-biphénylyl)-1 H-imidazol-2-yl)-2-azabicyclo[3.1.0]hex-2-yl)carbonyl)-2-méthylpropyl)acétamide;
((1S)-1-(((1R,3S,5R)-3-(4-(4'-(2-((1R,3S,5R)-2-((2S)-2-((tert-butoxycarbonyl)amino)-3-méthylbutanoyl)-2-azabicyclo[3.1.0]hex-3-yl)-1H-imidazol-4-yl)-4-biphénylyl)-1H-imidazol-2-yl)-2-azabicyclo[3.1.0]hex-2-yl)carbonyl-2-méthylpropyl)carbamate de tert-butyle;
(2S)-1-((1R,3S,5R)-3-(4-(4'-(2-((1R,3S,5R)-2-((2S)-2-amino-3-méthylbutanoyl)-2-azabicyclo[3.1.0]hex-3-yl)-1H-imidazol-5-yl)-4-biphénylyl)-1H-imidazol-2-yl)-2-azabicyclo[3.1.0]hex-2-yl)-3-méthyl-1-oxo-2-butanamme;
N-((1S)-1-(((1R,3S,5R)-3-(4-(4'-(2-((1R,3S,5R)-2-((2S)-2-((cyclopropylcarbonyl)amino-3-méthylbutanoyl)-2-azabicyclo[3.1.0]hex-3-yl)-1H-imidazol-4-yl)-4-biphénylyl)-1H-imidazol-2-yl)-2-azabicyclo[3.1.0]hex-2-yl)carbonyl)-2-méthylpropyl)cyclopropanecarbamate;
((1R)-1-(((1R,3S,5R)-3-(4-(4'-(2-((1R,3S,5R)-2-((2R)-2-((tert-butoxycarbonyl)amino)-3-méthylbutanoyl)-2-azabicyclo[3.1.0]hex-3-yl)-1H-imidazol-4-yl)-4-biphénylyl)-1H-imidazol-2-yl)-2-azabicyclo[3.1.0]hex-2-yl)carbonyl)-2-méthylpropyl)carbamate de tert-butyle;
N-((1R)-1-(((1R,3S,5R)-3-(4-(4'-(2-((1R,3S,5R)-2-((2R)-2-acétamido-3-méthylbutanoyl)-2-azabicyclo[3.1.0]hex-3-yl)-1H-imidazol-4-yl)-4-biphénylyl)-1H-imidazol-2-yl)-2-azabicyclo[3.1.0]hex-2-yl)carbonyl)-2-méthylpropyl)acétamide;
N,N'-(4,4'-biphényldiylbis(1H-imidazole-4,2-diyl(1R,3S,5R)-2-azabicyclo[3.1.0]hexane-3,2-diyl((2S)-3-méthyl-1-oxo-1,2-butanediyl)))di(2-pyrimidinamine);
((1S)-1-(((6S)-6-(4-(4'-(2-((6S)-5-((2S)-2-((méthoxycarbonyl)amino)-3-méthylbutanoyl)-5 -azaspiro[2.4]hept-6-yl)-1H-imidazol-4-yl)-4-biphénylyl)-1H-imidazol-2-yl)-5-azaspiro[2.4]hept-5-yl)carbonyl)-2-méthylpropyl)carbamate de méthyle;
((1S)-1-(((2S,5S)-2-(4-(4'-(2-((2S,5S)-1-((2S)-2-((méthoxycarbonyl)amino)-3-méthylbutanoyl)-5-méthyl-2-pyrrolidinyl)-1H-imidazol-4-yl)-4-biphénylyl)-1H-imidazol-2-yl)-5-méthyl-1-pyrrolidinyl)carbonyl)-2-méthylpropyl)carbamate de méthyle;
(2-((6S)-6-(4-(4'-(2-((6S)-5-(((méthoxycarbonyl)amino)acétyl)-5-azaspiro[2.4]hept-6-yl)-1H-imidazol-4-yl)-4-biphénylyl)-1H-imidazol-2-yl)-5-azaspiro[2.4]hept-5-yl)-2-oxoéthyl)carbamate de méthyle;
((1S)-2-((6S)-6-(4-(4'-(2-((6S)-5-(N-(méthoxycarbonyl)-L-alanyl)-5-azaspiro[2.4]hept-6-yl)-1H-imidazol-4-yl)-4-biphénylyl)-1H-imidazol-2-yl)-5-azaspiro[2.4]hept-5-yl)-1-méthyl-2-oxoéthyl)carbamate de méthyle;
(4,4'-biphényldiylbis(1H-imidazole-4,2-diyl(6S)-5-azaspiro[2.4]heptane-6,5-diyl((2S)-1-oxo-1,2-butanediyl)))biscarbamate de diméthyle;
((1R)-1-(((6S)-6-(4-(4'-(2-((6S)-5-((2R)-2-((méthoxycarbonyl)amino)-3-méthylbutanoyl)-5-azaspiro[2.4]hept-6-yl)-1H-imidazol-4-yl)-4-biphénylyl)-1H-imidazol-2-yl)-5-azaspiro[2.4]hept-5-yl)carbonyl)-2-méthylpropyl)carbamate de méthyle;
(4,4'-biphényldiylbis(1H-imidazole-4,2-diyl(6S)-5-azaspiro[2.4]heptane-6,5-diyl((1R)-2-oxo-1-phényl-2,1-éthanediyl)))biscarbamate de diméthyle;
((1R)-1-(((2S,5S)-2-(4-(4'-(2-((2S,5S)-1-((2R)-2-((méthoxycarbonyl)amino)-3-méthylbutanoyl)-5-méthyl-2-pyrrolidinyl)-1H-imidazol-4-yl)-4-biphénylyl)-1H-imidazol-2-yl)-5-méthyl-1-pyrrolidinyl)carbonyl)-2-méthylpropyl)carbamate de méthyle;
(4,4'-biphényldiylbis(1H-imidazole-4,2-diyl((2S,5S)-5-méthyl-2,1-pyrrolidinediyl)((1R)-2-oxo-1-phényl-2,1-éthanediyl)))biscarbamate de diméthyle;
(4,4'-biphényldiylbis(1H-imidazole-4,2-diyl((2S,5S)-5-méthyl-2,1-pyrrolidinediyl)((2S)-1-oxo-1,2-butanediyl)))biscarbamate de diméthyle;
(2-((2S,5S)-2-(4-(4'-(2-((2S,5S)-1-(((méthoxycarbonyl)amino)acétyl)-5-méthyl-2-pyrrolidinyl)-1H-imidazol-4-yl)-4-biphénylyl)-1 H-imidazol-2-yl)-5-méthyl-1-pyrrolidinyl)-2-oxoéthyl)carbamate de méthyle;
(2-((2S,5S)-2-(4-(4'-(2-((2S,5S)-1-(2-((méthoxycarbonyl)amino)-2-méthylpropanoyl)-5-méthyl-2-pyrrolidinyl)-1H-imidazol-4-yl)-4-biphénylyl)-1H-imidazol-2-yl)-5-méthyl-1-pyrrolidinyl)-1,1-diméthyl-2-oxoéthyl)carbamate de méthyle;
((1S)-2-((2S,5S)-2-(4-(4'-(2-((2S,5S)-1-(N-(méthoxycarbonyl)-L-alanyl)-5-méthyl-2-pyrrolidinyl)-1H-imidazol-4-yl)-4-biphénylyl)-1H-imidazol-2-yl)-5-méthyl-1-pyrrolidinyl)-1-méthyl-2-oxoéthyl)carbamate de méthyle;
4,4'-(4,4'-biphényldiyt)bis(2-((2S,5S)-5-méthyl-l-(3-méthylbutanoyl)-2-pyrrolidinyl)-1H-imidazole);
4,4'-(4,4'-biphényldiyl)bis(2-((2S,5S)-5-méthyl-1-(phénylacétyl)-2-pyrrolidinyl)-1H-imidazole);
(2R,2'R)-1,1'-(4,4'-biphényldiylbis(1H-imidazole-4,2-diyl((2S,5S)-5-méthyl-2,1-pyrrolidinediyl)))bis(3-méthyl-1-oxo-2-butanol);
(2S,2'S)-1,1'-(4,4'-biphényldiylbis(1H-imidazole-4,2-diyl((2S,5S)-5-méthyl-2,1-pyrrolidinediyl)))bis(3-méthyl-1-oxo-2-butanol);
2-((2S,5S)-1-acétyl-5-méthyl-2-pyrrolidinyl)-4-(4'-(2-((2S,5S)-1-acétyl-5-méthyl-2-pyrrolidinyl)-1H-imidazol-5-yl)-4-biphénylyl)-1H-imidazole;
(2S)-2-(4-(4'-(2-((2S)-1-(tert-butoxycarbonyl)-2-pyrrolidinyl)-4-(1,3-dioxan-2-ylméthyl)-1H-imidazol-5-yl)-4-biphénylyl)-1H-imidazol-2-yl)-1-pyrrolidinecarboxylate de tert-butyle;
(2S)-2-(4-(4'-(2-((2S)-1-(tert-butoxycarbonyl)-2-pyrrolidinyl)-4-(1,3-dioxolan-2-ylméthyl)-1H-imidazol-5-yl)-4-biphénylyl)-1H-imidazol-2-yl)-1-pyrrolidinecarboxylate de tert-butyle;
(2S)-2-(4-(4'-(2-((2S)-1-(tert-butoxycarbonyl)-2-pyrrolidinyl)-1H-imidazol-4-yl)-4-biphénylyl)-5-(2-méthoxy-2-oxoéthyl)-1H-imidazol-2-yl)-1-pyrrolidinecarboxylate de tert-butyle;
(2S)-2-(4-(4'-(2-((2S)-1-(tert-butoxycarbonyl)-2-pyrrolidinyl)-1H-imidazol-4-yl)-4-biphénylyl)-5-propyl-1H-imidazol-2-yl)-1-pyrrolidinecarboxylate de tert-butyle;
2-((2S)-1-(tert-butoxycarbonyl)-2-pyrrolidinyl)-5-(4'-(2-((2S)-1-(tert-butoxycarbonyl)-2-pyrrolidinyl)-1H-imidazol-5-yl)-4-biphénylyl)-1H-imidazole-4-carboxylate d'éthyle;
(2S)-2-(4-(4'-(2-((2S)-1-(tert-butoxycarbonyl)-2-pyrrolidinyl)-1H-imidazol-4-yl)-3'-fluoro-4-biphénylyl)-1H-imidazol-2-yl)-4,4-difluoro-1-pyrrolidinecarboxylate de tert-butyle;
2-((2S)-4,4-difluoro-2-pyrrolidinyl)-4-(3'-fluoro-4'-(2-((2S)-2-pyrrolidinyl)-1H-imidazol-4-yl)-4-biphénylyl)-1H-imidazole;
(1R)-2-((2S)-2-(4-(4'-(2((2S)-1-((2R)-2-(diéthylamino)-2-phénylacétyl)-2-pyrrolidinyl)-1H-imidazol-4-yl)-4-biphénylyl)-5-méthyl-1H-imidazol-2-yl)-1-pyrrolidinyl)-N,N-diéthyl-2-oxo-1-phényléthanamine;
1-((1R)-2-((2S)-2-(4-(4'-(4-méthyl-2-((2S)-1-((2R)-2-phényl-2-(1-pipéridinyl)acétyl)-2-pyrrolidinyl)-1H-imidazol-5-yl)-4-biphénylyl)-1H-imidazol-2-yl)-1-pyrrolidinyl)-2-oxo-1-phényléthyl)pipéridine;
((1R)-2-((2S)-2-(4-(4'-(2-((2S)-1-((2R)-2-((méthoxycarbonyl)amino)-2-phénylacétyl)-2-pyrrolidinyl)-1H-imidazol-4-yl)-4-biphénylyl)-5-méthyl-1H-imidazol-2-yl)-1-pyrrolidinyl)-2-oxo-1-phényléthyl)carbamate de méthyle;
((1S)-2-((2S)-2-(4-(4'-(2-((2S)-1-(N-(méthoxycarbonyl)-L-alanyl)-2-pyrrolidinyl)-4-méthyl-1H-imidazol-5-yl)-4-biphénylyl)-1H-imidazol-2-yl)-1-pyrrolidinyl)-1-méthyl-2-oxoéthyl)carbamate de méthyle;
((1S)-1-(((2S)-2-(4-(4'-(2-((2S)-1-((2S)-2-((méthoxycarbonyl)amino)-3-méthylbutanoyl)-2-pyrrolidinyl)-1H-imidazol-4-yl)-4-biphénylyl)-5-méthyl-1H-imidazol-2-yl)-1-pyrrolidinyl)carbonyl)-2-méthylpropyl)carbamate de méthyle;
((1S,2R)-2-méthoxy-1-(((2S)-2-(4-(4'-(2-((2S)-1-(N-(méthoxycarbonyl)-O-méthyl-L-thréonyl)-2-pyrrolidinyl)-4-méthyl-1H-imidazol-5-yl)-4-biphénylyl)-1H-imidazol-2-yl)-1-pyrrolidinyl)carbonyl)propyl)carbamate de méthyle;
(1R, 1'R)-2,2'-(4,4'-biphényldiylbis((4-méthyl-1H-imidazole-5,2-diyl)(2S)-2,1-pyrrolidinediyl))bis(N,N-diméthyl-2-oxo-1-phényléthanamine);
(1R, 1'R)-2,2'-(4,4-biphényldiylbis((4-méthyl-1H-imidazole-5,2-diyl)(2S)-2,1-pyrrolidinediyl))bis(N,N-diéthyl-2-oxo-1-phényléthanamine);
1,1'-(4,4'-biphényldiylbis((4-méthyl-1H-imidazole-5,2-diyl)(2S)-2,1-pyrrolidinediyl((1R)-2-oxo-1-phényl-2,1-éthanediyl)))dipipéridine;
(4,4'-biphényldiylbis((4-méthyl-1H-imidazole-5,2-diyl)(2S)-2,1-pyrrolidinediyl((1R)-2-oxo-1-phényl-2,1-éthanediyl)))biscarbamate de diméthyle;
((1S)-2-((2S)-2-(4-(4'-(2-((2S)-1-(N-(méthoxycarbonyl)-L-alanyl)-2-pyrrolidinyl)-5-méthyl-1H-imidazol-4-yl)-4-biphénylyl)-5-méthyl-1H-imidazol-2-yl)-1-pyrrolidinyl)-1-méthyl-2-oxoéthyl)carbamate de méthyle;
((1S)-1-(((2S)-2-(4-(4'-(2-((2S)-1-((2S)-2-((méthoxycarbonyl)amino)-3-méthylbutanoyl)-2-pyrrolidinyl)-4-méthyl-1H-imidazol-5-yl)-4-biphénylyl)-5-méthyl-1H-imidazol-2-yl)-1-pyrrolidinyl)carbonyl)-2-méthylpropyl)carbamate de méthyle;
((1S,2R)-2-méthoxy-1-(((2S)-2-(4-(4'-(2-((2S)-1-(N-(méthoxycarbonyl)-O-méthyl-L-thréonyl)-2-pyrrolidinyl)-5-méthyl-1H-imidazol-4-yl)-4-biphénylyl)-5-méthyl-1H-imidazol-2-yl)-1-pyrrolidinyl)carbonyl)propyl)carbamate de méthyle;
((1S)-1-(((2S)-2-(4-(1,3-dioxan-2-ylméthyl)-5-(4'-(2-((2S)-1-((2S)-2-((méthoxycarbonyl)amino)-3-méthylbutanoyl)-2-pyrrolidinyl)-1H-imidazol-4-yl)-4-biphénylyl)-1H-imidazol-2-yl)-1-pyrrolidinyl)carbonyl)-2-méthylpropyl)carbamate de méthyle;
((1S,2R)-1-(((2S)-2-(4-(2,2-diméthoxyéthyl)-5-(4'-(2-((2S)-1-(N-(méthoxycarbonyl)-O-méthyl-L-thréonyl)-2-pyrrolidinyl)-1H-imidazol-4-yl)-4-biphénylyl)-1H-imidazol-2-yl)-1-pyrrolidinyl)carbonyl)-2-méthoxypropyl)carbamate de méthyle;
((1S)-1-(((2S)-2-(4-(2,2-diméthoxyéthyl)-5-(4'-(2-((2S)-1-((2S)-2-((méthoxycarbonyl)amino)-3-méthylbutanoyl)-2-pyrrolidinyl)-1H-imidazol-4-yl)-4-biphénylyl)-1H-imidazol-2-yl)-1-pyrrolidinyl)carbonyl)-2-méthylpropyl)carbamate de méthyle;
2-((2S)-1-(N-(méthoxycarbonyl)-O-méthyl-L-thréonyl)-2-pyrrolidinyl)-4-(4'-(2-((2S)-1-(N-(méthoxycarbonyl)-O-méthyl-L-thréonyl)-2-pyrrolidinyl)-1H-imidazol-4-yl)-4-biphénylyl)-1H-imidazol-5-yl)acétate de méthyle;
2-((2S)-1-(N-(méthoxycarbonyl)-L-valyl)-2-pyrrolidinyl)-4-(4'-(2-((2S)-1-(N-(méthoxycarbonyl)-L-valyl)-2-pyrrolidinyl)-1H-imidazol-4-yl)-4-biphénylyl)-1 H-imidazol-5-yl)acétate de méthyle;
((1S)-2-((2S)-2-(4-(4'-(2-((2S)-1-(N-(méthoxycarbonyl)L-alanyl)-2-pyrrolidinyl)-4-propyl-1H-imidazol-5-yl)-4-biphénylyl)-1H-imidazol-2-yl)-1-pyrrolidinyl)-1-méthyl-2-oxoéthyl)carbamate de méthyle;
(1R)-2-((2S)-2-(4-(4'-(2-((2S)-1-((2R)-2-(diéthylamino)-2-phénylacétyl)-2-pyrrolidinyl)-1H-imidazol-4-yl)-4-biphénylyl)-5-propyl-1H-imidazol-2-yl)-1-pyrrolidinyl)-N,N-diéthyl-2-oxo-1-phényléthanamine;
((1S)-1-(((2S)-2-(4-(4'-(2-(2S)-1-((2S)-2-(méthoxycarbonyl)amino)-3-méthylbutanoyl)-2-pyrrolidinyl)-1H-imidazol-4-yl)-4-biphénylyl)-5-propyl-1H-imidazol-2-yl)-1-pyrrolidinyl)carbonyl)-2-méthylpropyl)carbamate de méthyle;
((1S)-1-(((2S)-4,4-difluoro-2-(4-(3'-fluoro-4'-(2-((2S)-1-((2S)-2-(méthoxycarbonyl)amino)-3-méthylbutanoyl)-2-pyrrolidinyl)-1H-imidazol-4-yl)-4-biphénylyl)-1H-imidazol-2-yl)-1-pyrrolidinyl)carbonyl)-2-méthylpropyl)carbamate de méthyle;
(1R)-2-((2S)-2-(4-(4'-(2-((2S)-1-((2R)-2-(diéthylamino)-2-phénylacétyl)-4,4-difluoro-2-pyrrolidinyl)-1H-imidazol-4-yl)-3-fluoro-4-biphénylyl)-1H-imidazol-2-yl)-1-pyrrolidinyl)-N,N-diéthyl-2-oxo-1-phényléthanamine;
((1S)-1-(((2S)-2-(4-hydroxyméthyl)-5-(4'-(2-((2S)-1-((2S)-2-(méthoxycarbonyl)amino)-3-méthylbutanoyl)-2-pyrrolidinyl)-1H-imidazol-4-yl)-4-biphénylyl)-1H-imidazol-2-yl)-1-pyrrolidinyl)carbonyl)-2-méthylpropyl)carbamate de méthyle;
((1S)-2-((2S)-2-(4-(4'-(4-(hydroxyméthyl)-2-((2S)-1-(N-(méthoxycarbonyl)-L-alanyl)-2-pyrrolidinyl)-1H-imidazol-5-yl)-4-biphénylyl)-1H-imidazol-2-yl)-1-pyrrolidinyl)-1-méthyl-2-oxoéthyl)carbamate de méthyle;
(N,N'-(4,4'-biphényldiylbis(1H-imidazole-4,2-diyl(2S)-2,1-pyrrolidinediyl((2S)-3-méthyl-l-oxo-1,2-butanediyl)))di(2-pyrimidinamine);
N,N'-(4,4'-biphényldiylbis(1H-imidazole-4,2-diyl(2S)-2,1-pyrrolidinediyl((1S)-1-cyclopropyl-2-oxo-1,2-éthanediyl)))di(2-pyrimidinamine);
N,N'-(4,4'-biphényldiylbis(1H-imidazole-4,2-diyl(2S)-2,1-pyrrolidinediyl((2S)-1-oxo-1,2-propanediyl)))di(2-pyrimidinamine);
N,N'-(4,4'-biphényldiylbis(1H-imidazole-4,2-diyl(2S)-2,1-pyrrolidinediyl((2S)-1-oxo-1,2-butanediyl)))di(2-pyrimidinamine);
N,N'-(4,4'-biphényldiylbis(1H-imidazole-4,2-diyl(2S)-2,1-pyrrolidinediyl((2S)-3-méthoxy-1-oxo-1,2-propanediyl)))di(2-pyrimidinamine); et
N,N'-(4,4'-biphényldiylbis(1H-imidazole-4,2-diyl(2S)-2,1-pyrrolidinediyl((2S,3R)-3-méthoxy-1-oxo-1,2-butanediyl)))di(2-pyrimidinamine); 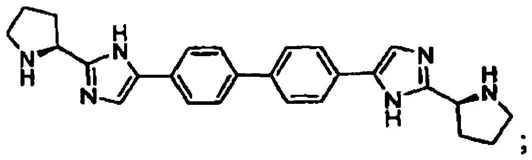 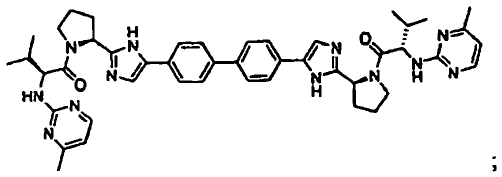 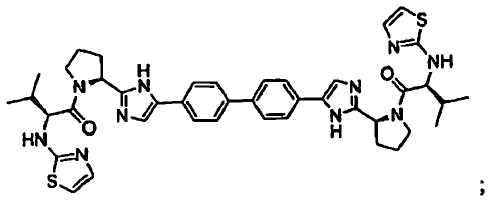 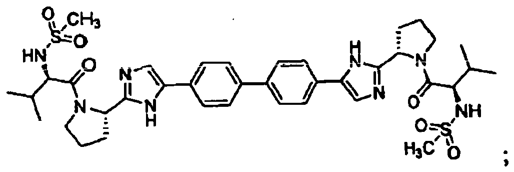 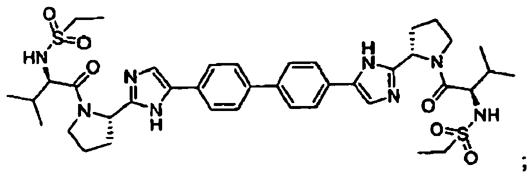 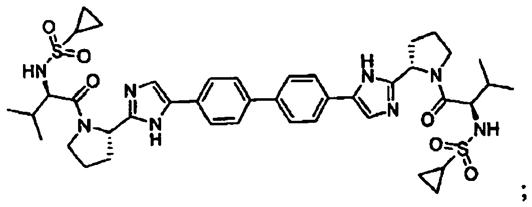 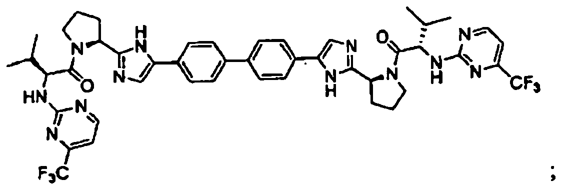 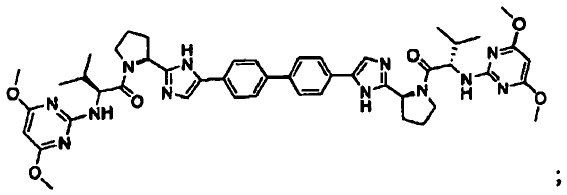 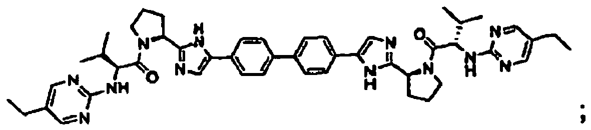 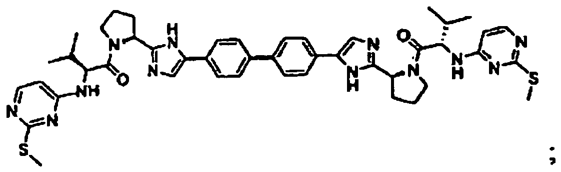 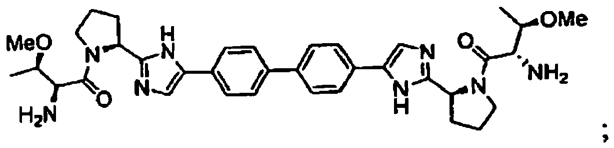 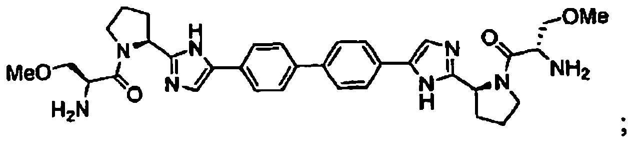 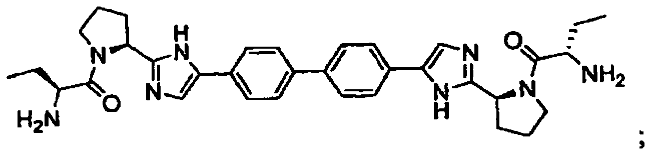 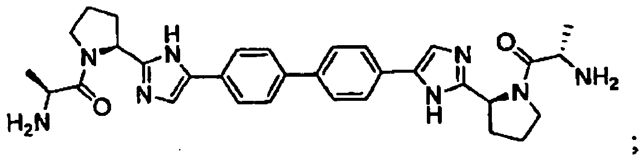 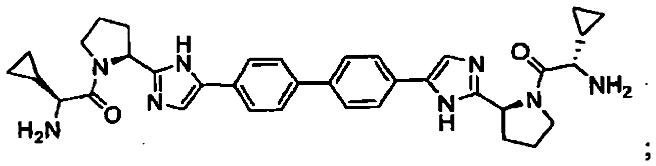 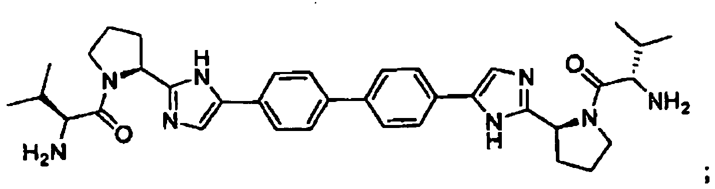 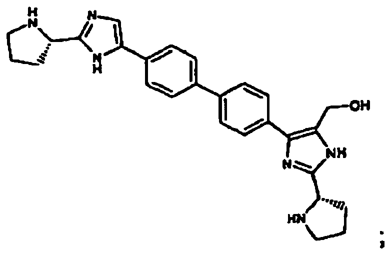 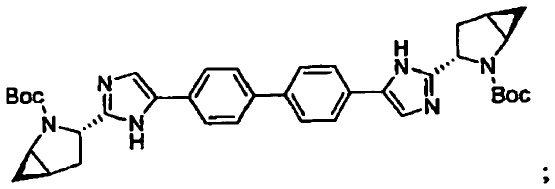 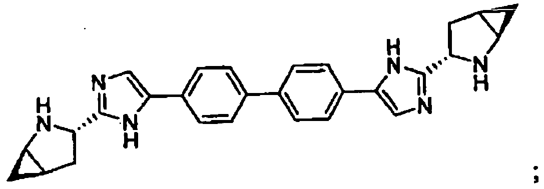 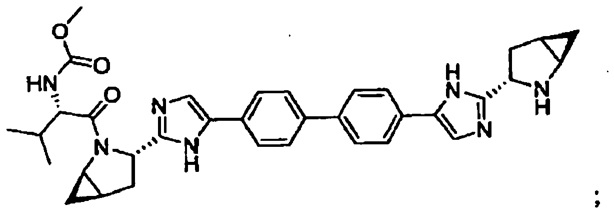 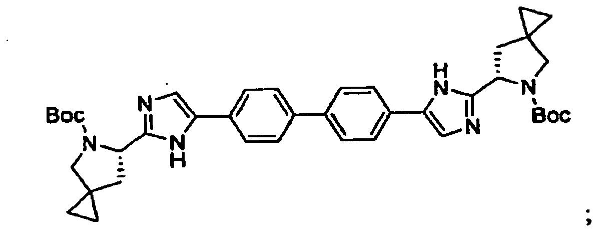 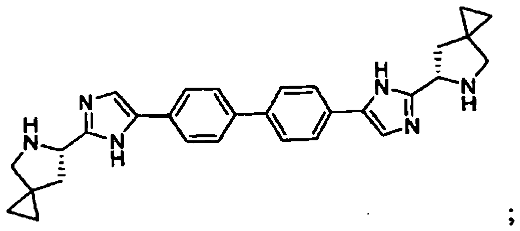 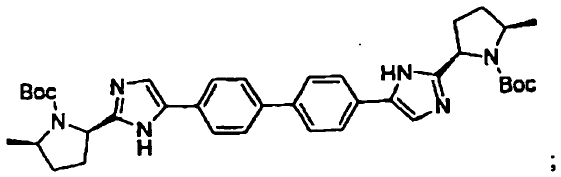 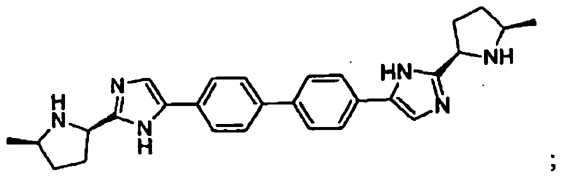 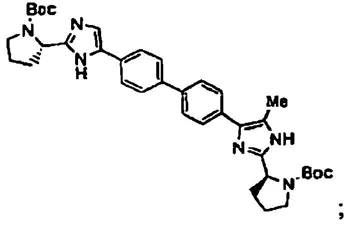 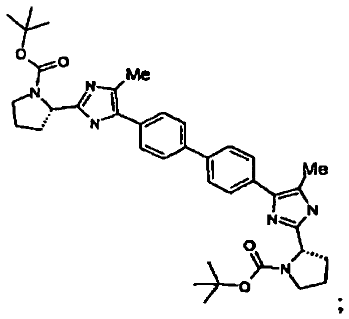 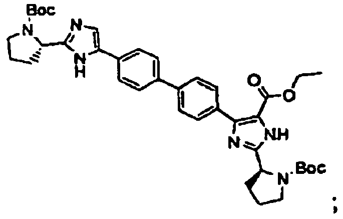 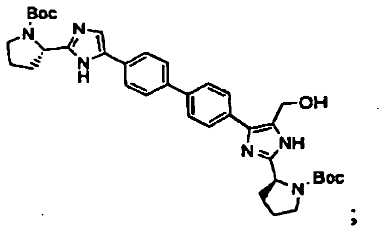 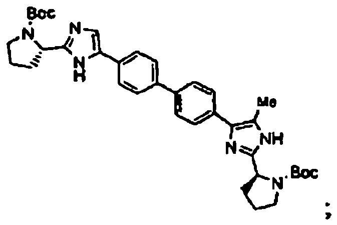 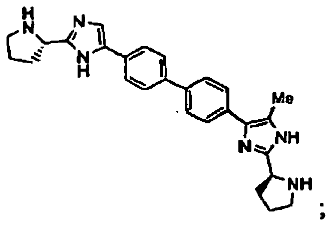 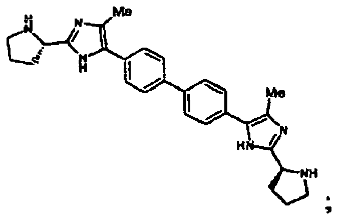 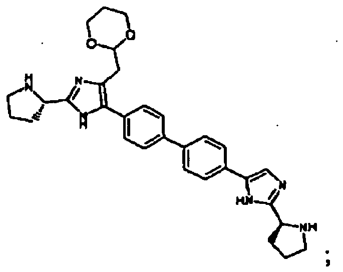 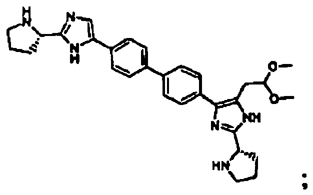 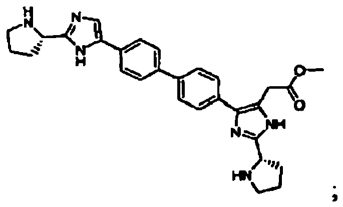 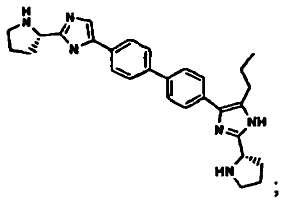 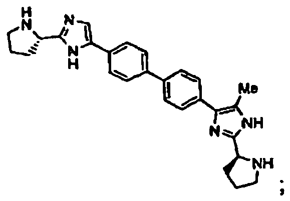 et 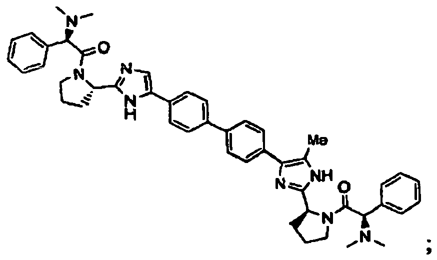 ou un de leurs sels pharmaceutiquement acceptables.

2. Composition comprenant un composé selon la revendication 1 ou un de ses sels pharmaceutiquement acceptables et un véhicule pharmaceutiquement acceptable.

3. Composition selon la revendication 2, comprenant en outre un ou deux composés supplémentaires ayant une activité anti-VHC.

4. Composition selon la revendication 3, dans laquelle au moins l'un des composés supplémentaires est un interféron ou une ribavirine.

5. Composition selon la revendication 4, dans laquelle l'interféron est sélectionné parmi l'interféron alpha 2B, l'interféron alpha pégylé, l'interféron consensus, l'interféron alpha 2A et l'interféron lymphoblastoïde tau.

6. Composition selon la revendication 3, dans laquelle au moins l'un des composés supplémentaires est sélectionné parmi l'interleukine 2, l'interleukine 6, l'interleukine 12, un composé qui active le développement d'une réponse des cellules T auxiliaires de type 1, un ARN interférant, un ARN anti-sens, l'imiquimod, la ribavirine, un inhibiteur d'inosine-5'-monophosphate déshydrogénase, l'amantadine et la rimantadine.

7. Composition selon la revendication 3, dans laquelle au moins l'un des composés supplémentaires est efficace pour inhiber la fonction d'une cible sélectionnée parmi la métalloprotéase du VHC, la sérine protéase du VHC, la polymérase du VHC, l'hélicase du VHC, la protéine NS4B du VHC, l'entrée du VHC, l'assemblage du VHC, la sortie du VHC, la protéine NS5A du VHC et l'IMPDH, pour traiter une infection par le VHC.

8. Composé selon la revendication 1 ou un sel pharmaceutiquement acceptable de celui-ci, à utiliser dans le traitement d'une infection par le VHC chez un patient.

9. Composé à utiliser selon la revendication 8, comprenant en outre utiliser un ou deux composés supplémentaires ayant une activité anti-VHC, avant, après ou en même temps que le composé selon la revendication 1 ou 2 ou un de ses sels pharmaceutiquement acceptables.

10. Composé à utiliser selon la revendication 9, où au moins l'un des composés supplémentaires est un interféron ou une ribavirine.

11. Composé à utiliser selon la revendication 10, où l'interféron est sélectionné parmi l'interféron alpha 2B, l'interféron alpha pégylé, l'interféron consensus, l'interféron alpha 2A et l'interféron lymphoblastoïde tau.

12. Composé à utiliser selon la revendication 9, où au moins l'un des composés supplémentaires est sélectionné parmi l'interleukine 2, l'interleukine 6, l'interleukine 12, un composé qui active le développement d'une réponse des cellules T auxiliaires de type 1, un ARN interférant, un ARN anti-sens, l'imiquimod, la ribavirine, un inhibiteur d'inosine-5'-monophosphate déshydrogénase, l'amantadine et la rimantadine.

13. Composé à utiliser selon la revendication 9, où au moins l'un des composés supplémentaires est efficace pour inhiber la fonction d'une cible sélectionnée parmi la métalloprotéase du VHC, la sérine protéase du VHC, la polymérase du VHC, l'hélicase du VHC, la protéine NS4B du VHC, l'entrée du VHC, l'assemblage du VHC, la sortie du VHC, la protéine NSSA du VHC et l'IMPDH, pour traiter une infection par le VHC.
